(19) Europäisches Patentamt / European Patent Office / Office européen des brevets

(11) **EP 4 775 575 A1**

(12) **EUROPEAN PATENT APPLICATION**
published in accordance with Art. 153(4) EPC


(43) Date of publication:
**15.07.2026 Bulletin 2026/29**

(21) Application number: **24862049.4**

(22) Date of filing: **05.09.2024**

(51) International Patent Classification (IPC):
***C07D 405/14*** *(2006.01)*

(52) Cooperative Patent Classification (CPC):
**A61K 31/506; A61K 31/519; A61K 31/52; A61K 31/527; A61K 31/529; A61K 31/542; A61P 3/00; A61P 9/00; A61P 29/00; A61P 31/00; A61P 35/00; A61P 35/02; A61P 37/02; A61P 37/06; C07D 401/14;** (Cont.)

(86) International application number:
**PCT/CN2024/117282**

(87) International publication number:
**WO 2025/051211 (13.03.2025 Gazette 2025/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.09.2023 CN 202311138856**
**05.03.2024 CN 202410248443**



(71) Applicant: **TYK Medicines, Inc.**
**Huzhou, Zhejiang 313100 (CN)**

(72) Inventors:
- **LIANG, Apeng**
  **Huzhou, Zhejiang 313100 (CN)**
- **WANG, Kai**
  **Huzhou, Zhejiang 313100 (CN)**
- **CHEN, Shaoqing**
  **Huzhou, Zhejiang 313100 (CN)**
- **LI, Jun**
  **Huzhou, Zhejiang 313100 (CN)**
- **WU, Yusheng**
  **Huzhou, Zhejiang 313100 (CN)**
- **LI, Meihua**
  **Huzhou, Zhejiang 313100 (CN)**
- **YIN, Zhou**
  **Huzhou, Zhejiang 313100 (CN)**
- **LONG, Yi**
  **Huzhou, Zhejiang 313100 (CN)**
- **NIU, Chengshan**
  **Zhengzhou, Henan 451162 (CN)**

(74) Representative: **dompatent**
**Partnerschaft von Patentanwälten und Rechtsanwälten mbB**
**Deichmannhaus am Dom**
**Bahnhofsvorplatz 1**
**50667 Köln (DE)**


(54) **COMPOUND USED AS CDK4 KINASE INHIBITOR AND USE THEREOF**


(57) The present invention relates to a compound used as a CDK4 kinase inhibitor and the use thereof. Specifically, the compound of the present invention has a structure as shown in formula I or formula II, wherein the definitions of various groups and substituents are as described in the description. The compound of the present invention can be used as an inhibitor of a cyclin-dependent kinase (CDK4) for treating or preventing proliferative diseases (such as cancer), and in particular for regulating and treating related diseases caused by the abnormal activity of cyclin-dependent kinase (CDK4).


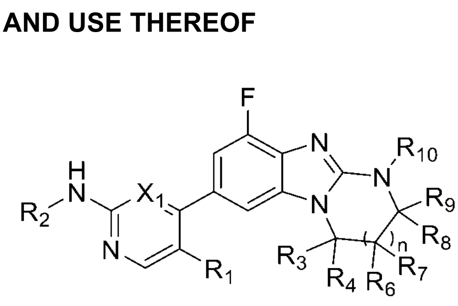

I

EP 4 775 575 A1

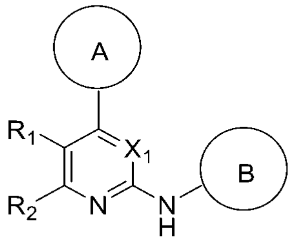

II

(52) Cooperative Patent Classification (CPC): (Cont.)
**C07D 403/04; C07D 405/14; C07D 413/14; C07D 471/14; C07D 473/00; C07D 487/04; C07D 487/08; C07D 487/10; C07D 487/14; C07D 513/04; C07D 519/00**

## Description

### TECHNICAL FIELD

The present invention relates to the field of pharmaceutical technology, and specifically to a compound used as CDK4 kinase inhibitor, and use thereof in modulating CDK4 kinase activity or treating CDK4-related diseases, in particular cancer.

### BACKGROUND OF THE INVENTION

Protein kinases regulate various biological functions, including DNA replication, transcription, translation, cell cycle progression, energy metabolism, migration, and cell growth, making them ideal targets for treating proliferative diseases and conditions, including cancer. There is still a demand for compounds that can selectively inhibit protein kinase activity and are effective as therapeutic anti-proliferative agents.

Cyclin-dependent kinases (CDKs) belong to the serine/threonine kinase family, which exert physiological functions by binding to the corresponding cyclins to form active dimeric complexes that cause cell growth and proliferation. To date, more than 20 types of CDKs have been identified, which are categorized into two main groups according to their functions: CDKs that regulate the cell cycle and CDKs that regulate cellular transcription, among which CDKs 1 - 6, 14-18 and their cyclin partners (such as Cyclins A, B, D1, D2, D3, E, F etc.) are involved in cell cycle regulation and are considered as cell cycle regulatory factors; CDKs 7-13, 19-20, and their cyclin partners (such as Cyclins C, H, K, L1, L2, T1, T2, etc.) are involved in the regulation of cell transcription and are considered transcriptional regulatory factors. CDKs are therefore involved in the regulation of cell cycle control, apoptosis, differentiation, and transcription. It has now been shown that CDK inhibitors can be used in the treatment of various diseases including cancer.

CDK4 and CDK6 are involved in regulating the cell cycle from G1 to S phase upon binding to Cyclin D. Abnormalities in the CyclinD-CDK4/6-Rb pathway have been reported to be associated with progression of resistance to endocrine therapies. Currently, several CDK4/6 inhibitors, such as palbociclib, ribociclib, abemaciclib, etc., have been approved for marketing and are used in combination with endocrine therapies for the treatment of hormone receptor (HR)-positive, human epidermal growth factor 2 (HER2)-negative advanced or metastatic breast cancer. However, hematologic toxicities such as neutropenia and/or gastrointestinal toxicities often occur during treatment with CDK4/6 inhibitors, leading to drug discontinuation or intermittent administration, which seriously affects the efficacy and compliance of the drugs. Currently, there are research data suggesting that the activity of cyclin D3-CDK6 may be associated with these side effects. In view of the toxic and side effects of current dual-target CDK4/6 inhibitors, the development of a selective CDK4 inhibitor may have better safety and efficacy.

### SUMMARY OF THE INVENTION

The present invention provides a novel compound with CDK4 kinase inhibitory activity and better pharmacodynamic and pharmacokinetic properties.

In the first aspect, the present invention provides a compound used as a CDK4 kinase inhibitor, wherein the compound is a compound of formula II, or a pharmaceutically acceptable salt, a stereoisomer, a tautomer, a hydrate, a solvate, an isotope compound, or a prodrug thereof,

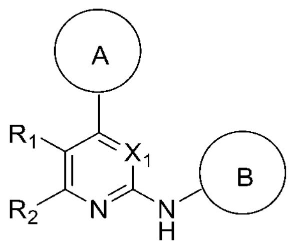
formula II

wherein,

- $X_1$ is selected from the group consisting of N, and $CR_3$;
- $R_1$ is selected from the group consisting of H, $CF_3$, F, Cl, Br, methyl, ethyl, isopropyl, and cyclopropyl;
- $R_2$ is selected from the group consisting of H, F, $CF_3$, $CF_2H$, $NH_2$, and methyl;
- or, $R_1$ and $R_2$ together with the C to which they are attached form a 5-membered heteroaryl containing two N atoms;
- each $R_3$ is selected from the group consisting of H, $CF_3$, F, Cl, Br, methyl, and cyano;

ring A is selected from the group consisting of

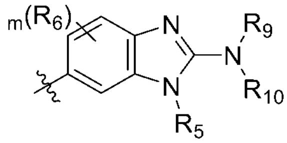,

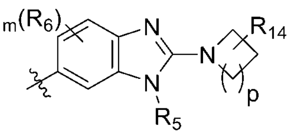, 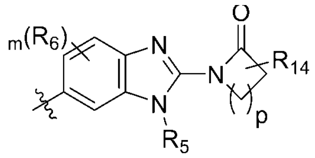, 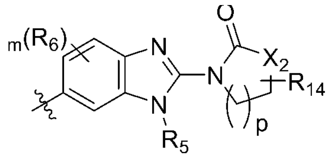,

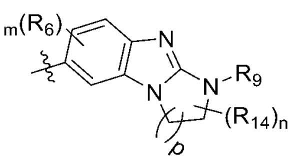, 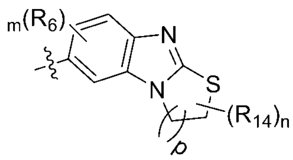, 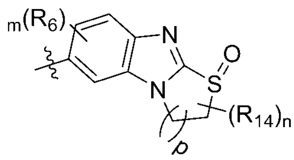,

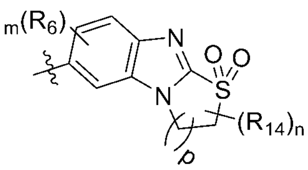, 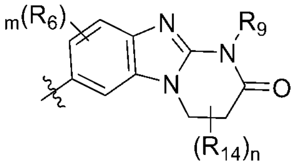, 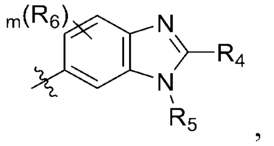, 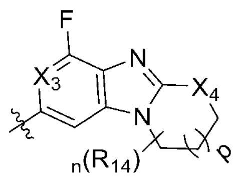,

and

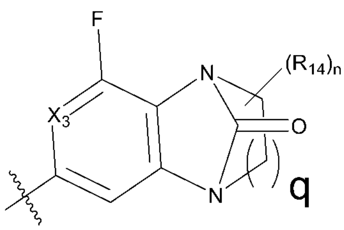

wherein, $X_2$ is O or NR, $X_3$ is N or $CR_3$, and $X_4$ is NR, SO or $SO_2$;
ring B is selected from the group consisting of

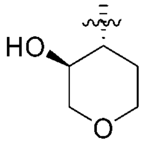, 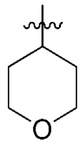, 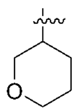, 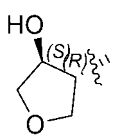,

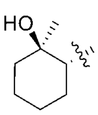, 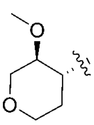, 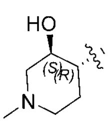, 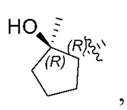, 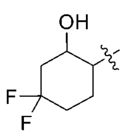, 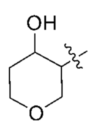, 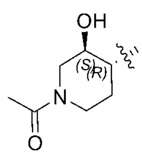, 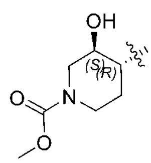,

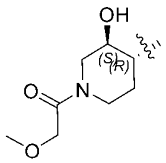 , 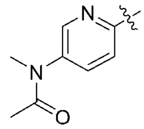 , 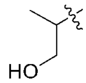 , 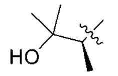 , 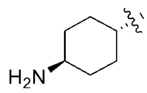 , 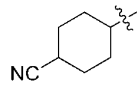 , 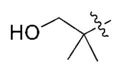 ,

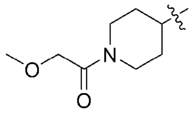 , 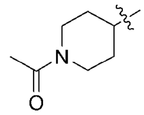 , 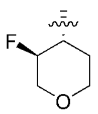 , 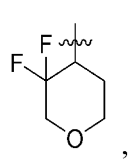 , 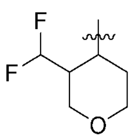 , 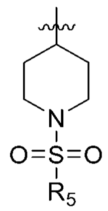 , 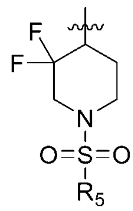 ,

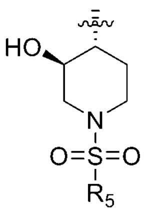 , 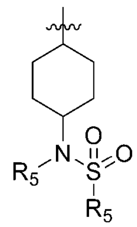 , 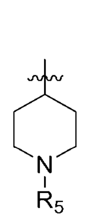 , 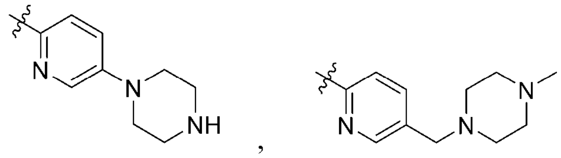 ,

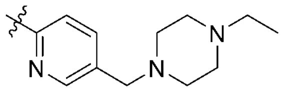 , 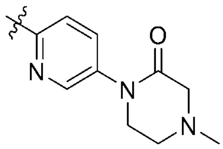 , 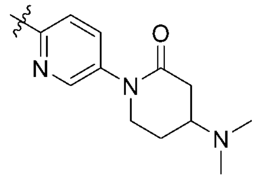 , 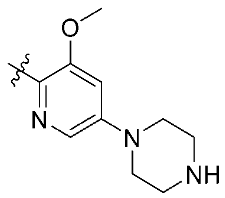 ,

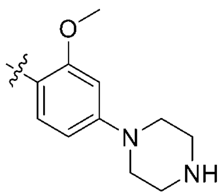 , 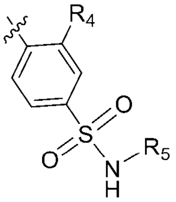 , 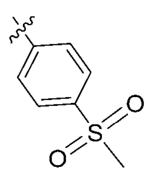 , 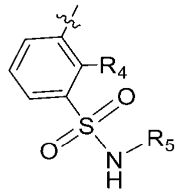 , 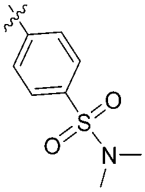 , 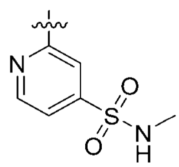 ,

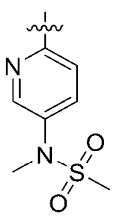 , 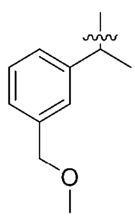 , 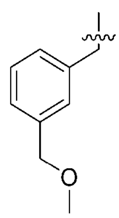 , 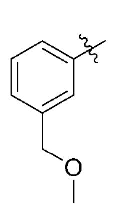 , 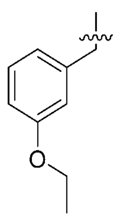 , 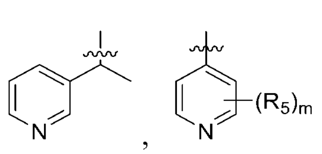 , 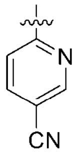 ,

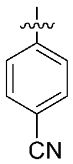 , 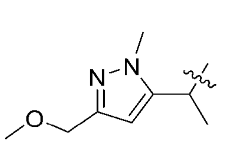 , 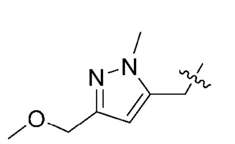 , 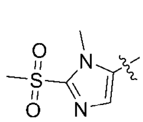 , 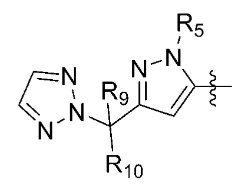 ,

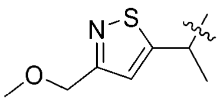, 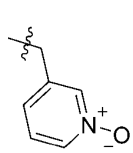, 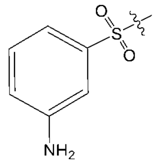, 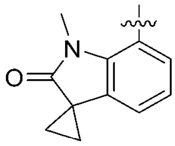, 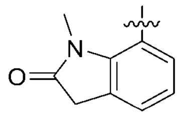, 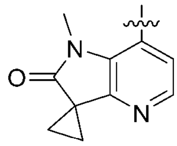,

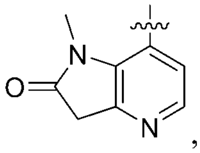, 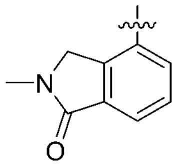, 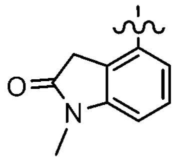, and 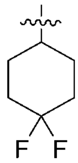;

wherein, each $R_4$ is independently selected from the group consisting of H, cyano, halogen, substituted or unsubstituted C1-C6 alkyl, and substituted or unsubstituted C3-C6 cycloalkyl;

each $R_5$, $R_6$, $R_9$, $R_{10}$ and $R_{14}$ is each independently selected from the group consisting of H, halogen, hydroxyl, amino,

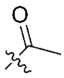, 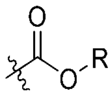, 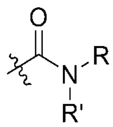,

oxo, substituted or unsubstituted C1-C6 alkyl, substituted or unsubstituted C3-C6 cycloalkyl, substituted or unsubstituted phenyl, substituted or unsubstituted $C_{1-6}$ alkoxy, and substituted or unsubstituted $-NR_mR_n$; or, two $R_{14}$ connected to the same C form a C3-C6 cycloalkyl together with the C to which they are attached;

or, $R_9$ and $R_{10}$ together with the N to which they are attached form

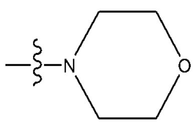

or

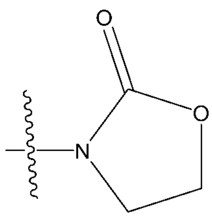;

each R and R' is each independently selected from the group consisting of H, C1-C6 alkyl, halogenated C1-C6 alkyl, C3-C6 cycloalkyl, halogenated C3-C6 cycloalkyl, and phenyl;

each $R_m$ is selected from the group consisting of H, $C_{1-6}$ alkyl, $C_{3-6}$ cycloalkyl, $C_{6-10}$ aryl, and 6-10 membered heteroaryl containing 1, 2, or 3 heteroatoms selected from N, O, and S;

each $R_n$ is $C_{1-6}$ alkyl;

the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;

each m, n, and p is each independently selected from the group consisting of 0, 1, 2, 3, and 4;

q is selected from the group consisting of 1, 2, and 3.

In another preferred embodiment,

$R_1$ is selected from the group consisting of H, $CF_3$, F, Cl, Br, methyl, ethyl, isopropyl, and cyclopropyl;

$R_2$ is selected from the group consisting of H, F, $CF_3$, $CF_2H$, $NH_2$, and methyl;

ring A is selected from the group consisting of

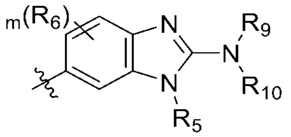,

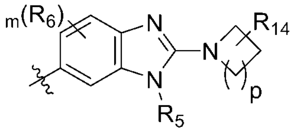, 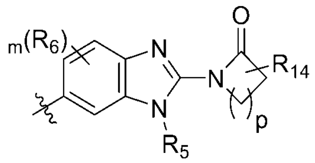, 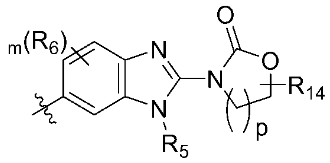,

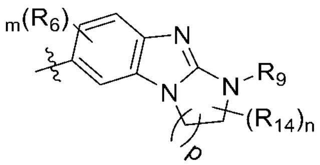, 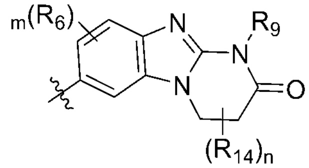, 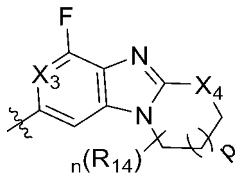,

and

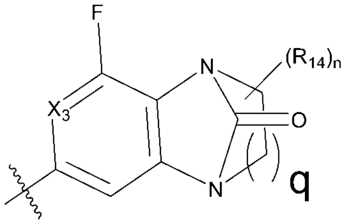

wherein, $X_2$ is O or NR, $X_3$ is N or $CR_3$, and $X_4$ is NR, SO or $SO_2$;
ring B is selected from the group consisting of

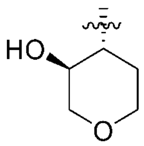, 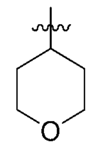, 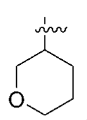, 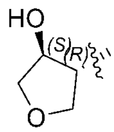,

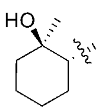, 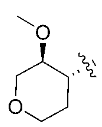, 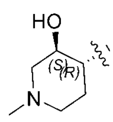, 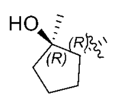, 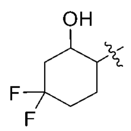, 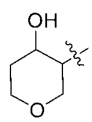, 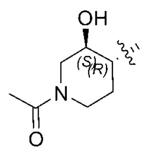, 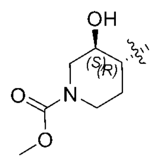,

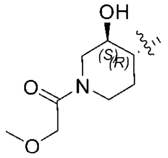, 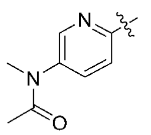, 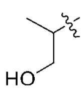, 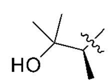, 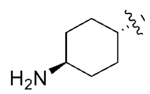, 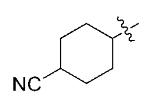, 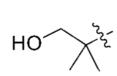,

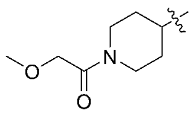, 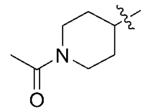, 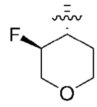, 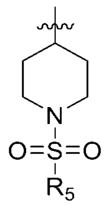, 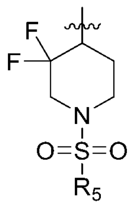, 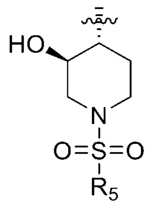, 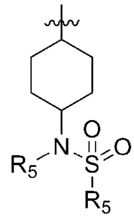,

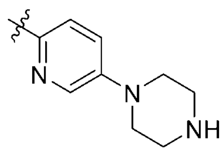, 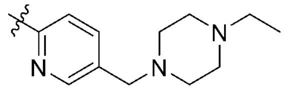, 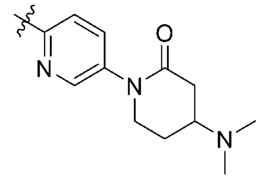, 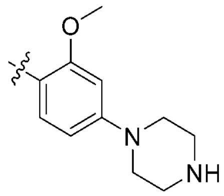, 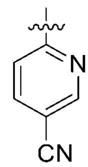,

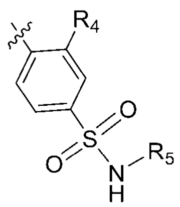, 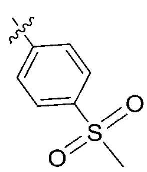, 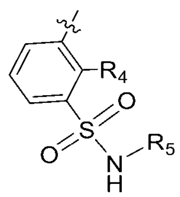, 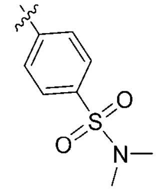, 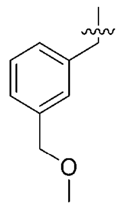, 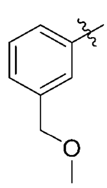, 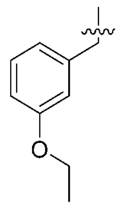,

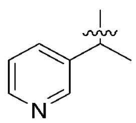, 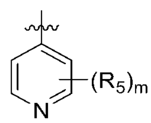, 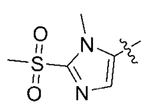, 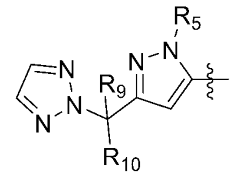, 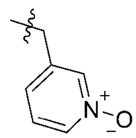, 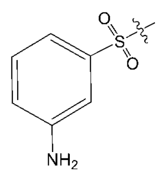,

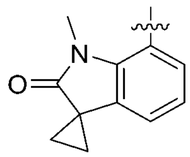, 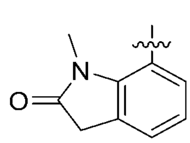, 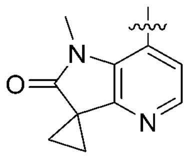, 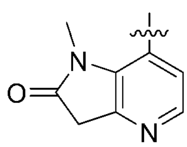, 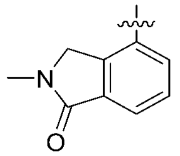,

and

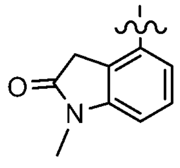;

wherein, each $R_4$ is independently selected from the group consisting of H, cyano, halogen, substituted or unsubstituted C1-C6 alkyl, and substituted or unsubstituted C3-C6 cycloalkyl;

each $R_5$, $R_6$, $R_9$, $R_{10}$ and $R_{14}$ is each independently selected from the group consisting of H, halogen, hydroxyl, amino,

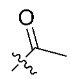, 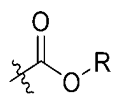, 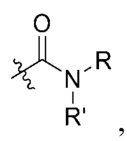,

substituted or unsubstituted C1-C6 alkyl, substituted or unsubstituted C3-C6 cycloalkyl, and substituted or unsubstituted phenyl;

or, $R_9$ and $R_{10}$ together with the N to which they are attached form

tituted phenyl;

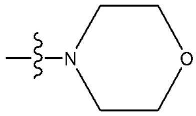
;

R and R' is each independently selected from the group consisting of H, C1-C6 alkyl, halogenated C1-C6 alkyl, C3-C6 cycloalkyl, halogenated C3-C6 cycloalkyl, and phenyl;
the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;
each m, n, and p is each independently selected from the group consisting of 0, 1, 2, 3, and 4;
q is selected from the group consisting of 1, 2, and 3.

In another preferred embodiment,

$R_1$ is selected from the group consisting of Cl, and Br;
$R_2$ is H;
ring A is selected from the group consisting of

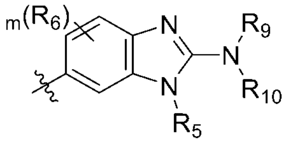
,

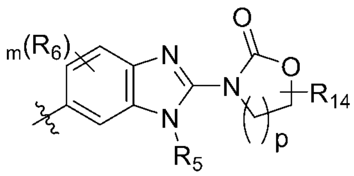
,
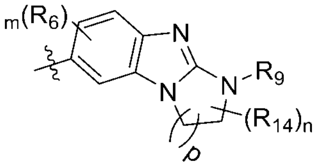
, and
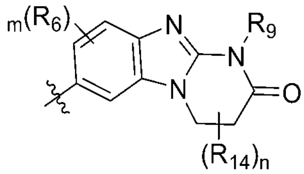
;

ring B is selected from the group consisting of

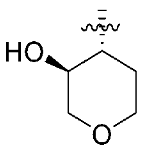
,
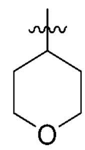
,
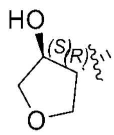
,
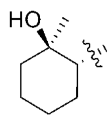
,

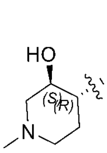
,
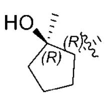
,
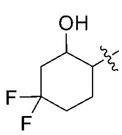
,
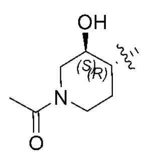
,
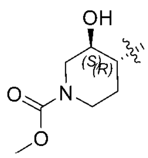
,
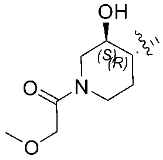
,
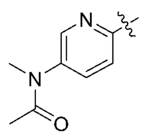
,
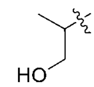
,

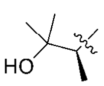
,
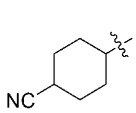
,
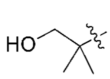
,
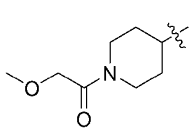
,
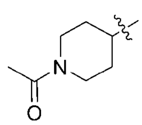
,
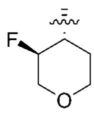
,
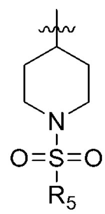
,
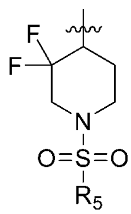
,

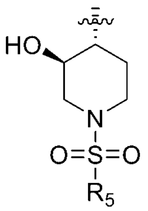, 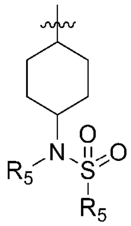, 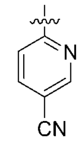, 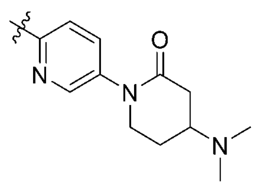, 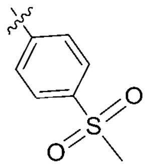, 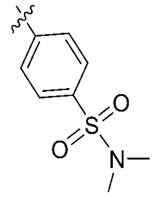,

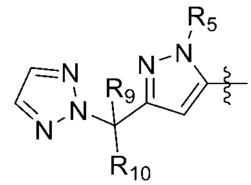, and 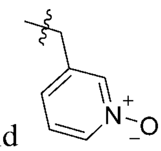;

wherein,

each $R_5$, $R_6$, $R_9$, $R_{10}$ and $R_{14}$ is each independently selected from the group consisting of H, halogen, hydroxyl, amino,

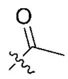, 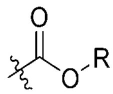, 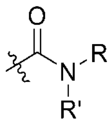,

substituted or unsubstituted C1-C6 alkyl, substituted or unsubstituted C3-C6 cycloalkyl, and substituted or unsubstituted phenyl;

or, $R_9$ and $R_{10}$ together with the N to which they are attached form

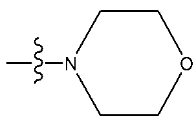;

R and R' is each independently selected from the group consisting of H, C1-C6 alkyl, halogenated C1-C6 alkyl, C3-C6 cycloalkyl, halogenated C3-C6 cycloalkyl, and phenyl;

the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;

each m, n, and p is each independently selected from the group consisting of 0, 1, 2, 3, and 4.

In another preferred embodiment,

$R_1$ and $R_2$ together with the C to which they are attached form a 5-membered heteroaryl containing two N atoms;

ring A is selected from the group consisting of

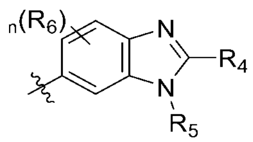, 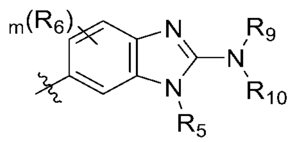,

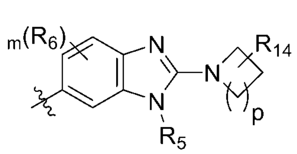, 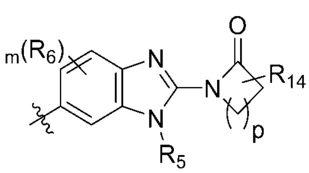, 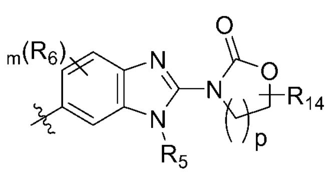,

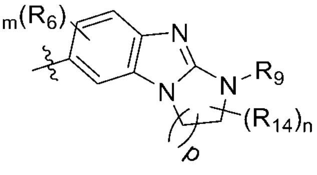 , and 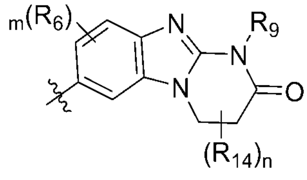 ,

ring B is selected from the group consisting of

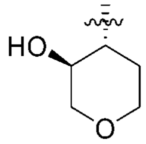 , 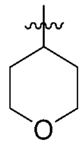 , 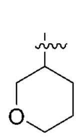 , 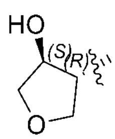 ,

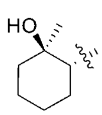 , 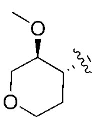 , 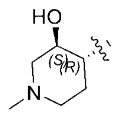 , 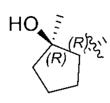 , 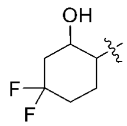 , 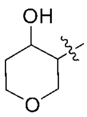 , 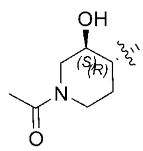 , 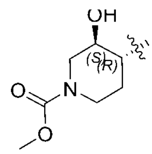 ,

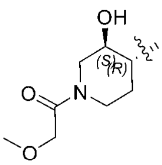 , 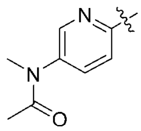 , 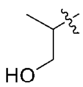 , 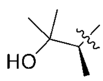 , 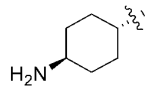 , 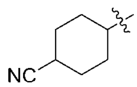 , 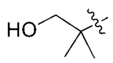 ,

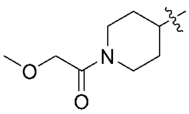 , 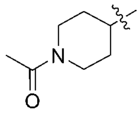 , 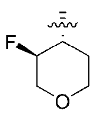 , 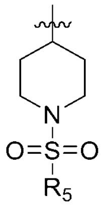 , 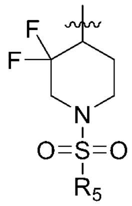 , 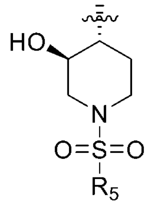 , 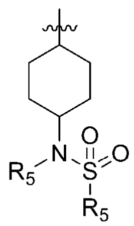 , 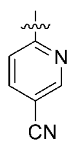 ,

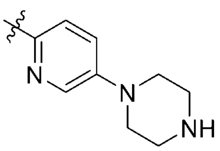 , 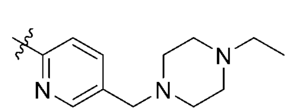 , 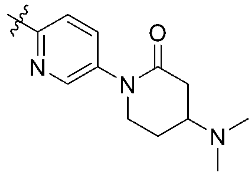 , 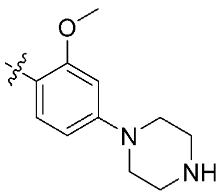 ,

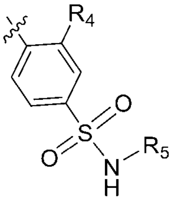 , 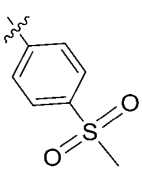 , 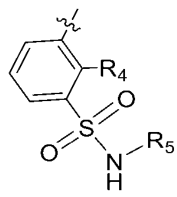 , 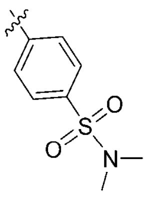 , 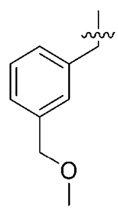 , 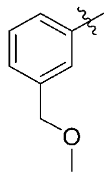 , 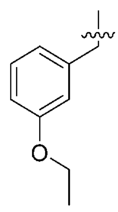 ,

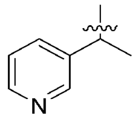, 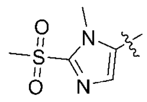, 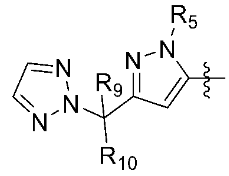, 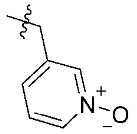, 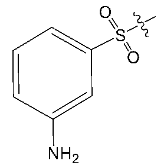, 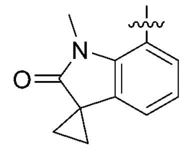,

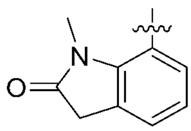, 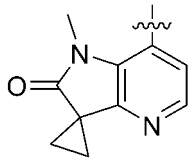, 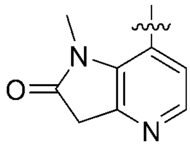, 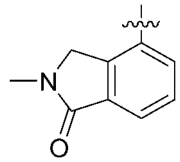, and 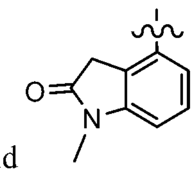;

wherein, each $R_4$ is independently selected from the group consisting of H, substituted or unsubstituted C1-C6 alkyl, and substituted or unsubstituted C3-C6 cycloalkyl;

each $R_5$, $R_6$, $R_9$, $R_{10}$ and $R_{14}$ is each independently selected from the group consisting of H, halogen, hydroxyl, amino,

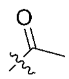, 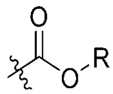, 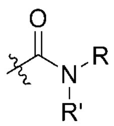,

substituted or unsubstituted C1-C6 alkyl, substituted or unsubstituted C3-C6 cycloalkyl, and substituted or unsubstituted phenyl;

each R and R' is each independently selected from the group consisting of H, C1-C6 alkyl, halogenated C1-C6 alkyl, C3-C6 cycloalkyl, halogenated C3-C6 cycloalkyl, and phenyl;

the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;

each m, n, and p is each independently selected from the group consisting of 0, 1, 2, 3, and 4.

In another preferred embodiment,

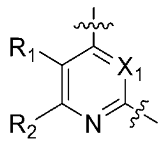

has the following structure

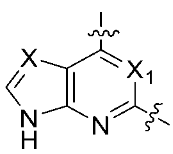;

X is selected from the group consisting of N, and $CR_3$;

$R_3$ is selected from the group consisting of H, $CF_3$, F, Cl, Br, methyl, and cyano.

In another preferred embodiment, $X_1$ is selected from the group consisting of N, and $CR_3$;

$R_1$ is Cl;

$R_2$ is H;

$R_3$ is H;

ring A is

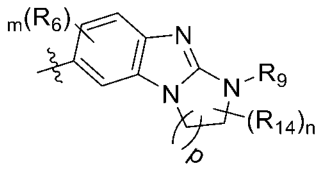

;

ring B is

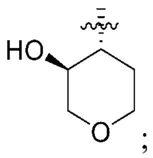

;

each $R_6$, $R_9$ and $R_{14}$ is each independently selected from the group consisting of H, halogen, substituted or unsubstituted C1-C6 alkyl, and substituted or unsubstituted phenyl;
the substituted in $R_6$, $R_9$ and $R_{14}$ each independently refers to be substituted by 1, 2 or 3 substituents selected from the group consisting of halogen, and cyano;
each m, n, and p is each independently selected from the group consisting of 0, 1, 2, 3, and 4.

In another preferred embodiment, $X_1$ is selected from the group consisting of N, and $CR_3$;
$R_3$ is H.

In another preferred embodiment, $R_1$ is Cl.

In another preferred embodiment, $R_2$ is H.

In another preferred embodiment, each $R_6$ and $R_{14}$ is each independently selected from the group consisting of H, halogen, and substituted or unsubstituted C1-C6 alkyl;
the substituted in $R_6$ and $R_{14}$ each independently refers to be substituted by 1, 2 or 3 substituents selected from the group consisting of halogen.

In another preferred embodiment, $R_9$ is selected from the group consisting of substituted or unsubstituted C1-C6 alkyl, and substituted or unsubstituted phenyl;
the substituted in $R_9$ each independently refers to be substituted by 1, 2 or 3 substituents selected from the group consisting of halogen, and cyano.

In another preferred embodiment, each m, n, and p is each independently selected from the group consisting of 0, 1, 2, 3, and 4.

In the second aspect, the present invention provides a compound used as a CDK4 kinase inhibitor, wherein the compound is a compound of formula I, or a pharmaceutically acceptable salt, a stereoisomer, a tautomer, a hydrate, a solvate, an isotope compound, or a prodrug thereof,

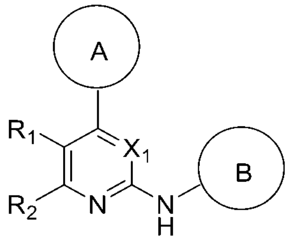

formula I

$X_1$ is selected from the group consisting of N, and $CR_3$;
$R_1$ is selected from the group consisting of H, $CF_3$, F, Cl, Br, methyl, ethyl, isopropyl, and cyclopropyl;
$R_2$ is selected from the group consisting of H, F, $CF_3$, $CF_2H$, $NH_2$, and methyl;
or, $R_1$ and $R_2$ together with the C to which they are attached form a 5-membered heteroaryl containing two N atoms;
$R_3$ is selected from the group consisting of H, $CF_3$, F, Cl, Br, methyl, and cyano;
ring A is selected from the group consisting of

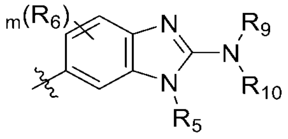,

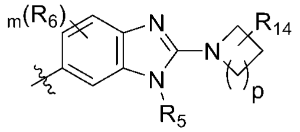, 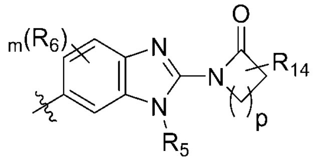, 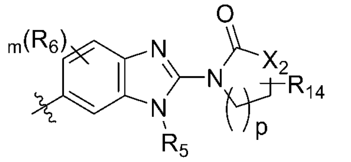,

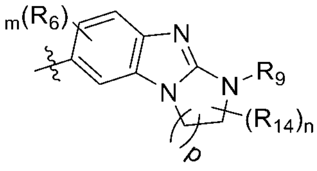, 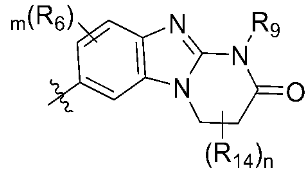, and 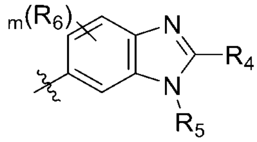,

wherein, $X_2$ is O or NR;

ring B is selected from the group consisting of

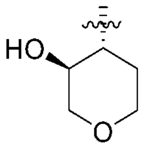, 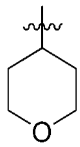, 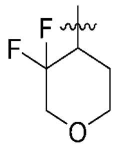, 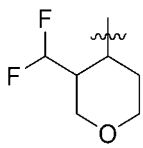,

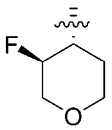, 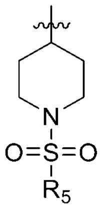, 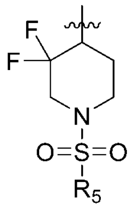, 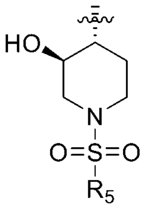, 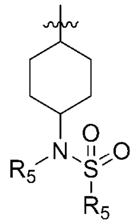, 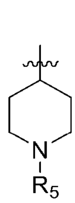, 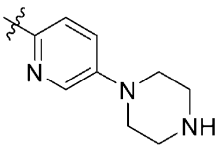,

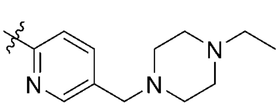, 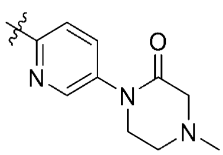, 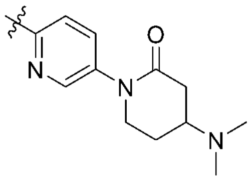, 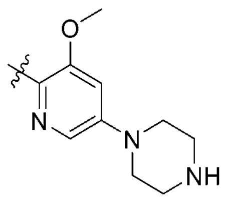,

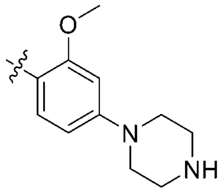, 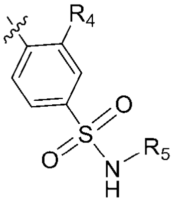, 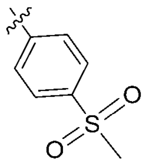, 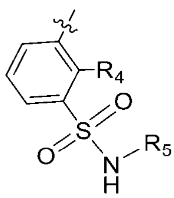, 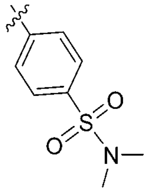, 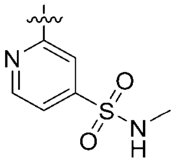,

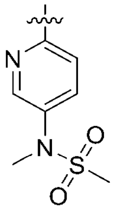, 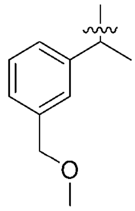, 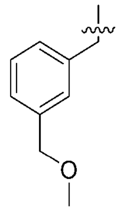, 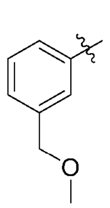, 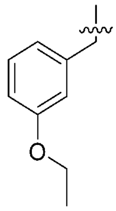, 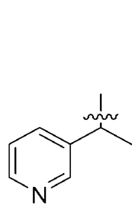, 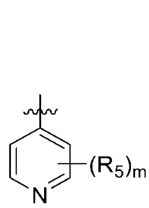, 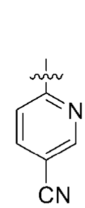,

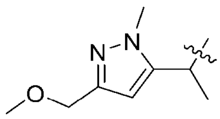, 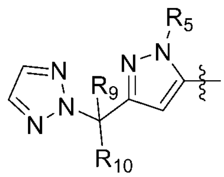, 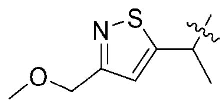, 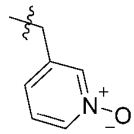, 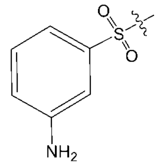,

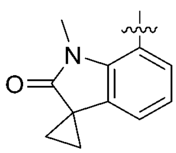, 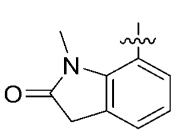, 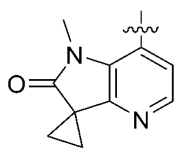, 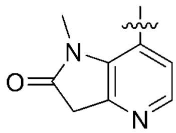, 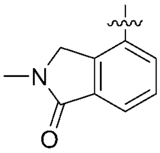, 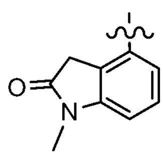,

and

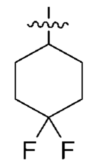;

wherein, each $R_4$ is independently selected from the group consisting of H, cyano, halogen, substituted or unsubstituted C1-C6 alkyl, and substituted or unsubstituted C3-C6 cycloalkyl;

each $R_5$, $R_6$, $R_9$, $R_{10}$ and $R_{14}$ is each independently selected from the group consisting of H, halogen, hydroxyl, amino,

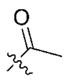, 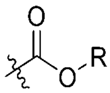, 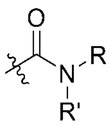,

substituted or unsubstituted C1-C6 alkyl, substituted or unsubstituted C3-C6 cycloalkyl, and substituted or unsubstituted phenyl;

or, $R_9$ and $R_{10}$ together with the N to which they are attached form

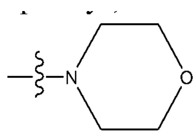

or

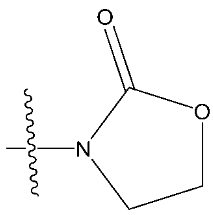;

each R and R' is each independently selected from the group consisting of H, C1-C6 alkyl, halogenated C1-C6 alkyl, C3-C6 cycloalkyl, halogenated C3-C6 cycloalkyl, and phenyl;
the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;
each m, n, and p is each independently selected from the group consisting of 0, 1, 2, 3, and 4.

In another preferred embodiment,

$R_1$ is selected from the group consisting of H, $CF_3$, F, Cl, Br, methyl, ethyl, isopropyl, and cyclopropyl;
$R_2$ is selected from the group consisting of H, F, $CF_3$, $CF_2H$, $NH_2$, and methyl;
ring A is selected from the group consisting of

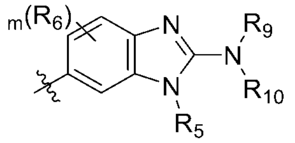
,

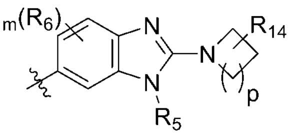
,
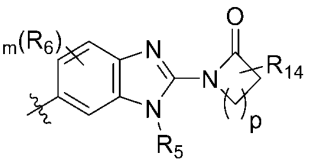
,
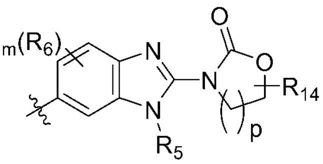
,

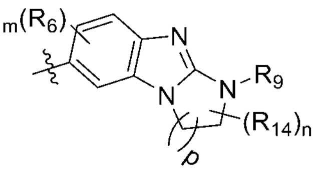
,
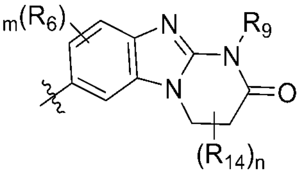
,

ring B is selected from the group consisting of

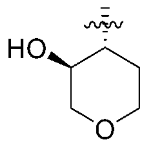
,
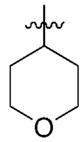
,
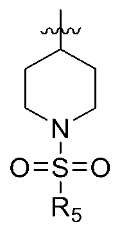
,
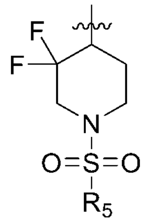
,

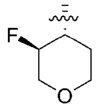
,
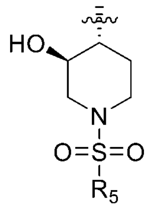
,
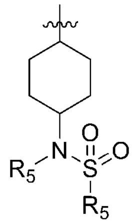
,
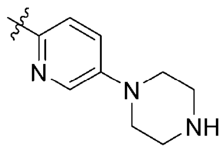
,
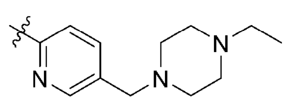
,

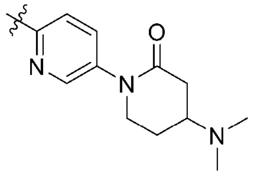, 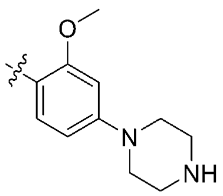, 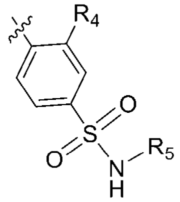, 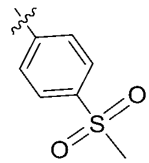, 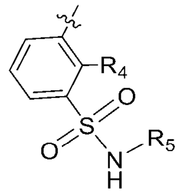,

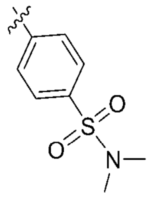, 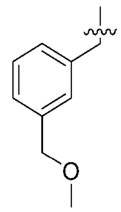, 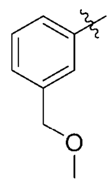, 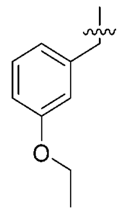, 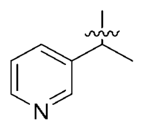, 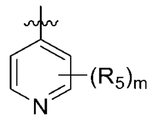, 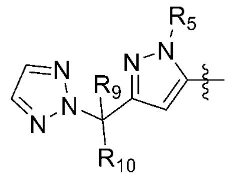,

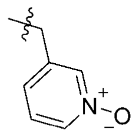, 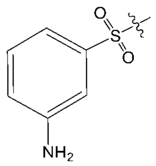, 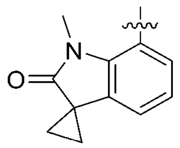, 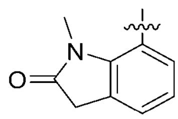, 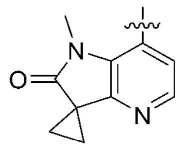, 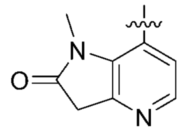,

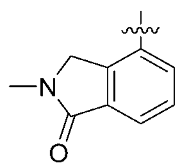, and 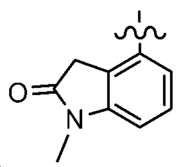;

wherein, each $R_4$ is independently selected from the group consisting of H, cyano, halogen, substituted or unsubstituted C1-C6 alkyl, and substituted or unsubstituted C3-C6 cycloalkyl;

each $R_5$, $R_6$, $R_9$, $R_{10}$ and $R_{14}$ is each independently selected from the group consisting of H, halogen, hydroxyl, amino,

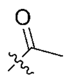, 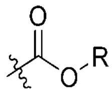, 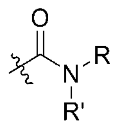,

substituted or unsubstituted C1-C6 alkyl, substituted or unsubstituted C3-C6 cycloalkyl, and substituted or unsubstituted phenyl;

or, $R_9$ and $R_{10}$ together with the N to which they are attached form

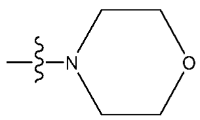;

R and R' is each independently selected from the group consisting of H, C1-C6 alkyl, halogenated C1-C6 alkyl, C3-C6 cycloalkyl, halogenated C3-C6 cycloalkyl, and phenyl;

the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;

each m, n, and p is each independently selected from the group consisting of 0, 1, 2, 3, and 4.

In another preferred embodiment,

$R_1$ is selected from the group consisting of $CF_3$, F, Cl, and Br;

$R_2$ is H;
ring A is selected from the group consisting of

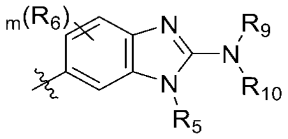,

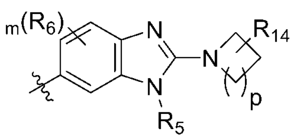, 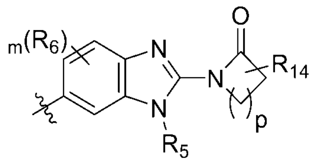, 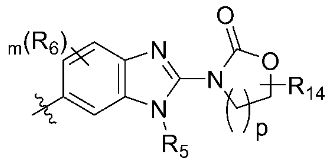,

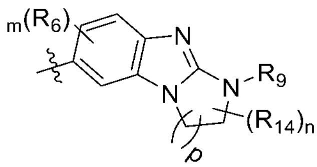, and 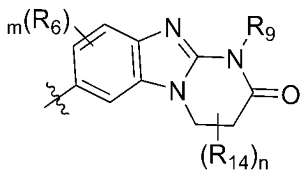;

ring B is selected from the group consisting of

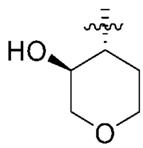, 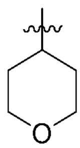, 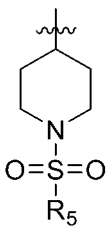, 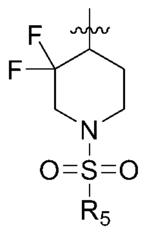,

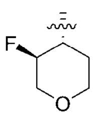, 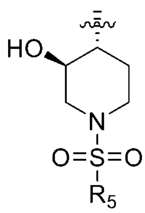, 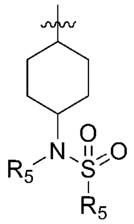, 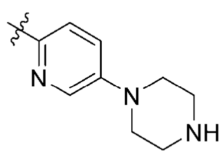, 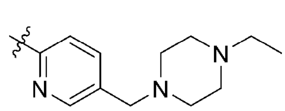,

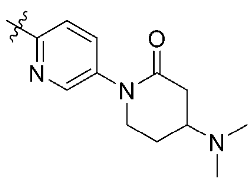, 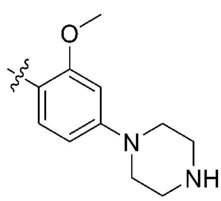, 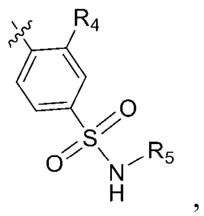, 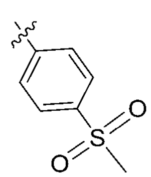, 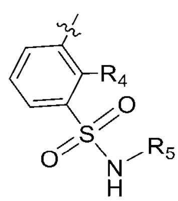,

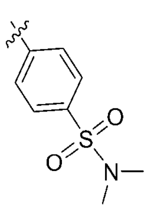, 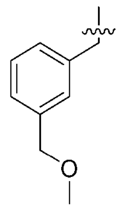, 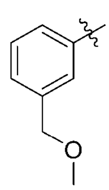, 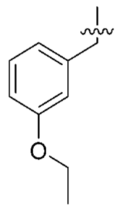, 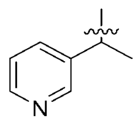, 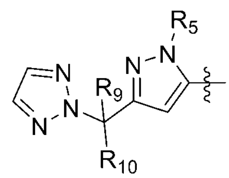, 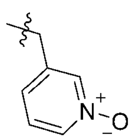,

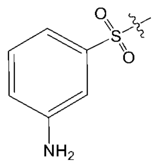, 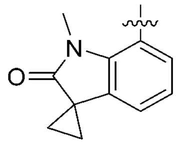, 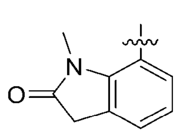, 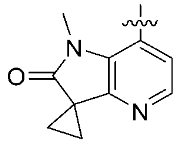, 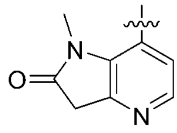, 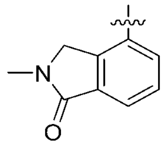,

and

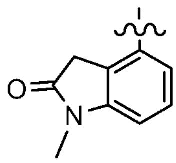;

wherein, each $R_4$ is independently selected from the group consisting of H, substituted or unsubstituted C1-C6 alkyl, and substituted or unsubstituted C3-C6 cycloalkyl;
each $R_5$, $R_6$, $R_9$, $R_{10}$ and $R_{14}$ is each independently selected from the group consisting of H, halogen, hydroxyl, amino,

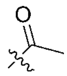, 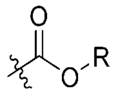, 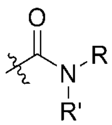,

substituted or unsubstituted C1-C6 alkyl, substituted or unsubstituted C3-C6 cycloalkyl, and substituted or unsubstituted phenyl;
or, $R_9$ and $R_{10}$ together with the N to which they are attached form

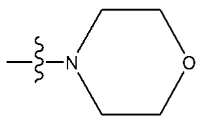;

R and R' is each independently selected from the group consisting of H, C1-C6 alkyl, halogenated C1-C6 alkyl, C3-C6 cycloalkyl, halogenated C3-C6 cycloalkyl, and phenyl;
the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;
each m, n, and p is each independently selected from the group consisting of 0, 1, 2, 3, and 4.

In another preferred embodiment,

$R_1$ and $R_2$ together with the C to which they are attached form a 5-membered heteroaryl containing two N atoms;
ring A is selected from the group consisting of

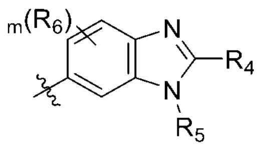, 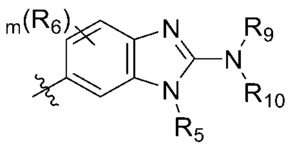,

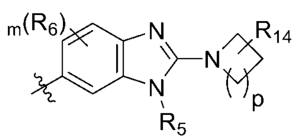, 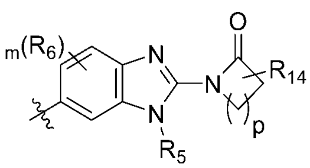, 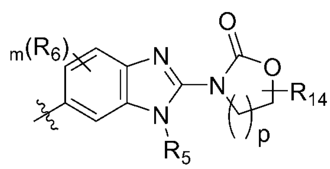,

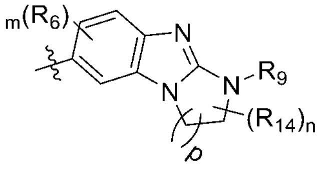 , and 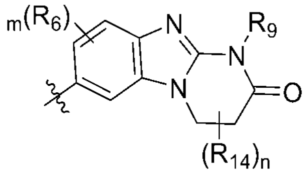 ,

ring B is selected from the group consisting of

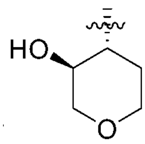 , 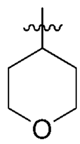 , 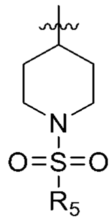 , 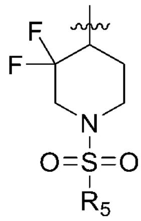 ,

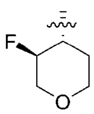 , 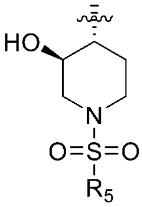 , 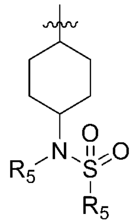 , 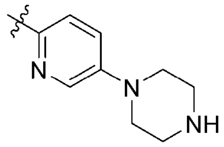 , 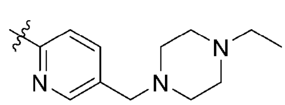 ,

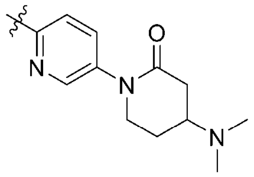 , 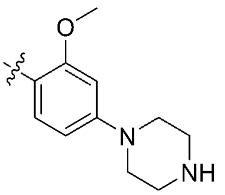 , 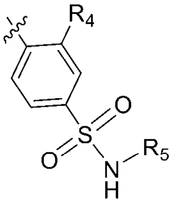 , 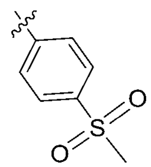 , 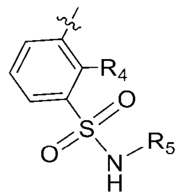 ,

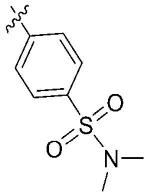 , 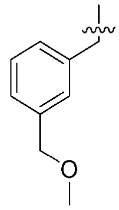 , 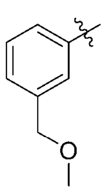 , 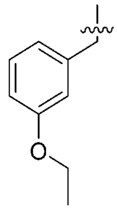 , 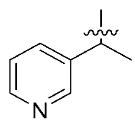 , 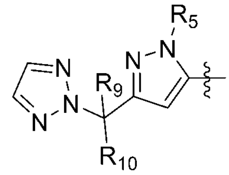 , 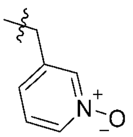 ,

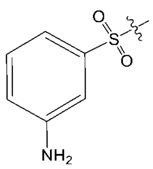 , 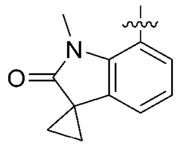 , 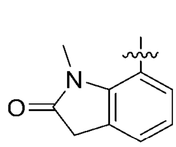 , 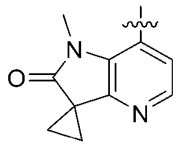 , 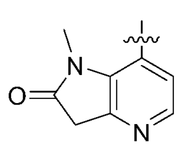 , 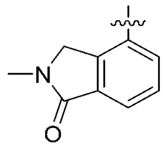 ,

and

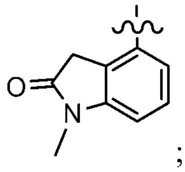 ;

wherein, each $R_4$ is independently selected from the group consisting of H, substituted or unsubstituted C1-C6 alkyl, and substituted or unsubstituted C3-C6 cycloalkyl;

each $R_5$, $R_6$, $R_9$, $R_{10}$ and $R_{14}$ is each independently selected from the group consisting of H, halogen, hydroxyl, amino,

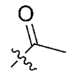, 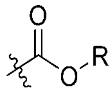, 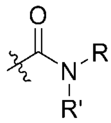,

substituted or unsubstituted C1-C6 alkyl, substituted or unsubstituted C3-C6 cycloalkyl, and substituted or unsubstituted phenyl;

R and R' is each independently selected from the group consisting of H, C1-C6 alkyl, halogenated C1-C6 alkyl, C3-C6 cycloalkyl, halogenated C3-C6 cycloalkyl, and phenyl;

the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;

each m, n, and p is each independently selected from the group consisting of 0, 1, 2, 3, and 4.

In another preferred embodiment,

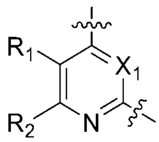

has the following structure

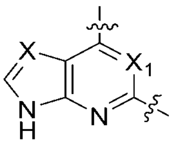;

X is selected from the group consisting of N, and $CR_3$;

$R_3$ is selected from the group consisting of H, $CF_3$, F, Cl, Br, methyl, and cyano.

In the third aspect, the present invention provides a compound, wherein the compound is a compound of formula I, or a pharmaceutically acceptable salt, a stereoisomer, a tautomer, a hydrate, a solvate, an isotope compound, or a prodrug thereof,

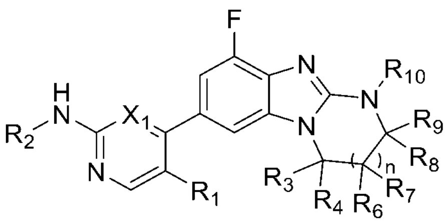

formula I

wherein:

$X_1$ is selected from the group consisting of N and $CR_{11}$;

$R_1$ is selected from the group consisting of H, halogen, cyano, $C_{1-6}$ alkyl, and $C_{1-6}$ haloalkyl;

$R_2$ is selected from the group consisting of

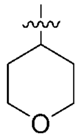, 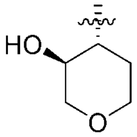, 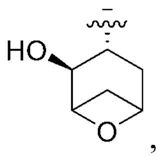, 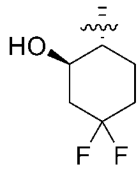,

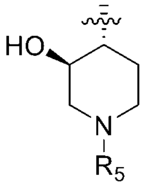, 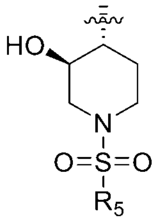, 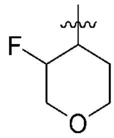, 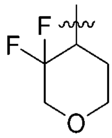, 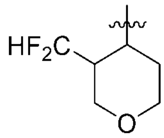, 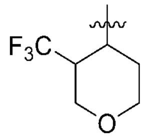, and 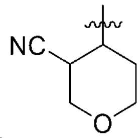;

$R_5$ is selected from the group consisting of H, halogen, hydroxyl, amino,

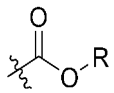, 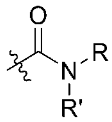,

$C_{1-6}$ alkyl, $C_{3-6}$ cycloalkyl, and phenyl;
R and R' are each independently selected from the group consisting of H and $C_{1-6}$ alkyl;
$R_3$, $R_4$, $R_6$, $R_7$, $R_8$, and $R_9$ are each independently selected from the group consisting of H, $C_{1-6}$ alkyl, $C_{3-6}$ cycloalkyl, hydroxyl, amino, ketocarbonyl, $C_{1-6}$ alkoxy, $-COC_{1-6}$ alkoxy, $-COC_{1-6}$ alkyl, $C_{1-6}$ haloalkyl, $C_{1-6}$ haloalkoxy, hydroxyl substituted $C_{1-6}$ alkyl, 5-8 membered heterocycloalkyl containing 1, 2, or 3 heteroatoms selected from N, O, and S, and 5-8 membered heteroaryl containing 1, 2, or 3 heteroatoms selected from N, O, and S;
$R_{10}$ is selected from the group consisting of H, $C_{1-6}$ alkyl, deuterated $C_{1-6}$ alkyl, $C_{3-8}$ cycloalkyl, $C_{1-6}$ alkoxy, $-COC_{1-6}$ alkoxy, $-COC_{1-6}$ alkyl, $C_{6-10}$ aryl, 5-8 membered heteroaryl containing 1, 2, or 3 heteroatoms selected from N, O, and S, 4-8 membered heterocyclyl containing 1, 2, or 3 heteroatoms selected from N, O, and S,

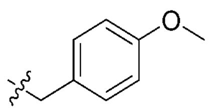,

$-S(=O)_2-C_{1-6}$ alkyl, and -CO-CN; or, $R_{10}$ together with adjacent $R_8$ or $R_9$ forms a 5-8 membered heterocyclyl containing 1, 2, or 3 heteroatoms selected from N, O, and S, wherein the alkyl, aryl, cycloalkyl, and heterocyclyl are optionally substituted with one or more groups selected from the group consisting of halogen, cyano, hydroxyl, $C_{1-6}$ alkyl, $C_{1-6}$ haloalkyl, $-CO-C_{1-6}$ alkyl, $C_{1-6}$ alkoxy, amino, -COOH, $-CONH_2$, $-CF_3C(F)_2$, and 5-8 membered heterocyclyl containing 1, 2, or 3 heteroatoms selected from N, O and S which is optionally substituted with $C_{1-6}$ alkyl, $-CH(F)_2$, $-COO-C_{1-6}$ alkyl, or $-S(O)_2-C_{6-10}$ aryl;
$R_{11}$ is selected from the group consisting of H, halogen, cyano, $C_{1-6}$ alkyl, $C_{1-6}$ haloalkyl;
n is independently selected from the group consisting of 0, 1 and 2.

In another preferred embodiment, the compound is a compound of formula I, or a pharmaceutically acceptable salt, a stereoisomer, a tautomer, a hydrate, a solvate, an isotope compound, or a prodrug thereof,

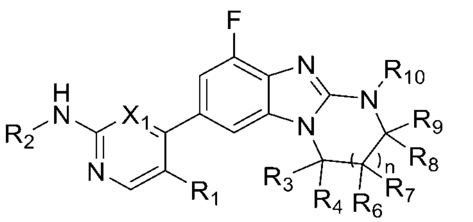

formula I

wherein:

$X_1$ is selected from the group consisting of N and $CR_{11}$;
$R_1$ is selected from the group consisting of H, halogen (such as F, Cl, and Br), cyano, $C_{1-6}$ alkyl (such as methyl, and ethyl), and $C_{1-6}$ haloalkyl (such as $CF_3$);
$R_2$ is selected from the group consisting of

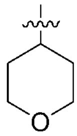, 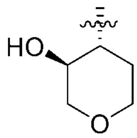, 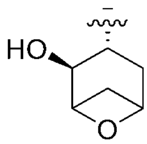, 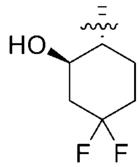,

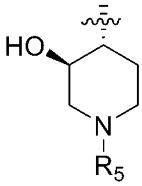, 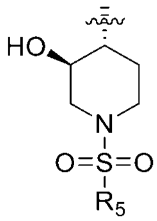, 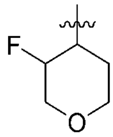, 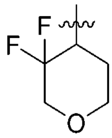, 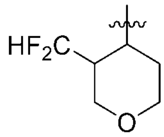, 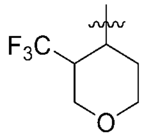, and 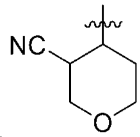;

$R_5$ is selected from the group consisting of H, halogen, hydroxyl, amino,

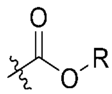, 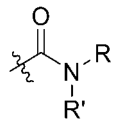,

$C_{1-6}$ alkyl, $C_{3-6}$ cycloalkyl, and phenyl;
R and R' are each independently selected from the group consisting of H and $C_{1-6}$ alkyl;
$R_3$, $R_4$, $R_6$, $R_7$, $R_8$, and $R_9$ are each independently selected from the group consisting of H, $C_{1-6}$ alkyl (such as methyl, ethyl, and isopropyl), $C_{3-6}$ cycloalkyl (such as

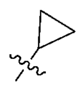

), hydroxyl, amino, ketocarbonyl, $C_{1-6}$ alkoxy (such as methoxy), -COC$_{1-6}$ alkoxy (such as

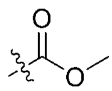

), -COC$_{1-}$alkyl (such as

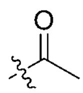

), $C_{1-6}$ haloalkyl (such as $CF_3$), $C_{1-6}$ haloalkoxy, hydroxyl substituted $C_{1-6}$ alkyl, 5-8 membered heterocycloalkyl containing 1, 2, or 3 heteroatoms selected from N, O, and S, and 5-8 membered heteroaryl containing 1, 2, or 3 heteroatoms selected from N, O, and S;
$R_{10}$ is selected from the group consisting of H, $C_{1-6}$ alkyl (such as methyl, ethyl, and isopropyl), deuterated $C_{1-6}$ alkyl (such as deuterated methyl), $C_{3-8}$ cycloalkyl (such as

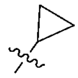,

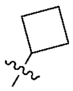, 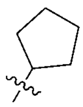, and 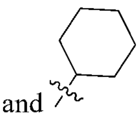

), $C_{1-6}$ alkoxy (such as methoxy), -$COC_{1-6}$ alkoxy (such as

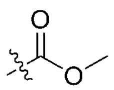

), - $COC_{1-6}$ alkyl (such as

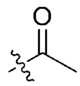

), $C_{6-10}$ aryl, 5-8 membered heteroaryl containing 1, 2, or 3 heteroatoms selected from N, O, and S, 4-8 membered heterocyclyl containing 1, 2, or 3 heteroatoms selected from N, O, and S, and

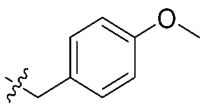;

or, $R_{10}$ together with adjacent $R_8$ or $R_9$ forms a 5-8 membered heterocyclyl containing 1, 2, or 3 heteroatoms selected from N, O, and S, wherein the alkyl, aryl, cycloalkyl, and heterocyclyl are optionally substituted with one or more groups selected from the group consisting of halogen, cyano, hydroxyl, $C_{1-6}$ alkyl, -CO-$C_{1-6}$ alkyl, $C_{1-6}$ alkoxy (such as -O-methyl), amino (such as -$NH_2$), -COOH, -$CONH_2$, -$CF_3C(F)_2$, and 5-8 membered heterocyclyl containing 1, 2, or 3 heteroatoms selected from N, O and S which is optionally substituted with $C_{1-6}$ alkyl, -$CH(F)_2$, -COO-$C_{1-6}$ alkyl (such as -COO-$C(CH_3)_3$), or -$S(O)_2$-$C_{6-10}$ aryl;

$R_{11}$ is selected from the group consisting of H, halogen, (such as F, Cl, and Br), cyano, $C_{1-}$alkyl (such as methyl, and ethyl), and $C_{1-6}$ haloalkyl (such as $CF_3$);

n is independently selected from the group consisting of 0, 1 and 2.

In another preferred embodiment, $X_1$ is selected from the group consisting of N and $CR_{11}$.

In another preferred embodiment, $R_1$ is selected from the group consisting of H, halogen (such as F, Cl, and Br), cyano, $C_{1-6}$ alkyl (such as methyl, and ethyl), and $C_{1-6}$ haloalkyl (such as $CF_3$).

In another preferred embodiment, $R_2$ is selected from the group consisting of

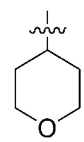,

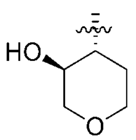, 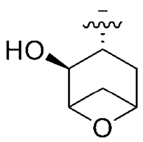, 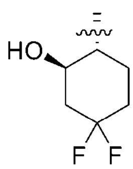, 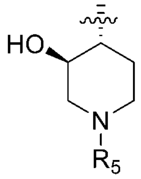, 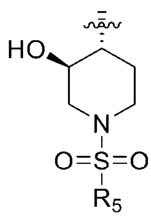, 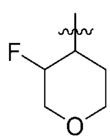, 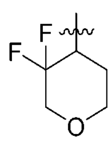,

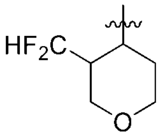, 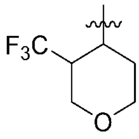, and 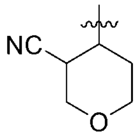.

In another preferred embodiment, $R_2$ is selected from the group consisting of

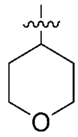,

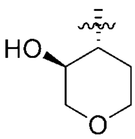, 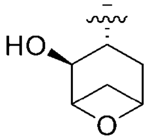, 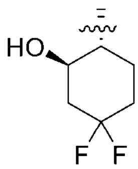, 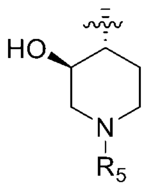, 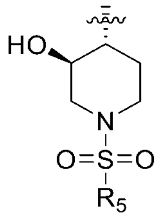, 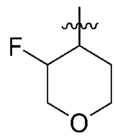, 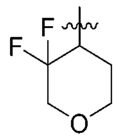,

and

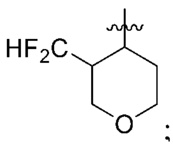;

$R_{10}$ is selected from the group consisting of H, $C_{1-6}$ alkyl (such as methyl, ethyl, and isopropyl), deuterated $C_{1-6}$ alkyl (such as deuterated methyl), $C_{3-8}$ cycloalkyl (such as

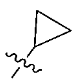,

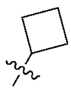, 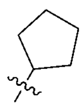, and 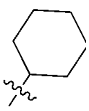

), $C_{1-6}$ alkoxy (such as methoxy), -$COC_{1-6}$ alkoxy (such as

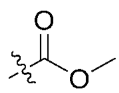

), - $COC_{1-6}$ alkyl (such as

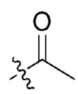

), $C_{6-10}$ aryl, 5-8 membered heteroaryl containing 1, 2, or 3 heteroatoms selected from N, O, and S, 5-8 membered heterocyclyl containing 1, 2, or 3 heteroatoms selected from N, O, and S, and

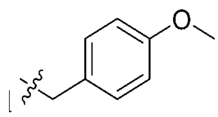;

or, $R_{10}$ together with adjacent $R_8$ or $R_9$ forms a 5-8 membered heterocyclyl containing 1, 2, or 3 heteroatoms selected from N, O, and S, wherein the alkyl, aryl, cycloalkyl, and heterocyclyl are optionally substituted with one or more groups selected from the group consisting of halogen, cyano, hydroxyl, $C_{1-6}$ alkyl, -CO-$C_{1-}$alkyl, $C_{1-6}$ alkoxy (such as -O-methyl), amino (such as -$NH_2$), -COOH, -$CONH_2$, -$CF_3C(F)_2$, and 5-8 membered heterocyclyl containing 1, 2, or 3 heteroatoms selected from N, O and S which is optionally substituted with $C_{1-6}$ alkyl, -$CH(F)_2$, -COO-$C_{1-6}$ alkyl (such as -COO-$C(CH_3)_3$), or -$S(O)_2$-$C_{6-10}$ aryl.

In another preferred embodiment, $R_1$ is selected from the group consisting of Cl and Br.

In another preferred embodiment, $R_2$ is selected from the group consisting of

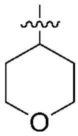,

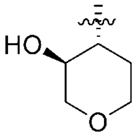, 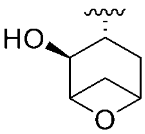, 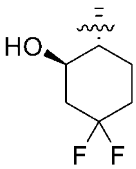, 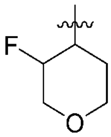, 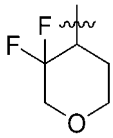, 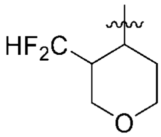, 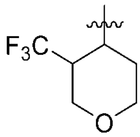,

and

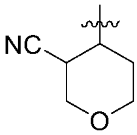.

In another preferred embodiment, $R_1$ is selected from the group consisting of Cl and Br; $R_2$ is selected from the group consisting of

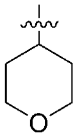, 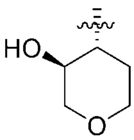, 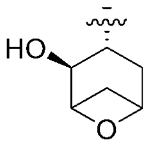, 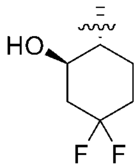,

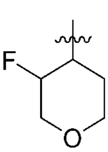, 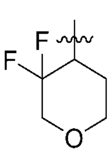, 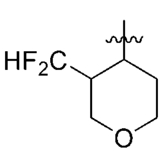, 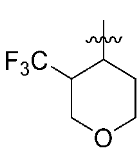, and 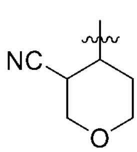.

In another preferred embodiment, $R_3$, $R_4$, $R_6$, $R_7$, $R_8$, and $R_9$ are each independently selected from the group consisting of H, $C_{1-6}$ alkyl (such as methyl, ethyl, and isopropyl), $C_{3-6}$ cycloalkyl (such as

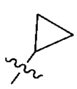

), hydroxyl, amino, $C_{1-6}$ alkoxy (such as methoxy), -$COC_{1-6}$ alkoxy (such as

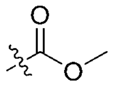

), $-COC_{1-6}$ alkyl (such as

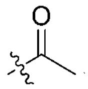

), $C_{1-6}$ haloalkyl (such as $CF_3$), $C_{1-6}$ haloalkoxy, hydroxyl substituted $C_{1-6}$ alkyl, and 5-8 membered heterocycloalkyl containing 1, 2, or 3 heteroatoms selected from N, O, and S.

In another preferred embodiment, $R_3$, $R_4$, $R_6$, $R_7$, $R_8$, and $R_9$ are each independently selected from the group consisting of H, $C_{1-6}$ alkyl (such as methyl, ethyl, and isopropyl), $C_{3-6}$ cycloalkyl (such as

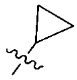

), $C_{1-6}$ haloalkyl (such as $CF_3$), $C_{1-6}$ haloalkoxy, and hydroxyl substituted $C_{1-6}$ alkyl.

In another preferred embodiment, $R_3$, $R_4$, $R_6$, $R_7$, $R_8$, and $R_9$ are each independently selected from the group consisting of H, $C_{1-6}$ alkyl (such as methyl, ethyl, and isopropyl), $C_{3-6}$ cycloalkyl (such as

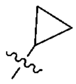

), and $C_{1-6}$ haloalkyl (such as $CF_3$).

In another preferred embodiment, $R_{10}$ is selected from the group consisting of H, $C_{1-6}$ alkyl (such as methyl, ethyl, and isopropyl), deuterated $C_{1-6}$ alkyl (such as deuterated methyl), $C_{3-8}$ cycloalkyl (such as

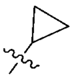 , 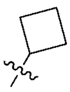 , 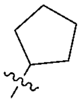 , and 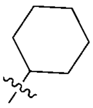

), $C_{6-10}$ aryl, 4-8 membered heterocyclyl containing 1, 2, or 3 heteroatoms selected from N, O, and S, and

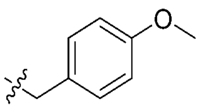 ;

or, $R_{10}$ together with adjacent $R_8$ or $R_9$ forms a 5-8 membered heterocyclyl containing 1, 2, or 3 heteroatoms selected from N, O, and S, wherein the alkyl, aryl, cycloalkyl, and heterocyclyl are optionally substituted with one or more groups selected from the group consisting of halogen, cyano, hydroxyl, $C_{1-6}$ alkyl, amino (such as $-NH_2$), and $-CF_3C(F)_2$.

In another preferred embodiment, $R_{10}$ is selected from the group consisting of $C_{1-6}$ alkyl (such as methyl, ethyl, and isopropyl), deuterated $C_{1-6}$ alkyl (such as deuterated methyl), and $C_{6-10}$ aryl; wherein the alkyl and aryl are optionally substituted with one or more groups selected from the group consisting of halogen, cyano, hydroxyl, $C_{1-6}$ alkyl, and $-CF_3C(F)_2$.

In another preferred embodiment, $R_{10}$ is selected from the group consisting of $C_{1-6}$ alkyl (such as methyl, ethyl, and isopropyl), and $C_{6-10}$ aryl; wherein the alkyl and aryl are optionally substituted with one or more groups selected from the group consisting of halogen, cyano, hydroxyl, and $C_{1-6}$ alkyl.

In another preferred embodiment, $R_{11}$ is selected from the group consisting of H, halogen, (such as F, Cl, and Br), cyano, $C_{1-6}$ alkyl (such as methyl, and ethyl), and $C_{1-6}$ haloalkyl (such as $CF_3$).

In another preferred embodiment, $R_{11}$ is H.

In another preferred embodiment, n is 1.

In another preferred embodiment, the pharmaceutically acceptable salt is an inorganic acid salt or an organic

acid salt.

In another preferred embodiment, the inorganic acid salt is selected from the group consisting of hydrochloride, hydrobromide, hydroiodate, sulfate, bisulfate, nitrate, phosphate, and acid phosphate.

In another preferred embodiment, the organic acid salt is selected from the group consisting of formate, acetate, trifluoroacetate, propionate, pyruvate, glycolate, oxalate, malonate, fumarate, maleate, lactate, malate, citrate, tartrate, methanesulfonate, ethane sulfonate, benzenesulfonate, p-toluenesulfonate, salicylate, picrate, glutamate, ascorbate, camphorate, and camphorsulfonate.

In another preferred embodiment, the compound is selected from the group consisting of:

| | | |
|---|---|---|
| 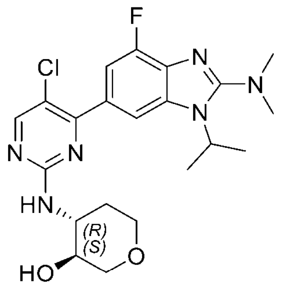 TB-1 | 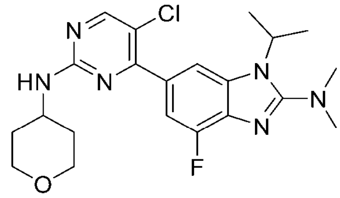 TB-2 | 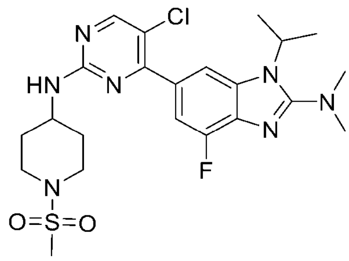 TB-3 |

| | | |
|---|---|---|
| 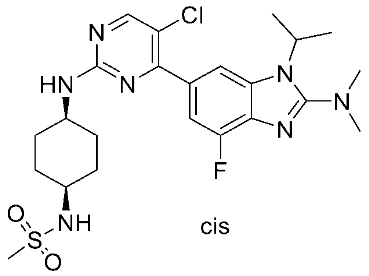 cis<br>TB-4 | 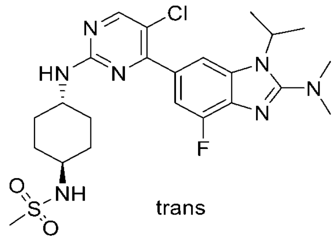 trans<br>TB-5 | 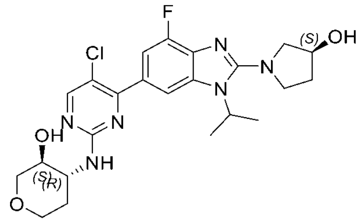<br>TB-6 |
| 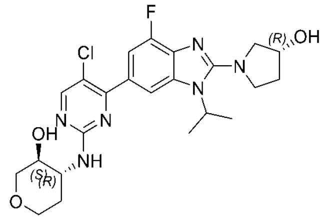<br>TB-7 | 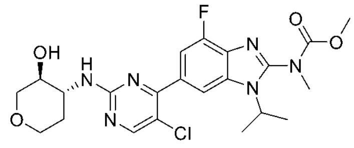<br>TB-8 | 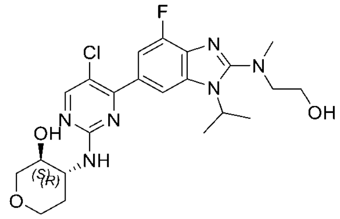<br>TB-9 |
| 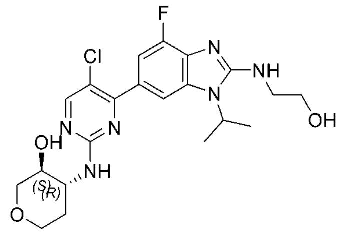<br>TB-10 | 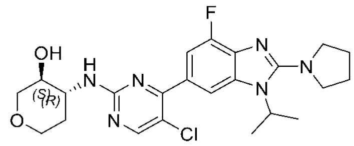<br>TB-11 | 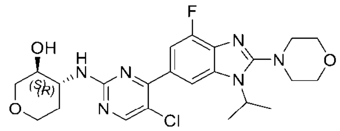<br>TB-12 |
| 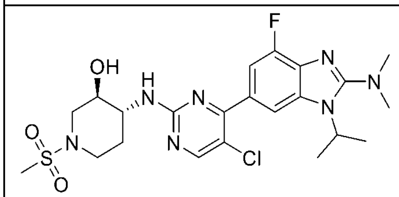<br>TB-13 | 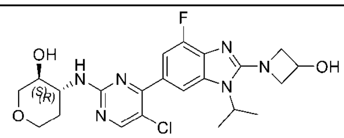<br>TB-14 | 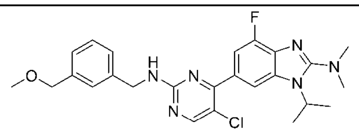<br>TB-15 |
| 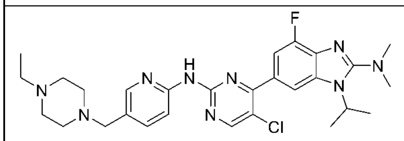<br>TB-16 | 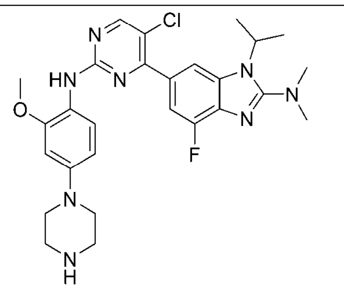<br>TB-17 | 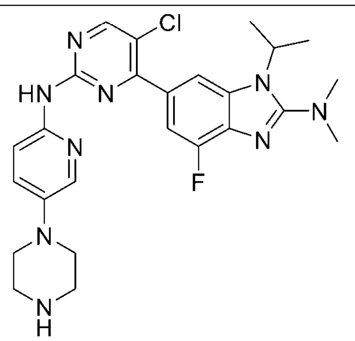<br>TB-18 |

| | | |
|---|---|---|
| 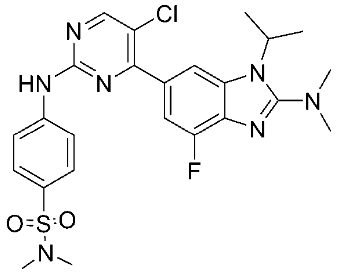 TB-19 | 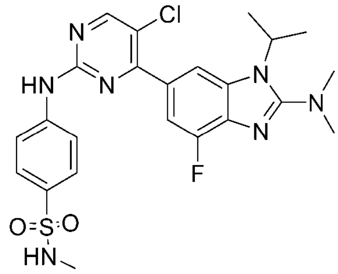 TB-20 | 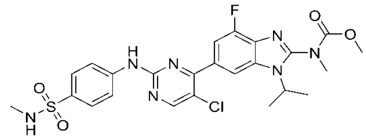 TB-21 |
| 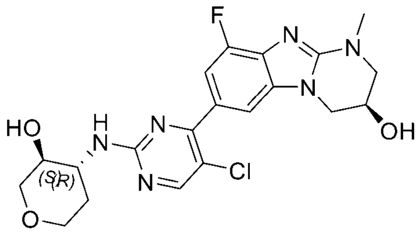 TB-22 | 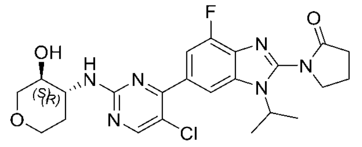 TB-23 | 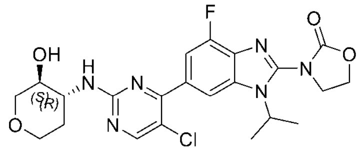 TB-24 |
| 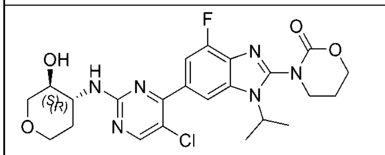 TB-25 | 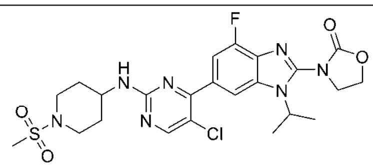 TB-26 | 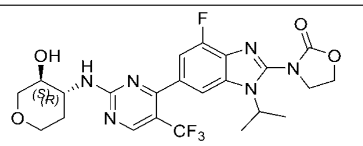 TB-27 |
| 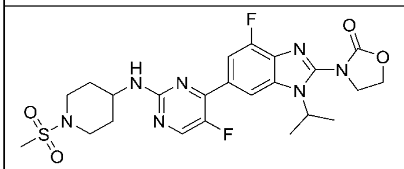 TB-28 | 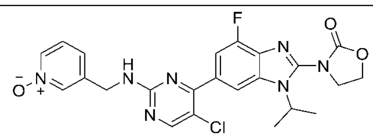 TB-29 | 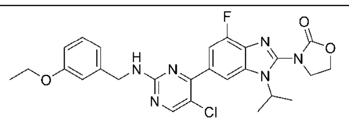 TB-30 |
| 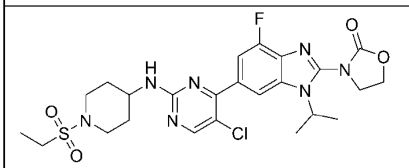 TB-31 | 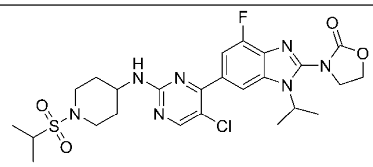 TB-32 | 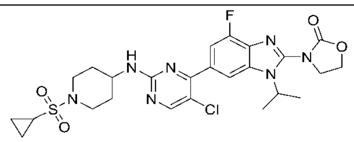 TB-33 |
| 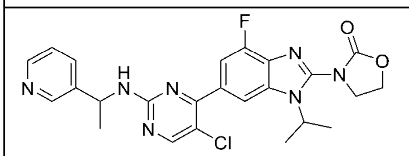 TB-34 | 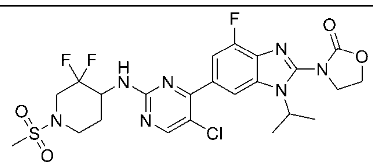 TB-35 | 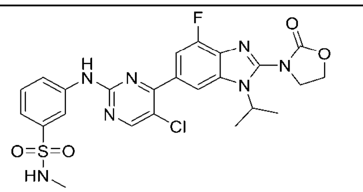 TB-36 |

| | | |
|---|---|---|
| 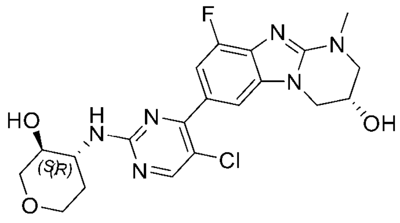 TB-37 | 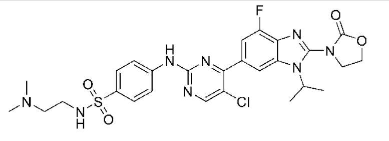 TB-38 | 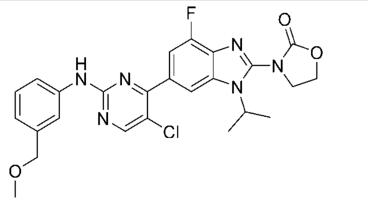 TB-39 |
| 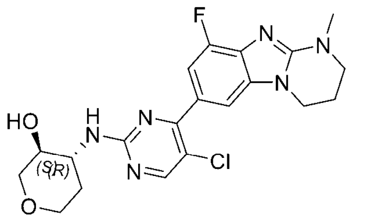 TB-40 | 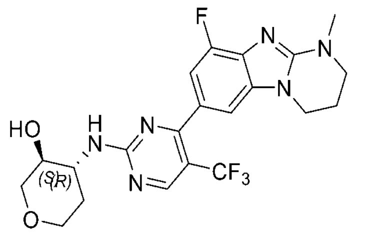 TB-41 | 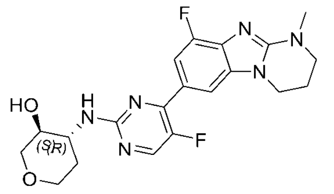 TB-42 |
| 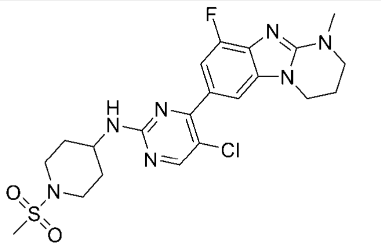 TB-43 | 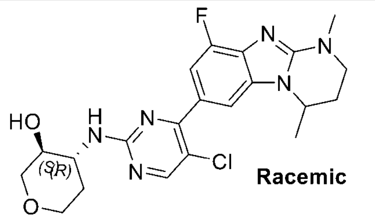 Racemic TB-44 | 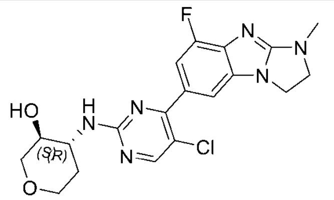 TB-45 |
| 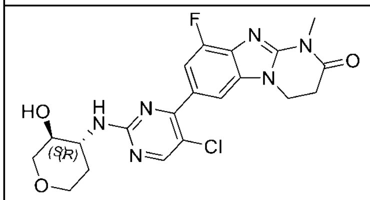 TB-46 | 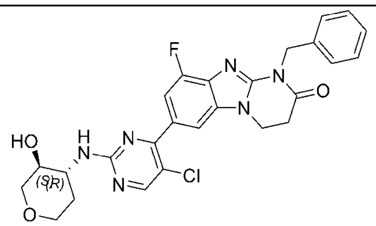 TB-47 | 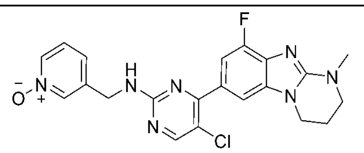 TB-48 |
| 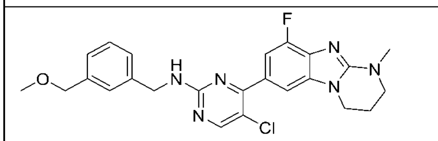 TB-49 | 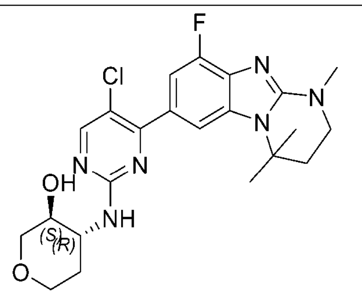 TB-50 | 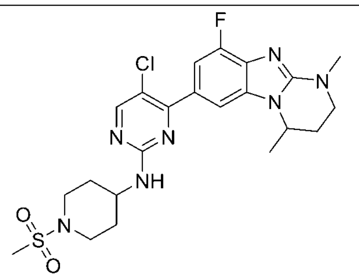 TB-51 |
| 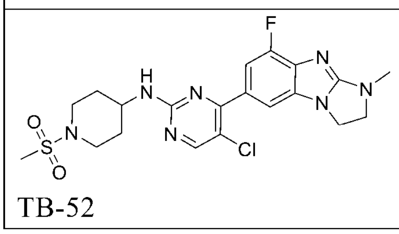 TB-52 | 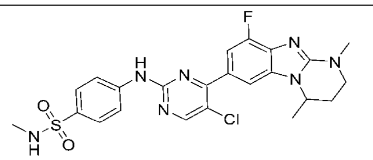 TB-53 | 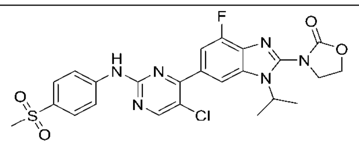 TB-54 |

| | | |
|---|---|---|
| 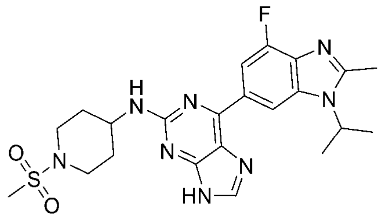 TB-55 | 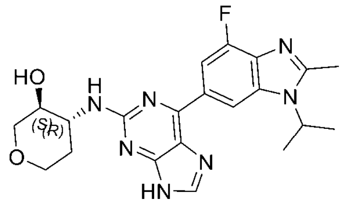 TB-56 | 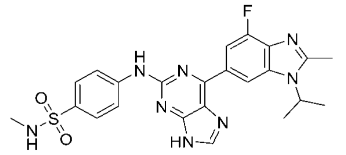 TB-57 |
| 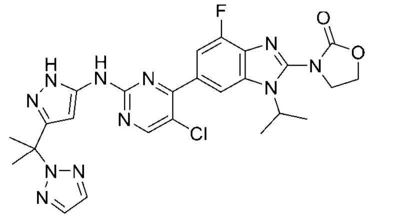 TB-58 | 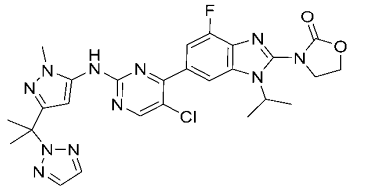 TB-59 | 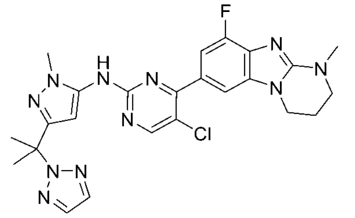 TB-60 |
| 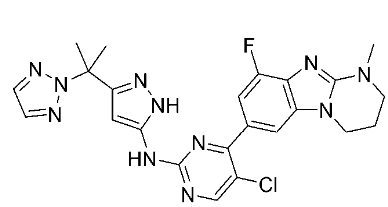 TB-61 | 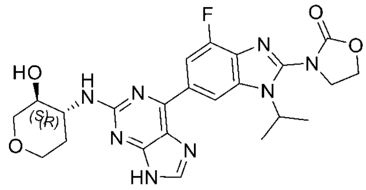 TB-62 | 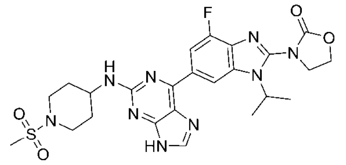 TB-63 |
| 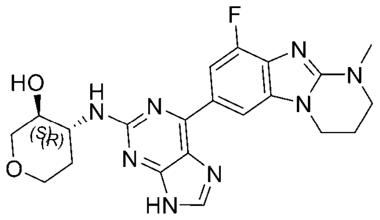 TB-64 | 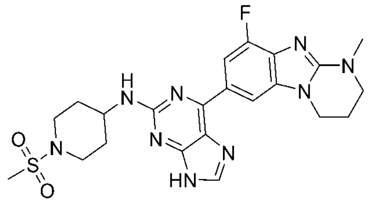 TB-65 | 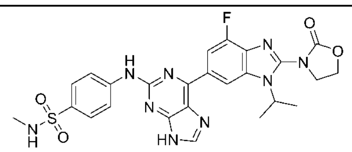 TB-66 |
| 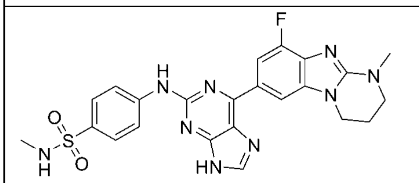 TB-67 | 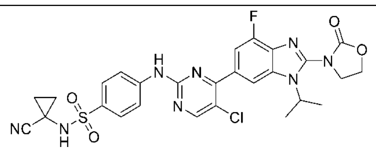 TB-68 | 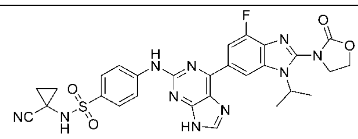 TB-69 |
| 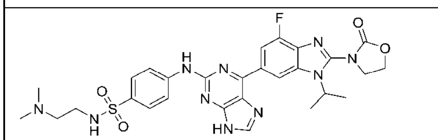 TB-70 | 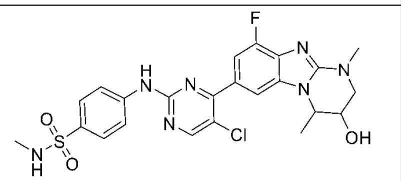 TB-71 | 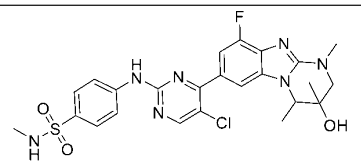 TB-72 |

| | | |
|---|---|---|
| 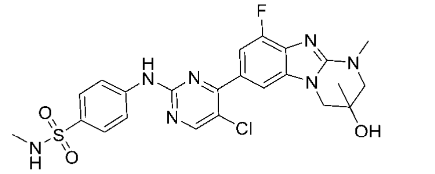 TB-73 | 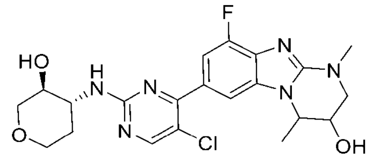 TB-74 | 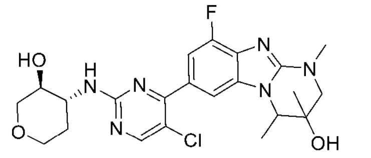 TB-75 |
| 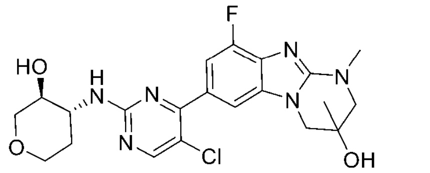 TB-76 | 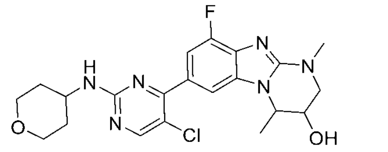 TB-77 | 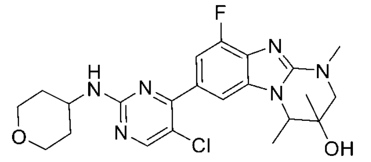 TB-78 |
| 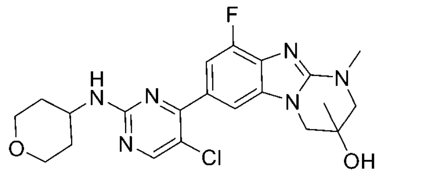 TB-79 | 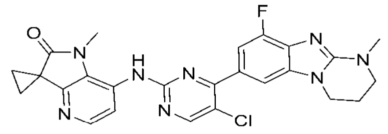 TB-80 | 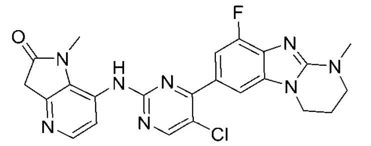 TB-81 |
| 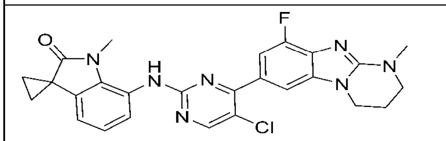 TB-82 | 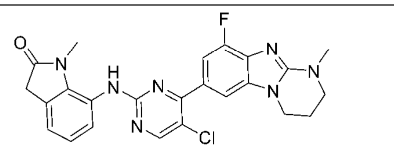 TB-83 | 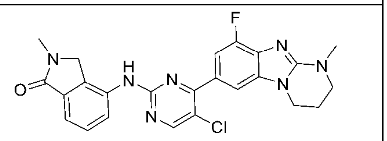 TB-84 |
| 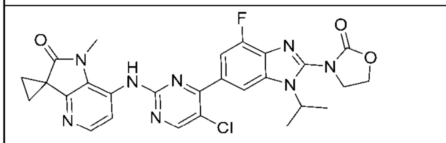 TB-85 | 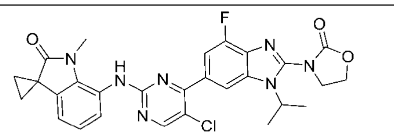 TB-86 | 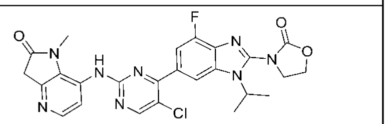 TB-87 |
| 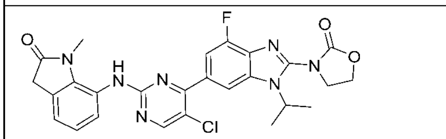 TB-88 | 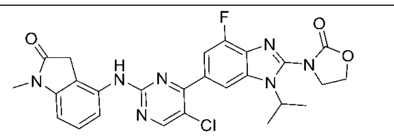 TB-89 | 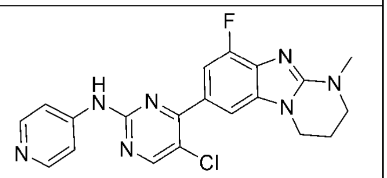 TB-90 |
| 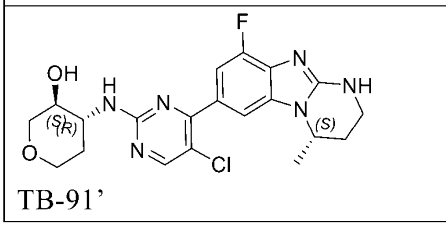 TB-91' | 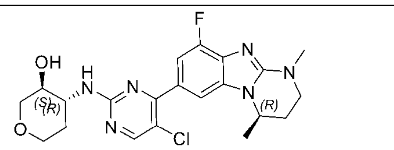 TB-91 | 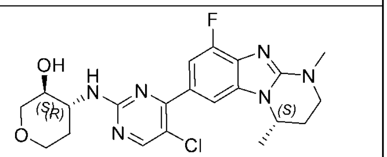 TB-92 (or TA-1) |

| | | |
|---|---|---|
| 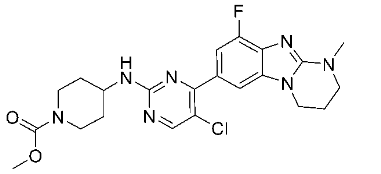<br>TB-93 | 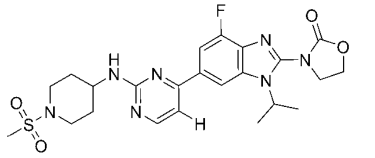<br>TB-94 | 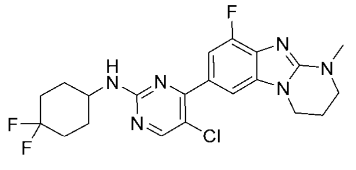<br>TB-95 |
| 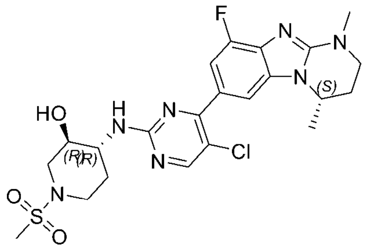<br>TB- 96 | 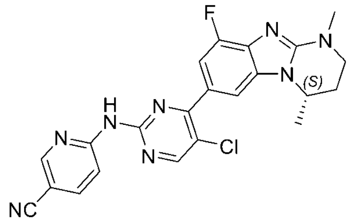<br>TB-97 | 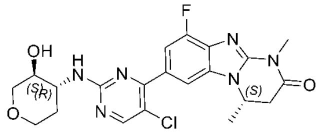<br>TB-98 |
| 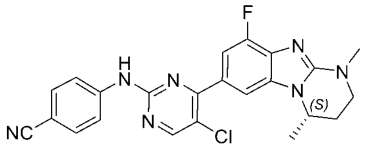<br>TB- 99 | 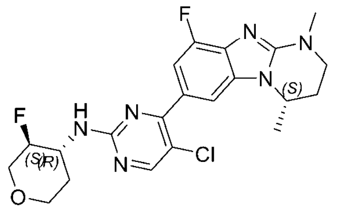<br>TB-100 | 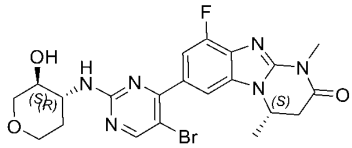<br>TB-101 |
| 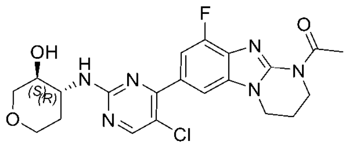<br>TB-102 | 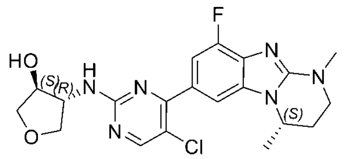<br>TB-103 | 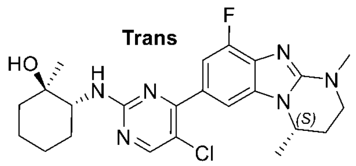<br>TB-104 |
| 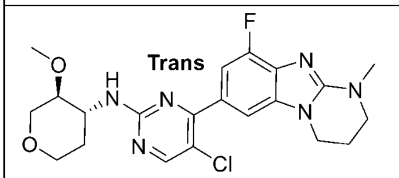<br>TB-105 | 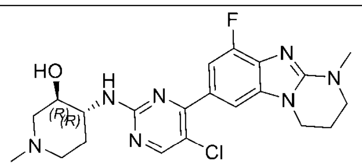<br>TB-106 | 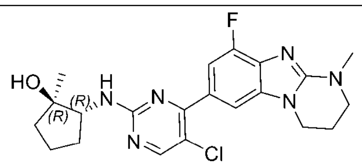<br>TB-107 |
| 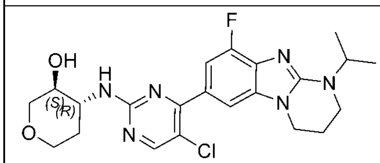<br>TB-108 | 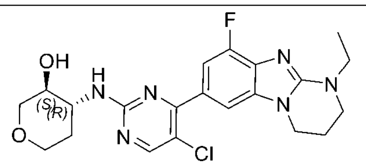<br>TB-109 | 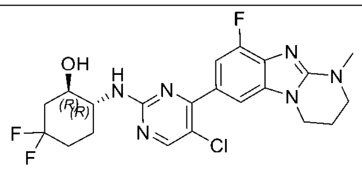<br>TB-110 |
| 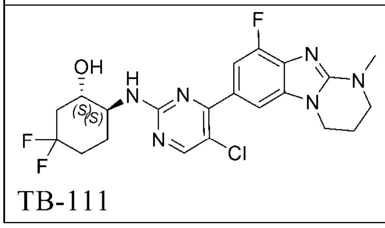<br>TB-111 | 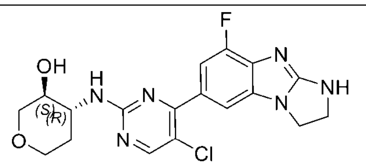<br>TB-112 | 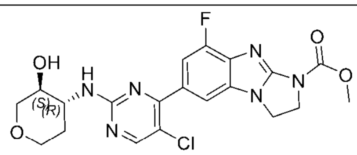<br>TB-113 |

| | | |
|---|---|---|
| 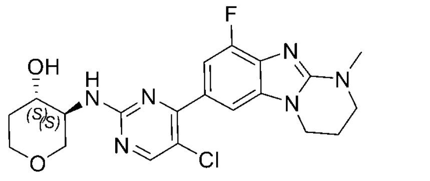 TB-114 | 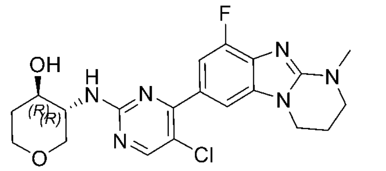 TB-115 | 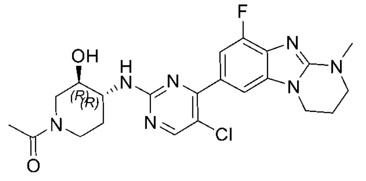 TB-116 |
| 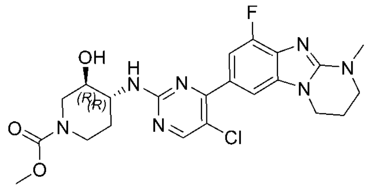 TB-117 | 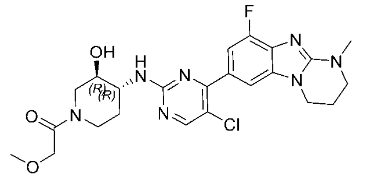 TB-118 | 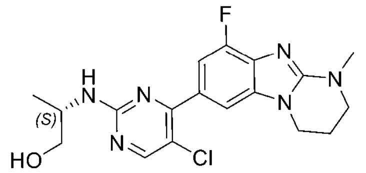 TB-119 |
| 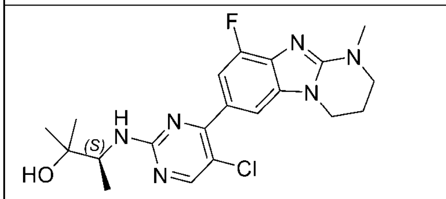 TB-120 | 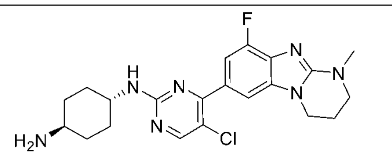 TB-121 | 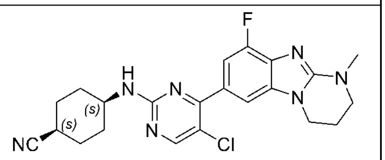 TB-122 |
| 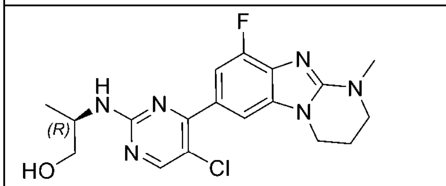 TB-123 | 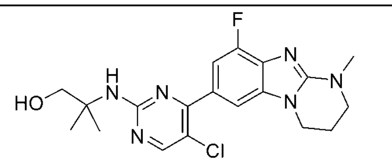 TB-124 | 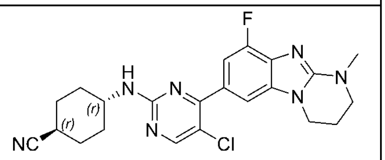 TB-125 |
| 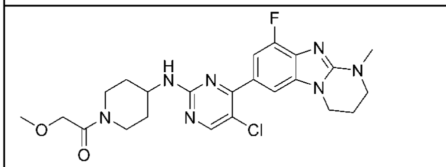 TB-126 | 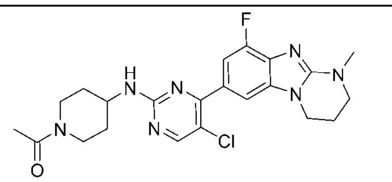 TB-127 | 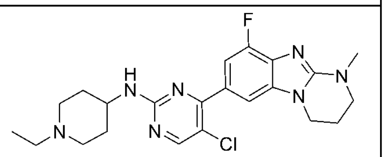 TB-128 |
| 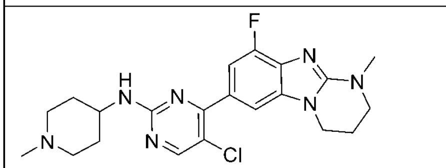 TB-129 | 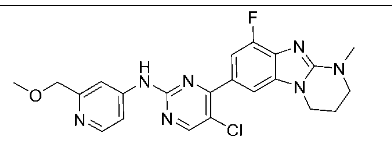 TB-130 | 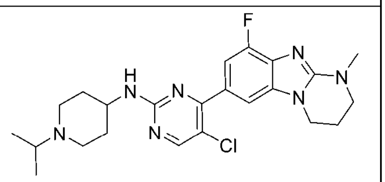 TB-131 |
| 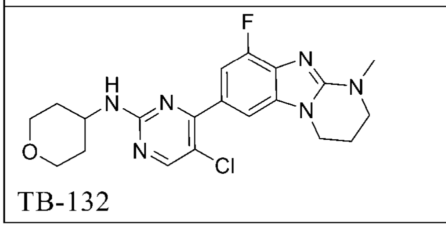 TB-132 | 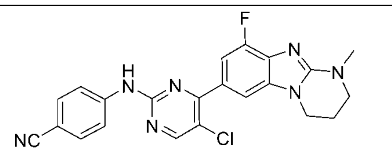 TB-133 | Racemic 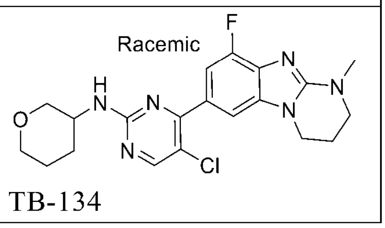 TB-134 |

| | | |
|---|---|---|
| 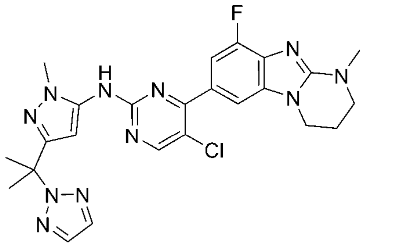 TB-135 | 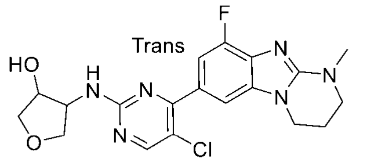 TB-136 | 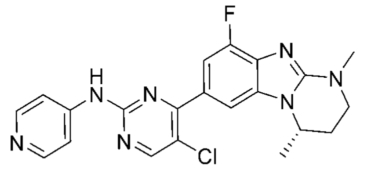 TB-137 |
| 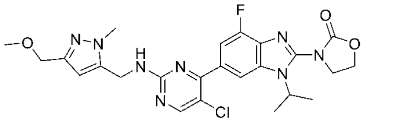 TB-138 | 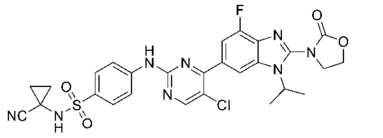 TB-139 | 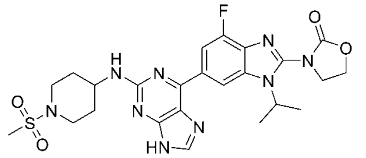 TB-140 |
| 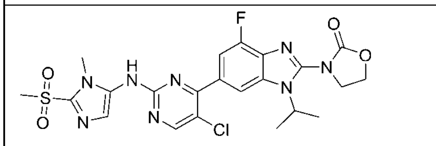 TB-141 | 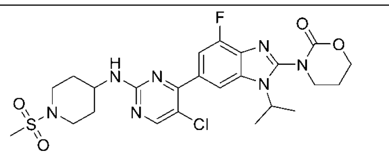 TB-142 | 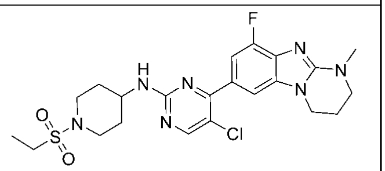 TB-143 |
| 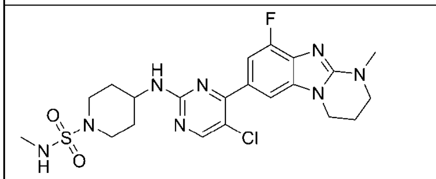 TB-144 | 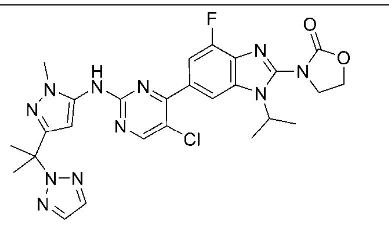 TB-145 | 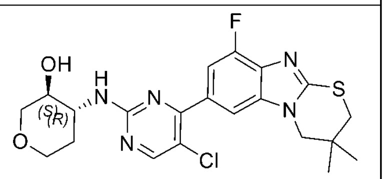 TB-146 |
| 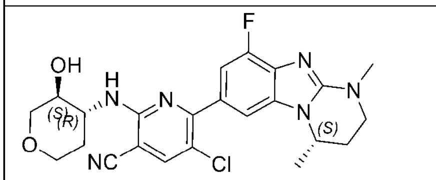 TB-147 | 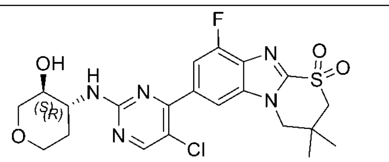 TB-148 | 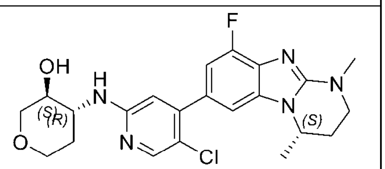 TB-149 (or TA-11) |
| 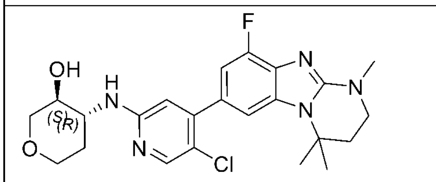 TB-150 | 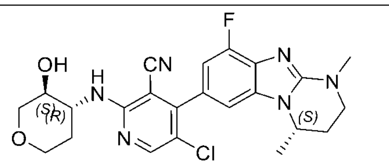 TB-151 | 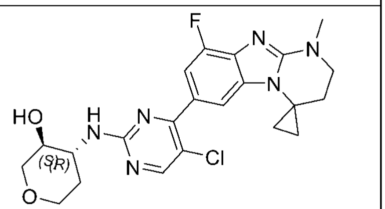 TB-152 |
| 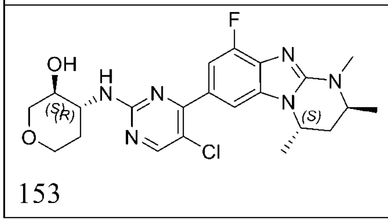 TB-153 | 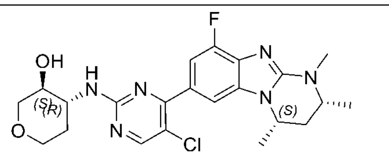 TB-154 | 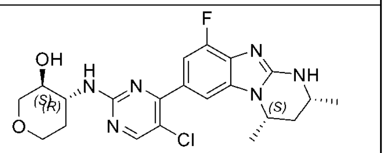 TB-155 |

| | | |
|---|---|---|
| 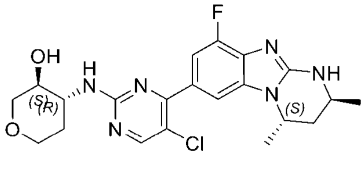 TB-156 | 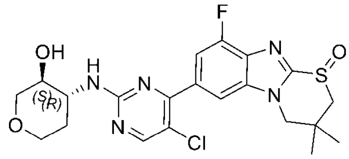 TB-157 | 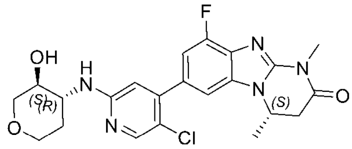 TB-158 |
| 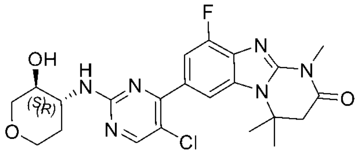 TB-159 | 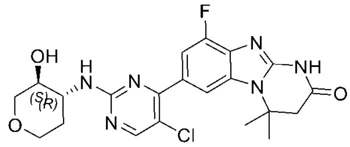 TB-160 | 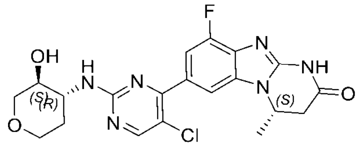 TB-161 |
| 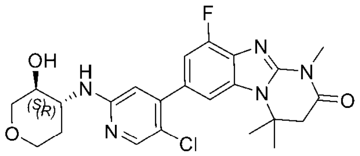 TB-162 | 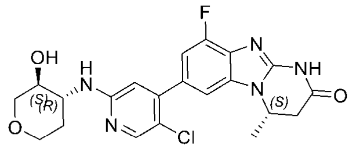 TB-163 | 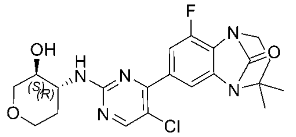 TB-164 |
| 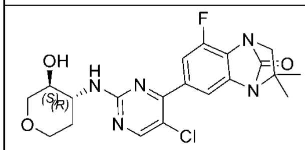 TB-165 | 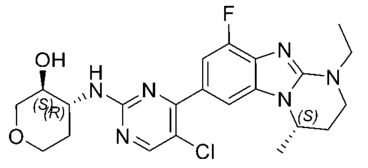 TA-2 | 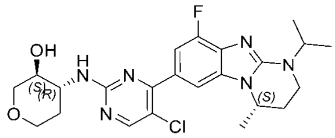 TA-3 |
| 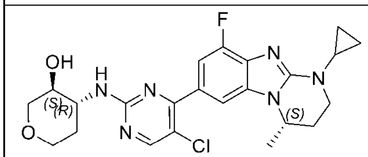 TA-4 | 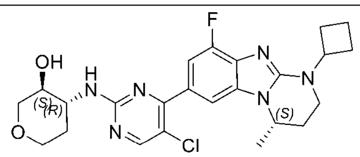 TA-5 | 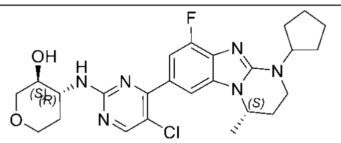 TA-6 |
| 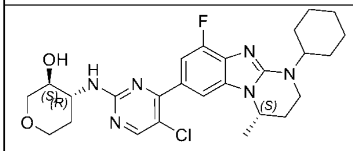 TA-7 | 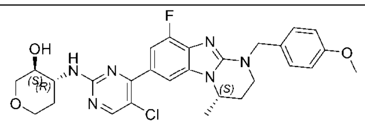 TA-8 | 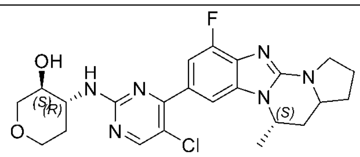 TA-9 |
| 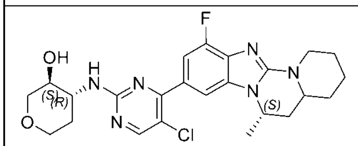 TA-10 | | 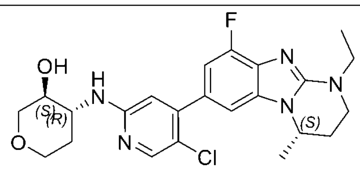 TA-12 |

| | | |
|---|---|---|
| 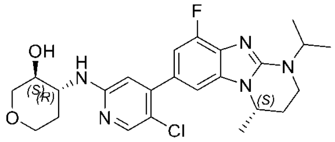 TA-13 | 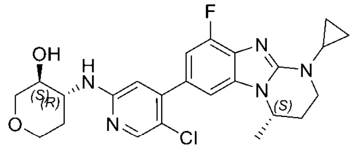 TA-14 | 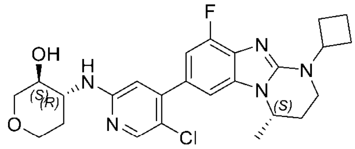 TA-15 |
| 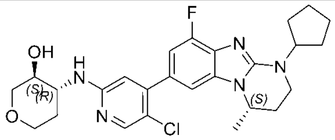 TA-16 | 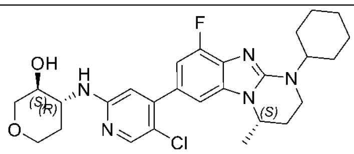 TA-17 | 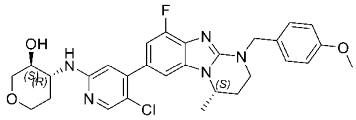 TA-18 |
| 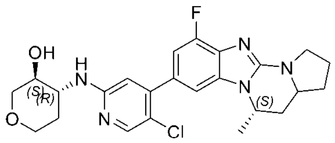 TA-19 | 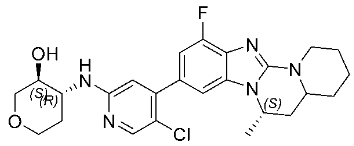 TA-20 | 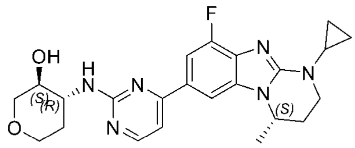 TA-21 |
| 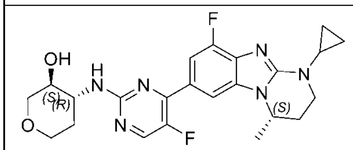 TA-22 | 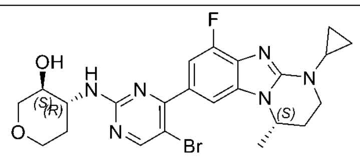 TA-23 | 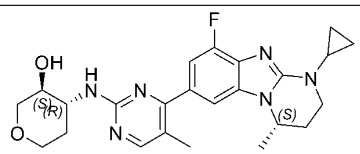 TA-24 |
| 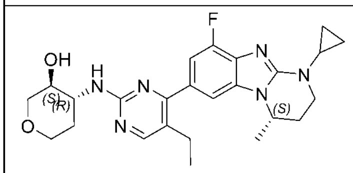 TA-25 | 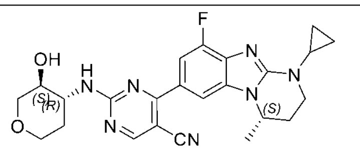 TA-26 | 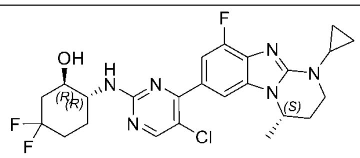 TA-27 |
| 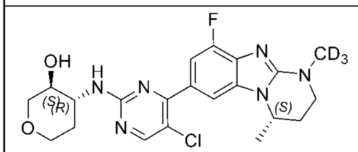 TA-28 | 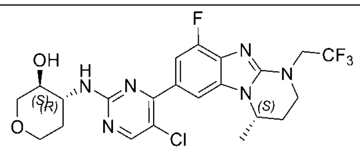 TA-29 | 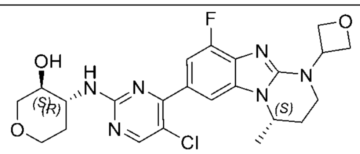 TA-30 |
| 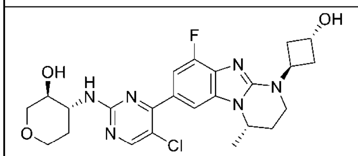 TA-31 | 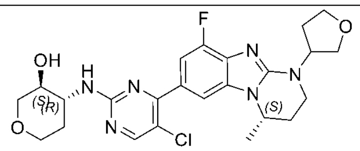 TA-32 | 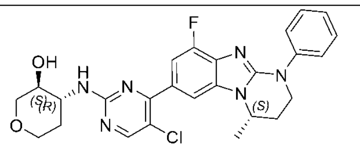 TA-33 |

| | | |
|---|---|---|
| 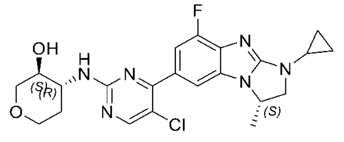 TA-34 | 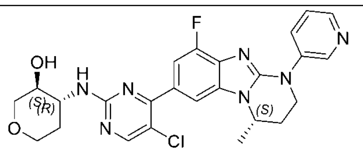 TA-35 | 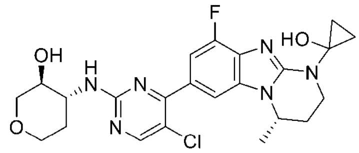 TA-36 |
| 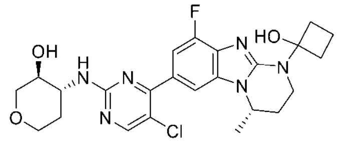 TA-37 | 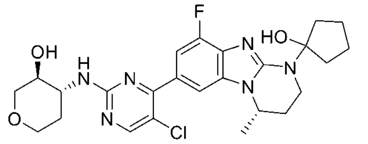 TA-38 | 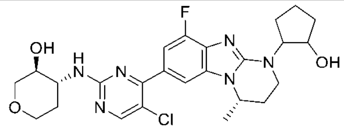 TA-39 |
| 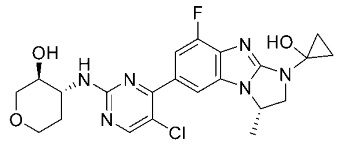 TA-40 | 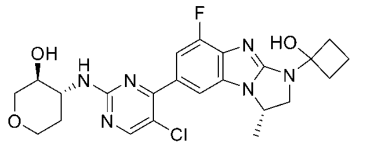 TA-41 | 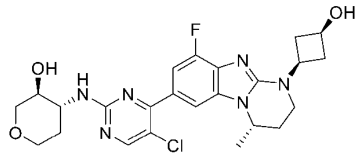 TA-42 |
| 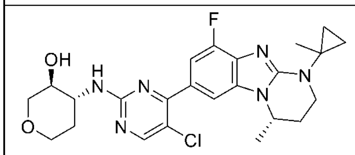 TA-43 | 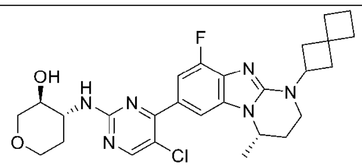 TA-44 | 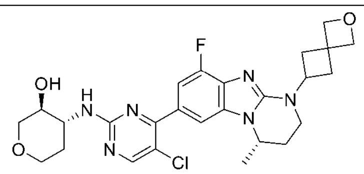 TA-45 |
| 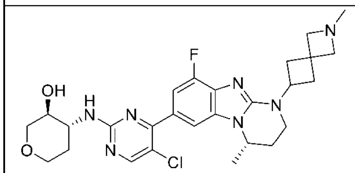 TA-46 | 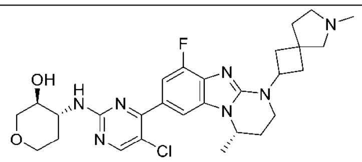 TA-47 | 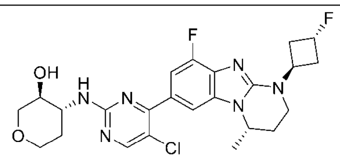 TA-48 |
| 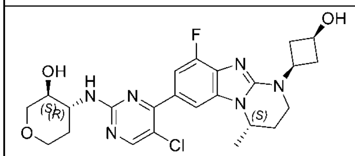 TA-49 | 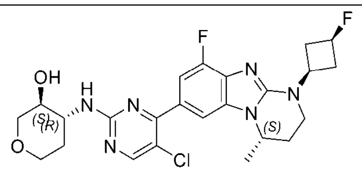 TA-50 | 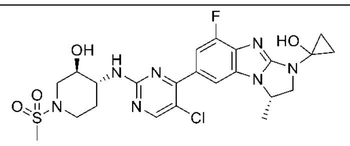 TA-51 |
| 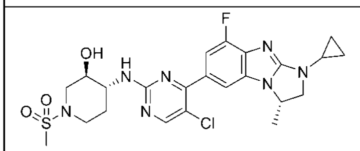 TA-52 | 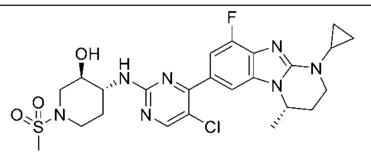 TA-53 | 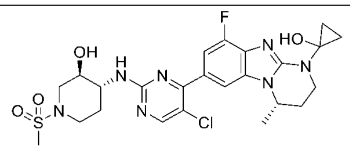 TA-54 |

| | | |
|---|---|---|
| 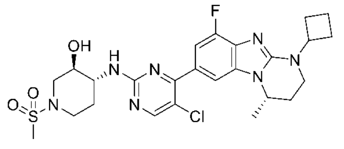 TA-55 | 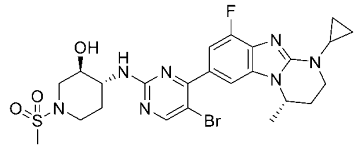 TA-56 | 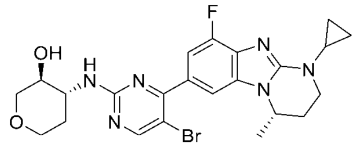 TA-57 |
| 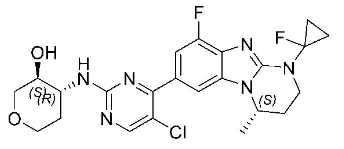 TC-1 | 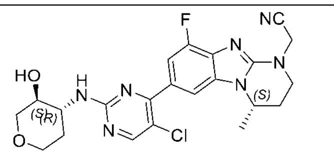 TC-2 | |
| 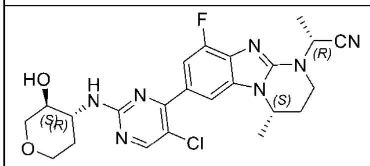 TC-3 | 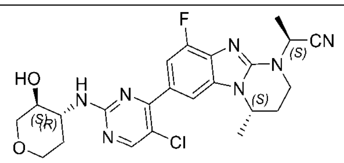 TC-4 | 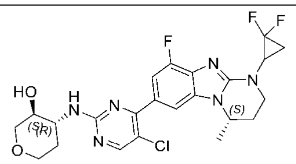 TC-5 |
| 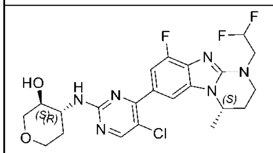 TC-6 | 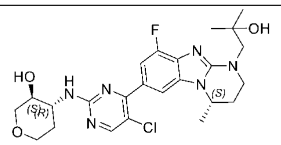 TC-7 | 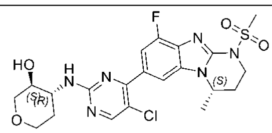 TC-8 |
| 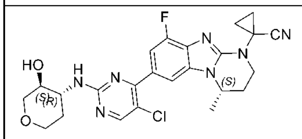 TC-9 | 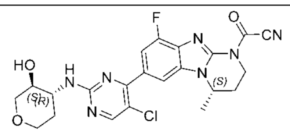 TC-10 | 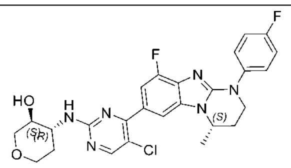 TC-11 |
| 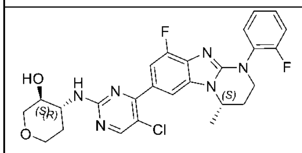 TC-12 | 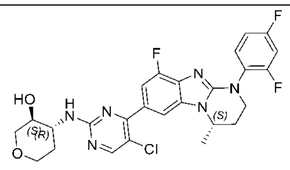 TC-13 | 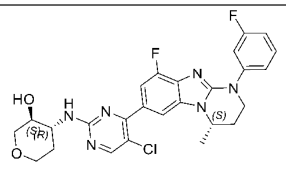 TC-14 |
| 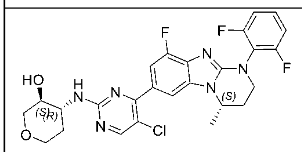 TC-15 | 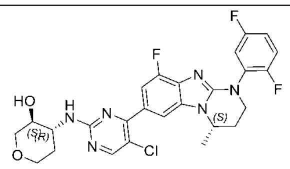 TC-16 | 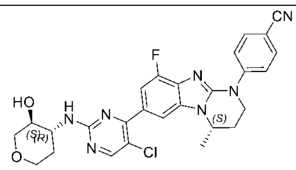 TC-17 |

| | | |
|---|---|---|
| 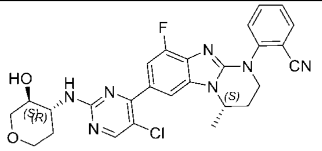 TC-18 | 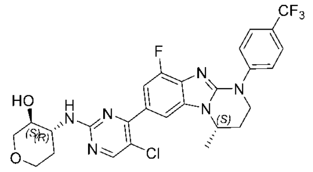 TC-19 | 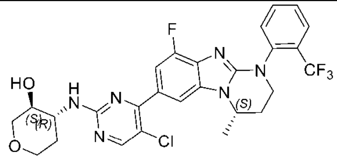 TC-20 |
| 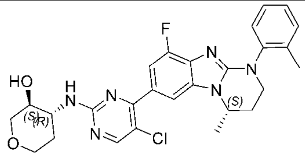 TC-21 | 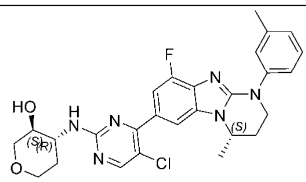 TC-22 | 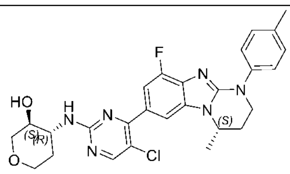 TC-23 |
| 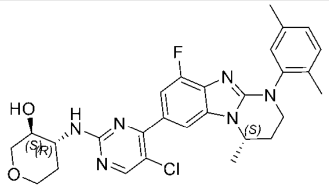 TC-24 | 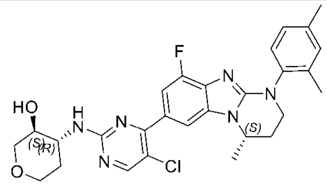 TC-25 | 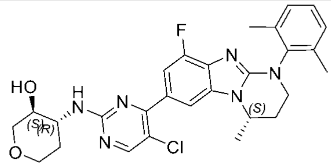 TC-26 |
| 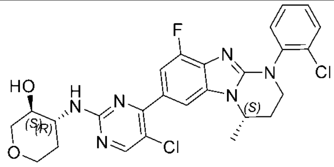 TC-27 | 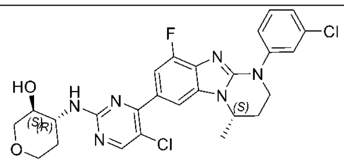 TC-28 | 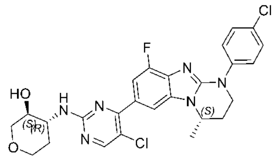 TC-29 |
| 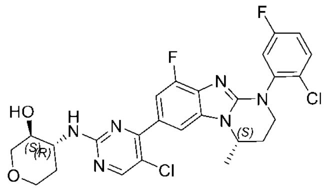 TC-30 | 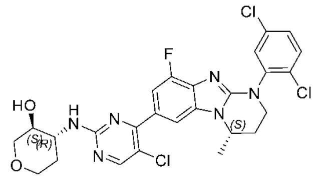 TC-31 | 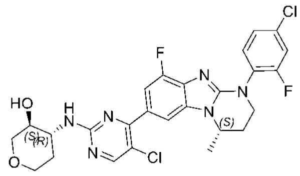 TC-32 |
| 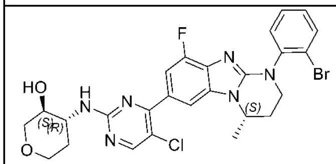 TC-33 | 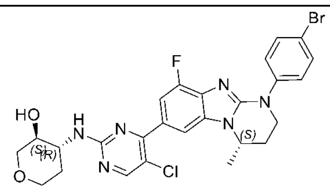 TC-34 | 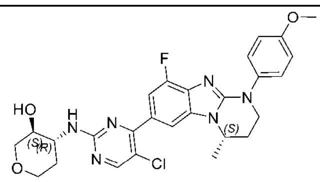 TC-35 |
| 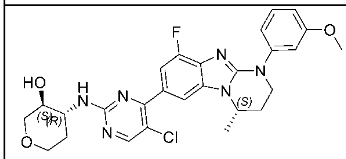 TC-36 | 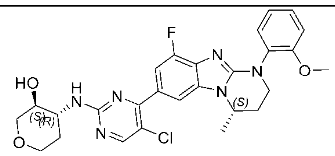 TC-37 | 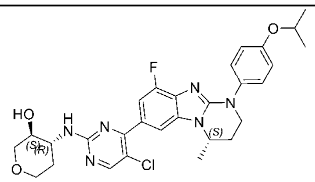 TC-38 |

| | | |
|---|---|---|
| 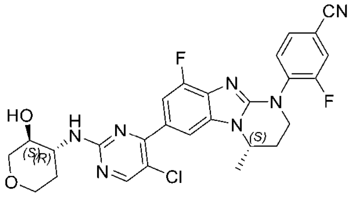 TC-39 | 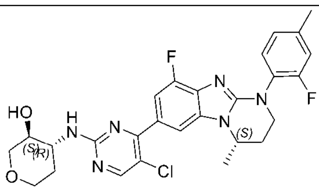 TC-40 | 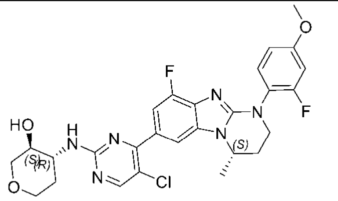 TC-41 |
| 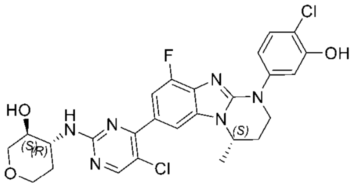 TC-42 | 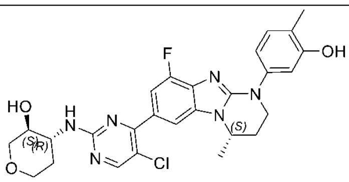 TC-43 | 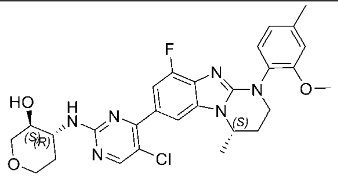 TC-44 |
| 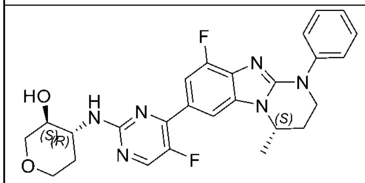 TC-45 | 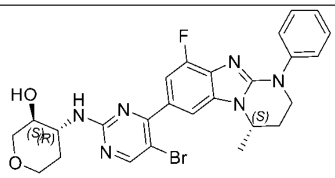 TC-46 | 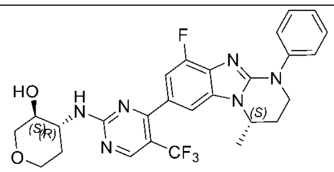 TC-47 |
| 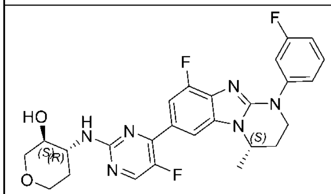 TC-48 | 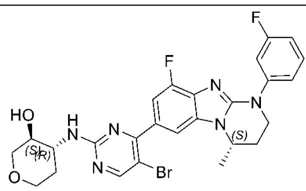 TC-49 | 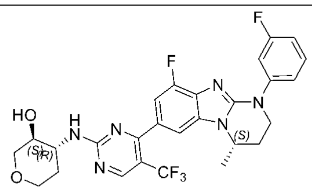 TC-50 |
| 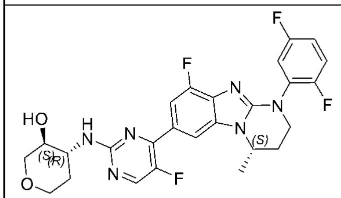 TC-51 | 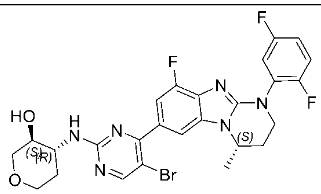 TC-52 | 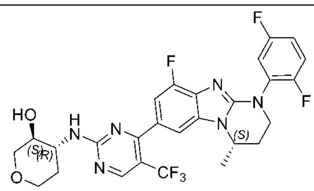 TC-53 |
| 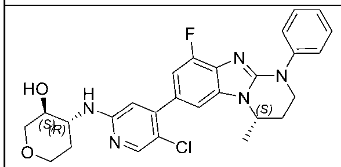 TC-54 | 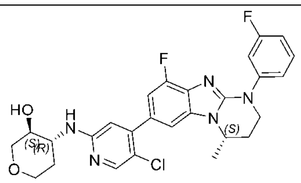 TC-55 | 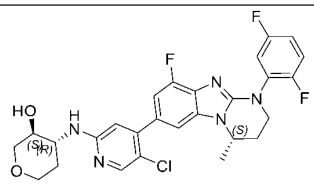 TC-56 |

In another preferred embodiment, the pharmaceutically acceptable salt is an inorganic acid salt or an organic acid salt.

In another preferred embodiment, the inorganic acid salt is selected from the group consisting of hydrochloride, hydrobromide, hydroiodate, sulphate, bisulfate, nitrate, phosphate, and acid phosphate.

In another preferred embodiment, the organic acid salt is selected from the group consisting of formate, acetate, trifluoroacetate, propionate, pyruvate, hydroxyacetate, oxalate, malonate, fumarate, maleate, lactate, malate, citrate, tartrate, methanesulfonate, ethanesulfonate, benzenesulfonate, p-toluenesulfonate, salicylate, picrate, glutamate, ascorbate, camphorate, and camphorsulfonates.

In the fourth aspect, the present invention provides a pharmaceutical composition comprising a safe and effective amount of the compound according to the first, second, and third aspect of the present invention, and a pharmaceutically acceptable carrier.

In the fifth aspect, the present invention provides use of the compound according to the first, second, and third

aspect of the present invention for the manufacture of a medicament for use as a CDK4 kinase inhibitor.

In the sixth aspect, the present invention provides use of the compound according to the first, second, and third aspect of the present invention for the manufacture of a medicament for modulating CDK4 kinase activity or treating a CDK4-related disease.

In another preferred embodiment, the CDK4-related disease is a disease with high CDK4 expression. In another preferred embodiment, the CDK4-related disease is selected from the group consisting of inflammation, cancer, cardiovascular disease, infection, immune disease, and metabolic disease.

In another preferred embodiment, the cancer is selected from the group consisting of lung cancer, breast cancer, prostate cancer, colorectal cancer, liver cancer, pancreatic cancer, ovarian cancer, leukemia, neuroblastoma, gastric cancer, renal cancer, esophageal cancer, uterine cancer, and liposarcoma.

In another preferred embodiment, the inflammation is selected from the group consisting of dermatitis, keratitis, conjunctivitis, prostatitis, hepatitis, and enteritis.

In another preferred embodiment, the infection is selected from the group consisting of bacterial infection and viral infection.

In another preferred embodiment, the bacterial infection is selected from the group consisting of gram-positive bacterial infection, gram-negative bacterial infection, mycoplasma infection, fungal infection.

In another preferred embodiment, the viral infection is selected from the group consisting of coronavirus infection, influenza A, influenza B, and avian influenza.

In another preferred embodiment, the immune disease is selected from the group consisting of systemic lupus erythematosus, rheumatoid arthritis, scleroderma, and ulcerative colitis.

It should be understood that within the scope of the present invention, the above-described technical features of the present invention and the technical features described in detail in the following (e.g., examples) may be combined with each other to constitute a new or preferred technical solution. Due to space limitations, they are not enumerated one by one herein.

## DETAILED DESCRIPTION OF THE INVENTION

After extensive and in-depth research, the present inventors unexpectedly discovered a class of compounds with excellent CDK4 kinase inhibitory activity. In addition, the compounds have excellent inhibitory activity and selectivity against CDK4 kinase and have better pharmacodynamic/pharmacokinetic properties. On above basis, the present invention has been completed.

### Terms

Unless otherwise specified, the following terms used in this application (including the specification and claims) have the definitions given below.

When a substituent is described by a conventional chemical formula written from left to right, the substituent also includes a chemically equivalent substituent obtained when the structural formula is written from right to left. For example, $-CH_2O-$ is equivalent to $-OCH_2-$.

As used herein, the bond represented by

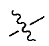

or --- in the group represents the position where the group is connected to other parts of the compound or molecule.

"Alkyl", alone or as part of other groups, refers to a monovalent linear or branched saturated hydrocarbon group containing 1 to 12 carbon atoms composed only of carbon and hydrogen atoms (i.e. $C_{1-12}$ alkyl). The alkyl is preferably $C_{1-6}$ alkyl (i.e. alkyl containing 1, 2, 3, 4, 5 or 6 carbon atoms), more preferably $C_{1-4}$ alkyl (i.e. alkyl containing 1, 2, 3 or 4 carbon atoms). Examples of alkyl include but are not limited to methyl, ethyl, propyl, isopropyl, isobutyl, *sec*-butyl, *tert*-butyl, amyl, *n*-hexyl, octyl and dodecyl, etc. Unless otherwise specified, in the present invention, alkyl is also intended to comprise a substituted alkyl, i.e. alkyl bearing one or more substituents, in particular 1-4 substituents, at any position. Unless otherwise specified, in the present application, "substituted alkyl" includes a haloalkyl. As used herein, "haloalkyl" refers to an alkyl as defined herein in which one or more hydrogen atoms are replaced by the same or different halogens. Examples of haloalkyl include $-CH_2Cl$, $-CH_2CF_3$, $-CH_2CCl_3$, perfluoroalkyl (e.g.$-CF_3$), etc..

"Alkylene" refers to divalent group of an alkyl as defined herein, e.g. $-CH_2-$, $-CH_2CH_2-$ and $-CH_2CH_2CH_2-$.

"Alkoxy", alone or as part of other groups, refers to an alkyl to which an oxy is attached, which has a structure of alkyl O-, wherein the alkyl has the definition described above, and preferably the alkoxy is $C_{1-6}$ alkoxy (i.e. $-OC_{1-6}$ alkyl).

Alkoxy includes but is not limited to methoxy, ethoxy, propoxy, *tert*-butoxy, etc. "Haloalkoxy" refers to the formula -OR, wherein, R is a haloalkyl as defined herein. Examples of haloalkoxy include but are not limited to trifluoromethoxy, difluoromethoxy, and 2,2,2-trifluoroethoxy, etc. "Thioalkyl" refers to an alkyl in which the carbon in the alkyl is substituted by S, S(O) or $S(O)_2$.

"Alkenyl", alone or as part of other groups, refers to an aliphatic group containing at least one carbon-carbon double bond, typically having between 2 and 20 carbon atoms (i.e. $C_{2-20}$ alkenyl). The alkenyl is preferably $C_{2-6}$ alkenyl (i.e. alkenyl containing 2, 3, 4, 5 or 6 carbon atoms). The alkenyl includes but is not limited to for example, vinyl, propenyl, butenyl, 1-methyl-2-buten-1-yl, etc. Unless otherwise defined, in the present invention, the alkenyl also includes substituted alkenyl.

"Alkenylene" refers to an alkenyl as defined above having two connection points. For example, "vinylidene" refers to the group -CH=CH-. The alkenylene is preferably $C_{2-6}$ alkenylene (i.e. alkenylene containing 2, 3, 4, 5 or 6 carbon atoms). Unless otherwise defined, the alkenylene may also be in an unsubstituted form or in a substituted form having one or more substituents.

"Alkynyl", alone or as part of other groups, refers to a straight or branched hydrocarbon chain containing two or more carbon atoms and characterized by one or more triple bonds, typically having 2 to 20 carbon atoms (i.e. $C_{2-20}$ alkynyl). The alkynyl is preferably $C_{2-6}$ alkynyl (i.e. alkynyl having 2, 3, 4, 5 or 6 carbon atoms). Alkynyl includes but is not limited to ethynyl, propargyl and 3-hexynyl. One of the triple-bond carbons can optionally be the connection point of alkynyl substituent. In the present invention, unless otherwise defined, the alkynyl also includes substituted alkynyl.

"Alkynylene" refers to an alkynyl as defined above having two connecting points. For example, "ethynylene" refers to the group: -C≡C-. The alkynylene is preferably $C_{2-6}$ alkynylene (i.e. alkynylene containing 2, 3, 4, 5 or 6 carbon atoms). Unless otherwise defined, the alkynylene may also be in an unsubstituted form or in a substituted form having one or more substituents.

"Aliphatic group" refers to straight-chain, branched-chain or cyclic hydrocarbon group including saturated and unsaturated group, such as alkyl, alkenyl and alkynyl.

"Aromatic ring system" or "aromatic ring" refers to monocyclic, bicyclic or polycyclic hydrocarbon ring system in which at least one ring is aromatic. Preferably, the "aromatic ring system" or "aromatic ring" has 6-12 ring atoms, i.e. $C_{6-12}$ aromatic ring, examples of which include benzene ring, naphthalene ring, anthracene ring, etc.

"Aryl", alone or as part of other groups, refers to a monovalent group of an aromatic ring system (aromatic ring). Representative aryl includes all aromatic ring systems, such as phenyl, naphthyl and anthryl; and ring systems in which aromatic carbocyclic rings are fused with one or more non-aromatic carbocyclic rings, such as indanyl, phthalimidyl, naphthylimidyl or tetrahydronaphthyl, etc. In the present invention, the aryl is preferably $C_{6-12}$ aryl. In the present invention, unless otherwise defined, aryl also includes substituted aryl.

"Arylalkyl" or "aralkyl" refers to an alkyl moiety in which the hydrogen atom in alkyl is substituted by an aryl. An aralkyl includes a group which one or more hydrogen atoms in alkyl are substituted by aryl, wherein aryl and alkyl are defined as above. Examples of "arylalkyl" or "aralkyl" include benzyl, 2-phenylethyl, 3-phenylpropyl, 9-fluorenyl, diphenylmethyl and triphenylmethyl, etc.

"Aryloxy" refers to -O-(aryl), wherein the aryl moiety is as defined herein.

"Heteroalkyl" refers to an alkyl in which carbon atoms are replaced, it has one or more skeleton chain atoms selected from atoms other than carbon, such as oxygen, nitrogen, sulfur, phosphorus, or a combination thereof. Numeric ranges may be given for example $C_{1-6}$ heteroalkyl refers to the number of carbons in the chain which includes 1 to 6 carbon atoms. For example, - $CH_2OCH_2CH_3$ is called "C3" heteroalkyl. The connection with the rest of the molecule can be through heteroatom or carbon in heteroalkyl chain. "Heteroalkylene" refers to a divalent alkyl in which carbon atoms are replaced, it has one or more skeleton chain atoms selected from atoms other than carbon, such as oxygen, nitrogen, sulfur, phosphorus, or a combination thereof. Unless otherwise defined, "heteroalkyl" and "heteroalkylene" include both substituted and unsubstituted forms.

"Carbocyclic ring system" or "carbocyclic ring" refers to a monocyclic, dicyclic or polycyclic hydrocarbon ring system in which each ring is fully saturated or contains one or more unsaturated units, but none of the rings are aromatic. Preferably, "carbocyclic ring system" or "carbocyclic ring" has 6-12 ring atoms, i.e. $C_{6-12}$ carbocyclic ring. "Carbocyclyl" refers to a monovalent group of carbocyclic ring system or carbocyclic ring as defined above. Preferably, carbocyclic group has 6-12 ring atoms, i.e. $C_{6-12}$ carbocyclyl. Examples of carbocyclyl include cycloalkyl (e.g. cyclopentyl, cyclobutyl, cyclopropyl, cyclohexyl, etc.) and cycloalkenyl (e.g. cyclopentenyl, cyclohexenyl, cyclopentadienyl, etc.) and the like.

"Cycloalkyl" refers to a monovalent saturated carbocyclic group consisting of monocyclic or dicyclic ring, it has 3-12 (i.e. $C_{3-12}$ cycloalkyl), preferably 3-10 (i.e. $C_{3-10}$ cycloalkyl), more preferably 3-8 ring atoms (i.e. $C_{3-8}$ cycloalkyl), most preferably 3, 4, 5 or 6 ring atoms (i.e. $C_{3-6}$ cycloalkyl). Unless otherwise defined, the cycloalkyl may optionally be substituted with one or more substituents. Preferably, the substituent of the cycloalkyl may be independently hydroxyl, alkyl, alkoxy, halogen, haloalkyl, amino, monoalkylamino or dialkylamino. Examples of cycloalkyl include but are not limited to cyclopropyl, cyclobutyl, cyclopentyl, cyclohexyl and cycloheptyl, etc.

"Cycloalkoxy" refers to the formula -OR, wherein R is a cycloalkyl as defined herein. Exemplary cycloalkyloxy

include cyclopropyloxy, cyclobutyloxy, cyclopentyloxy and cyclohexyloxy, etc. "Cycloalkyl alkyl" or "cycloalkyl alkylene" means - alkylene (cycloalkyl) in which cycloalkyl and alkylene are as defined above. "Cycloalkyl alkyl" or "cycloalkyl alkylene" is bonded to the parent molecular structure via an alkyl (alkylene).

"Heteroaromatic ring system" or "heteroaromatic ring" refers to a monocyclic (e.g. 5 or 6 membered), dicyclic (6-12 membered), or polycyclic system in which at least one ring is aromatic ring containing at least one heteroatom (e.g. N, O, or S) as ring atom and the remaining ring atoms are all carbon. In some cases, aromatic ring that contains at least one heteroatom contain 1, 2, 3 or 4 ring heteroatoms in the ring. Except for aromatic ring containing at least one heteroatom as ring atom, the remaining rings in the "heteroaromatic ring system" or "heteroaromatic ring" may be saturated, partially unsaturated or completely unsaturated.

"Heteroaryl", alone or as part of other groups, refers to a monovalent group of the "heteroaromatic ring system" or "heteroaromatic ring" as defined above. The connection point of the heteroaryl should be located on the aromatic ring. Examples of heteroaryl include but are not limited to imidazolyl, aoxazolyl, isoxazolyl, thiazolyl, isothiazolyl, oxadiazolyl, thiadiazolyl, pyrazinyl, thienyl, furanyl, pyranyl, pyridyl, pyrrolyl, pyrazolyl, pyrimidinyl, quinolinyl, isoquinolinyl, benzofuranyl, benzofuranyl, benzothienyl, benzothiopyranyl, benzimidazolyl, benzoxazolyl, benzoxadiazolyl, benzothiazolyl, benzothiadiazolyl, benzopyranyl, indolyl, isoindolyl, triazolyl, triazinyl, quinoxalinyl, purinyl, quinazolinyl, quinazinyl, naphthyridinyl, pterridinyl, carbazolyl, azepinyl, diazepinyl and acridinyl, etc.. Heteroarylene refers to heteroaryl as defined above having two connecting points. Unless otherwise defined, heteroaryl includes substituted or unsubstituted forms.

"Heterocyclic ring system" or "heterocyclic ring" refers to a monocyclic, bicyclic and polycyclic system where at least one ring is saturated or partially unsaturated (but not aromatic) and the ring contains at least one heteroatom as ring atom. The heterocyclic ring system or heterocyclic ring can be attached to the side group of any heteroatom or carbon atom to produce a stable structure and any ring atom can be optionally substituted.

"Heterocyclyl" refers to a monovalent group of the heterocyclic ring system or heterocyclic ring as defined above. It usually refers to stable monocyclic (such as 3-8 membered, i.e. 3 membered, 4 membered, 5 membered, 6 membered, 7 membered or 8 membered) or bicyclic (such as 5-12 membered, i.e. 5 membered, 6 membered, 7 membered, 8 membered, 9 membered, 10 membered, 11 membered or 12 membered) or polycyclic (such as 7-14 membered, i.e. 7 membered, 8 membered, 9 membered, 10 membered, 11 membered, 12 membered, 13 membered or 14 membered) ring, including fused ring, spiro ring and/or bridge ring structure, which is saturated and partially unsaturated and contains carbon atoms and 1, 2, 3 or 4 heteroatoms independently selected from N, O and S as ring atom. The heterocyclyl preferably 3 to 14 membered heterocyclyl, more preferably 3 to 8 membered heterocyclyl, most preferably 4 to 6 membered heterocyclyl. Representative heterocyclyl include the following ring systems, where (1) each ring is non-aromatic and at least one ring contains heteroatom, for example, tetrahydrofuryl, tetrahydropyranyl, tetrahydrothienyl, pyrrolidinyl, pyrrolidonyl, piperidinyl, pyrrolinyl, decahydroquinolinyl, oxazolidinyl, piperazinyl, dioxanyl, dioxolanyl, diazepinyl, oxazepinyl, thiazepinyl, morpholinyl and quinuclidinyl; (2) at least one ring is non-aromatic and contains heteroatom and at least one other ring is an aromatic carbocyclic ring, for example, 1,2,3,4-tetrahydroquinolinyl, 1,2,3,4-tetrahydroisoquinolinyl; and (3) at least one ring is non-aromatic and contains heteroatom, and at least one other ring is aromatic and contains heteroatom, for example, 3,4-dihydro-1H-pyrano[4,3-c] pyridine and 1,2,3,4-tetrahydro-2,6-naphthyridine. Heterocyclylene refers to heterocyclyl as defined above having two connecting points. In the present invention, the heterocyclylene is preferably bicyclic, wherein one ring is a heteroaryl and connected to the other parts of the general formula through the heteroaryl. In the present invention, the heterocyclylene is preferably a 5-6-membered monocyclic heterocyclylene or an 8-10-membered bicyclic heterocyclylene. Unless otherwise defined, "heterocyclyl" and " heterocyclylene" include substituted or unsubstituted forms. When the heterocyclyl is saturated, it can also be referred to heterocycloalkyl.

"Heterocyclyl alkyl" refers to an alkyl substituted with a heterocyclyl, wherein the heterocyclyl and alkyl are defined as above.

"Alkylamino" refers to a group having a structure of alkyl-NR-, where R is H, or the alkyl, cycloalkyl, aryl, heteroaryl, etc. as defined above.

"Cycloalkylamino" refers to a group of formula -NRaRb, wherein Ra is H, the alkyl as defined herein or the cycloalkyl as defined herein, and Rb is the cycloalkyl as defined herein; or Ra and Rb together with the N atom to which they are attached form a 3-10-membered N-containing monocyclic or bicyclic heterocyclyl, such as tetrahydropyrrolyl. As used herein, $C_3$-$C_8$ cycloalkylamino refers to an amino containing 3-8 carbon atoms.

As used herein, the "ester group" refers to a structure of -C(O)-O-R or R-C(O)-O-, wherein R independently represents hydrogen, alkyl, cycloalkyl, aryl, heteroaryl, heterocyclyl as defined above.

As used herein, the term "amido" refers to a group with a structure of -CONRR', wherein R and R' can independently represent hydrogen, alkyl or substituted alkyl, cycloalkyl or substituted cycloalkyl, aryl or substituted aryl, heterocycle or substituted heterocycle as defined above. R and R' may be the same or different in the dialkylamino segments.

As used herein, the term "sulfonamido" refers to a group with a structure of $-SO_2NRR'$, wherein R and R' can

independently represent hydrogen, alkyl or substituted alkyl, cycloalkyl or substituted cycloalkyl, aryl or substituted aryl, heterocycle or substituted heterocycle as defined above. R and R' may be the same or different in the dialkylamino segments.

"Ketocarbonyl" refers to R-C(=O)-, wherein R is alkyl, cycloalkyl, etc. as defined above.

When the substituent is a non-terminal substituent, it is a diradical of the corresponding substituent, such as alkyl corresponding to alkylene, cycloalkyl corresponding to cycloalkylene, heterocyclyl corresponding to heterocyclylene, alkoxy corresponding to alkyleneoxy, etc..

In the present invention, each of the above-mentioned groups of alkyl, alkoxy, cycloalkyl, heteroalkyl, aryl, heteroaryl, cycloheteroalkyl, alkenyl, alkynyl, heterocycle, heterocyclyl, etc. may be substituted or unsubstituted.

In the present invention, the term "substituted" refers to the substitution of one or more hydrogen atoms on a specific group by specific substituents. The specific substituents are the substituents described correspondingly in the preceding text, or the substituents appearing in the examples. Unless otherwise specified, a substituted group may have a substituent selected from a specific group at any substitutable position of the group, and the substituent may be the same or different at each position. Those skilled in the art should understand that the combinations of substituents contemplated by the present invention are those that are stable or chemically achievable. Typical substituents include but are not limited to one or more of the following groups: such as hydrogen, deuterium, halogen (such as monohalogenated substituent or polyhalogenated substituent, and the latter such as trifluoromethyl or alkyl containing $CI_3$), cyano, nitro, oxo (such as =O), trifluoromethyl, trifluoromethoxy, cycloalkyl, alkenyl, alkynyl, heterocycle, aromatic ring, $OR_a$, $SR_a$, $S(=O)R_e$, $S(=O)_2R_e$, $P(=O)_2R_e$, $S(=O)_2OR_e$, $P(=O)_2OR_e$, $NR_bR_c$, $NR_bS(=O)_2R_e$, $NR_bP(=O)_2R_e$, $S(=O)_2NR_bR_c$, $P(=O)_2NR_bR_c$, $C(=O)OR_d$, $C(=O)R_a$, $C(=O)NR_bR_c$, $OC(=O)R_a$, $OC(=O)NR_bR_c$, $NR_bC(=O)OR_e$, $NR_dC(=O)NR_bR_c$, $NR_dS(=O)_2NR_bR_c$, $NR_dP(=O)_2NR_bR_c$, $NR_bC(=O)R_a$ or $NR_bP(=O)_2R_e$, wherein $R_a$ can independently represent hydrogen, deuterium, alkyl, cycloalkyl, alkenyl, alkynyl, heterocycle or aromatic ring, $R_b$, $R_c$ and $R_d$ can independently represent hydrogen, deuterium, alkyl, cycloalkyl, heterocycle or aromatic ring, or $R_b$ and $R_c$ together with the N atom form a heterocycle, $R_e$ can independently represent hydrogen, alkyl, cycloalkyl, alkenyl, alkynyl, heterocycle or aromatic ring. The above typical substituents, such as alkyl, cycloalkyl, alkenyl, cycloalkenyl, alkynyl, heterocycle or aromatic ring, may be optionally substituted. The substituent is such as (but is not limited to): halogen, hydroxyl, cyano, carboxy (-COOH), $C_1$-$C_6$ alkyl, $C_2$-$C_6$ alkenyl, $C_2$-$C_6$ alkynyl, $C_3$-$C_8$ cycloalkyl, 3-12 membered heterocyclyl, aryl, heteroaryl, $C_1$-$C_8$ aldehydyl, $C_2$-$C_{10}$ acyl, $C_2$-$C_{10}$ ester group, amino, $C_1$-$C_6$ alkoxy, $C_1$-$C_{10}$ sulfonyl, and $C_1$-$C_6$ uramido, etc..

"Cyano" refers to -CN.

"Nitro" refers to $-NO_2$.

"Hydroxyl" refers to -OH.

"Amino" refers to $-NH_2$ or RNH-, wherein R is ketocarbonyl, sulfonyl, sulfonamido, $R_a$-C(=O)-, $R_aR_b$N-C (=O)- etc., wherein $R_a$ and $R_b$ are alkyl, cycloalkyl, aryl or heteroaryl, etc..

"Halogen (halogenated)" refers to any halogen group, e.g. -F, -Cl, -Br or -I.

"Deuterated substance" refers to the compound obtained by replacing one hydrogen atom (H) or multiple hydrogen atoms (H) with deuterium atoms (D) in a compound.

In the present invention, the term "more" independently refers to 2, 3, 4, or 5.

The structural formula of the carbamate group is -NH-C(=O)-O-R, wherein R is alkyl, aryl, heteroaryl, etc..

It should be understood that when a certain group exists at multiple different positions in a compound, its definition at each position is independent and can be the same or different. That is to say, the term "selected from the group consisting of" has the same meaning as the term "each independently selected from the group consisting of".

## Active Ingredient

As used herein, the terms "compound of the present invention" or "active ingredient of the present invention" are used interchangeably to refer to a compound of formula I or formula II, or a pharmaceutically acceptable salt, a hydrate, a solvate, an isotope compound (such as a deuterated compound) or a prodrug thereof. The term also includes a racemate and an optical isomer.

The compound of formula I has the following structure:

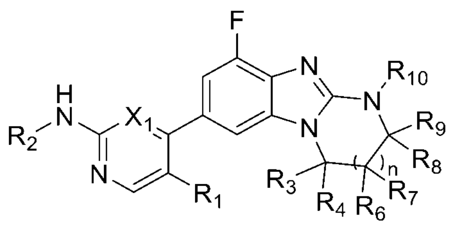

formula I

wherein, the definitions of each group are as described above.

The compound of formula II has the following structure:

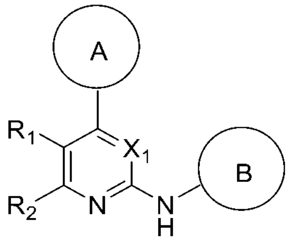

formula II

wherein, the definitions of each group are as described above.

The salts that may be formed by the compound in the present invention are also within the scope of the present invention. Unless otherwise stated, the compound in the present invention is understood to include its salt. The term "salt" as used herein refers to an acidic or basic salt formed with inorganic or organic acid and base. Further, when the compound in the present invention contains a base fragment, including but not limited to pyridine or imidazole, when it contains an acid segment including but not limited to carboxylic acid, the zwitter-ion ("inner salt") may be formed is included within the range of the term "salt". Pharmaceutically acceptable (i.e., non-toxic, physiologically acceptable) salt is preferred, although other salts are also useful and may be used, for example, in the separation or purification steps of the preparation process. The compound of the present invention may form a salt, for example, by reacting compound I with a certain amount (such as an equivalent amount) of an acid or base, followed by salting out in a medium, or lyophilization in aqueous solution.

The base fragment contained in the compounds of the present invention includes but is not limited to amines or pyridine or imidazole rings, and may form salt with organic or inorganic acid. Typical acids that form salts include acetate (such as acetic acid or trihalogenated acetic acid, such as trifluoroacetic acid), adipate, alginate, ascorbate, aspartate, benzoate, benzene sulfonate, disulfate, borate, butyrate, citrate, camphorate, camphor sulfonate, cyclopentane propionate, diethylene glycolate, lauryl sulfate, ethane sulphonate, fumarate, gluceptate, glycerophosphate, hemisulphate, enanthate, caproate, hydrochloride, hydrobromide, hydriodate, isethionate(e.g., 2-hydroxyl-ethesulfonate), lactate, maleate, mesylate, naphthalenesulfonate (e.g., 2-naphthalenesulfonate), nicotinate, nitrate, oxalate, pectate, persulfate, phenylpropionate (e.g., 3-phenylpropionate), phosphate, picrate, pivalate, propionate, salicylate, succinate, sulfate (e.g., formed with sulfuric acid), sulfonate, tartrate, thiocyanate, toluenesulfonate (e.g., tosilate), dodecanoate, etc..

Some compounds of the invention may contain acidic fragments including, but not limited to carboxylic acid which may form salts with various organic or inorganic bases. Salt formed by typical base includes ammonium salt, alkali metal salt (such as sodium, lithium and potassium salts), alkaline earth metal salt (such as calcium and magnesium salts), and salt formed by organic bases (such as organic amines), such as benzathine, dicyclohexylamine, hydrabamine (salt formed with N,N-bis(dehydroabietyl) ethylenediamine), N-methyl-D-glucosamine, *N*-methyl-D-glucoamide, tert-butyllamine, and the salt formed with amino acids such as arginine, lysine, etc.. Basic nitrogen-containing groups can form quaternary ammonium salts with halides, such as small molecular alkyl halides (such as chlorides, bromides and iodides of methyl, ethyl, propyl and butyl), dialkyl sulfate (such as dimethyl, diethyl, dibutyl, and dipentyl sulfates), long chain halides (such as chlorides, bromides and iodides of decyl, dodecyl, tetradecyl, and tetradecyl), aralkyl halides (such as bromides of benzyl and phenyl), etc..

The prodrug and solvate of the compound in the present invention are also included within the scope of the present invention.

The term "prodrug" herein refers to a compound that, upon administration for the treatment of a related disease, undergoes a chemical transformation via metabolic or chemical processes to yield a compound, salt, or solvate of the present invention. The compounds of the invention include solvates such as hydrates.

The compound, salt or solvate in the present invention may be present in tautomeric forms such as amide and imino ether. All of these tautomers are part of the present invention.

All stereoisomers of the compounds (e.g., those asymmetric carbon atoms that may be present due to various substitutions), including their enantiomeric forms and diastereomeric forms, all belong to the envisaged scope of the present invention. The independent stereoisomer in the present invention may not coexist with other isomers (e.g., as a pure or substantially pure optical isomer with special activity), or may be a mixture (e.g., racemate), or a mixture formed with all other stereoisomers or a part thereof. The chiral center of the present invention has two configurations of S or R, which is defined by International Union of Pure and Applied Chemistry (IUPAC) in 1974. The racemization form can be solved by physical methods, such as fractional crystallization, or separation crystallization by derivation into diastereomers, or separation by chiral column chromatography. Individual optical isomer can be obtained from racemate by appropriate methods, including but not limited to conventional methods, such as recrystallization after salting with optically active acids.

Weight content of compound in the present invention obtained by preparation, separation and purification in turn is equal to or greater than 90%, such as equal to or greater than 95%, equal to or greater than 99% ("very pure" compound), which is listed in the description of the text. In addition, the "very pure" compound of the present invention is also part of the present invention.

All configuration isomers of the compound of the present invention are within the scope, whether in mixture, pure or very pure form. The definition of the compound of the present invention comprises cis (*Z*) and trans (*E*) olefin isomers, and cis and trans isomers of carbocycle and heterocycle.

In the entire specification, the groups and substituents can be selected to provide stable fragments and compounds.

Specific functional groups and chemical term definitions are described in detail below. For the purposes of the present invention, the chemical elements are consistent with those in Periodic Table of the Elements, CAS version, Handbook of Chemistry and Physics, 75th Ed. The definition of a particular functional group is also described therein. In addition, the basic principles of organic chemistry as well as specific functional groups and reactivity are described in "Organic Chemistry", Thomas Sorrell, University Science Books, Sausalito: 1999, the entire content of which is incorporated herein by reference.

Some compounds of the present invention may exist in specific geometric or stereoisomeric forms. The present invention covers all compounds, including their cis and trans isomers, R and S enantiomers, diastereomers, (D) type isomers, (L) type isomers, racemic mixtures and other mixtures. In addition, asymmetric carbon atom can represent substituent, such as alkyl. All isomers and mixtures thereof are included in the present invention.

According to the invention, mixtures of isomers may contain a variety of ratios of isomers. For example, mixtures with only two isomers may have the following combinations: 50:50, 60:40, 70:30, 80:20, 90:10, 95:5, 96:4, 97:3, 98:2, 99:1, or 100:0, all ratios of the isomers are within the scope of the present invention. Similar ratios readily understood by those of ordinary skill in the art and ratios for mixtures of more complex isomers are also within the scope of the present invention.

The invention also includes isotope labeled compounds, which are equivalent to the original compounds disclosed herein. However, in practice, it usually occurs when one or more atoms are replaced by atoms with a different atomic weight or mass number. Examples of isotopes that can be incorporated into the compounds of the present invention include hydrogen, carbon, nitrogen, oxygen, phosphorus, sulfur, fluorine and chlorine isotopes, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$, respectively. Compounds of the present application, or enantiomers, diastereomers, isomers, or pharmaceutically acceptable salts or solvates thereof which contain the aforementioned isotopes or other isotope atoms are all within the scope of the invention. Some isotope-labeled compounds in the present invention, such as the radioactive isotopes of $^{3}H$ and $^{14}C$, are also included and are useful in experiments on the tissue distribution of drugs and substrates. Tritium ($^{3}H$) and Carbon-14 ($^{14}C$), which are relatively easy to prepare and detect, are the preferred choice. In addition, heavier isotope substitutions such as deuterium, i.e., $^{2}H$, have advantages in certain therapies due to their good metabolic stability, such as increased half-life or reduced dosage in vivo, and thus may be preferred in certain situations. Isotope-labeled compounds can be prepared by conventional methods through replacing non-isotopic reagents with readily available isotope-labeled reagents using the disclosed scheme shown in the Example.

If the synthesis of a specific enantiomer of the compound of the invention is to be designed, it can be prepared by asymmetric synthesis, or derivatized with chiral auxiliary reagent, separating the resulting diastereomeric mixture and removing the chiral auxiliary reagent to obtain a pure enantiomer. In addition, if a molecule contains a basic functional group, such as an amino acid, or an acidic functional group, such as a carboxyl group, a diastereomer salt can be formed with a suitable optically active acids or bases, followed by separation by conventional means, such as crystallization or chromatography, to obtain a pure enantiomer.

As described herein, the compound in the present invention may be substituted with any number of substituents or functional groups to extend its scope. In general, whether the term "substituted" appears before or after the term "optionally", the general formula that includes substituents in the present invention means the substitution of a specified structural substituent for a hydrogen radical. When multiple locations in a particular structure are replaced by multiple specific substituents, each location of the substituents can be the same or different. The term "substituted" as used herein includes all substitution that allows organic compounds to be substituted. Broadly speaking, the allowable substituents include non-cyclic, cyclic, branched, non-branched, carbocyclic and heterocyclic, aromatic ring and non-aromatic organic compounds. In the present invention, such as heteroatomic nitrogen, its valence state may be supplemented by a hydrogen substituent or by any permitted organic compound described above. Furthermore, the invention does not unintentionally limit the substituted organic compounds in any way. The present invention considers that a combination of substituents and variable groups is good for the treatment of diseases in the form of stable compounds. The term "stable" herein refers to a stable compound which is sufficient for maintaining the integrity of the compound structure within a sufficiently long time, preferably being effective in a sufficiently long time, which is hereby used for the above purposes.

The metabolites of the compounds of the present invention and pharmaceutically acceptable salts thereof, and prodrugs that can be converted into the compounds of the present invention and pharmaceutically acceptable salts thereof in vivo, are also included in the claims.

In another preferred embodiment, in the said compounds, each of ring A, ring B, $R_1$, $R_2$ and $X_1$ is the corresponding specific group in the specific compound, respectively.

In another preferred embodiment, in the said compounds, each of $R_1$, $R_2$, $X_1$, $R_3$, $R_4$, $R_6$, $R_7$, $R_8$, $R_9$, $R_{10}$ and n is the corresponding specific group in the specific compound, respectively.

**Preparation Method**

Methods for preparing compounds of formula I or formula II are described in the following schemes and examples. Raw materials and intermediates are purchased from commercial sources, prepared by known steps, or otherwise described. In some cases, the sequence of steps to perform the reaction scheme may be changed to facilitate the reaction or avoid unwanted side reaction products.

The preparation method of the compound of formula I or formula II of the present invention is more specifically described below, but these specific methods do not constitute any limitation of the invention. The compound of the invention may also optionally be conveniently prepared by combining the various synthetic methods described in this specification or known in the art, such a combination may be easily performed by a skilled person in the art to which the invention belongs.

Typically, the preparation process of the compounds of the present invention is as follows, in which unless otherwise specified, the raw materials and reagents used can be purchased through commercial channels, prepared by known steps, or otherwise described.

Generally, in the preparation process, each reaction is usually carried out under the protection of inert gas and in an appropriate solvent at 0 to 150 °C, and the reaction time is usually 2-24 hours.

The preferred preparation method is as follows:

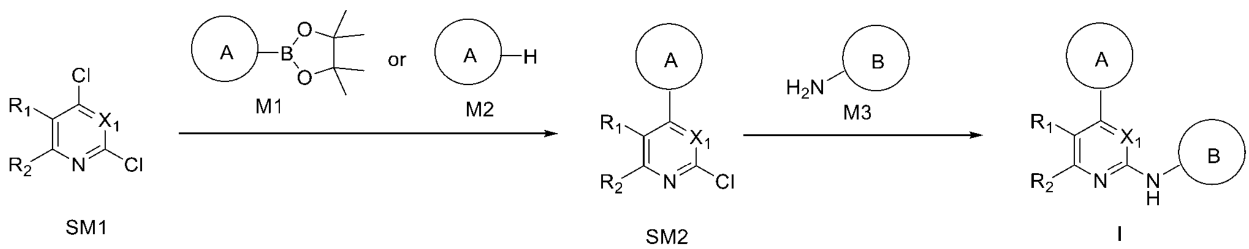


Step 1: In a solvent (such as 1,2-dichloroethane, dioxane, tetrahydrofuran), SM1 and M1 or M2 undergo palladium-catalyzed coupling or nucleophilic substitution reaction at 70-120 °C to generate SM2;

Step 1: In an inert solvent (such as DMF, dioxane, ethylene glycol dimethyl ether, N-methylpyrrolidone, tetrahydrofuran, etc.), under alkaline conditions (such as diisopropylethylamine, potassium acetate, DBU, LiHDMS, etc.) or in the presence of catalyst and ligand (such as $Pd(PPh_3)_4$, $Pd_2(dba)_3$\t-BuXphos, etc.), M3 is reacted with SM2 to generate T (i.e. compound of formula II).

In each of the above formulas, the definitions of ring A, ring B, $R_1$, $R_2$, and $X_1$ are as described above.

The preferred preparation method is as follows:

Method:

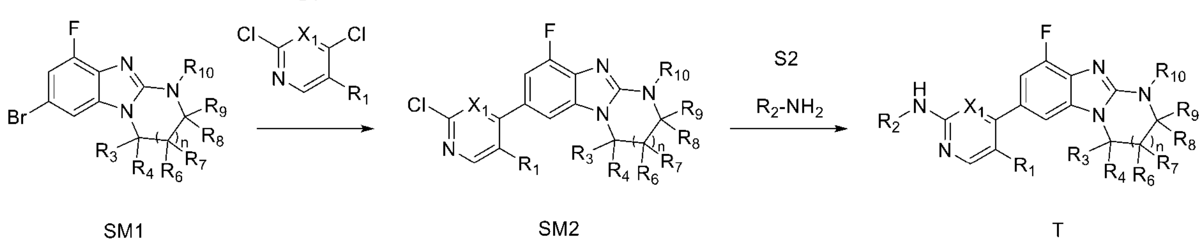


Step 1: In a solvent (1,4-dioxane, tetrahydrofuran, 1,2-dichloroethane), SM1 and S1 undergo palladium-catalyzed coupling or nucleophilic substitution reaction at 70-120 °C to generate SM2;

Step 2: In a solvent (1,4-dioxane, tetrahydrofuran, toluene), SM2 and S2 undergo palladium-catalyzed coupling or nucleophilic substitution reaction under alkaline conditions to obtain product T.

In each of the above formula, the definitions of $R_1$, $R_2$, $R_3$, $R_4$, $R_6$, $R_7$, $R_8$, $R_9$, $R_{10}$, $X_1$ and n are as described above.

Unless otherwise specified, the above starting materials can be purchased from commercial sources or synthesized according to reported literatures.

**Pharmaceutical Compositions and Administration Method**

The pharmaceutical composition of the present invention can be used for preventing and/or treating the following diseases: inflammation, cancer, cardiovascular disease, infection, immune disease, and metabolic disease.

The compound of formula I or formula II can be used in combination with other drugs known to treat or improve similar conditions. When administered in combination, the original administration method and dosage for the drug can remain unchanged, while the compound of formula I or formula II may be administered simultaneously or subsequently. Pharmaceutical composition simultaneously containing one or more known drugs and the compound of formula I or formula II may be preferred when administered in combination with one or more other drugs. The drug combination also includes administering the compound of formula I or formula II and one or more other known drugs at overlapping time. When the compound of formula I or formula II is used in combination with one or more other drugs, the dose of the compound of formula I or formula II or known drug may be lower than their respective dosages when used alone.

The drug or active ingredients that can be used in combination with the compounds of formula I or formula II include but are not limited to PD-1 inhibitor (such as nivolumab, pembrolizumab, JS-001, SHR-120, BGB-A317, IBI-308, GLS-010, GB-226, STW204, HX008, HLX10, BAT 1306, AK105, LZM 009 or the biological analogue thereof, etc.), PD-L1 inhibitor (such as durvalumab, atezolizumab, CS1001, KN035, HLX20, SHR-1316, BGB-A333, JS003, CS1003, KL-A167, F 520 , GR1405, MSB2311 or the biological analogue thereof, etc.), CD20 antibody (such as rituximab, obinutuzumab, ofatumumab, tositumomab, ibritumomab, etc.), CD47 antibody (such as Hu5F9-G4, CC-90002, TTI-621, TTI-622, OSE-172, SRF-231, AIX-148, NI-1701, SHR-1603, IBI188, IMM01), ALK inhibitor (such as Ceritinib, Alectinib, Brigatinib, Lorlatinib, Ocatinib), PI3K inhibitor (such as Idelalisib, Dactolisib, Taselisib, Buparlisib, etc.), BTK inhibitor (such as Ibrutinib, Tirabrutinib, Acalabrutinib, etc.), EGFR inhibitor (such as Afatinib, Gefitinib, Erlotinib , Lapatinib, Dacomitinib, Icotinib, Canertinib, etc.), VEGFR inhibitor (such as Sorafenib, Pazopanib, Regorafenib, Cabozantinib, Sunitinib, Donafenib, etc.), HDAC inhibitor (such as Givinostat, Droxinostat, Entinostat, Dacinostat, Tacedinaline, etc.), CDK inhibitor (such as Palbociclib, Ribociclib, Abemaciclib, Lerociclib, etc.), MEK inhibitor (such as Selumetinib (AZD6244), Trametinib (GSK1120212), PD0325901, U0126, AS-703026, PD184352 (CI-1040), etc.), Akt inhibitor (such as MK-2206, Ipatasertib, Capivasertib, Afuresertib, Uprosertib, etc.), mTOR inhibitor (such as Vistusertib, etc.), SHP2 inhibitor (such as RMC-4630, JAB-3068, TNO 155, etc.), IGF-1R inhibitor (such as Ceritinib, Okatinib, Linsitinib, BMS-754807, GSK1838705A, etc.), ER antagonist or degradant (such as tamoxifen, fulvestrant, etc.), aromatase inhibitor (such as letrozole, etc.), BCL2 or BCL-XL inhibitor (such as ABT-199, ABT-263, etc.), Hedgehog inhibitor (such as vismodegib, cyclopamine, etc.), chemotherapy drug (such as cisplatin, etoposide, topotecan, etc.), PARP inhibitor (such as Olaparib, Veliparib, Rucaparib, etc.), ATR/ATM inhibitor (such as Ceralasertib, Berzosertib, etc.) or combinations thereof.

The dosage forms of the pharmaceutical composition of the present invention include (but are not limited to): injection, tablet, capsule, aerosol, suppository, pellicle, pill, liniment for external use, controlled release or sustained-release or nano formulation.

The pharmaceutical composition of the present invention comprises a safe and effective amount of a compound of the present invention or a pharmacologically acceptable salt thereof, and a pharmacologically acceptable excipient or carrier. In which, "safe and effective amount" is meant that the amount of the compound is sufficient to significantly improve the condition without causing serious side effects. Generally, the pharmaceutical composition contains 1-2000 mg of the compound of the present invention/dose, more preferably, 10-1000 mg of the compound of the present invention/dose. Preferably, the "one dose" is one capsule or tablet.

"Pharmaceutically acceptable carrier" means one or more compatible solid or liquid fillers or gelatinous materials which are suitable for human use and should be of sufficient purity and sufficiently low toxicity. "Compatibility" as used herein means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agent (such as sodium dodecyl sulfate), coloring agents, flavoring agents, stabilizers, antioxidants, preservatives, pyrogen-free water, etc..

The administration mode of the compound or pharmaceutical composition of the present invention is not particularly limited, and representative administration modes include, but are not limited to, oral, intratumoral, rectal, parenteral (intravenous, intramuscular or subcutaneous) and topical administration.

Solid dosage forms for oral administration include capsules, tablets, pills, powders and granules. In these solid dosage forms, the active compound is mixed with at least one conventional inert excipient (or carrier), such as sodium citrate or dicalcium phosphate, or mixed with any of the following components: (a) fillers or compatibilizer, such as starch, lactose, sucrose, glucose, mannitol and silicic acid; (b) binders, such as hydroxylmethyl cellulose, alginate, gelatin, polyvinylpyrrolidone, sucrose and arabic gum; (c) humectant, such as, glycerol; (d) disintegrants, such as agar, calcium carbonate, potato starch or tapioca starch, alginic acid, certain composite silicates, and sodium carbonate; (e) dissolution-retarding agents, such as paraffin; (f) absorption accelerators, such as quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, lauryl sodium sulfate, or the mixtures thereof. In capsules, tablets and pills, the dosage forms may also contain buffering agents.

Solid dosage forms such as tablets, dragees, capsules, pills and granules can be prepared with coatings and shells such as enteric coatings and other materials known in the art. They may contain opacifying agents and the release of the active compound or compound in such compositions may be released in a portion of the digestive tract in a delayed manner. Examples of embedding components that can be employed are polymeric materials and waxy materials. If necessary, the active compound may also be in microencapsulated form with one or more of the above-mentioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the compositions may contain adjuvants such as wetting agents, emulsifying and suspending agents, sweetening agents, flavoring agents and spices.

In addition to the active compound, the suspension may contain suspending agent, such as ethoxylated isooctadecanol, polyoxyethylene sorbitol and dehydrated sorbitan ester, microcrystalline cellulose, aluminum methoxide and agar, or the mixture of these substances etc..

The compositions for parenteral injection may comprise physiologically acceptable sterile aqueous or anhydrous solutions, dispersions, suspensions or emulsions and sterile powders which can be re-dissolved into sterile injectable solutions or dispersions. Suitable aqueous and non-aqueous carriers, diluents, solvents or excipients include water, ethanol, polyols and any suitable mixtures thereof.

Dosage forms for the compounds of the present invention for topical administration include ointments, powders, patches, sprays and inhalants. The active ingredient is mixed under sterile conditions with a physiologically acceptable carrier and any preservatives, buffers, or propellants which may be required if necessary.

The treatment method of the present invention can be administered alone or in combination with other therapeutic means or therapeutic drugs.

When the pharmaceutical composition is used, a safe and effective amount of the compound of the present invention is administered to a mammal (such as a human) in need of treatment, wherein the dosage at the time of administration is the pharmaceutically effective dosage, for a human having a body weight of 60kg, the daily dose is usually 1 ~2000mg, preferably 50~ 1000mg. Of course, specific doses should be determined by taking into account factors such as the administration route, the health of the patient, etc., which are within the skill of the skilled physician.

The present invention also provides a method for preparing pharmaceutical composition, comprising the step of mixing a pharmaceutically acceptable carrier with the compound of formula I or formula II, or the crystalline form, the pharmaceutically acceptable salt, the hydrate or the solvate thereof, thus forming the pharmaceutical composition.

The invention also provides a treatment method, comprising the step of administering the compound of formula I or formula II, or the crystalline form, the pharmaceutically acceptable salt, the hydrate or the solvate thereof, or the pharmaceutical composition of the present invention to a subject in need thereof to inhibit CDK4.

Compared with prior art, the present invention has the following main advantages:

(1) The compounds of the present invention have excellent inhibitory ability and selectivity against CDK4 protein kinase;

(2) The compounds of the present invention have lower toxic side effects;

(3) The compounds of the present invention have better pharmacodynamic and pharmacokinetic properties;

(4) The compounds of the present invention have excellent anti-proliferation performance against cancer cells, especially breast cancer cells and ovarian cancer cells.

The present invention is further described hereafter in combination with specific examples. It should be understood that these examples are only used to illustrate the present invention and not to limit the scope of the present invention. The experimental methods without specific conditions described in the following examples are generally

performed according to conventional conditions, for example, conditions described in J. Sambrook et al., Molecular Cloning: A Laboratory Manual (New York: Cold Spring Harbor Laboratory Press 1989), or conditions recommended by the manufacturer. Unless otherwise stated, percentages and parts are calculated by weight.

Unless otherwise defined, all professional and scientific terms used herein have the same meanings as those familiar to those skilled in the art. In addition, any methods and materials similar or equivalent to those described herein can be applied to the method of the present invention. The preferred embodiments and materials described herein are for demonstration purposes only.

**Example B1**

Synthesis of compound TB-1 of the present invention:

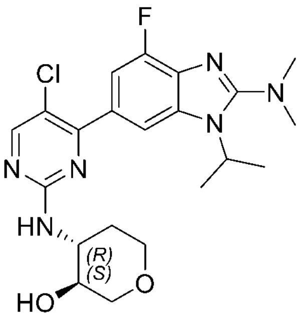

The synthesis route was as follows:

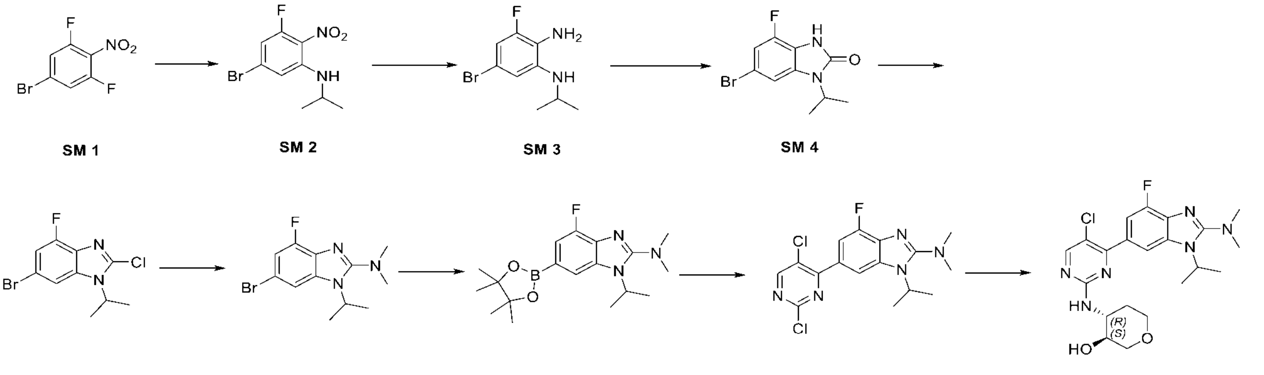

SM 1 SM 2 SM 3 SM 4

SM 5 SM 6 SM 7 SM 8 TB-1

The experimental process was as follows:

Step 1:

Compound SM **1** (1.0 g, 1.0 e.q.) was dissolved in ultra-dry tetrahydrofuran solvent to form a clear solution, cooled in an ice water bath, solid cesium carbonate (1.37 g, 1.0 e.q.) was added, and isopropylamine (0.25 g, 1.0 e.q.) was added dropwise under stirring conditions. After the dropwise addition was completed, the mixture was allowed to warm up naturally and reacted. After the reaction was completed as monitored by TLC, the solid was removed by suction filtration, and the filter cake was washed with an appropriate amount of EA. Water was added, the mixture was extracted and separated, the organic phase was dried over anhydrous sodium sulfate. After suction filtration, the mixture was concentrated under reduced pressure and purified by column chromatography to obtain yellow solid SM **2** (750 mg, 64.7%).

$^1$H NMR (400 MHz, Chloroform-*d*) δ 6.76 (t, *J* = 1.9 Hz, 1H), 6.57 (dd, *J* = 10.9, 2.0 Hz, 1H), 3.73 (dq, *J* = 13.0, 6.5 Hz, 1H), 1.31 (d, *J* = 6.3 Hz, 6H).

Step 2:

Compound SM **2** (0.75 g, 1.0 e.q.) was dissolved in a mixed solvent of anhydrous ethanol and water (v: v = 4:1), iron powder (0.91 g, 6.0 e.q.) and solid ammonium chloride (0.29 g, 2.0 e.q.) was added in sequence, and the mixture was

heated up to 90 °C for reaction. After the reaction was completed, most of the solvent was removed by concentration under reduced pressure, the mixture was extracted using saturated sodium bicarbonate solution and EA, and separated. The organic phase was dried over anhydrous sodium sulfate, filtered and concentrated under reduced pressure, and purified by column chromatography to obtain compound SM **3** (0.5 g, 74.7%)

$^1$H NMR (400 MHz, Chloroform-*d*) $\delta$ 6.64 (dd, *J* = 9.4, 2.1 Hz, 1H), 6.53 (t, *J* = 1.8 Hz, 1H), 3.56 (p, *J* = 6.3 Hz, 1H), 3.50 (s, 1H), 3.15 (s, 2H), 1.23 (d, *J* = 6.2 Hz, 6H).

Step 3:

Compound SM **3** (1.3 g, 1.0 e.q.) and CDI (2.2 g, 2.5 e.q.) were dissolved sequentially in ultra-dry DMF solvent to form a clear solution, the mixture was heated up to 110 °C for reaction. After the reaction was completed as monitored by TLC, the mixture was cooled to room temperature, extracted with EA and water and separated, the organic phase was dried over anhydrous sodium sulfate, filtered and concentrated under reduced pressure, and purified by column chromatography to obtain compound SM **4** (1.2 g, 83.3%).

$^1$H NMR (400 MHz, Chloroform-*d*) $\delta$ 9.82 (s, 1H), 7.08 (s, 1H), 7.02 (dd, *J* = 8.0, 4.0 Hz, 1H), 4.70 (m, 1H), 1.53 (d, *J* = 8.0 Hz, 6H).

Step 4:

Compound SM **4** (1.2 g, 1.0 e.q.) was dissolved in dry toluene to form a clear solution, phosphorus oxychloride (7.1 g, 1.0 e.q.) was added, the mixture was heated up to 90 °C and reacted for two days. After the reaction was completed, the mixture was cooled to room temperature and poured into ice water to quench. The mixture was extracted with EA and separated, the organic phase was dried over anhydrous sodium sulfate, filtered and concentrated under reduced pressure, and purified by column chromatography to obtain compound SM **5** (1.2 g, 94%).

$^1$H NMR (400 MHz, Chloroform-*d*) $\delta$ 7.45 (d, *J* = 2.0 Hz, 1H), 7.14 (dd, *J* = 8.0, 2.0 Hz, 1H), 4.88 (m, 1H), 1.64 (d, *J* = 8.0 Hz, 6H).

Step 5:

Compound SM **5** (1.23 g, 1.0 e.q.) was dissolved in ultra-dry DMF to form a clear solution, dimethylamine hydrochloride (3.5 g, 10.0 e.q.) and triethylamine (8.8 g, 20.0 e.q.) were added in sequence, and the mixture was heated up to 110 °C for reaction. After the reaction was completed, the mixture was cooled to room temperature, extracted with EA and water and separated. The organic phase was dried over anhydrous sodium sulfate, filtered, concentrated under reduced pressure and purified by column chromatography to obtain compound SM **6** (1.2 g, 94.5%), LCMS: $[M+H]^+$ = 299.9, 301.8.

Step 6:

Under nitrogen protection, compound SM **6** (0.27 g, 1.0 e.q.), bis(pinacolato)diboron (0.46 g, 2.0 e.q.), Pd(dppf)$Cl_2$ (33 mg, 0.05 e.q.), and potassium acetate (0.26 g, 3.0 e.q.) were sequentially added to a dry 50 mL three-necked flask. The flask was purged with nitrogen three times, and an ultra-dry 1,4-dioxane solvent was added. The mixture was heated to reflux and reacted. After the reaction was completed, the mixture was cooled to room temperature, extracted with EA and water and separated. The organic phase was dried over anhydrous sodium sulfate, filtered, concentrated under reduced pressure and purified by column chromatography to obtain compound SM 7 (0.3 g, 97%). LCMS: $[M+H]^+$ = 348.3.

Step 7:

Under nitrogen protection, compound SM 7 (0.39 g, 1.0 e.q.), 2,4,5-trichloropyrimidine (0.27 g, 1.3 e.q.), tetratriphenylphosphine palladium (0.13 g, 0.1 e.q.), and solid sodium carbonate (0.36 g, 3.0 e.q.) were sequentially added to a 50 mL three-necked flask. A mixture of 1,4-dioxane and water (v: v = 10:3) was added. The flask was purged with nitrogen three times. The mixture was heated to reflux. After the reaction was completed, the mixture was cooled to room temperature, extracted with EA and water, and separated. The organic phase was dried over anhydrous sodium sulfate, filtered, concentrated under reduced pressure and purified by column chromatography to obtain compound SM **8** (0.2 g, 49%). LCMS: $[M+H]^+$ = 368.2.

Step 8:

Under nitrogen condition, compound SM **8** (0.17 g, 1.0 e.q.), (3s, 4r)-4-aminooxan-3-ol hydrochloride (0.14 g, 2.0

e.q.) and DIPEA (0.24 g, 4.0 e.q.) were sequentially added to DMSO solvent, the mixture was heated to 90 °C and reacted. After the reaction was completed, the mixture was cooled to room temperature, extracted with saturated saline, water, and EA sequentially, and separated. The organic phase was dried over anhydrous sodium sulfate, filtered and concentrated under reduced pressure, and purified by column chromatography to obtain compound TB-1 (90 mg, 43.3%) with a HPLC purity of 99.7%, LCMS: $[M + H]^+ = 449.4$.

$^1$H NMR (400 MHz, Chloroform-*d*) $\delta$ 8.30 (s, 1H), 7.71 (d, *J* = 1.4 Hz, 1H), 7.41 (dd, *J* = 11.4, 1.4 Hz, 1H), 5.29 (d, *J* = 6.0 Hz, 1H), 4.98 (s, 1H), 4.68 (h, *J* = 7.0 Hz, 1H), 4.02 (ddd, *J* = 27.1, 11.7, 4.7 Hz, 2H), 3.92 - 3.77 (m, 1H), 3.63 (td, *J* = 9.5, 4.9 Hz, 1H), 3.46 (td, *J* = 11.9, 2.2 Hz, 1H), 3.17 (dd, *J* = 11.4, 9.8 Hz, 1H), 2.98 (s, 6H), 2.07 (s, 1H), 1.62 (dd, J = 7.0, 1.9 Hz, 6H).

The following compounds were synthesized by referring to the synthesis method of Example B1:

| Compound | Structure | LC-MS | NMR |
|---|---|---|---|
| TB-2 | 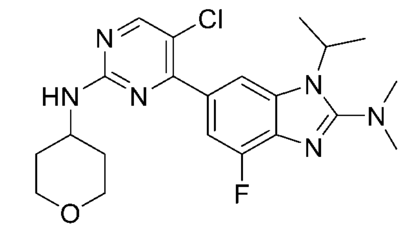 6-(5-chloro-2-((tetrahydro-2*H*-pyran-4-yl)amino)pyrimidin-4-yl)-4-fluoro-1-isopropyl-*N*,*N*-dimethyl-1*H*-benzo[d]imidazol-2-amine | $[M+H]^+$=433.4 | $^1$H NMR (400 MHz, Chloroform-*d*) $\delta$ 8.30 (s, 1H), 7.77 (s, 1H), 7.43 (d, *J* = 12.0 Hz, 1H), 5.16 (d, *J* = 8.0 Hz, 1H), 4.70 (m, 1H), 4.07 (m, 1H), 4.00 (m, 2H), 3.54 (td, *J* = 12.0, 4.0 Hz, 2H), 2.98 (s, 6H), 2.11 - 2.03 (m, 2H), 1.67 (m, 2H), 1.62 (d, *J* = 8.0 Hz, 6H). |
| TB-3 | 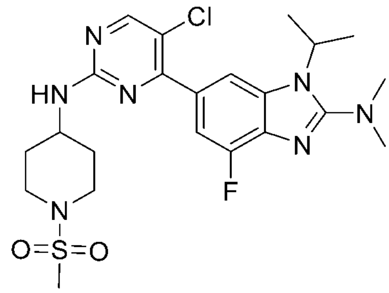 6-(5-chloro-2-((1-(methylsulfonyl)piperidin-4-yl)amino)pyrimidin-4-yl)-4-fluoro-1-isopropyl-*N*,*N*-dimethyl-1*H*-benzo[*d*]imidazol-2-amine | $[M+H]^+$=510.5 | $^1$H NMR (400 MHz, Chloroform-*d*) $\delta$ 8.30 (s, 1H), 7.77 (s, 1H), 7.41 (d, *J* = 8.0 Hz, 1H), 5.18 (d, *J* = 8.0 Hz, 1H), 4.70 (m, 1H), 4.03 - 3.95 (m, 1H), 3.80 - 3.73 (m, 2H), 2.98 (s, 6H), 2.81 (s, 2H), 2.20 (dd, *J* = 12.0, 4.0Hz, 2H), 1.66 (m, 4H), 1.62 (d, *J* = 8.0 Hz, 6H). |
| TB-4 | 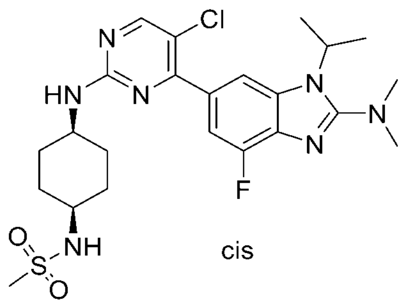 cis | $[M+H]^+$=524.5 | $^1$H NMR (400 MHz, Chloroform-*d*) $\delta$ 8.29 (s, 1H), 7.77 (s, 1H), 7.42 (d, *J* = 12.0 Hz, 1H), 5.29 (d, *J* = 8.0 Hz, 1H), 4.69 (m, 1H), 4.55 (d, *J* = 8.0 Hz, 1H), 4.01 (m, 1H), 3.55 (m, 1H), 3.00 (s, 3H), 2.98 (s, 6H), 1.89 - 1.83 (m, 4H), 1.71 (m, 4H), 1.62 (d, *J* = 4.0 Hz, 6H). |
| TB-5 | 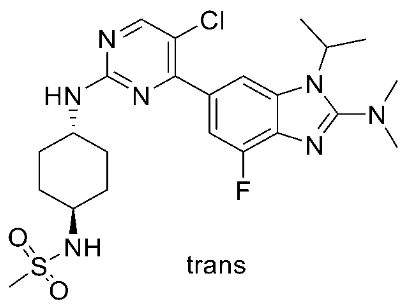 trans | $[M+H]^+$=524.4 | $^1$H NMR (400 MHz, Chloroform-*d*) $\delta$ 8.28 (s, 1H), 7.76 (s, 1H), 7.42 (d, *J* = 12.0 Hz, 1H), 5.11 (d, *J* = 8.0 Hz, 1H), 4.69 (m, 1H), 4.29 (m, 1H), 3.85 - 3.75 (m, 1H), 3.35 (m, 1H), 2.99 (s, 3H), 2.98 (s, 6H), 2.24 - 2.18 (m, 2H), 2.17 - 2.10 (m, 2H), 1.68 (m, 4H), 1.62 (d, *J* = 8.0 Hz, 6H). |

(continued)

| Compound | Structure | LC-MS | NMR |
|---|---|---|---|
| TB-6 | 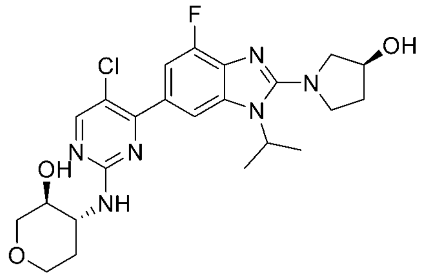<br>(*S*)-1-(6-(5-chloro-2-(((3*S*,4*R*)-3-hydroxytetrahydro-2*H*-pyran-4-yl)amino)pyrimidin-4-yl)-4-fluoro-1-isopropyl-1*H*-benzo[*d*]imidazol-2-yl)pyrrolidin-3-ol | $[M+H]^+$=491.3 | $^1$H NMR (400 MHz, Chloroform-*d*) $\delta$ 8.27 (s, 1H), 7.68 (d, *J* = 4.0 Hz, 1H), 7.40 (d, *J* = 12.0 Hz, 1H), 5.48 (d, *J* =4.0 Hz, 1H), 4.75 (m, 1H), 4.60 m, 1H), 4.05 (dd, *J* = 12.0, 4.0 Hz, 1H), 3.98 (dd, *J* = 12.0, 4.0 Hz, 1H), 3.93 - 3.87 (m, 1H), 3.85 (m, 1H), 3.78 (q, *J* = 12.0 Hz, 1H), 3.70 (dt, *J* = 12.0,4.0 Hz, 1H), 3.62 (qd, *J* = 16.0,8.0,4.0 Hz, 3H), 3.46 (td, *J* = 12.0,4.0 Hz, 1H), 3.18 (t, *J* =12.0 Hz, 1H), 2.26 (dt, *J* = 44.0,4.0 Hz, 1H), 2.18 - 2.09 (m, 2H), 2.09 - 1.98 (m, 2H), 1.67 (d, *J* = 8.0 Hz, 3H), 1.60 (d, *J* = 8.0 Hz, 3H). |
| TB-7 | 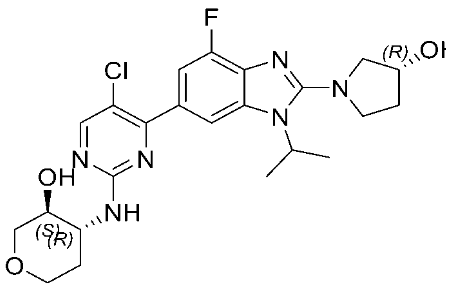<br>(*R*)-1-(6-(5-chloro-2-(((3*S*,4*R*)-3-hydroxytetrahydro-2*H*-pyran-4-yl)amino)pyrimidin-4-yl)-4-fluoro-1-isopropyl-1*H*-benzo[*d*]imidazol-2-yl)pyrrolidin-3-ol | $[M+H]^+$=491.4 | |
| TB-8 | 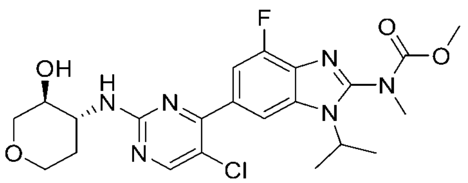<br>methyl (6-(5-chloro-2-(((3*S*,4*R*)-3-hydroxytetrahydro-2*H*-pyran-4-yl)amino)pyrimidin-4-yl)-4-fluoro-1-isopropyl-1*H*-benzo[*d*]imidazol-2-yl)(methyl)carbamate | $[M+H]^+$=493.3 | $^1$H NMR (400 MHz, Chloroform-*d*) $\delta$ 8.34 (s, 1H), 7.84 (s, 1H), 7.46 (d, *J* = 12.0 Hz, 1H), 5.30 (d, *J* = 4.0 Hz, 1H), 4.84 (brs, 1H), 4.57 (m, 1H), 4.02 (dd, *J* = 12.0, 4.0 Hz, 2H), 3.85 (m, 1H), 3.75 (s, 3H), 3.69 - 3.58 (m, 1H), 3.46 (td, *J* = 12.0, 1.6 Hz 1H), 3.39 (s, 3H), 3.17 (t, *J* = 8.0 Hz, 1H), 1.74 (td, *J* = 8.0, 4.0 Hz, 2H), 1.64 (d, *J* = 8.0 Hz, 6H). |

(continued)

| Compound | Structure | LC-MS | NMR |
|---|---|---|---|
| TB-9 | 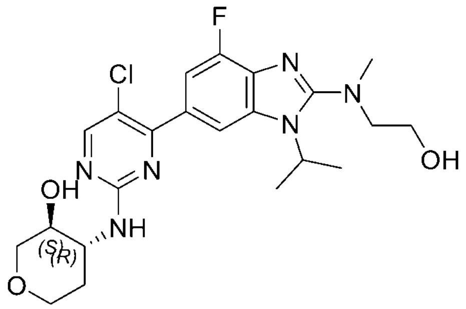 (3*S*,4*R*)-4-((5-chloro-4-(4-fluoro-2-((2-hydroxyethyl)(methyl)amino)-1-isopropyl-1*H*-benzo[*d*]imidazol-6-yl)pyrimidin-2-yl)amino)tetrahydro-2*H*-pyran-3-ol | $[M+H]^+$=479.4 | $^1$H NMR (400 MHz, Chloroform-*d*) $\delta$ 8.30 (s, 1H), 7.71 (d, *J* = 1.2 Hz, 1H), 7.41 (dd, *J* = 12.0, 1.2 Hz, 1H), 5.32 (d, *J* = 4.0 Hz, 1H), 4.74 (m, 1H), 4.05 (dd, *J* = 12.0, 4.0 Hz, 1H), 3.98 (dd, *J* = 12.0, 4.0 Hz, 1H), 3.92 (t, *J* = 4.0 Hz, 2H), 3.85 (m, 1H), 3.63 (td, *J* = 8.0, 4.0 Hz, 1H), 3.53 (t, *J* = 4.0 Hz, 2H), 3.46 (td, *J* = 12.0, 2.0 Hz, 1H), 3.17 (t, *J* = 8.0 Hz, 2H), 3.04 (s, 3H), 2.19 - 1.94 (m, 2H), 1.77 - 1.65 (m, 2H), 1.63 (dd, *J* = 8.0, 1.2 Hz, 6H). |
| TB-10 | 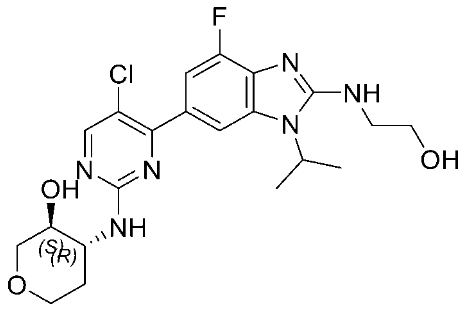 (3*S*,4*R*)-4-((5-chloro-4-(4-fluoro-2-((2-hydroxyethyl)amino)-1-isopropyl-1*H*-benzo[*d*]imidazol-6-yl)pyrimidin-2-yl)amino)tetrahydro-2*H*-pyran-3-ol | $[M+H]^+$=526.7 | |
| TB-11 | 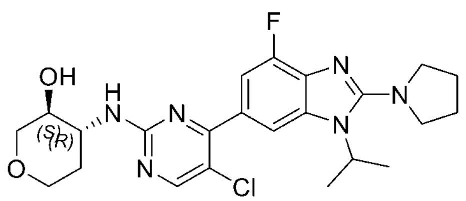 (3*S*,4*R*)-4-((5-chloro-4-(4-fluoro-1-isopropyl-2-(pyrrolidin-1-yl)-1*H*-benzo[*d*]imidazol-6-yl)pyrimidin-2-yl)amino)tetrahydro-2*H*-pyran-3-ol | $[M+H]^+$=475.7 | $^1$H NMR (400 MHz, Chloroform-*d*) $\delta$ 8.28 (s, 1H), 7.68 (d, *J* = 1.2 Hz, 1H), 7.41 (d, *J* = 11.2 Hz, 1H), 5.30 (d, *J* = 6.0 Hz, 1H), 4.72 (m, 1H), 4.05 (dd, *J* = 11.6, 5.2 Hz, 1H), 3.98 (dd, *J* = 11.2, 4.4 Hz, 1H), 3.84 (m, 1H), 3.65 - 3.61 (m, 4H), 3.45 (td, *J* = 11.9, 2.2 Hz, 1H), 3.21 - 3.12 (m, 1H), 2.03 (m, 1H), 2.03 - 1.98 (m, 4H), 1.76 - 1.67 (m, 2H), 1.63 (dd, *J* = 6.8, 2.0 Hz, 6H). |

(continued)

| Compound | Structure | LC-MS | NMR |
|---|---|---|---|
| TB-12 | 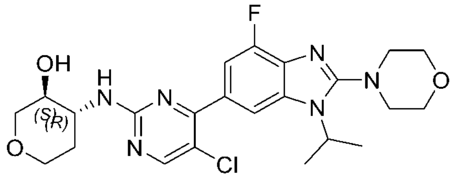 (3*S*,4*R*)-4-((5-chloro-4-(4-fluoro-1-isopropyl-2-morpholino-1*H*-benzo[*d*]imidazol-6-yl)pyrimidin-2-yl)amino)tetrahydro-2*H*-pyran-3-ol | $[M+H]^+$=491.7 | $^1$H NMR (400 MHz, Chloroform-*d*) $\delta$ 8.31 (s, 1H), 7.74 (d, *J* = 1.2 Hz, 1H), 7.42 (dd, *J* = 11.2, 0.8 Hz, 1H), 5.27 (d, *J* = 6.0 Hz, 1H), 4.93 (s, 1H), 4.68 (m, 1H), 4.05 (dd, *J* = 11.2, 4.8 Hz, 1H), 3.99 (dd, *J* = 11.2, 4.8 Hz, 1H), 3.95 - 3.88 (m, 4H), 3.84 (dq, *J* = 11.0, 5.4 Hz, 1H), 3.63 (q, *J* = 9.2 Hz, 1H), 3.46 (td, *J* = 11.9, 2.3 Hz, 1H), 3.37 - 3.26 (m, 4H), 3.17 (t, *J* = 10.6 Hz, 1H), 2.05 (ddd, *J* = 11.1, 4.8, 2.5 Hz, 1H), 1.73 (td, *J* = 12.3, 4.8 Hz, 1H), 1.63 (dd, *J* = 6.9, 1.7 Hz, 6H). |
| TB-13 | 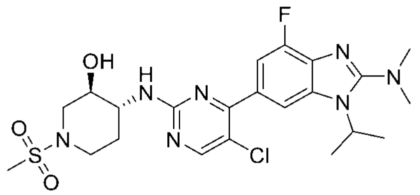 (3*R*,4*R*)-4-((5-chloro-4-(2-(dimethylamino)-4-fluoro-1-isopropyl-1*H*-benzo[*d*]imidazol-6-yl)pyrimidin-2-yl)amino)-1-(methylsulfonyl)piperidin-3-ol | $[M+H]^+$=526.7 | $^1$H NMR (400 MHz, Chloroform-*d*) $\delta$ 8.30 (s, 1H), 7.70 (d, *J* = 1.4 Hz, 1H), 7.39 (dd, *J* = 11.2, 1.4 Hz, 1H), 5.33 (d, *J* = 5.2 Hz, 1H), 4.98 (s, 1H), 4.69 (m, 1H), 3.95 (ddd, *J* = 11.7, 4.5, 2.2 Hz, 1H), 3.88 - 3.78 (m, 1H), 3.75 (m, 2H), 2.98 (s, 6H), 2.82 (s, 3H), 2.76 (dd, *J* = 12.2, 2.7 Hz, 1H), 2.53 (t, *J* = 10.2 Hz, 1H), 2.17 (dd, *J* = 13.2, 3.5 Hz, 1H), 1.80 - 1.71 (m, 1H), 1.62 (d, *J* = 7.2 Hz, 6H). |
| TB-14 | 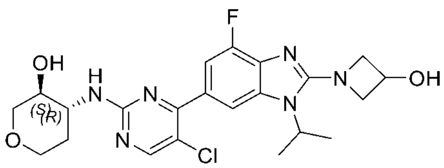 1-(6-(5-chloro-2-(((3*S*,4*R*)-3-hydroxytetrahydro-2*H*-pyran-4-yl)amino)pyrimidin-4-yl)-4-fluoro-1-isopropyl-1*H*-benzo[*d*]imidazol-2-yl)azetidin-3-ol | $[M+H]^+$=477.4 | |
| TB-15 | 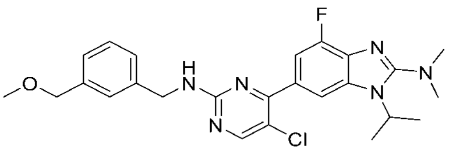 6-(5-chloro-2-((3-(methoxymethyl)benzyl)amino)pyrimidin-4-yl)-4-fluoro-1-isopropyl-*N*,*N*-dimethyl-1*H*-benzo[*d*]imidazol-2-amine | $[M+H]^+$=483.6 | $^1$H NMR (400 MHz, Chloroform-*d*) $\delta$ 8.30 (s, 1H), 7.77 (s, 1H), 7.43 (d, *J* = 12.0 Hz, 1H), 7.35 (s, 1H), 7.34 - 7.28 (m, 2H), 7.24 (m, 1H), 5.59 (t, *J* = 6.0 Hz, 1H), 4.70 (m, 1H), 4.67 (d, *J* = 6.0 Hz, 2H), 4.45 (s, 2H), 3.39 (s, 3H), 2.97 (s, 6H), 1.60 (d, *J* = 4.0 Hz, 6H). |

**Example B2**

Synthesis of compound TB-16 of the present invention:

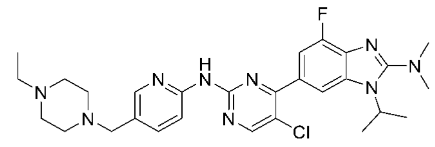

The synthesis route was as follows:

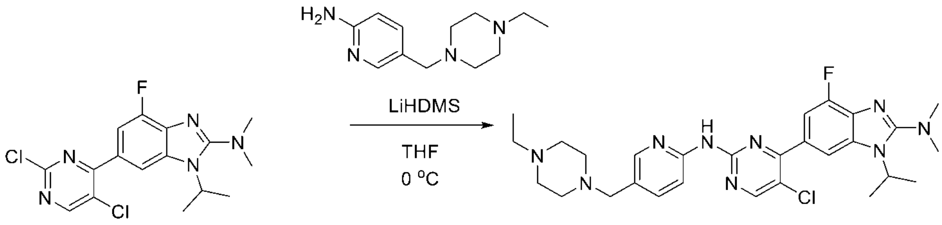


SM 1

TB-16

The experimental process was as follows:

Synthesis of compound **TB-16**

Under nitrogen protection, 5-[(4-ethylpiperazin-1-yl)methyl]pyridine-2-amine (180 mg, 1.5 e.q.) was dissolved in 4 mL of ultra-dry tetrahydrofuran solvent to form a clear solution and placed in an ice water bath. 1 M LiHDMS solution in tetrahydrofuran (0.81 mL, 1.5 e.q.) was added dropwise and the mixture was reacted in an ice water bath for half an hour. Compound SM **1** (100 mg, 1.0 e.q.) was then added and the mixture was incubated for reaction. After the reaction was completed, the mixture was extracted with EA and water and separated. The organic phase was dried over anhydrous sodium sulfate, filtered, concentrated under reduced pressure, and purified by preparative separation to obtain 120 mg of compound TB-**16**, with a HPLC purity of 98.14% and a yield of 82.0%, LCMS: $[M + H]^+$ =552.5.

$^1$H NMR (400 MHz, Chloroform-*d*) $\delta$ 8.53 (s, 1H), 8.47 (s, 1H), 8.36 - 8.32 (d, *J* = 8.0 Hz, 1H), 8.21 (d, *J* = 4.0 Hz, 1H), 7.80 (d, *J* = 1.6 Hz, 1H), 7.57 (dd, *J* = 8.0, 4.0 Hz, 1H), 7.48 (dd, *J* = 12.0, 1.6 Hz, 1H), 4.65 (p, *J* = 8.0 Hz, 1H), 3.48 (s, 2H), 2.94 (s, 6H), 2.73 (m, 8H), 1.58 (d, *J* = 8.0 Hz, 6H), 1.25 (t, *J* = 6 Hz, 3H), 1.19 (m, 2H).

The following compounds were synthesized by referring to the synthesis method of Example B2:

| Compound | Structure | LC-MS | NMR |
|---|---|---|---|
| TB-17 | 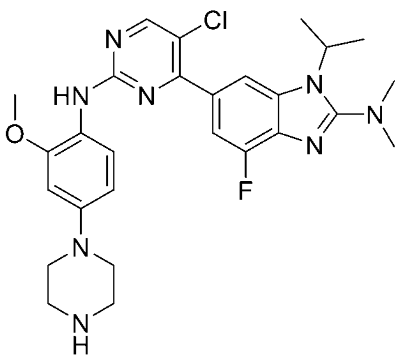 6-(5-chloro-2-((2-methoxy-4-(piperazin-1-yl)phenyl)amino) pyrimidin-4-yl)-4-fluoro-1-isopropyl-*N*,*N*-dimethyl-1*H*-benzo [*d*]imidazol-2-amine | $[M+H]^+$=539.5 | $^1$H NMR (400 MHz, Chloroform-*d*) $\delta$ 8.34 (s, 1H), 7.77 (d, *J* = 4.0 Hz, 1H), 7.60 (s, 1H), 7.49 - 7.44 (d, *J* = 12.0 Hz, 1H), 6.50 - 6.44 (m, 2H), 4.64 (p, *J* = 8.0 Hz, 1H), 3.83 (s, 3H), 3.33 (m, 8H), 2.93 (s, 6H), 1.57 (d, *J* = 4.0 Hz, 6H). |

(continued)

| Compound | Structure | LC-MS | NMR |
|---|---|---|---|
| TB-18 | 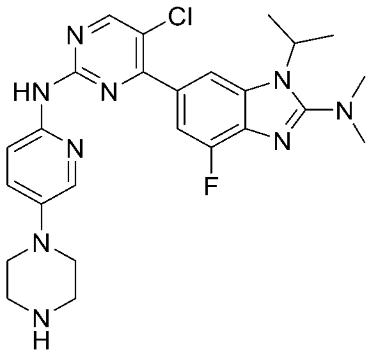 6-(5-chloro-2-((5-(piperazin-1-yl)pyridin-2-yl)amino)pyrimidin-4-yl)-4-fluoro-1-isopropyl-*N*,*N*-dimethyl-1*H*-benzo[*d*]imidazol-2-amine | $[M+H]^+$=510.4 | $^1H$ NMR (400 MHz, DMSO-$d_6$) $\delta$ 9.9s (s, 1H), 8.62 (s, 1H), 8.12 - 8.02 (m, 2H), 7.91 (s, 1H), 7.47 (dd, *J* = 12, 1.3 Hz, 1H), 7.42 (dd, *J* = 10.0, 4.0 Hz, 1H), 4.69 (p, *J* = 8.0 Hz, 1H), 3.24 (m, 4H), 3.11 (m, 4H), 2.93 (s, 6H), 1.58 (d, *J* = 8.0 Hz, 6H). |
| TB-19 | 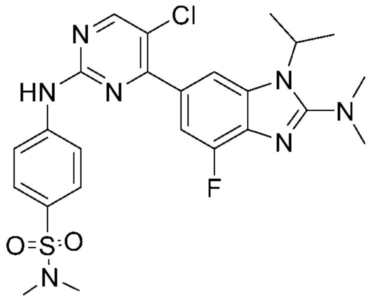 4-((5-chloro-4-(2-(dimethylamino)-4-fluoro-1-isopropyl-1*H*-benzo[*d*]imidazol-6-yl)pyrimidin-2-yl)amino)-*N,N*-dimethylbenzenesulfonamide | $[M+H]^+$=532.4 | $^1H$ NMR (400 MHz, Chloroform-d) $\delta$ 8.50 (s, 1H), 7.87 (d, *J* = 4.0 Hz, 1H), 7.85 (d, *J* = 4.0 Hz, 2H), 7.73 (d, *J* = 8.0 Hz, 2H), 7.59 (s, 1H), 7.54 (dd, *J* = 12.0, 1.6 Hz, 1H), 4.72 (m, 1H), 3.00 (s, 6H), 2.70 (s, 6H), 1.65 (d, *J* = 8.0 Hz, 6H). |
| TB-20 | 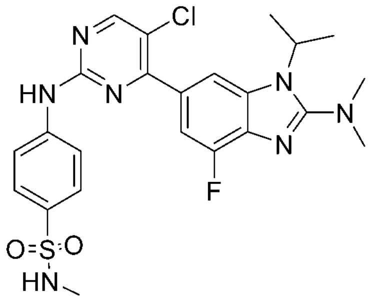 4-((5-chloro-4-(2-(dimethylamino)-4-fluoro-1-isopropyl-1*H*-benzo[*d*]imidazol-6-yl)pyrimidin-2-yl)amino)-*N*-methylbenzenesulfonamide | $[M+H]^+$=518.5 | |

(continued)

| Compound | Structure | LC-MS | NMR |
|---|---|---|---|
| TB-21 | 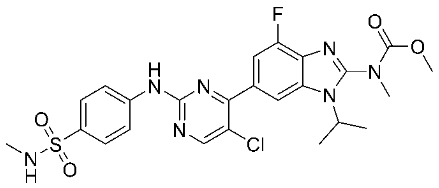 methyl (6-(5-chloro-2-((4-(*N*-methylsulfamoyl)phenyl)amino) pyrimidin-4-yl)-4-fluoro-1-isopropyl-1*H*-benzo[*d*]imidazol-2-yl)(methyl)carbamate | $[M+H]^+$=562.2 | $^1$H NMR (400 MHz, Chloroform-*d*) $\delta$ 8.54 (s, 1H), 7.98 (d, *J* = 0.8 Hz, 1H), 7.83 (q, *J* = 8.8 Hz, 4H), 7.62 (s, 1H), 7.58 (d, *J* = 12.0 Hz, 1H), 4.60 (m, 1H), 4.41 (q, *J* = 4.0 Hz, 1H), 3.77 (s, 3H), 3.41 (s, 3H), 2.66 (d, *J* = 4.0 Hz, 3H), 1.67 (d, *J* = 6.8 Hz, 6H). |

**Example B3**

Synthesis of compound TB-23 of the present invention:

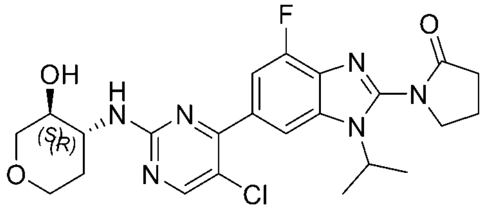

The synthesis route is as follows:

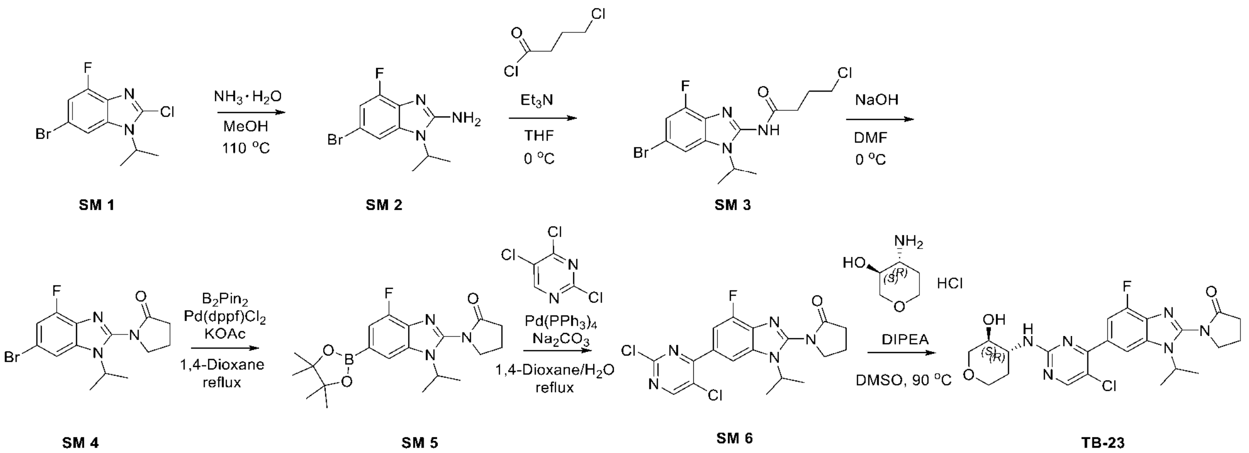


The experimental process was as follows:

Step 1:

Compound SM **1** (1.5 g, 1.0 e.q.) was dissolved in a mixed solvent of ammonia and methanol (45 mL, v: v = 2:1), and the mixture was heated up to 110 °C in a sealed autoclave to react for 24 hours. After the reaction was completed, the mixture was cooled to room temperature, the reaction solvent was concentrated under reduced pressure, and the resulting mixture was directly separated and purified by silica gel column chromatography to obtain 1.1 g of compound SM **2** with a yield of 78.6%, LCMS: $[M+H]^+$ = 272.0, 274.0.

Step 2:

Under an ice water bath, compound SM **2** (170 mg, 1.0 e.q.) was dissolved in 2 mL of ultra-dry THF solvent to form a clear solution. Then, triethylamine (316 mg, 5.0 e.q.) and 4-chlorobutyryl chloride (221 mg, 2.5 e.q.) were added in sequence, the reaction was carried out in the ice water bath. After the reaction was completed, the mixture was extracted with EA and water, and separated. The organic phase was dried over anhydrous sodium sulfate, filtered. The filtrate was concentrated under reduced pressure, separated and purified by silica gel column chromatography to obtain 180 mg of compound SM **3** with a yield of 76.6%, LCMS: $[M+H]^+$ = 376.1, 378.1.

Step 3:

Under ice water bath condition, compound SM **3** (1.1 g, 1.0 e.q.) was dissolved in 22 mL of ultra-dry DMF solvent to form a clear solution, then NaOH solid (0.21 g, 1.8 e.q.) was added and the mixture was maintained in an ice bath, stirred and reacted. After the reaction was completed, the mixture was extracted with saturated ammonium chloride and EA, and separated. The organic phase was dried over anhydrous sodium sulfate, filtered. The filtrate was concentrated under reduced pressure, then separated and purified by silica gel column chromatography to obtain 340 mg of compound SM 4 with a yield of 34%, LCMS: $[M+H]^+$ = 340.1, 342.1.

Step 4:

Under nitrogen protection, compound SM 4 (360 mg, 1.0 e.q.), bis(pinacolato)diboron (537 mg, 2.0 e.q.), $Pd(dppf)Cl_2$ (38.7 mg, 0.05 e.q.), and potassium acetate (311 mg, 3.0 e.q.) were sequentially added to a dry 50 mL three-necked flask. The flask was purged with nitrogen three times, and ultra-dry 1,4-dioxane solvent was added. The mixture was heated to reflux and reacted. After the reaction was completed, the mixture was cooled to room temperature, extracted with EA and water, and separated. The organic phase was dried over anhydrous sodium sulfate, filtered, concentrated under reduced pressure, separated and purified by column chromatography to obtain compound SM 5 (310 mg, 75.6%). LCMS: $[M+ H]^+$ = 388.3.

Step 5:

Under nitrogen protection, compound SM **5** (310 mg, 1.0 e.q.), 2,4,5-trichloropyrimidine (200 mg, 1.5 e.q.), tetratriphenylphosphine palladium (92.5 mg, 0.1 e.q.), and solid sodium carbonate (254.5 mg, 3.0 e.q.) were sequentially added to a 50 mL three-necked flask. 13 mL of a mixture of 1,4-dioxane and water (v: v = 10:3) was added, and the flask was purged with nitrogen three times. The mixture was heated to reflux. After the reaction was completed, the mixture was cooled to room temperature, extracted with EA and water, and separated. The organic phase was dried over anhydrous sodium sulfate, filtered, concentrated under reduced pressure, separated and purified by column chromatography to obtain compound SM **6** (240 mg, 73.6%), LCMS: $[M+H]^+$ = 408.1.

Step 6:

Under nitrogen condition, compound SM **6** (180 mg, 1.0 e.q.), (3s, 4r)-4-aminooxan-3-ol hydrochloride (102 mg, 1.5 e.q.), and DIPEA (228 mg, 4.0 e.q.) were sequentially added to 3 mL of DMSO solvent, the mixture was heated to 90 °C and reacted. After the reaction was completed, the mixture was cooled to room temperature, extracted with saturated saline, water, and EA sequentially, and separated. The organic phase was dried over anhydrous sodium sulfate, filtered and concentrated under reduced pressure, separated and purified by column chromatography to obtain compound TB-**23** (120 mg, 56.0%) with a HPLC purity of 96.75%, LCMS: $[M + H]^+$ = 489.4.

$^1H$ NMR (400 MHz, Chloroform-*d*) $\delta$ 8.32 (s, 1H), 7.88 (d, *J* = 1.2 Hz, 1H), 7.45 (dd, *J* = 12.0, 1.2 Hz, 1H), 5.33 (d, *J* = 8.0 Hz, 1H), 4.82 (s, 1H), 4.61 (m, 1H), 4.11 (t, *J* = 8.0 Hz, 3H), 4.05 (dd, *J* = 12.0, 4.0 Hz, 1H), 3.98 (dd, *J* = 12.0, 4.0 Hz, 1H), 3.92 - 3.79 (m, 1H), 3.63 (td, *J* = 8.0, 4.0 Hz, 1H), 3.46 (td, *J* = 12.0, 2.0 Hz, 1H), 3.17 (t, *J* = 8.0, 1H), 2.63 (t, *J* = 8.0 Hz, 2H), 2.32 (p, *J* = 8.0 Hz, 2H), 1.78 - 1.69 (m, 2H), 1.68 (d, *J* = 8.0 Hz, 6H).

The following compounds were synthesized by referring to the synthesis method of Example B3:

| Compound | Structure | LC-MS | NMR |
|---|---|---|---|
| TB-24 | 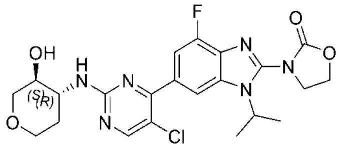 | [M+H]+=491.3 | |
| TB-25 | 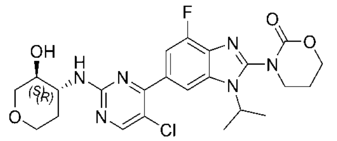 | [M+H]+=505.1 | |
| TB-26 | 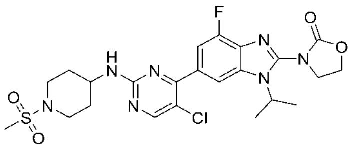 | [M+H]+=552.1 | |
| TB-27 | 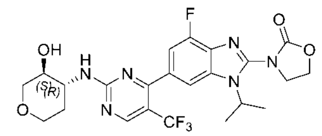 | [M+H]+=525.2 | |
| TB-28 | 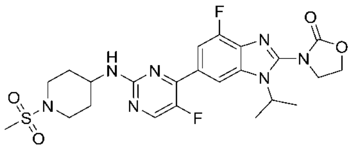 | [M+H]+=536.1 | |
| TB-31 | 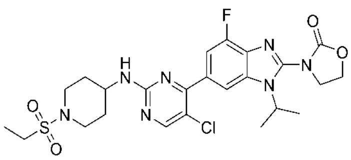 | [M+H]+=566.2 | $^1$H NMR (400 MHz, Chloroform-*d*) $\delta$ 8.33 (s, 1H), 7.93 (s, 1H), 7.48 (d, *J* = 12.0 Hz, 1H), 5.19 (d, *J* = 8.0 Hz, 1H), 4.76 (m, 1H), 4.65 (t, *J* = 8.0 Hz, 2H), 4.38 (t, *J* = 8.0 Hz, 2H), 4.07 - 3.93 (m, 1H), 3.87 - 3.74 (m, 2H), 3.06 - 3.00 (m, 2H), 2.98 (q, *J* = 8.0 Hz, 2H), 2.18 (dd, *J* = 12.0, 4.0 Hz, 2H), 1.74 (dd, *J* = 8.0, 4.0 Hz, 2H), 1.70 (d, *J* = 8.0 Hz, 6H), 1.38 (t, *J* = 8.0 Hz, 3H). |
| TB-32 | 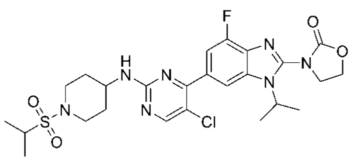 | [M+H]+=580.1 | $^1$H NMR (400 MHz, Chloroform-d) $\delta$ 8.33 (s, 1H), 7.92 (s, 1H), 7.47 (d, *J* = 12.0 Hz, 1H), 5.19 (d, J = 8.0 Hz, 1H), 4.79 - 4.72 (m, 1H), 4.65 (t, J = 8.0 Hz, 2H), 4.38 (t, J = 8.0 Hz, 2H), 4.06 - 3.95 (m, 1H), 3.84 (dd, J = 16.0, 4.0 Hz, 2H), 3.25 - 3.16 (m, 1H), 3.09 (t, J = 12.0 Hz, 2H), 2.20 - 2.10 (m, 2H), 1.70 (d, J = 4.0 Hz, 6H), 1.63 - 1.57 (m, 2H), 1.35 (d, J = 8.0 Hz, 6H). |
| TB-33 | 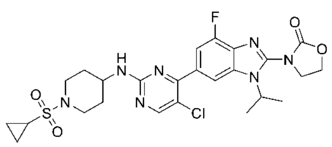 | [M+H]+=578.3 | $^1$H NMR (400 MHz, Chloroform-*d*) $\delta$ 8.33 (s, 1H), 7.93 (s, 1H), 7.48 (d, *J* = 12.0 Hz, 1H), 5.19 (d, *J* = 8.0 Hz, 1H), 4.80 - 4.73 (m, 1H), 4.64 (t, *J* = 8.0 Hz, 2H), 4.38 (t, *J* = 8.0 Hz, 2H), 4.02 - 3.96 (m, 1H), 3.78 (dd, *J* = 12.0, 4.0 Hz, 2H), 3.05 (td, *J* = 12.0, 4.0 Hz, 2H), 2.31 - 2.24 (m, 1H), 2.20 (dd, *J* = 8.0, 4.0 Hz, 2H) |

(continued)

| Compound | Structure | LC-MS | NMR |
|---|---|---|---|
| TB-35 | 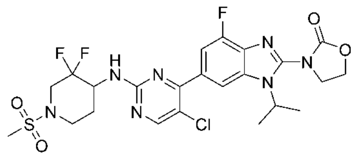 | $[M+H]^+$=588.2 | |
| TB-94 | 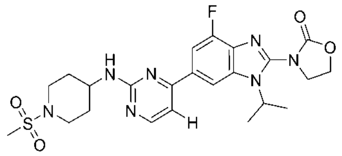 | $[M+H]^+$=518.2 | |
| TB-142 | 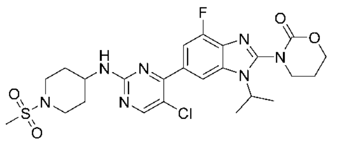 | $[M+H]^+$=566.2 | |

**Example B4**

Synthesis of compound **TB-36** of the present invention:

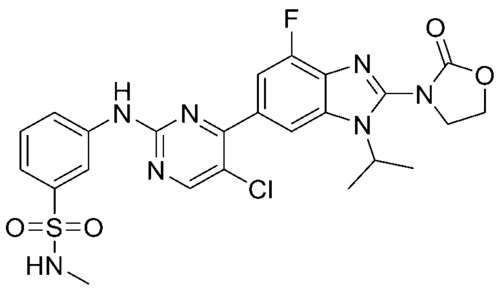

The synthesis route was as follows:

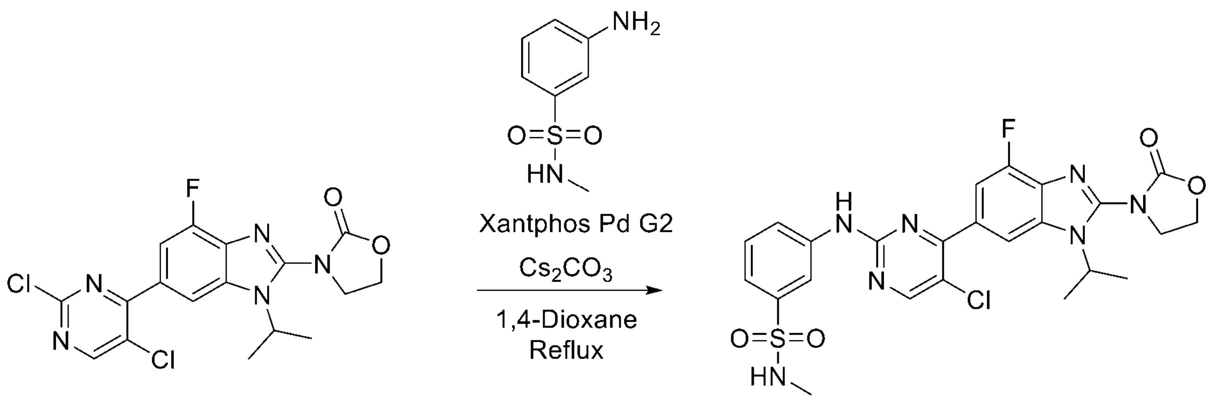


**Int-1** **TB-36**

The experimental process was as follows:

Step 1:

Under nitrogen protection, Int-**1** (200 mg, 1.0 e.q.), N-methyl-3-aminobenzenesulfonamide (136.0 mg, 1.5 e.q.), cesium carbonate (476.5 mg, 3.0 e.q.), and Xantphos Pd G2 catalyst (21.6 mg, 0.05 e.q.) were added to a 25 mL three-necked flask, followed by the addition of 10 mL of ultra-dry 1,4-dioxane solvent. After the flask was purged with nitrogen three times, the mixture was heated to reflux and reacted. After the reaction was completed, the mixture was cooled to room temperature, extracted with EA and water, and separated. The organic phase was dried over anhydrous sodium sulfate, filtered, concentrated under reduced pressure, and subjected to preparative chromatography to separate the isomers to obtain compound TB-**36** (20 mg, 7.3%) with HPLC purity of 97.18%, LCMS: $[M+H]^+$ = 561.0.

$^1$H NMR (400 MHz, Chloroform-*d*) $\delta$ 8.51 (s, 1H), 8.25 (t, *J* = 2.0 Hz, 1H), 8.03 (d, *J* = 1.3 Hz, 1H), 7.84 (d, *J* = 8.3 Hz, 1H), 7.62 - 7.43 (m, 4H), 4.79 (p, *J* = 6.9 Hz, 1H), 4.66 (t, *J* = 7.7 Hz, 2H), 4.51 (q, *J* = 5.4 Hz, 1H), 4.40 (t, *J* = 7.7 Hz, 2H), 2.69 (d, *J* = 5.4 Hz, 3H), 1.72 (d, *J* = 6.9 Hz, 6H).

The following compounds were synthesized by referring to the synthesis method of Example B4:

| Compound | Structure | LC-MS | NMR |
|---|---|---|---|
| TB-38 | 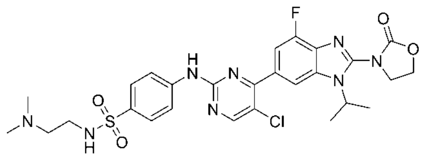 4-((5-chloro-4-(4-fluoro-1-isopropyl-2-(2-oxooxazolidin-3-yl)-1*H*-benzo[*d*]imidazol-6-yl)pyrimidin-2-yl)amino)-*N*-(2-(dimethylamino)ethyl)benzenesulfonamide | $[M+H]^+$= 617.6 | $^1$H NMR (400 MHz, Methanol-$d_4$) $\delta$ 8.61 (s, 1H), 8.17 (d, *J* = 1.2 Hz, 1H), 8.00 (d, *J* = 8.9 Hz, 2H), 7.78 (d, *J* = 8.9 Hz, 2H), 7.64 (dd, *J* = 12.0, 1.2 Hz, 1H), 4.86 (s, 1H), 4.70 (t, *J* = 8.0 Hz, 2H), 4.63 (s, 1H), 4.31 (t, *J* = 8.0 Hz, 2H), 3.05 (t, *J* = 6.4 Hz, 2H), 2.79 (t, *J* = 6.4 Hz, 2H), 2.52 (s, 6H), 1.72 (d, *J* = 7.0 Hz, 6H). |
| TB-39 | 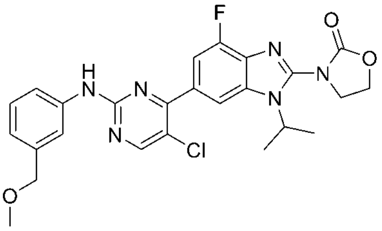 3-(6-(5-chloro-2-((3-(methoxymethyl)phenyl)amino)pyrimidin -4-yl)-4-fluoro-1-isopropyl-1*H*-benzo[d]imidazol-2-yl)oxazolidin-2-one | $[M+H]^+$= 511.3 | $^1$H NMR (400 MHz, Chloroform-d) $\delta$ 8.47 (s, 1H), 8.03 (d, *J* = 1.2 Hz, 1H), 7.65 (dd, *J* = 8.0, 1.2 Hz, 1H), 7.64 - 7.61 (m, 1H), 7.60 (s, 1H), 7.32 (t, *J* = 8.0 Hz, 1H), 7.03 (d, *J* = 4.0 Hz, 1H), 4.77 (m, 1H), 4.65 (t, *J* = 8.0Hz, 2H), 4.47 (s, 2H), 4.39 (t, *J* = 8.0 Hz, 2H), 3.41 (s, 3H), 1.72 (d, *J* = 8.0 Hz, 6H). |
| TB-54 | 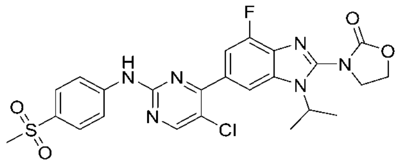 3-(6-(5-chloro-2-((4-(methylsulfonyl)phenyl)amino)pyrimidin-4-yl)-4-fluoro-1-isopropyl-1*H*-benzo[*d*]imidazol-2-yl)oxazolidin-2-one | $[M+H]^+$= 545.3 | $^1$H NMR (400 MHz, Chloroform-d) $\delta$ 8.55 (s, 1H), 8.01 (d, *J* = 1.2 Hz, 1H), 7.88 (s, 4H), 7.67 (s, 1H), 7.58 (dd, *J* = 12.0, 1.2 Hz, 1H), 4.79 (m, 1H), 4.66 (t, *J* = 8.0 Hz, 2H), 4.40 (t, *J* = 8.0 Hz, 2H), 3.05 (s, 3H), 1.72 (d, *J* = 8.0 Hz, 6H). |
| TB-68 | 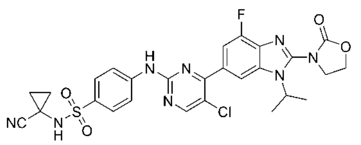 | $[M+H]^+$= 611.5 | $^1$H NMR (400 MHz, Chloroform-*d*) $\delta$ 8.52 (s, 1H), 8.02 (d, *J* = 1.4 Hz, 1H), 7.94 - 7.83 (m, 4H), 7.75 (s, 1H), 7.65 - 7.54 (m, 1H), 5.68 (s, 1H), 4.79 (p, *J* = 6.8 Hz, 1H), 4.66 (t, *J* = 7.7 Hz, 2H), 4.50 - 4.30 (m, 2H), 1.72 (d, *J* = 6.9 Hz, 6H), 1.56 (q, *J* = 5.4, 5.0 Hz, 2H), 1.42 (dd, *J*= 5.5, 2.8 Hz, 2H). |

(continued)

| Compound | Structure | LC-MS | NMR |
|---|---|---|---|
| TB-70 | 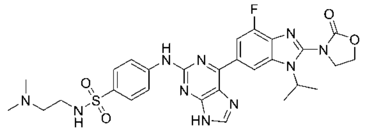 | [M+H]+= 623.3 | |
| TB-139 | 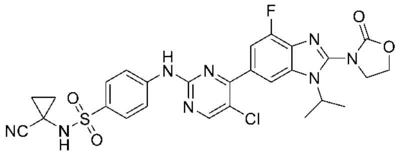 4-((5-chloro-4-(4-fluoro-1-isopropyl-2-(2-oxooxazolidin-3-yl)-1*H*-benzo[*d*]imidazol-6-yl)pyrimidin-2-yl)amino)-*N*-(1-cyanocyclopropyl)benzenesulfonamide | [M+H]+= 611.5 | $^1$H NMR (400 MHz, Chloroform-*d*) $\delta$ 8.52 (s, 1H), 8.02 (d, *J* = 1.4 Hz, 1H), 7.94 - 7.83 (m, 4H), 7.75 (s, 1H), 7.65 - 7.54 (m, 1H), 5.68 (s, 1H), 4.79 (p, *J* = 6.8 Hz, 1H), 4.66 (t, *J* = 7.7 Hz, 2H), 4.50 - 4.30 (m, 2H), 1.72 (d, *J* = 6.9 Hz, 6H), 1.56 (q, *J* = 5.4, 5.0 Hz, 2H), 1.42 (dd, *J* = 5.5, 2.8 Hz, 2H). |
| TB-84 | 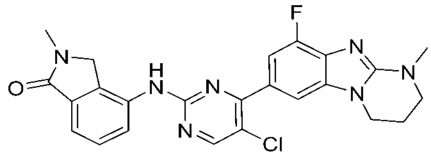 4-((5-chloro-4-(9-fluoro-1-methyl-1,2,3,4-tetrahydrobenzo[4,5]imidazo[1,2-a]pyrimidin-7-yl)pyrimidin-2-yl)amino)-2-methylisoindolin-1-one | [M+H]+= 478.5 | $^1$H NMR (400 MHz, Chloroform-*d*) $\delta$ 8.40 (s, 1H), 8.09 (d, *J* = 8.0 Hz, 1H), 7.61 (d, *J* = 7.4 Hz, 1H), 7.53 (dd, *J* = 11.8, 1.5 Hz, 1H), 7.47 (t, *J* = 7.8 Hz, 1H), 7.41 (d, *J* = 1.5 Hz, 1H), 7.08 (s, 1H), 4.36 (s, 2H), 3.99 (t, *J* = 6.1 Hz, 2H), 3.44 (t, *J* = 5.6 Hz, 2H), 3.27 (s, 3H), 3.19 (s, 3H), 2.35 - 2.25 (m, 2H). |
| TB-90 | 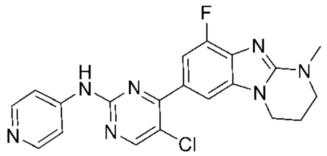 | [M+H]+= 410.2 | |
| TB-53 | 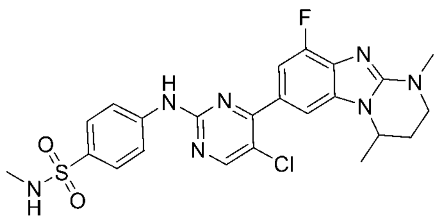 4-((5-chloro-4-(9-fluoro-1,4-dimethyl-1,2,3,4-tetrahydrobenzo[4,5]imidazo[1,2-a]pyrimidin-7-yl)pyrimidin-2-yl)amino)-N-methylbenzenesulfonamide | [M+H]+= 516.5 | $^1$H NMR (400 MHz, Chloroform-*d*) $\delta$ 8.45 (s, 1H), 7.81 - 7.74 (m, 4H), 7.69 (s, 1H), 7.54 - 7.46 (m, 2H), 4.65 (q, *J* = 5.6 Hz, 1H), 4.60 - 4.46 (m, 1H), 3.60 (td, *J* = 12.0, 3.2 Hz, 1H), 3.36 - 3.30 (m, 1H), 3.29 (s, 3H), 2.65 (d, *J* = 5.6 Hz, 3H), 2.41 (m, 1H), 2.04 - 1.97 (m, 1H), 1.52 (d, *J* = 6.4 Hz, 3H). |
| TB-130 | 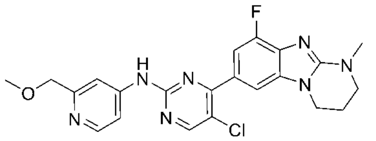 5-chloro-4-(9-fluoro-1-methyl-1,2,3,4-tetrahydrobenzo[4,5]imidazo[1,2-a]pyrimidin-7-yl)-*N*-(2-(methoxymethyl)pyridin-4-yl)pyrimidin-2-amine | [M+H]+= 454.3 | |

(continued)

| Compound | Structure | LC-MS | NMR |
|---|---|---|---|
| TB-133 | 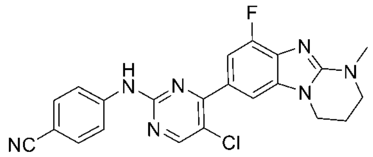 4-((5-chloro-4-(9-fluoro-1-methyl-1,2,3,4-tetrahydrobenzo[4,5]imidazo[1,2-*a*]pyrimidin-7-yl)pyrimidin-2-yl)amino)benzonitrile | $[M+H]^+$= 434.5 | $^1$H NMR (400 MHz, Chloroform-*d*) $\delta$ 8.46 (s, 1H), 7.78 (d, *J* = 8.8 Hz, 2H), 7.60 (d, *J* = 8.8 Hz, 2H), 7.55 (s, 1H), 7.52 (dd, *J* = 12.0, 1.5 Hz, 1H), 7.46 (d, *J* = 1.5 Hz, 1H), 4.03 (t, *J* = 6.0 Hz, 2H), 3.45 (t, *J* = 6.0 Hz, 2H), 3.28 (s, 3H), 2.31 (p, *J* = 6.0 Hz, 2H). |
| TB-135 | 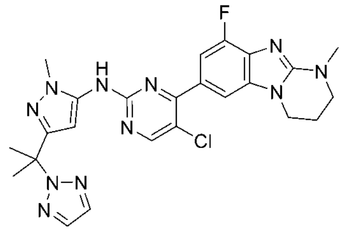 N-(3-(2-(2H-1,2,3-triazol-2-yl)propan-2-yl)-1-methyl-1*H*-pyrazol-5-yl)-5-chloro-4-(9-fluoro-1-methyl-1,2,3,4-tetrahydrobenzo[4,5]imidazo[1,2-a]pyrimidin-7-yl)pyrimidin-2-amine | $[M+H]^+$= 522.4 | $^1$H NMR (400 MHz, Chloroform-*d*) $\delta$ 8.39 (s, 1H), 7.63 (d, *J* = 1.2 Hz, 1H), 7.58 (d, *J* = 1.2 Hz, 1H), 7.56 - 7.51 (m, 1H), 7.43 (d, *J* = 1.6 Hz, 1H), 6.92 (s, 1H), 6.28 (s, 1H), 3.99 (t, *J* = 6.0 Hz, 2H), 3.79 (s, 3H), 3.52 - 3.42 (m, 2H), 3.30 (s, 3H), 2.32 (p, *J* = 6.2 Hz, 2H), 2.09 (s, 6H). |
| TB-137 | 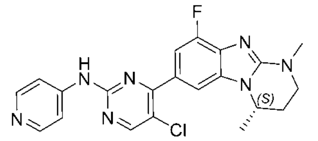 (*S*)-5-chloro-4-(9-fluoro-1,4-dimethyl-1,2,3,4-tetrahydrobenzo[4,5]imidazo[1,2-a]pyrimidin-7-yl)-*N*-(pyridin-4-yl)pyrimidin-2-amine | $[M+H]^+$= 424.3 | |
| TB-141 | 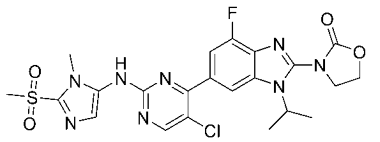 3-(6-(5-chloro-2-((1-methyl-2-(methylsulfonyl)-1*H*-imidazol-5-yl)amino)pyrimidin-4-yl)-4-fluoro-1-isopropyl-1*H*-benzo[*d*]imidazol-2-yl)oxazolidin-2-one | $[M+H]^+$= 549.2 | $^1$H NMR (400 MHz, Chloroform-d) $\delta$ 8.45 (s, 1H), 7.93 (d, *J* = 1.2 Hz, 1H), 7.56 - 7.38 (m, 1H), 7.13 (s, 1H), 7.07 (s, 1H), 4.76 (m, 1H), 4.64 (t, *J* = 7.6 Hz, 2H), 4.38 (t, *J* = 7.6 Hz, 2H), 3.83 (s, 3H), 3.38 (s, 3H), 1.69 (d, *J* = 8.0 Hz, 6H). |
| TB-145 | 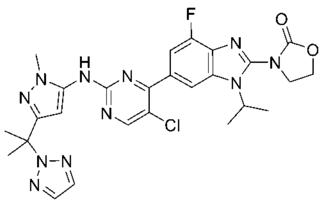 3-(6-(2-((3-(2-(2*H*-1,2,3-triazol-2-yl)propan-2-yl)-1-methyl-1*H*-pyrazol-5-yl)amino)-5-chloropyrimidin-4-yl)-4-fluoro-1-isopropyl-1*H*-benzo[*d*]imidazol-2-yl)oxazolidin-2-one | $[M+H]^+$= 580.1 | $^1$H NMR (400 MHz, Chloroform-*d*) $\delta$ 8.46 (s, 1H), 7.96 (d, *J* = 1.1 Hz, 1H), 7.65 (d, *J* = 0.8 Hz, 1H), 7.57 (d, *J* = 0.8 Hz, 1H), 7.49 (dd, *J* = 11.2, 0.9 Hz, 1H), 6.90 (s, 1H), 6.21 (s, 1H), 4.76 (p, *J* = 6.9 Hz, 1H), 4.68 - 4.59 (m, 2H), 4.45 - 4.33 (m, 2H), 3.79 (s, 3H), 2.08 (s, 6H), 1.68 (d, *J* = 6.8 Hz, 6H). |

**Example B5'**

Synthesis of compound TB-40 of the present invention:

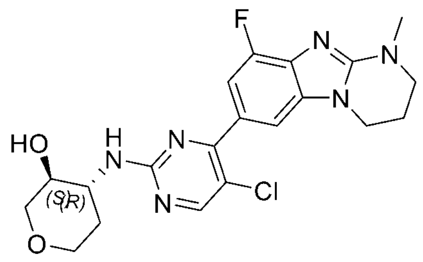

The synthesis route was as follows:

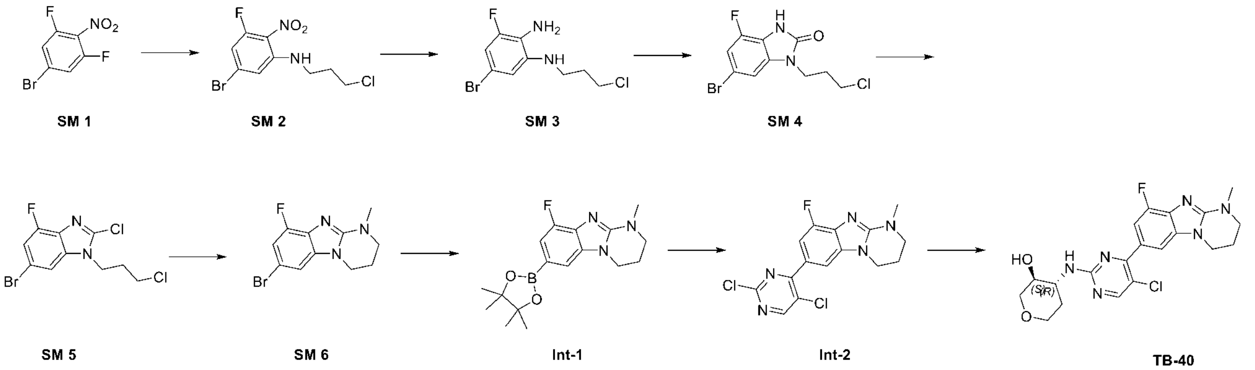

SM 1 SM 2 SM 3 SM 4

SM 5 SM 6 Int-1 Int-2 TB-40

The experimental process was as follows:

Step 1:

Compound SM **1** (5.0 g, 1.0 e.q.) was dissolved in ultra-dry tetrahydrofuran solvent to form a clear solution, the mixture was placed in an ice water bath, cesium carbonate solid (17.1 g, 2.5 e.q.) was added, and 3-chloropropylamine hydrochloride (2.7 g, 1.0 e.q.) was added under stirring. After the completion of addition, the mixture was allowed to warm to room temperature naturally and reacted. After the reaction was completed as monitored by TLC, the solid was removed by filtration, and the filter cake was washed with an appropriate amount of EA. Water was added, the mixture was extracted and separated, the organic phase was dried over anhydrous sodium sulfate. After filtered, the mixture was concentrated under reduced pressure, separated and purified by column chromatography to obtain yellow solid SM 2 (4.7 g, 71.9%).

$^1H$ NMR (400 MHz, Chloroform-*d*) $\delta$ 7.35 (s, 1H), 6.81 (t, *J* = 2.0 Hz, 1H), 6.64 (dd, *J* = 12.0, 2.0 Hz, 1H), 3.67 (t, *J* = 4.0 Hz, 2H), 3.46 (q, *J* = 8.0 Hz, 2H), 2.16 (p, *J* = 8.0 Hz, H).

Step 2:

Compound SM **2** (4.3 g, 1.0 e.q.) was dissolved in a mixed solvent of methanol and water (v: v = 4:1), iron powder (3.9 g, 5.0 e.q.) and solid ammonium chloride (1.5 g, 2.0 e.q.) were added, and the mixture was heated up to reflux and reacted. After the reaction was completed as monitored by TLC, the mixture was cooled to room temperature and filtered. The filter cake was washed with an appropriate amount of EA, and the filtrate was concentrated under reduced pressure to remove most of the solvent. The mixture was extracted with saturated sodium bicarbonate solution and EA, and separated. The organic phase was dried over anhydrous sodium sulfate, filtered and concentrated under reduced pressure, separated and purified by column chromatography to obtain compound SM **3** (2.0 g, 51.4%).

Step 3:

Compound SM **3** (1.3 g, 1.0 e.q.) and CDI (2.2 g, 2.5 e.q.) were dissolved sequentially in ultra-dry DMF solvent to form a clear solution, the mixture was heated up to 90 °C and reacted. After the reaction was completed as monitored by TLC, the mixture was cooled to room temperature, extracted with EA and water and separated. The organic phase was dried over anhydrous sodium sulfate, filtered and concentrated under reduced pressure, separated and purified by column chromatography to obtain compound SM **4** (1.2 g, 83.3%).

Step 4:

Compound SM **4** (4.0 g, 1.0 e.q.) was dissolved in dry toluene to form a clear solution, phosphorus oxychloride (36 mL, 30.0 e.q.) was added, and the mixture was heated up to reflux for two days. After the reaction was completed as monitored by TLC, the mixture was cooled to room temperature, poured into ice water for quenching, extracted with EA, and separated. The organic phase was dried over anhydrous sodium sulfate, filtered and concentrated under reduced pressure, separated and purified by column chromatography to obtain compound SM **5** (137 mg, 24.9%), LCMS: $[M+H]^+$=325.3, 327.3.

Step 5:

Compound SM 5 (1.25 g, 1.0 e.q.) was added to 60 mL of 30% methylamine ethanol solution, the mixture was heated to 60 °C and reacted. After the reaction was completed, the mixture was cooled to room temperature after reaction, concentrated under reduced pressure to remove most of the solvent, extracted with saturated saline and EA, and separated. The organic phase was dried over anhydrous sodium sulfate, filtered. The organic phase was concentrated, separated and purified by silica gel column chromatography to obtain compound SM 6 (500 mg, 48.9%)

$^1$H NMR (400 MHz, Chloroform-*d*) $\delta$ 6.98 (dd, *J* = 10.0, 1.7 Hz, 1H), 6.93 (d, *J* = 1.7 Hz, 1H), 3.93 (t, *J*= 6.2 Hz, 2H), 3.48 - 3.31 (m, 2H), 3.22 (s, 3H), 2.26 (p, *J* = 6.0 Hz, 2H).

Step 6:

Under nitrogen protection, compound SM **6** (0.26 g, 1.0 e.q.), bis(pinacolato)diboron (0.46 g, 2.0 e.q.), Pd(dppf)$Cl_2$ (34 mg, 0.05 e.q.), and potassium acetate (0.27 g, 3.0 e.q.) were sequentially added to a dry 50 mL three-necked flask. The flask was purged with nitrogen three times, and ultra-dry 1,4-dioxane solvent was added. The mixture was heated to reflux and reacted. After the reaction was completed as monitored by TLC, the mixture was cooled to room temperature, extracted with Ea and water, and separated. The organic phase was dried over anhydrous sodium sulfate, filtered, concentrated under reduced pressure, separated and purified by column chromatography to obtain compound Int-**1** (0.21 g, 69.3%), LCMS: $[M+H]^+$=332.6.

Step 7:

Under nitrogen protection, compound Int-1 (0.21 g, 1.0 e.q.), 2,4,5-trichloropyrimidine (0.21 g, 2.0 e.q.), tetratriphenylphosphine palladium (71.5 mg, 0.1 e.q.), and solid sodium carbonate (0.2 g, 3.0 e.q.) were sequentially added to a 50 mL three-necked flask. A mixture of 1,4-dioxane and water (v: v=10:3) was added, and the flask was purged with nitrogen three times. The mixture was heated to reflux and reacted. After the reaction was completed as monitored by TLC, the mixture was cooled to room temperature, extracted with Ea and water, and separated. The organic phase was dried over anhydrous sodium sulfate, filtered, concentrated under reduced pressure, separated and purified by column chromatography to obtain compound Int-**2** (0.13 g, 59.2%), LCMS: $[M+H]^+$=352.5.

Step 8:

Under nitrogen condition, compound Int-**2** (100 mg, 1.0 e.q.), (3s, 4r)-4-aminooxan-3-ol hydrochloride (87.2 mg, 2.0 e.q.), and DIPEA (0.2 mL, 4.0 e.q.) were sequentially added to DMSO solvent and the mixture was heated to 90 °C and reacted. After the reaction was completed, the mixture was cooled to room temperature, extracted with saturated saline solution, water, and EA in sequence, and separated. The organic phase was dried over anhydrous sodium sulfate, filtered, and concentrated under reduced pressure, separated and purified by preparative liquid-phase to obtain the compound TB-**40** (50 mg, 40.7%) with an HPLC purity of 91.7%, LCMS: $[M+H]^+$= 433.4.

$^1$H NMR (400 MHz, Chloroform-d) $\delta$ 8.25 (s, 1H), 7.42 (dd, *J*= 11.8, 1.2 Hz, 1H), 7.32 (d, *J* = 1.2 Hz, 1H), 5.27 (d, *J* = 6.0 Hz, 1H), 5.12 (s, 1H), 4.05 (m, 2H), 4.00 (t, *J*= 6.0 Hz, 2H), 3.89 - 3.77 (m, 1H), 3.62 (td, *J* = 9.6, 4.8 Hz, 1H), 3.50 - 3.43 (m, 1H), 3.42 (t, *J* = 5.6 Hz, 2H), 3.26 (s, 3H), 3.24 - 3.14 (m, 1H), 2.28 (p, *J* = 5.8 Hz, 2H), 2.09 - 1.98 (m, 1H), 1.70 - 1.64 (m, 1H).

**Example B5**

Synthesis of Compound TB-92 of the Present Invention

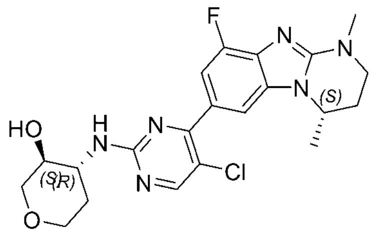

The synthetic route was as follows:

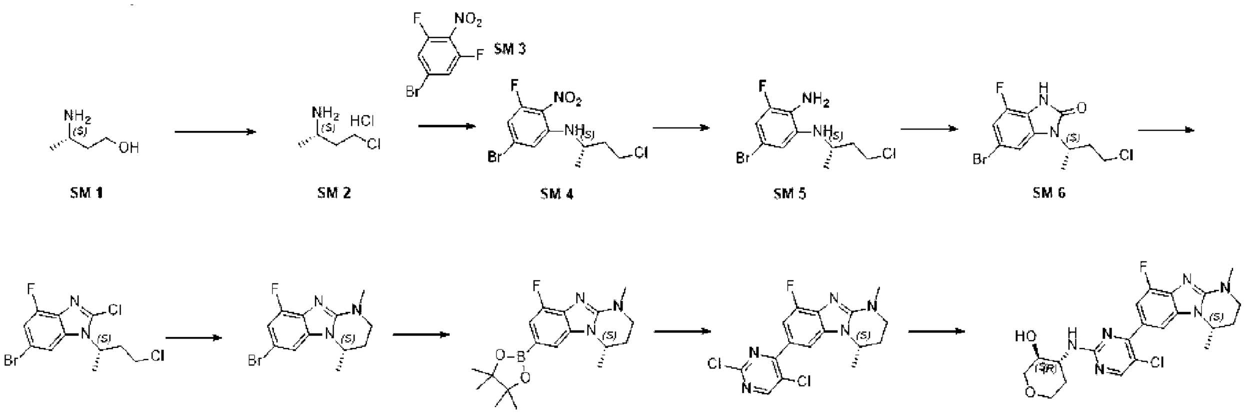

SM 3

SM 1 SM 2 SM 4 SM 5 SM 6

Int-3 SM 7 SM 8 Int-4 TB-92

The experimental procedure was as follows:

Step 1

Compound SM 1 (25.0 g, 1.0 eq.) and DMF (2.16 mL, 0.1 eq.) were dissolved in 500 mL of dry dichloromethane. The mixture was cooled to 0 °C in an ice-water bath, and $SOCl_2$ (30.5 mL, 1.5 eq.) was added dropwise slowly. After the addition was completed, the mixture was heated to 40 °C and reacted for 3 hours, then cooled to room temperature and reacted, during which white solid precipitated. After the reaction was completed as monitored by TLC, most of the solvent was removed by concentration under reduced pressure. The mixture was filtered to obtain white solid filter cake, the filter cake was washed successively with ethyl acetate and petroleum ether, and dried to obtain the product SM **2** (34 g, 84.2%).

Step 2

Compound SM **3** (56.0 g, 1.0 eq.) was dissolved in 500 mL of ultra-dry DMF. Potassium carbonate solid (97.6 g, 3.0 eq.) was added, followed by the addition of SM **2** (34.0 g, 1.0 eq.) under stirring. After the addition was completed, the mixture was heated to 80 °C and reacted. After the reaction was completed as monitored by TLC, the solid was removed by filtration, and the filter cake was washed with an appropriate amount of EA. Water was added for extraction and layer separation. The organic phase was dried over anhydrous sodium sulfate, filtered, concentrated under reduced pressure, separated and purified by column chromatography to obtain SM **4** as a yellow solid (58.5 g, 76.5%).

Step 3

Compound SM **4** (58.5 g, 1.0 eq.) was dissolved in a mixed solvent of ethanol and water (v:v = 3:1). Iron powder (60.3 g, 6.0 eq.) and ammonium chloride solid (19.2 g, 2.0 eq.) were added. The mixture was heated to reflux and reacted for 1 hour under nitrogen protection. After the reaction was completed as monitored by TLC, the mixture was cooled to room temperature and filtered. The filter cake was washed with an appropriate amount of EA. The filtrate was concentrated under reduced pressure to remove most of the solvent, extracted with saturated sodium bicarbonate solution and EA, and separated. The organic phase was dried over anhydrous sodium sulfate, filtered, concentrated under reduced pressure, separated and purified by column chromatography to obtain compound SM 5 (48.4 g, 91.7%).

Step 4

Compound SM **5** (48.0 g, 1.0 eq.) and CDI (66.3 g, 2.5 eq.) were dissolved successively in 720 mL of ultra-dry DMF to form a clear solution. The mixture was heated to 50 °C and reacted. After the reaction was completed as monitored by TLC, the mixture was cooled to room temperature, extracted with saturated sodium bicarbonate solution and EA for layer separation. The organic phase was dried over anhydrous sodium sulfate, filtered, concentrated under reduced pressure, separated and purified by column chromatography to obtain compound SM **6** (48.0 g, 91.9%).

Step 5

Compound SM **6** (48 g, 1.0 eq.) was added to 167 mL of phosphorus oxychloride, and the mixture was heated to 100 °C and reacted for 18 hours. After the reaction was completed as monitored by TLC, the mixture was cooled to room temperature, slowly poured into ice water to quench, and extracted with EA for layer separation. The organic phase was dried over anhydrous sodium sulfate, filtered, concentrated under reduced pressure, separated and purified by column chromatography to obtain compound Int-**3** (45.5 g, 89.7%).

Step 6

Under nitrogen protection, compound Int-**3** (10.0 g, 1.0 eq.), sodium iodide (22.0 g, 5.0 eq.) and 30% methylamine in ethanol (100 mL, 10.0 Vol) were added to 200 mL of DMF solution. The mixture was heated to 50 °C and reacted. After the reaction was completed, the mixture was cooled to room temperature, and most of the solvent was removed by concentration under reduced pressure. the mixture was extracted with saturated brine and EA for layer separation. The organic phase was dried over anhydrous sodium sulfate, filtered. The organic phase was concentrated under reduced pressure, separated and purified by silica gel column chromatography to afford compound SM **7** (4.8 g, 54.5%). LCMS: $[M+H]^+$ = 298.1, 300.1.

Step 7

Under nitrogen protection, compound SM 7 (4.8 g, 1.0 eq.), bis(pinacolato)diboron (8.1 g, 2.0 eq.), $Pd(dppf)Cl_2$ (585 mg, 0.05 eq.) and potassium acetate (4.7 g, 3.0 eq.) were added successively to a dry 100 mL three-necked flask. The atmosphere was replaced by nitrogen three times, and ultra-dry 1,4-dioxane (48 mL, 10.0 Vol) was added. The mixture was heated to 110 °C and reacted. After the reaction was completed as monitored by TLC, the mixture was cooled to room temperature, and extracted with EA and water for layer separation. The organic phase was dried over anhydrous sodium sulfate, filtered, concentrated under reduced pressure, separated and purified by column chromatography to obtain compound SM **8** (4.7 g, 85.0%). LCMS: $[M+H]^+$ = 346.4.

Step 8

Under nitrogen protection, compound SM **8** (4.7 g, 1.0 eq.), 2,4,5-trichloropyrimidine (3.2 g, 1.3 eq.), tetrakis(triphenylphosphine)palladium (1.6 g, 0.1 eq.) and sodium carbonate solid (4.3 g, 3.0 eq.) were added successively to a 250 mL three-necked flask. A mixture of 1,4-dioxane and water (v:v = 10:3) was added, and the flask was purged with nitrogen three times. The mixture was heated to 110 °C and reacted. After the reaction was completed as monitored by TLC, the mixture was cooled to room temperature, and extracted with Ea and water for layer separation. The organic phase was dried over anhydrous sodium sulfate, filtered, concentrated under reduced pressure, separated and purified by column chromatography to obtain compound Int-**4** (3.5 g, 70.4%). LCMS: $[M+H]^+$ = 366.2.

Step 9

Compound Int-**4** (150 mg, 1.0 eq.), (3s,4r)-4-aminooxan-3-ol hydrochloride (121.2 mg, 2.0 eq.), DIPEA (0.28 mL, 4.0 eq.) and NMP solvent (1.5 mL) were added successively to a 10 mL microwave reaction tube. The mixture was heated to 130 °C under microwave and reacted for half an hour. After the reaction was completed as monitored by TLC, the mixture was extracted with EA and water for layer separation. The organic phase was dried over anhydrous sodium sulfate, concentrated under reduced pressure, separated and purified by silica gel column chromatography to obtain the target compound TB-**92** (80 mg, 43.7%). LCMS: $[M+H]^+$ = 447.3, HPLC purity = 98.8%.

$^1$H NMR (400 MHz, Chloroform-d) $\delta$ 8.19 (s, 1H), 7.34 (dd, J = 12.0, 1.6 Hz, 1H), 7.30 (d, J = 1.6 Hz, 1H), 5.20 (d, J = 8.0 Hz, 1H), 4.46 (dq, J = 12.0,4.0, 4.0 Hz, 1H), 3.98 (dd, J = 10.0, 6.0 Hz, 1H), 3.91 (dd, J = 12.0, 4.0 Hz, 1H), 3.81 - 3.71 (m, 1H), 3.59 - 3.48 (m, 2H), 3.38 (td, J = 11.9, 2.2 Hz, 1H), 3.24 (dd, J = 8.0, 4.0 Hz, 1H), 3.21 (s, 3H), 3.10 (dd, J = 12.0, 12.0 Hz, 1H), 2.33 (tt, J = 12.0, 4.0 Hz, 1H), 1.99 - 1.89 (m, 3H), 1.43 (d, J = 8.0 Hz, 3H).

The following compounds were synthesized by referring to the synthesis method of Example B5:

| Comp. | Structure | LC-MS | NMR |
|---|---|---|---|
| TB-37 | 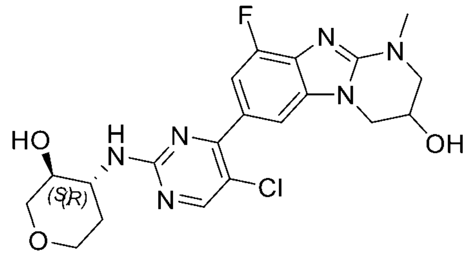 7-(5-chloro-2-(((3*S*,4*R*)-3-hydroxytetrahydro-2*H*-pyran-4-yl)amino)pyrimidin-4-yl)-9-fluoro-1-methyl-1,2,3,4-tetrahydrobenzo[4,5]imidazo[1,2-a]pyrimidin-3-ol | $[M+H]^+$= 449.2 | |
| TB-41 | 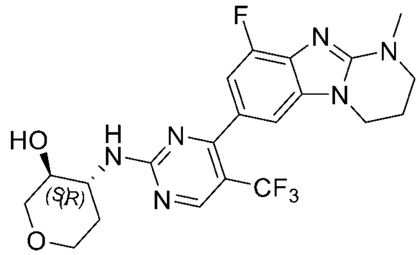 (3*S*,4*R*)-4-((4-(9-fluoro-1-methyl-1,2,3,4-tetrahydrobenzo[4,5]imidazo[1,2-*a*]pyrimidin-7-yl)-5-(trifluoromethyl)pyrimidin-2-yl)amino)tetrahydro-2*H*-pyran-3-ol | $[M+H]^+$= 467.6 | $^1$H NMR (400 MHz, Chloroform-d) $\delta$ 8.55 (s, 1H), 7.11 (d, *J* = 12.0 Hz, 1H), 7.03 (s, 1H), 5.60 (d, *J* = 5.2 Hz, 1H), 4.69 (s, 1H), 4.05 (m, 1H), 3.98 (t, *J* = 6.0 Hz, 2H), 3.96 (m, 2H), 3.63 (m, 1H), 3.47 (m, 1H), 3.42 (t, *J* = 5.2 Hz, 2H), 3.26 (s, 3H), 3.20 (m, 1H), 2.28 (p, *J* = 5.2 Hz, 2H), 2.07 (dd, *J* = 4.7, 2.3 Hz, 1H), 1.73 (d, *J* = 13.7 Hz, 1H). |
| TB-42 | 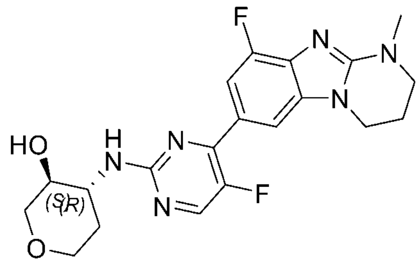 (3*S*,4*R*)-4-((5-fluoro-4-(9-fluoro-1-methyl-1,2,3,4-tetrahydrobenzo[4,5]imidazo[1,2-a]pyrimidin-7-yl)pyrimidin-2-yl)amino)tetrahydro-2*H*-pyran-3-ol | $[M+H]^+$= 417.6 | $^1$H NMR (400 MHz, Chloroform-d) $\delta$ 8.14 (d, *J* = 4.2 Hz, 1H), 7.66 (dd, *J* = 12.1, 1.5 Hz, 1H), 7.56 (d, *J* = 1.5 Hz, 1H), 5.17 (d, *J* = 5.8 Hz, 1H), 4.11 - 3.96 (m, 4H), 3.82 (td, *J* = 11.7, 10.7, 5.1 Hz, 1H), 3.67 - 3.61 (m, 1H), 3.53 - 3.40 (m, 3H), 3.27 (s, 3H), 3.27 - 3.17 (m, 1H), 2.30 (h, *J* = 5.9, 5.4 Hz, 3H), 2.08 - 2.01 (m, 1H), 1.73 - 1.68 (m, 2H). |
| TB-43 | 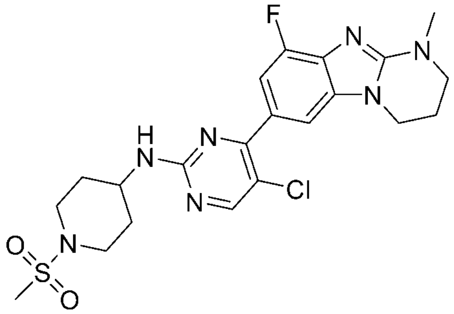 5-chloro-4-(9-fluoro-1-methyl-1,2,3,4-tetrahydrobenzo[4,5]imidazo[1,2-*a*]pyrimidin-7-yl)-*N*-(1-(methylsulfonyl)piperidin-4-yl)pyrimidin-2-amine | $[M+H]^+$= 494.8 | $^1$H NMR (400 MHz, Chloroform-*d*) $\delta$ 8.27 (s, 1H), 7.43 (dd, *J* = 12.0, 1.2 Hz, 1H), 7.40 (s, 1H), 5.14 (d, *J* = 8.0 Hz, 1H), 4.02 (t, *J* = 6.0 Hz, 2H), 4.00 - 3.91 (m, 1H), 3.75 (dt, *J* = 12.0 3.6 Hz, 2H), 3.43 (t, *J* = 5.6 Hz,2H), 3.27 (s, 3H), 2.93 (td, *J* = 12.0, 3.6 Hz, 2H), 2.81 (s, 3H), 2.33 - 2.24 (m, 2H), 2.18 (dd, *J*= 12.0, 3.6 Hz, 2H), 1.62 (dd, *J* = 12.0, 3.6 Hz, 2H). |

(continued)

| Comp. | Structure | LC-MS | NMR |
|---|---|---|---|
| TB-44 | 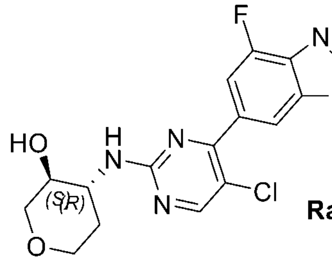 Racemic<br>(3*S*,4*R*)-4-((5-chloro-4-(9-fluoro-1,4-dimethyl-1,2,3,4-tetrahydrobenzo[4,5]imidazo[1,2-*a*]pyrimidin-7-yl)pyrimidin-2-yl)amino)tetrahydro-2*H*-pyran-3-ol | $[M+H]^+$= 447.2 | |
| TB-45 | 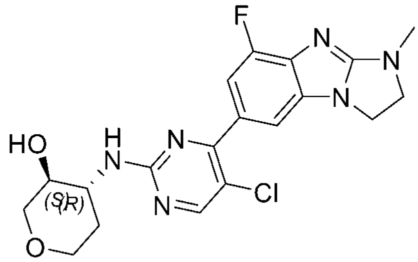<br>(3*S*,4*R*)-4-((5-chloro-4-(8-fluoro-1-methyl-2,3-dihydro-1*H*-benzo[*d*]imidazo[1,2-*a*]imidazol-6-yl)pyrimidin-2-yl)amino)tetrahydro-2*H*-pyran-3-ol | $[M+H]^+$= 419.2 | $^1$H NMR (400 MHz, DMSO-$d_6$) δ 8.43 (s, 1H), 7.55 (d, *J* = 4. 0Hz, 1H), 7.45 (d, *J* = 8.0 Hz, 1H), 7.37 (dd, *J* = 12.0, 4.0 Hz, 1H), 5.00 (d, *J* = 8.0 Hz, 1H), 4.26 (m, 2H), 4.04 (m, 2H), 3.91 - 3.82 (m, 3H), 3.55 (m, 1H), 3.11 (t, *J* = 12.0 Hz, 1H), 3.04 (s, 3H), 2.61 (s, 1H), 2.00 (m, 1H), 1.54 (m, 1H). |
| TB-48 | 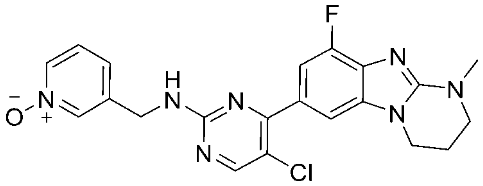<br>3-(((5-chloro-4-(9-fluoro-1-methyl-1,2,3,4-tetrahydrobenzo[4,5]imidazo[1,2-*a*]pyrimidin-7-yl)pyrimidin-2-yl)amino)methyl)pyridine 1-oxide | $[M+H]^+$= 440.6 | $^1$H NMR (400 MHz, Chloroform-d) δ 8.26 (s, 1H), 8.22 (s, 1H), 8.09 (dt, *J* = 4.3, 2.0 Hz, 1H), 7.39 (dd, *J* = 11.8, 1.5 Hz, 1H), 7.30 (d, *J* = 1.5 Hz, 1H), 7.21 (d, *J* = 4.8 Hz, 2H), 6.15 (s, 1H), 4.53 (d, *J* = 6.3 Hz, 2H), 4.02 (t, *J* = 6.1 Hz, 2H), 3.42 (t, *J* = 5.7 Hz, 2H), 3.26 (s, 3H), 2.28 (p, *J* = 6.0 Hz, 2H). |
| TB-49 | 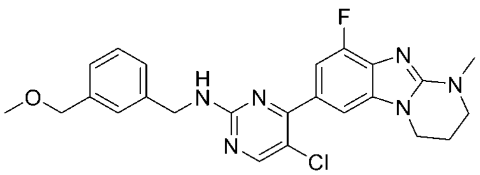<br>5-chloro-4-(9-fluoro-1-methyl-1,2,3,4-tetrahydrobenzo[4,5]imidazo[1,2-*a*]pyrimidin-7-yl)-*N*-(3-(methoxymethyl)benzyl)pyrimidin-2-amine | $[M+H]^+$= 467.5 | $^1$H NMR (400 MHz, Chloroform-*d*) δ 8.28 (s, 1H), 7.47 (d, *J*= 12.0 Hz, 1H), 7.38 (d, *J* = 1.2 Hz, 1H), 7.35 (s, 1H), 7.34 - 7.27 (m, 2H), 7.23 (s, 1H), 5.56 (t, *J* = 4.0 Hz, 1H), 4.66 (d, *J* = 4.0 Hz, 2H), 4.45 (s, 2H), 4.00 (t, *J* = 6.0 Hz, 2H), 3.42 (t, *J* = 6.0 Hz, 2H), 3.38 (s, 3H), 3.27 (s, 3H), 2.28 (p, *J* = 6.0 Hz, 2H). |

(continued)

| Comp. | Structure | LC-MS | NMR |
|---|---|---|---|
| TB-50 | 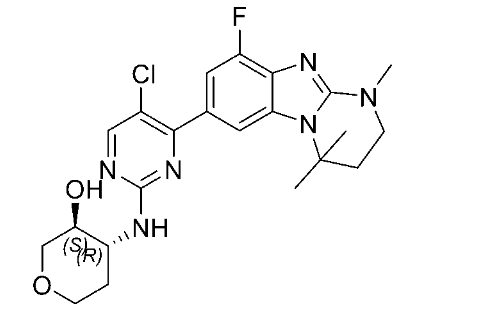 (3*S*,4*R*)-4-((5-chloro-4-(9-fluoro-1,4,4-trimethyl-1,2,3,4-tetrahydrobenzo[4,5]imidazo [1 ,2-*a*]pyrimidin-7-yl)pyrimidin-2-yl)amino)tetrahydro-2*H*-pyran-3-ol | $[M+H]^+$= 461.2 | |
| TB-51 | 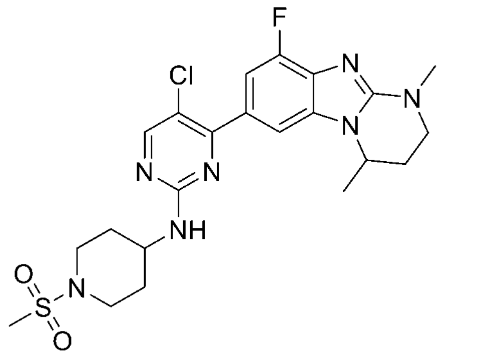 5-chloro-4-(9-fluoro-1,4-dimethyl-1,2,3,4-tetrahydrobenzo[4,5]imidazo[1,2-*a*]pyrimidin-7-yl)-*N*-(1-(methylsulfonyl)piperidin-4-yl)pyrimidin-2-amine | $[M+H]^+$= 508.50 | $^1$H NMR (400 MHz, Chloroform-*d*) $\delta$ 8.28 (s, 1H), 7.47 -7.38 (m, 2H), 5.11 (d, *J* = 8.0 Hz, 1H), 4.57 - 4.52 (m, 1H), 4.0 - 3.97 (m, 1H), 3.76 (d, *J* = 12.0 Hz, 2H), 3.59 (td, *J* = 12.0, 4.0 Hz, 1H), 3.33 - 3.29 (m, 1H), 3.28 (s, 3H), 2.93 (td, *J* = 12.0, 4.0 Hz, 2H), 2.81 (s, 3H), 2.44 - 2.35 (m, 1H), 2.18 (d, *J* = 12.0 Hz, 2H), 2.00 (dq, *J* = 12.0, 8.0, 4.0 Hz, 1H), 1.70 - 1.61 (m, 2H), 1.50 (d, *J* = 8.0 Hz, 3H). |
| TB-52 | 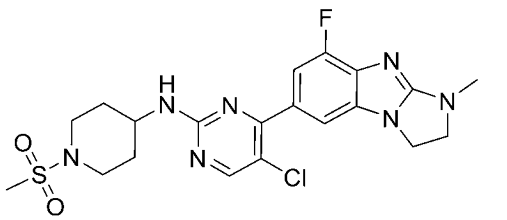 5-chloro-4-(8-fluoro-1-methyl-2,3-dihydro-1*H*-benzo[*d*]imidazo[1,2-*a*]imidazol-6-yl)-*N*-(1-(methylsulfonyl)piperidin-4-yl)pyrimidin-2-amine | $[M+H]^+$= 480.4 | $^1$H NMR (400 MHz, DMSO-$d_6$) $\delta$ 8.45 (s, 1H), 7.61 (s, 1H), 7.54 (s, 1H), 7.36 (dd, *J* = 12.0, 4.0 Hz, 1H), 4.25 (t, *J* = 16.0 Hz, 2H), 4.03 (t, *J* = 16.0 Hz, 2H), 3.94 (m, 1H), 3.59 (m, 2H), 3.04 (s, 3H), 2.93 (m, 5H), 2.04 (m, 2H), 1.63 (m, 2H). |
| TB-64 | 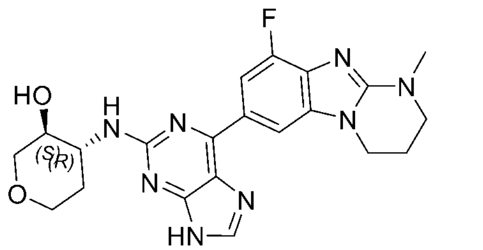 (3*S*,4*R*)-4-((6-(9-fluoro-1-methyl-1,2,3,4-tetrahydrobenzo[4,5]imidazo[1,2-a]pyrimidin-7-yl)-9*H*-purin-2-yl)amino)tetrahydro-2*H*-pyran-3-ol | $[M+H]^+$= 439.2 | |

(continued)

| Comp. | Structure | LC-MS | NMR |
|---|---|---|---|
| TB-74 | 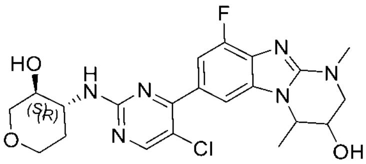 7-(5-chloro-2-(((3*S*,4*R*)-3-hydroxytetrahydro-2*H*-pyran-4-yl)amino)pyrimidin-4-yl)-9-fluoro-1,4-dimethyl-1,2,3,4-tetrahydrobenzo[4,5]imidazo [1,2-*a*]pyrimidin-3-ol | $[M+H]^+$= 463.2 | |
| TB-91 | 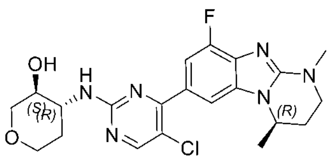 (3*S*,4*R*)-4-((5-chloro-4-((*R*)-9-fluoro-1,4-dimethyl-1,2,3,4-tetrahydrobenzo[4,5]imidazo [1,2-*a*]pyrimidin-7-yl)pyrimidin-2-yl)amino)tetrahydro-2*H*-pyran-3-ol | $[M+H]^+$= 447.2 | |
| TB-92 | 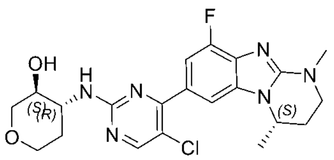 (3*S*,4*R*)-4-((5-chloro-4-((*S*)-9-fluoro-1,4-dimethyl-1,2,3,4-tetrahydrobenzo[4,5]imidazo [1,2-*a*]pyrimidin-7-yl)pyrimidin-2-yl)amino)tetrahydro-2*H*-pyran-3-ol | $[M+H]^+$= 447.2 | |
| TB-93 | 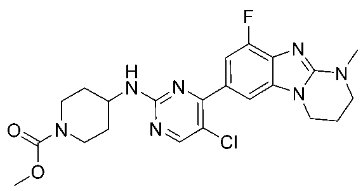 methyl 4-((5-chloro-4-(9-fluoro-1-methyl-1,2,3,4-tetrahydrobenzo[4,5]imidazo[1,2-*a*]pyrimidin-7-yl)pyrimidin-2-yl)amino)piperidine-1-carboxylate | $[M+H]^+$= 474.2 | |
| TB-95 | 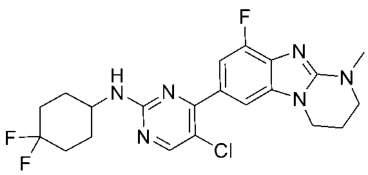 5-chloro-*N*-(4,4-difluorocyclohexyl)-4-(9-fluoro-1-methyl-1,2,3,4-tetrahydrobenzo[4,5]imidazo [1,2-*a*]pyrimidin-7-yl)pyrimidin-2-amine | $[M+H]^+$= 451.3 | $^1$H NMR (400 MHz, Methanol-$d_4$) $\delta$ 8.27 (s, 1H), 7.50 (d, *J* = 1.2 Hz, 1H), 7.40 (dd, *J* = 12.0, 1.2 Hz, 1H), 4.65 (s, 1H), 4.04 (t, *J* = 6.0 Hz, 2H), 3.96 (s, 1H), 3.47 (t, *J* = 6.0 Hz, 2H), 3.20 (s, 3H), 2.28 (p, *J* = 6.0 Hz, 2H), 2.08 (m, 4H), 1.99 - 1.84 (m, 2H), 1.76 - 1.62 (m, 2H). |
| TB-96 | 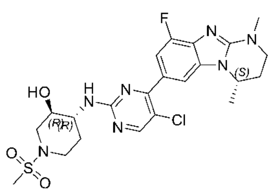 | $[M+H]^+$= 524.2 | |

(continued)

| Comp. | Structure | LC-MS | NMR |
|---|---|---|---|
| TB-100 | 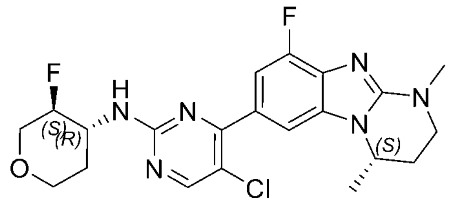 5-chloro-4-((S)-9-fluoro-1,4-dimethyl-1,2,3,4-tetrahydrobenzo[4,5]imidazo[1,2-*a*]pyrimidin-7-yl)-N-((3*S*,4*R*)-3-fluorotetrahydro-2*H*-pyran-4-yl) pyrimidin-2-amine | $[M+H]^+$= 449.3 | |
| TB-101 | 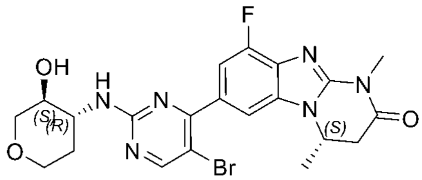 (*S*)-7-(5-bromo-2-(((3*S*,4*R*)-3-hydroxytetrahydro -2*H*-pyran-4-yl)amino)pyrimidin-4-yl)-9-fluoro-1,4-dimethyl-3,4-dihydrobenzo[4,5]imidazo [1,2-a]pyrimidin-2(1*H*)-one | $[M+H]^+$= 505.1 | |
| TB-102 | 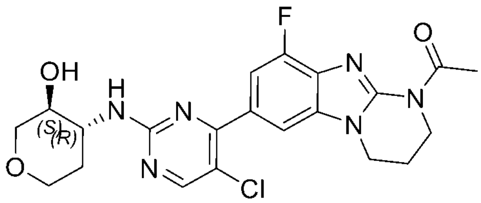 1-(7-(5-chloro-2-(((3*S*,4*R*)-3-hydroxytetrahydro-2*H*-pyran-4-yl)amino)pyrimidin-4-yl)-9-fluoro-3,4-dihydrobenzo[4,5]imidazo[1,2-*a*]pyrimidin-1(2*H*)-yl)ethan-1-one | $[M+H]^+$= 461.3 | $^1$H NMR (400 MHz, DMSO-d6) $\delta$ 8.41 (s, 1H), 7.72 (s, 1H), 7.44 (d, J = 12.4 Hz, 2H), 4.95 (d, J = 5.4 Hz, 1H), 4.19 (t, J = 6.1 Hz, 2H), 4.01 - 3.94 (m, 2H), 3.81 (dq, J = 10.4, 6.0, 5.3 Hz, 3H), 3.54 - 3.45 (m, 2H), 3.05 (t, J = 10.4 Hz, 1H), 2.72 (s, 3H), 2.19 (p, J = 5.9 Hz, 2H), 1.94 (m, 1H), 1.56 - 1.42 (m, 1H). |
| TB-103 | 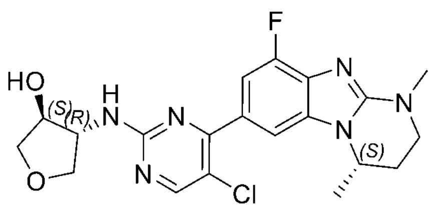 (3S,4*R*)-4-((5-chloro-4-((*S*)-9-fluoro-1,4-dimethyl-1,2,3,4-tetrahydrobenzo[4,5]imidazo [1,2-*a*]pyrimidin-7-yl)pyrimidin-2-yl)amino)tetrahydrofuran-3-ol | $[M+H]^+$= 433.2 | $^1$H NMR (400 MHz, Chloroform-*d*) $\delta$ 8.31 (s, 1H), 7.44 - 7.36 (m, 2H), 5.48 (d, *J* = 5.0 Hz, 1H), 4.53 (ddd, *J* = 11.5, 6.3, 3.3 Hz, 1H), 4.32 (ddd, *J* = 6.1, 3.9, 2.7 Hz, 1H), 4.27 (dd, *J* = 9.1, 6.4 Hz, 1H), 4.25 - 4.19 (m, 1H), 4.13 - 4.08 (m, 1H), 3.74 (ddd, *J* = 18.1, 9.4, 4.1 Hz, 2H), 3.58 (td, *J* = 12.3, 3.2 Hz, 1H), 3.34 - 3.28 (m, 1H), 3.27 (s, 3H), 2.38 (tt, *J* = 12.7, 5.1 Hz, 1H), 1.98 (dq, *J* = 13.8, 2.9 Hz, 1H), 1.49 (d, *J* = 6.6 Hz, 3H). |
| TB-104 | 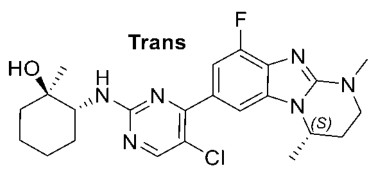 (1*R*,2*R*)-2-((5-chloro-4-((*S*)-9-fluoro-1,4-dimethyl-1,2,3,4-tetrahydrobenzo[4,5]imidazo [1,2-*a*]pyrimidin-7-yl)pyrimidin-2-yl)amino)-1-methylcyclohexan-1-ol | $[M+H]^+$= 459.4 | |

(continued)

| Comp. | Structure | LC-MS | NMR |
|---|---|---|---|
| TB-105 | 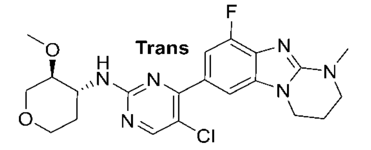 5-chloro-4-(9-fluoro-1-methyl-1,2,3,4-tetrahydrobenzo[4,5]imidazo[1,2-*a*]pyrimidin-7-yl)-*N*-((3*S*,4*R*)-3-methoxytetrahydro-2*H*-pyran-4-yl)pyrimidin-2-amine | $[M+H]^+$= 447.3 | $^1$H NMR (400 MHz, Chloroform-d) $\delta$ 8.28 (s, 1H), 7.44 (dd, *J* = 11.8, 1.5 Hz, 1H), 7.39 (s, 1H), 5.28 (d, *J* = 7.2 Hz, 1H), 4.10 (dd, *J* = 11.3, 4.0 Hz, 1H), 4.06 (d, *J* = 4.5 Hz, 1H), 4.02 (t, *J* = 6.1 Hz, 2H), 3.87 (dt, *J* = 11.8, 4.2 Hz, 1H), 3.60 - 3.52 (m, 1H), 3.44 (s, 3H), 3.42 (d, *J* = 5.9 Hz, 2H), 3.35 (dd, *J* = 11.4, 8.4 Hz, 1H), 3.26 (s, 3H), 3.23 (dd, *J* = 8.4, 4.0 Hz, 1H), 2.38 - 2.31 (m, 1H), 2.28 (q, *J* = 5.9 Hz, 2H), 1.61 - 1.53 (m, 1H). |
| TB-106 | 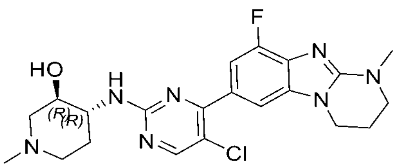 (3*R*,4*R*)-4-((5-chloro-4-(9-fluoro-1-methyl-1,2,3,4-tetrahydrobenzo[4,5]imidazo [1,2-*a*]pyrimidin-7-yl)pyrimidin-2-yl)amino)-1-methylpiperidin-3-ol | $[M+H]^+$= 446.3 | $^1$H NMR (400 MHz, Chloroform-*d*) $\delta$ 8.25 (s, 1H), 7.42 (dd, *J* = 11.8, 1.5 Hz, 1H), 7.33 (d, *J* = 1.5 Hz, 1H), 5.23 (d, *J* = 5.2 Hz, 1H), 4.02 (t, *J* = 6.1 Hz, 2H), 3.75 - 3.66 (m, 2H), 3.43 (t, *J* = 5.6 Hz, 2H), 3.27 (s, 3H), 3.06 (d, *J* = 11.3 Hz, 1H), 2.82 (d, *J* = 11.7 Hz, 1H), 2.34 (s, 3H), 2.28 (p, *J* = 5.9 Hz, 2H), 2.18 - 1.98 (m, 4H), 1.61 - 1.53 (m, 1H). |
| TB-107 | 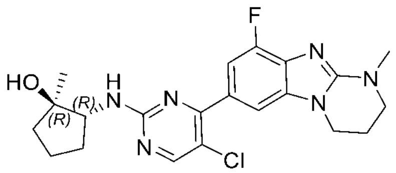 (1*R*,2*R*)-2-((5-chloro-4-(9-fluoro-1-methyl-1,2,3,4-tetrahydrobenzo[4,5]imidazo [1,2-*a*]pyrimidin-7-yl)pyrimidin-2-yl)amino)-1-methylcyclopentan-1-ol | $[M+H]^+$= 431.3 | $^1$H NMR (400 MHz, Methanol-$d_4$) $\delta$ 8.29 (s, 1H), 7.45 (s, 1H), 7.39 (dd, *J* = 12.0, 1.4 Hz, 1H), 4.61 (s, 1H), 4.18 (t, *J* = 8.2 Hz, 1H), 4.04 (t, *J* = 6.1 Hz, 2H), 3.52 - 3.43 (m, 2H), 3.20 (s, 3H), 2.26 (q, *J* = 5.4 Hz, 2H), 2.21 (dd, J = 8.9, 4.7 Hz, 1H), 1.92 - 1.55 (m, 6H), 1.19 (s, 3H). |
| TB-108 | 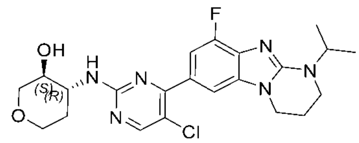 (3*S*,4*R*)-4-((5-chloro-4-(9-fluoro-1-isopropyl-1,2,3,4-tetrahydrobenzo[4,5]imidazo[1,2-*a*] pyrimidin-7-yl)pyrimidin-2-yl)amino)tetrahydro-2*H*-pyran-3-ol | $[M+H]^+$= 461.3 | $^1$H NMR (400 MHz, Chloroform-d) δ 8.19 (s, 1H), 7.35 (d, J = 12.0 Hz, 1H), 7.26 (s, 1H), 5.22 (d, J = 4.0 Hz, 1H), 4.91 (p, J = 6.8 Hz, 1H), 3.95 (dt, J = 20.0, 6.0 Hz, 4H), 3.75 (tt, J = 10.0, 4.0 Hz, 1H), 3.55 (td, J = 8.0, 4.0 Hz, 1H), 3.39 (td, J = 12.0, 4.0 Hz, 1H), 3.29 (t, J = 6.0 Hz, 2H), 3.11 (t, J = 10.0 Hz, 1H), 2.15 (p, J = 6.0 Hz, 2H), 2.01 - 1.92 (m, 1H), 1.59 (d, J = 6.0 Hz, 1H), 1.18 (d, J = 8.0 Hz, 6H). |
| TB-109 | 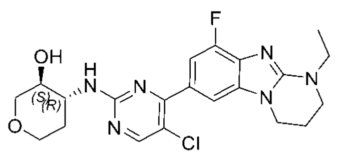 (3*S*,4*R*)-4-((5-chloro-4-(1-ethyl-9-fluoro-1,2,3,4-tetrahydrobenzo[4,5]imidazo[1,2-*a*]pyrimidin-7-yl)pyrimidin-2-yl)amino)tetrahydro-2*H*-pyran-3-ol | $[M+H]^+$= 447.3 | $^1$H NMR (400 MHz, Chloroform-*d*) δ 8.25 (s, 1H), 7.41 (dd, *J* = 11.8, 1.5 Hz, 1H), 7.32 (d, *J* = 1.5 Hz, 1H), 5.27 (d, *J* = 5.9 Hz, 1H), 4.01 (m, 4H), 3.89 - 3.78 (m, 1H), 3.74 (q, *J* = 7.1 Hz, 2H), 3.62 (td, J = 9.5, |

(continued)

| Comp. | Structure | LC-MS | NMR |
|---|---|---|---|
| | | | 4.9 Hz, 1H), 3.48 (d, *J* = 2.2 Hz, 1H), 3.43 (t, J = 5.8 Hz, 2H), 3.18 (t, *J* = 9.9 Hz, 1H), 2.26 (p, *J* = 6.0 Hz, 2H), 2.09 - 1.99 (m, 1H), 1.68 (m, 2H), 1.27 (t, *J* = 7.2 Hz, 3H). |
| TB-110 | 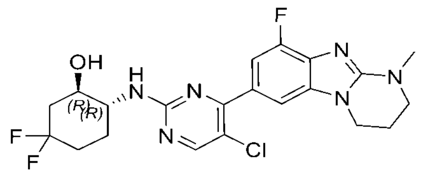 (1*R*,2*R*)-2-((5-chloro-4-(9-fluoro-1-methyl-1,2,3,4-tetrahydrobenzo[4,5]imidazo [1,2-*a*]pyrimidin-7-yl)pyrimidin-2-yl)amino)-5,5-difluorocyclohexan-1-ol | [M+H]$^+$= 467.2 | $^1$H NMR (400 MHz, Chloroform-d) $\delta$ 8.19 (s, 1H), 7.36 (dd, J = 12.0, 1.5 Hz, 1H), 7.26 (d, J = 4.0 Hz, 1H), 5.17 (d, J = 8.0 Hz, 1H), 3.94 (t, J = 12.0 Hz, 2H), 3.74 (m, 2H), 3.36 (t, J = 6.0 Hz, 2H), 3.20 (s, 3H), 2.54 - 2.45 (m, 1H), 2.22 (m, 2H), 2.05 (m, 2H), 1.93 - 1.84 (m, 2H), 1.84 - 1.74 (m, 2H). |
| TB-111 | 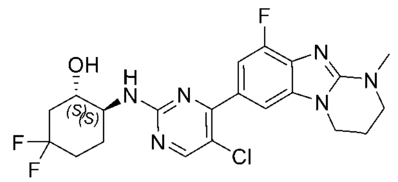 (1*S*,2*S*)-2-((5-chloro-4-(9-fluoro-1-methyl-1,2,3,4-tetrahydrobenzo[4,5]imidazo [1,2-*a*]pyrimidin-7-yl)pyrimidin-2-yl)amino)-5,5-difluorocyclohexan-1-ol | [M+H]$^+$= 467.1 | $^1$H NMR (400 MHz, Chloroform-d) $\delta$ 8.18 (s, 1H), 7.35 (d, J = 12.0 Hz, 1H), 7.26 (d, J = 4.0 Hz, 1H), 5.19 (d, J = 8.0 Hz, 1H), 3.93 (t, J = 4.0 Hz, 2H), 3.82 - 3.69 (m, 2H), 3.36 (t, J = 6.0 Hz, 2H), 3.19 (s, 3H), 2.56 - 2.44 (m, 1H), 2.21 (p, J = 6.0 Hz, 2H), 2.05 (td, J = 12.0, 12.0, 4.0 Hz, 2H), 1.88 (dq, J = 12.0, 4.0, 4.0 Hz, 1H), 1.84 - 1.72 (m, 2H). |
| TB-112 | 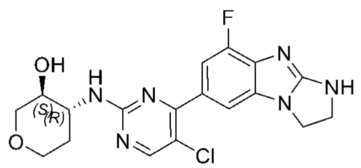 (3*S*,4*R*)-4-((5-chloro-4-(8-fluoro-2,3-dihydro-1*H*-benzo[*d*]imidazo[1,2-*a*]imidazol-6-yl)pyrimidin-2-yl)amino)tetrahydro-2*H*-pyran-3-ol | [M+H]$^+$= 405.1 | $^1$H NMR (400 MHz, DMSO-d6) $\delta$ 7.63 (d, J = 1.4 Hz, 1H), 7.53 (s, 2H), 7.45 (dd, J = 12.4, 1.5 Hz, 1H), 5.09 (d, J = 5.4 Hz, 1H), 4.35 (dd, J = 9.4, 6.7 Hz, 2H), 4.19 (dd, J = 9.3, 6.9 Hz, 2H), 4.01 - 3.92 (m, 3H), 3.64 (dd, J = 10.0, 5.1 Hz, 2H), 3.19 (t, J = 10.4 Hz, 1H), 2.09 (d, J = 13.1 Hz, 1H), 1.71 - 1.54 (m, 2H). |
| TB-113 | 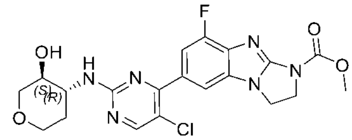 methyl 6-(5-chloro-2-(((3*S*,4*R*)-3-hydroxytetrahydro-2*H*-pyran-4-yl)amino)pyrimidin-4-yl)-8-fluoro-2,3-dihydro-1*H*-benzo[*d*]imidazo[1,2-a]imidazole-1-carboxylate | [M+H]$^+$= 463.3 | $^1$H NMR (400 MHz, DMSO-d6) $\delta$ 8.55 (s, 1H), 7.82 (s, 1H), 7.61 (s, 1H), 7.54 (d, J = 12.1 Hz, 1H), 5.08 (d, J = 5.3 Hz, 1H), 4.64 (dd, J = 9.7, 6.1 Hz, 2H), 4.54 - 4.40 (m, 2H), 4.00 (s, 3H), 3.97 (s, 2H), 3.19 (t, J = 10.4 Hz, 1H), 2.09 (s, 2H), 1.63 (d, J = 9.8 Hz, 2H), 1.46 (d, J = 15.4 Hz, 1H). |

(continued)

| Comp. | Structure | LC-MS | NMR |
|---|---|---|---|
| TB-114 | 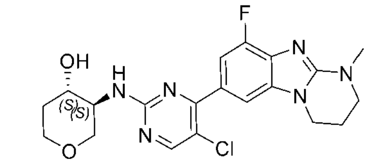 (3*S*,4*S*)-3-((5-chloro-4-(9-fluoro-1-methyl-1,2,3,4-tetrahydrobenzo[4,5]imidazo [1,2-*a*]pyrimidin-7-yl)pyrimidin-2-yl)amino)tetrahydro-2*H*-pyran-4-ol | $[M+H]^+$= 433.2 | $^1H$ NMR (400 MHz, Chloroform-*d*) $\delta$ 8.26 (s, 1H), 7.44 (dd, *J* = 11.8, 1.5 Hz, 1H), 7.35 (d, *J* = 1.5 Hz, 1H), 5.26 (d, *J* = 7.4 Hz, 1H), 4.14 (dd, *J* = 11.2, 4.4 Hz, 1H), 4.02 (t, *J* = 6.1 Hz, 2H), 3.96 - 3.90 (m, 1H), 3.77 (m, 1H), 3.48 (td, *J* = 11.7, 2.8 Hz, 1H), 3.43 (t, *J* = 6.1 Hz, 2H), 3.27 (s, 3H), 3.25 (d, *J* = 2.8 Hz, 1H), 2.28 (p, *J* = 5.9 Hz, 2H), 2.10 - 2.04 (m, 1H), 1.73 (s, 1H), 0.89 - 0.82 (m, 2H). |
| TB-115 | 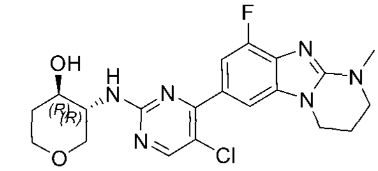 (3*R*,4*R*)-3-((5-chloro-4-(9-fluoro-1-methyl-1,2,3,4-tetrahydrobenzo[4,5]imidazo [1,2-*a*]pyrimidin-7-yl)pyrimidin-2-yl)amino)tetrahydro-2*H*-pyran-4-ol | $[M+H]^+$= 433.3 | $^1H$ NMR (400 MHz, Chloroform-*d*) $\delta$ 8.25 (s, 1H), 7.43 (d, *J* = 11.8 Hz, 1H), 7.36 - 7.31 (m, 1H), 5.28 (d, *J* = 7.4 Hz, 1H), 4.16 - 4.11 (m, 1H), 4.00 (t, *J* = 6.1 Hz, 2H), 3.92 (ddt, *J*= 15.9, 8.1, 4.3 Hz, 2H), 3.76 (td, *J* = 8.8, 4.5 Hz, 1H), 3.48 (ddd, *J* = 12.2, 10.4, 2.8 Hz, 1H), 3.42 (t, *J* = 5.7 Hz, 2H), 3.26 (s, 3H), 3.22 (s, 1H), 2.27 (p, *J* = 5.9 Hz, 2H), 2.13 - 1.99 (m, 2H), 0.88 - 0.83 (m, 1H). |
| TB-116 | 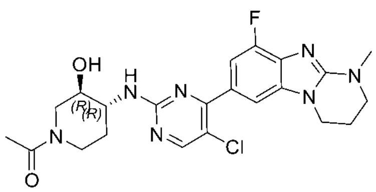 1-((3*R*,4*R*)-4-((5-chloro-4-(9-fluoro-1-methyl-1,2,3,4-tetrahydrobenzo[4,5]imidazo [1,2-*a*]pyrimidin-7-yl)pyrimidin-2-yl)amino)-3-hydroxypiperidin-1-yl)ethan-1-one | $[M+H]^+$= 474.2 | $^1H$ NMR (400 MHz, Chloroform-d) $\delta$ 8.26 (d, J = 2.3 Hz, 1H), 7.42 (dd, J = 11.7, 1.5 Hz, 1H), 7.33 (d, J = 1.6 Hz, 1H), 5.27 (d, J = 6.1 Hz, 1H), 4.68 - 4.59 (m, 1H), 4.06 - 3.94 (m, 3H), 3.87 - 3.76 (m, 1H), 3.53 (td, J = 9.6, 4.9 Hz, 1H), 3.43 (t, J = 5.6 Hz, 2H), 3.27 (s, 3H), 2.97 (dd, J = 13.5, 10.4 Hz, 1H), 2.70 - 2.53 (m, 1H), 2.28 (p, J = 6.0 Hz, 2H), 2.12 (d, J = 6.9 Hz, 3H), 2.05 (dt, J = 12.9, 3.8 Hz, 1H), 1.52 (qd, J = 12.4, 4.5 Hz, 2H). |
| TB-117 | 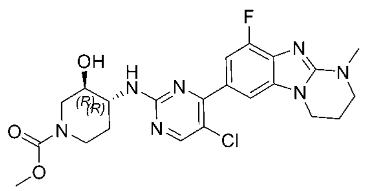 methyl (3*R*,4*R*)-4-((5-chloro-4-(9-fluoro-1-methyl-1,2,3,4-tetrahydrobenzo[4,5]imidazo [1,2-*a*]pyrimidin-7-yl)pyrimidin-2-yl)amino)-3-hydroxypiperidine-1-carboxylate | $[M+H]^+$= 490.2 | |

(continued)

| Comp. | Structure | LC-MS | NMR |
|---|---|---|---|
| TB-118 | 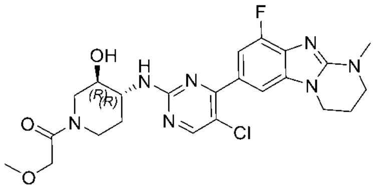 1-((3*R*,4*R*)-4-((5-chloro-4-(9-fluoro-1-methyl-1,2,3,4-tetrahydrobenzo[4,5]imidazo[1,2-a]pyrimidin-7-yl)pyrimidin-2-yl)amino)-3-hydroxypiperidin-1-yl)-2-methoxyethan-1-one | $[M+H]^+$= 504.3 | |
| TB-119 | 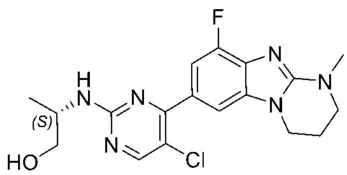 (*S*)-2-((5-chloro-4-(9-fluoro-1-methyl-1,2,3,4-tetrahydrobenzo[4,5]imidazo[1,2-*a*]pyrimidin-7-yl) pyrimidin-2-yl)amino)propan-1-ol | $[M+H]^+$= 391.5 | $^1$H NMR (400 MHz, Chloroform-d) δ 8.25 (s, 1H), 7.43 (dd, J = 11.8, 1.5 Hz, 1H), 7.34 (d, J = 1.5 Hz, 1H), 5.33 - 5.21 (m, 1H), 4.16 (qd, J = 6.7, 3.4 Hz, 1H), 4.01 (t, J = 6.1 Hz, 2H), 3.79 (dd, J = 11.0, 3.4 Hz, 1H), 3.63 (dd, J = 10.9, 6.5 Hz, 1H), 3.55 - 3.34 (m, 2H), 3.26 (s, 3H), 2.39 - 2.14 (m, 2H), 1.27 (d, J = 6.8 Hz, 3H). |
| TB-120 | 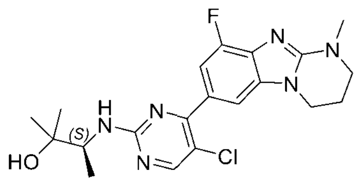 (*S*)-3-((5-chloro-4-(9-fluoro-1-methyl-1,2,3,4-tetrahydrobenzo[4,5]imidazo[1,2-*a*]pyrimidin-7-yl) pyrimidin-2-yl)amino)-2-methylbutan-2-ol | $[M+H]^+$= 419.7 | $^1$H NMR (400 MHz, Chloroform-d) δ 8.25 (s, 1H), 7.44 (dd, J = 11.8, 1.5 Hz, 1H), 7.37 (d, J = 1.6 Hz, 1H), 5.31 (d, J = 8.6 Hz, 1H), 4.05 (dt, J = 12.3, 6.4 Hz, 3H), 3.46 - 3.40 (m, 2H), 3.27 (s, 3H), 3.02 (s, 1H), 2.29 (p, J = 6.0 Hz, 3H), 1.29 (s, 3H), 1.25 (d, J = 1.6 Hz, 6H). |
| TB-121 | 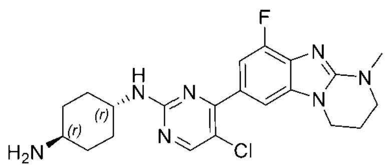 (1*r*,4*r*)-*N*[1]-(5-chloro-4-(9-fluoro-1-methyl-1,2,3,4-tetrahydrobenzo[4,5]imidazo[1,2-*a*]pyrimidin-7-yl)pyrimidin-2-yl)cyclohexane-1,4-diamine | $[M+H]^+$= 430.4 | |
| TB-122 | 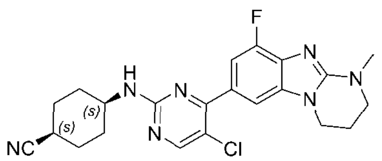 (1*s*,4*s*)-4-((5-chloro-4-(9-fluoro-1-methyl-1,2,3,4-tetrahydrobenzo[4,5]imidazo[1,2-*a*]pyrimidin-7-yl)pyrimidin-2-yl)amino)cyclohexane-1-carbonitrile | $[M+H]^+$= 440.7 | |

(continued)

| Comp. | Structure | LC-MS | NMR |
|---|---|---|---|
| TB-123 | 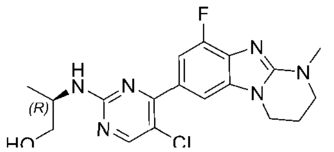 (*R*)-2-((5-chloro-4-(9-fluoro-1-methyl-1,2,3,4-tetrahydrobenzo[4,5]imidazo[1,2-*a*]pyrimidin-7-yl) pyrimidin-2-yl)amino)propan-1-ol | $[M+H]^+$= 391.2 | $^1H$ NMR (400 MHz, Chloroform-*d*) $\delta$ 8.25 (s, 1H), 7.43 (dd, *J* = 11.8, 1.5 Hz, 1H), 7.34 (d, *J* = 1.5 Hz, 1H), 5.26 (d, *J* = 6.9 Hz, 1H), 4.16 (ddt, *J* = 9.8, 6.5, 3.2 Hz, 1H), 4.01 (t, J = 6.1 Hz, 2H), 3.79 (dd, J = 10.9, 3.4 Hz, 1H), 3.64 (dd, *J* = 10.9, 6.5 Hz, 1H), 3.44 - 3.40 (m, 2H), 3.27 (s, 3H), 2.28 (p, *J* = 6.0 Hz, 2H), 1.28 (d, *J* = 6.8 Hz, 3H). |
| TB-124 | 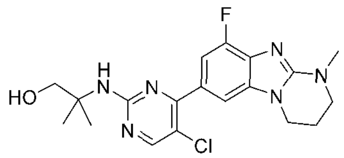 2-(5-chloro-4-(9-fluoro-1-methyl-1,2,3,4-tetrahydrobenzo[4,5]imidazo[1,2-*a*]pyrimidin-7-yl)pyrimidin-2-yl)amino)-2-methylpropan-1-ol | $[M+H]^+$= 405.3 | $^1H$ NMR (400 MHz, Chloroform-d) $\delta$ 8.24 (s, 1H), 7.41 (dd, J = 11.6, 1.4 Hz, 1H), 7.31 (d, J = 1.4 Hz, 1H), 5.63 (s, 1H), 5.34 (s, 1H), 4.02 (t, J = 6.0 Hz, 2H), 3.71 (s, 2H), 3.42 (t, J = 6.0 Hz, 2H), 3.27 (s, 3H), 2.32 - 2.24 (m, 2H), 1.39 (s, 6H). |
| TB-125 | 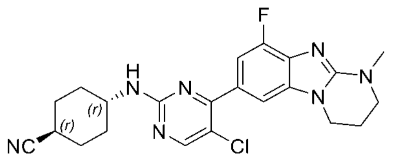 (1*r*,4*r*)-4-((5-chloro-4-(9-fluoro-1-methyl-1,2,3,4-tetrahydrobenzo[4,5]imidazo[1,2-*a*]pyrimidin-7-yl)pyrimidin-2-yl)amino)cyclohexane-1-carbonitrile | $[M+H]^+$= 440.3 | $^1H$ NMR (400 MHz, Chloroform-*d*) $\delta$ 8.26 (s, 1H), 7.44 (d, *J* = 12.0 Hz, 1H), 7.38 (s, 1H), 5.04 (d, *J* = 7.6 Hz, 1H), 4.02 (t, *J* = 6.0 Hz, 2H), 3.97 - 3.81 (m, 1H), 3.43 (t, *J* = 6.0 Hz, 2H), 3.27 (s, 3H), 2.49 (m, 1H), 2.29 (p, *J* = 6.0 Hz, 2H), 2.25 - 2.13 (m, 4H), 1.81 - 1.73 (m, 2H), 1.30 (q, *J* = 11.8 Hz, 2H). |
| TB-126 | 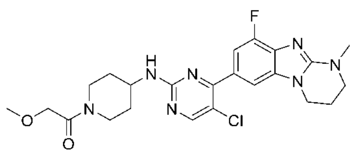 1-(4-((5-chloro-4-(9-fluoro-1-methyl-1,2,3,4-tetrahydrobenzo[4,5]imidazo[1,2-*a*]pyrimidin-7-yl)pyrimidin-2-yl)amino)piperidin-1-yl)-2-methoxyethan-1-one | $[M+H]^+$= 488.3 | $^1H$ NMR (400 MHz, Chloroform-d) $\delta$ 8.28 (s, 1H), 7.44 (d, J = 12.0 Hz, 1H), 7.39 (s, 1H), 5.11 (d, J = 8.0 Hz, 1H), 4.47 (d, J = 12.0 Hz, 1H), 4.14 - 4.10 (m, 2H), 4.08 (d, J = 6.2 Hz, 1H), 4.02 (t, J = 6.1 Hz, 2H), 3.87 (d, J = 13.9 Hz, 1H), 3.43 (s, 5H), 3.27 (s, 3H), 3.21 (t, J = 12.0 Hz, 1H), 2.93 (t, J = 12.0 Hz, 1H), 2.29 (m, 2H), 2.14 (t, J = 14.5 Hz, 2H), 1.44 (q, J = 12.0 Hz, 2H). |
| TB-127 | 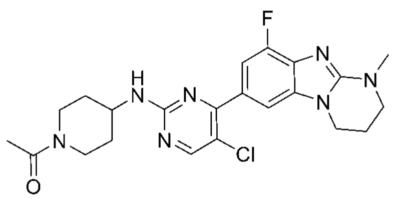 1-(4-((5-chloro-4-(9-fluoro-1-methyl-1,2,3,4-tetrahydrobenzo[4,5]imidazo[1,2-*a*]pyrimidin-7-yl)pyrimidin-2-yl)amino)piperidin-1-yl)ethan-1-one | $[M+H]^+$= 458.3 | $^1H$ NMR (400 MHz, Chloroform-d) $\delta$ 8.27 (s, 1H), 7.44 (dd, J = 11.7, 1.4 Hz, 1H), 7.39 (s, 1H), 5.12 (d, J = 7.8 Hz, 1H), 4.55 - 4.43 (m, 1H), 4.17 - 4.05 (m, 1H), 4.02 (t, J = 6.1 Hz, 2H), 3.80 (dd, J = 13.9, 2.0 Hz, 1H), 3.47 - 3.39 (m, 2H), 3.27 (s, 3H), 3.26 - 3.19 (m, 1H), 2.94 - 2.82 (m, 1H), 2.35 - 2.23 (m, 2H), 2.21 - 2.13 (m, 1H), 2.11 (s, 3H), 2.06 (s, 1H), 1.74 (s, 2H). |

(continued)

| Comp. | Structure | LC-MS | NMR |
|---|---|---|---|
| TB-128 | 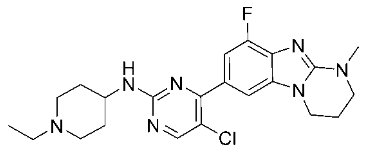 5-chloro-*N*-(1-ethylpiperidin-4-yl)-4-(9-fluoro-1-methyl-1,2,3,4-tetrahydrobenzo[4,5]imidazo [1,2-*a*]pyrimidin-7-yl)pyrimidin-2-amine | $[M+H]^+$= 444.3 | $^1H$ NMR (400 MHz, Chloroform-d) $\delta$ 8.26 (s, 1H), 7.48 - 7.42 (d,J = 12.2 Hz, 1H), 7.40 (s, 1H), 5.25 (d, J = 7.8 Hz, 1H), 4.17 - 4.06 (m, 1H), 4.03 (t, J = 6.1 Hz, 2H), 3.43 (t, J = 5.6 Hz, 2H), 3.32 (m, 2H), 3.27 (s, 3H), 2.90 (d, J = 7.4 Hz, 2H), 2.61 (m, 2H), 2.29 (p, J = 5.9 Hz, 4H), 2.14 (s, 2H), 1.39 (t, J = 7.2 Hz, 3H). |
| TB-129 | 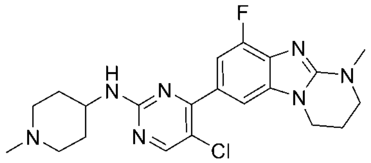 5-chloro-4-(9-fluoro-1-methyl-1,2,3,4-tetrahydrobenzo[4,5]imidazo[1,2-*a*]pyrimidin-7-yl)-*N*-(1-methylpiperidin-4-yl)pyrimidin-2-amine | $[M+H]^+$= 430.3 | $^1H$ NMR (400 MHz, Chloroform-d) $\delta$ 8.26 (s, 1H), 7.44 (d, *J* = 12.0 Hz, 1H), 7.39 (s, 1H), 5.36 (s, 1H), 4.02 (t, *J* = 6.0 Hz, 2H), 3.48 (m, 1H), 3.43 (t, *J* = 6.0 Hz, 2H), 3.26 (s, 3H), 2.92 (m, 2H), 2.78 (s, 3H), 2.29 (m, 2H), 2.26 (s, 2H), 1.27 (d, *J* = 2.4 Hz, 2H), 0.86 (m, 2H). |
| TB-131 | 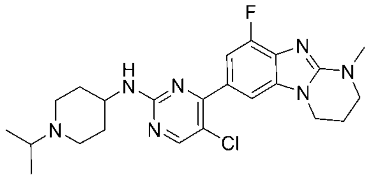 5-chloro-4-(9-fluoro-1-methyl-1,2,3,4-tetrahydrobenzo[4,5]imidazo[1,2-*a*]pyrimidin-7-yl)-*N*-(1-isopropylpiperidin-4-yl)pyrimidin-2-amine | $[M+H]^+$= 458.2 | $^1H$ NMR (400 MHz, Chloroform-d) $\delta$ 8.26 (s, 1H), 7.45 (d, *J* = 12.0 Hz, 1H), 7.41 (s, 1H), 5.31 (s, 1H), 4.09 (m, 1H), 4.03 (t, *J* = 6.0 Hz, 2H), 3.43 (t, *J* = 5.6 Hz, 2H), 3.40 (m, 2H), 3.27 (s, 3H), 2.89 (m, 2H), 2.36 (m, 2H), 2.28 (d, *J* = 5.8 Hz, 2H), 1.43 (d, *J* = 6.8 Hz, 6H), 0.86 (m, 2H). |
| TB-132 | 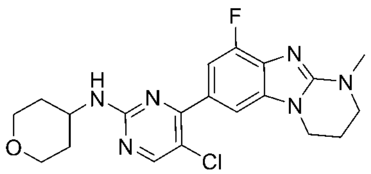 5-chloro-4-(9-fluoro-1-methyl-1,2,3,4-tetrahydrobenzo[4,5]imidazo[1,2-*a*]pyrimidin-7-yl)-*N*-(tetrahydro-2*H*-pyran-4-yl)pyrimidin-2-amine | $[M+H]^+$= 417.3 | $^1H$ NMR (400 MHz, Chloroform-*d*) $\delta$ 8.27 (s, 1H), 7.44 (d, *J* = 11.8 Hz, 1H), 7.39 (s, 1H), 5.11 (d, *J* = 7.9 Hz, 1H), 4.10 - 4.05 (m, 1H), 4.03 (d, *J* = 6.2 Hz, 2H), 3.98 (d, *J* = 3.7 Hz, 2H), 3.55 (td, *J* = 11.6, 2.2 Hz, 2H), 3.46 - 3.39 (m, 2H), 3.27 (s, 3H), 2.29 (p, *J* = 6.0 Hz, 2H), 2.08 - 2.02 (m, 2H), 1.62 - 1.50 (m, 2H). |
| TB-134 | Racemic 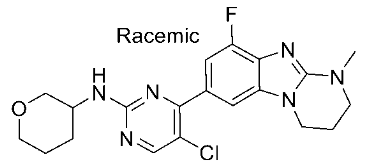 5-chloro-4-(9-fluoro-1-methyl-1,2,3,4-tetrahydrobenzo[4,5]imidazo[1,2-*a*]pyrimidin-7-yl)-*N*-(tetrahydro-2*H*-pyran-3-yl)pyrimidin-2-amine | $[M+H]^+$= 417.3 | $^1H$ NMR (400 MHz, Chloroform-*d*) $\delta$ 8.27 (s, 1H), 7.45 (d, *J* = 12.0 Hz, 1H), 7.39 (s, 1H), 5.38 (d, *J* = 8.0 Hz, 1H), 4.07 (s, 1H), 4.03 (t, *J* = 6.1 Hz, 2H), 3.94 (dd, *J* = 11.4, 3.2 Hz, 1H), 3.75 - 3.60 (m, 2H), 3.52 - 3.45 (m, 1H), 3.43 (t, *J* = 6.1 Hz, 2H), 3.27 (s, 3H), 2.37 - 2.22 (m, 2H), 1.99 (m, 1H), 1.85 - 1.78 (m, 1H), 1.72 - 1.63 (m, 2H). |

(continued)

| Comp. | Structure | LC-MS | NMR |
|---|---|---|---|
| TB-143 | 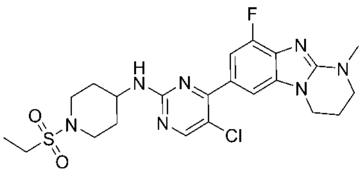 5-chloro-*N*-(1-(ethylsulfonyl)piperidin-4-yl)-4-(9-fluoro-1-methyl-1,2,3,4-tetrahydrobenzo[4,5]imidazo[1,2-*a*]pyrimidin-7-yl)pyrimidin-2-amine | $[M+H]^+$= 508.4 | $^1$H NMR (400 MHz, Chloroform-d) $\delta$ 8.27 (s, 1H), 7.44 (dd, *J* = 12.0, 1.2 Hz, 1H), 7.39 (s, 1H), 5.14 (d, *J* = 7.0 Hz, 1H), 4.02 (t, *J* = 6.0 Hz, 2H), 4.00 - 3.93 (m, 1H), 3.78 (dt, *J* = 12.0, 3.2 Hz, 2H), 3.43 (t, *J* = 5.6 Hz, 2H), 3.27 (s, 3H), 3.07 - 3.00 (m, 2H), 2.97 (q, *J* = 7.4, 2H), 2.34 - 2.25 (m, 2H), 2.16 (dd, *J* = 13.1, 3.7 Hz, 2H), 1.61 (td, *J* = 10.7, 6.9 Hz, 2H), 1.38 (t, *J* = 7.4 Hz, 3H). |
| TB-144 | 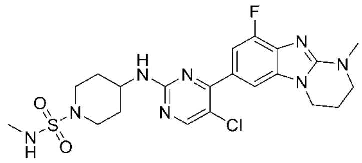 4-((5-chloro-4-(9-fluoro-1-methyl-1,2,3,4-tetrahydrobenzo[4,5]imidazo[1,2-*a*]pyrimidin-7-yl)pyrimidin-2-yl)amino)-*N*-methylpiperidine-1-sulfonamide | $[M+H]^+$= 509.2 | $^1$H NMR (400 MHz, Chloroform-d) $\delta$ 8.27 (s, 1H), 7.44 (d, *J* = 12.0 Hz, 1H), 7.39 (s, 1H), 5.22 (d, *J* = 8.0 Hz, 1H), 4.34 (m, 1H), 4.02 (t, *J* = 6.0 Hz, 2H), 4.00 - 3.89 (m, 1H), 3.68 (dt, *J* = 3.2 Hz, 12.5 Hz, 2H), 3.43 (t, *J*= 5.6 Hz, 2H), 3.27 (s, 3H), 3.01 (td, *J* = 12.0, 2.8 Hz, 2H), 2.71 (d, *J* = 5.2 Hz, 3H), 2.29 (p, *J* = 5.6 Hz, 2H), 2.13 (dd, *J* = 12.0, 4.0 Hz, 2H), 1.60 (ddd, *J* = 14.5, 10.7, 5.4 Hz, 2H). |
| TB-149(or TA-11) | 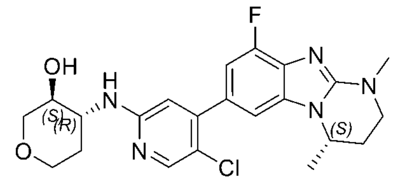 | [M+H]+ =446.2 | |
| TB-150 | 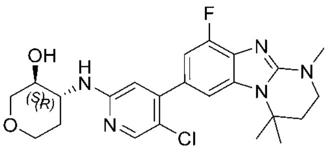 | [M+H]+ =460.3 | |
| TB-153 | 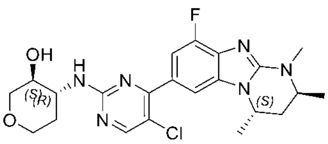 | [M+H]+ =461.2 | |
| TB-154 | 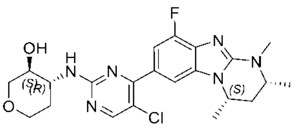 | [M+H]+ =461.2 | |

(continued)

| Comp. | Structure | LC-MS | NMR |
|---|---|---|---|
| TA-2 | 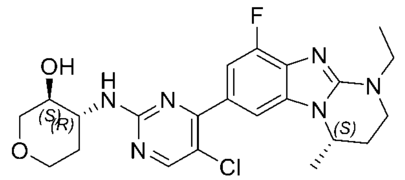 | [M+H]+ =447.3 | $^1$H NMR (400 MHz, Chloroform-d) $\delta$ 8.25 (s, 1H), 7.41 (dd, J = 11.8, 1.5 Hz, 1H), 7.32 (d, J = 1.5 Hz, 1H), 5.27 (d, J = 5.9 Hz, 1H), 4.01 (m, 4H), 3.89 - 3.78 (m, 1H), 3.74 (q, J = 7.1 Hz, 2H), 3.62 (td, J = 9.5, 4.9 Hz, 1H), 3.48 (d, J = 2.2 Hz, 1H), 3.43 (t, J = 5.8 Hz, 2H), 3.18 (t, J = 9.9 Hz, 1H), 2.26 (p, J = 6.0 Hz, 2H), 2.09 - 1.99 (m, 1H), 1.68 (m, 2H), 1.27 (t, J = 7.2 Hz, 3H). |
| TA-3 | 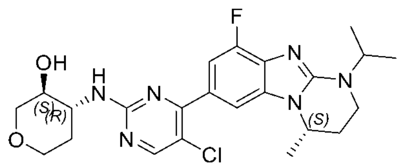 | [M+H]+ =461.3 | $^1$H NMR (400 MHz, Chloroform-d) $\delta$ 8.19 (s, 1H), 7.35 (d, J = 12.0 Hz, 1H), 7.26 (s, 1H), 5.22 (d, J = 4.0 Hz, 1H), 4.91 (p, J = 6.8 Hz, 1H), 3.95 (dt, J = 20.0, 6.0 Hz, 4H), 3.75 (tt, J = 10.0, 4.0 Hz, 1H), 3.55 (td, J = 8.0, 4.0 Hz, 1H), 3.39 (td, J = 12.0, 4.0 Hz, 1H), 3.29 (t, J = 6.0 Hz, 2H), 3.11 (t, J = 10.0 Hz, 1H), 2.15 (p, J = 6.0 Hz, 2H), 2.01 - 1.92 (m, 1H), 1.59 (d, J = 6.0 Hz, 1H), 1.18 (d, J = 8.0 Hz, 6H). |
| TA-4 | 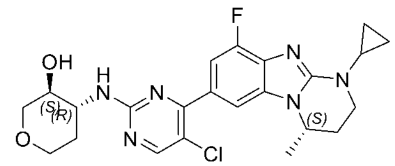 | [M+H]+ = 473.4 | $^1$H NMR (400 MHz, DMSO-d6) $\delta$ 8.02 (s, 1H), 7.12 (d, J = 1.4 Hz, 1H), 6.89 (dd, J = 11.7, 1.4 Hz, 1H), 6.76 (d, J = 7.3 Hz, 1H), 6.59 (s, 1H), 5.06 (d, J = 5.3 Hz, 1H), 4.70 - 4.53 (m, 1H), 3.78 (m, 3H), 3.52 (td, J = 12.3, 3.1 Hz, 1H), 3.40 (dd, J = 9.6, 4.9 Hz, 1H), 3.30 (dt, J = 11.9, 3.8 Hz, 1H), 3.12 (s, 3H), 3.07 (t, J = 11.1 Hz, 1H), 2.26 (m, 1H), 2.04 - 1.95 (m, 2H), 1.38 (d, J = 6.6 Hz, 3H). |
| TA-5 | 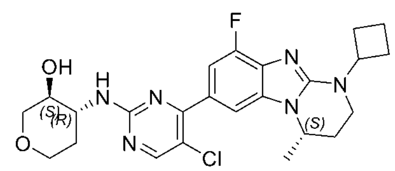 | [M+H]+ = 487.2 | |
| TA-6 | 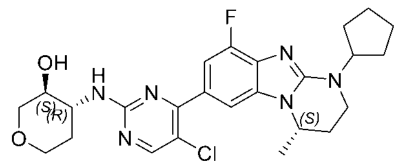 | [M+H]+ = 501.2 | $^1$H NMR (400 MHz, Chloroform-d) $\delta$ 8.26 (s, 1H), 7.41 (dd, J = 11.7, 1.5 Hz, 1H), 7.36 (d, J = 1.5 Hz, 1H), 5.28 (d, J = 5.8 Hz, 1H), 5.13 (p, J = 8.2 Hz, 1H), 4.51 (m, 1H), 4.05 (dd, J = 11.4, 5.0 Hz, 1H), 3.98 (dd, J = 11.6, 4.6 Hz, 1H), 3.81 (m, 1H), 3.62 (td, J = 9.5, 4.9 Hz, 1H), 3.44 (m, 2H), 3.35 (m, 1H), 3.21 - 3.12 (m, 1H), 2.24 (m, 1H), 2.06 - 1.96 (m, 4H), 1.74 - 1.65 (m, 8H), 1.50 (d, J = 6.6 Hz, 3H). |

(continued)

| Comp. | Structure | LC-MS | NMR |
|---|---|---|---|
| TA-7 | 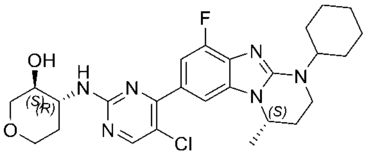 | [M+H]+ = 515.3 | |
| TA-8 | 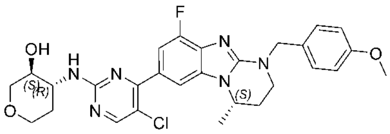 | [M+H]+ = 553.4 | $^1$H NMR (400 MHz, Chloroform-d) δ 8.26 (s, 1H), 7.42 (dd, J = 11.7, 1.5 Hz, 1H), 7.37 (d, J = 1.5 Hz, 1H), 7.27 (d, J = 7.1 Hz, 2H), 6.85 (d, J = 8.7 Hz, 2H), 5.33 (d, J = 5.8 Hz, 1H), 4.84 (q, J = 21.8, 2H), 4.52 (m, 1H), 4.04 (dd, J = 11.4, 4.9 Hz, 1H), 3.97 (dd, J = 11.7, 4.6 Hz, 1H), 3.86 - 3.81 (m, 1H), 3.79 (s, 3H), 3.62 (td, J = 9.5, 4.9 Hz, 1H), 3.48 - 3.34 (m, 2H), 3.18 (m, 2H), 2.37 (t, J = 8.1 Hz, 1H), 2.27 (m, 1H), 2.01 (m, 1H), 1.92 (dq, J = 13.9, 2.9 Hz, 1H), 1.69 (qd, J = 12.2, 4.8 Hz, 1H), 1.46 (d, J = 6.6 Hz, 3H). |
| TA-9 | 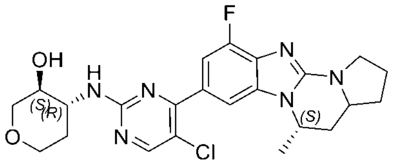 | [M+H]+ = 473.2 | |
| TA-10 | 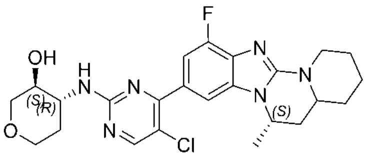 | [M+H]+ = 487.3 | |
| TA-12 | 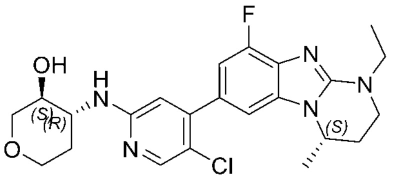 | [M+H]+ =460.2 | |
| TA-13 | 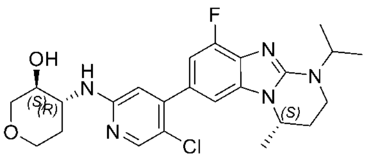 | [M+H]+ =474.2 | |
| TA-14 | 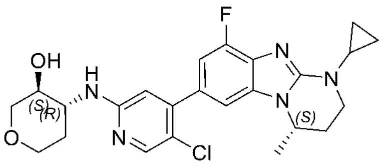 | [M+H]+ =472.3 | |
| TA-15 | 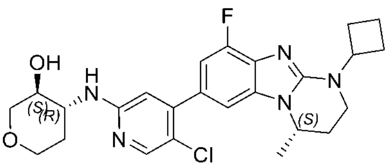 | [M+H]+ =486.2 | |

(continued)

| Comp. | Structure | LC-MS | NMR |
|---|---|---|---|
| TA-16 | 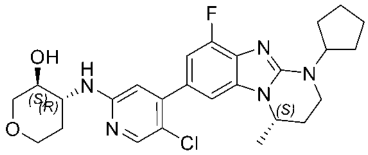 | [M+H]+ =500.1 | |
| TA-17 | 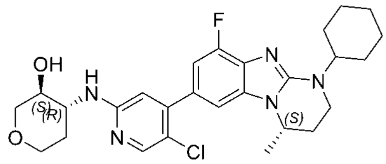 | [M+H]+ =514.2 | |
| TA-18 | 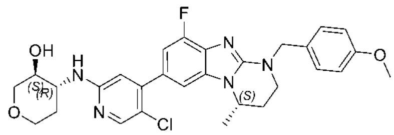 | [M+H]+ =552.3 | |
| TA-19 | 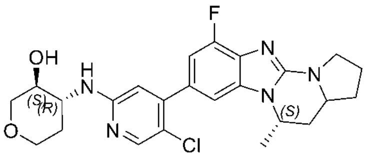 | [M+H]+ =472.2 | |
| TA-20 | 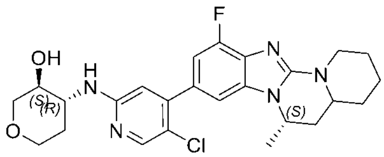 | [M+H]+ =486.3 | |
| TA-21 | 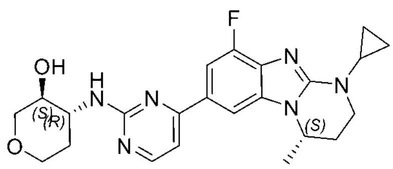 | [M+H]+ =439.2 | |
| TA-22 | 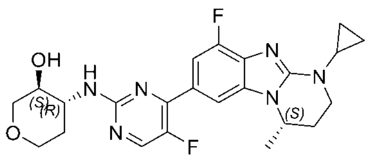 | [M+H]+ =457.2 | |
| TA-11 | 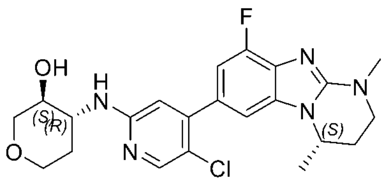 | [M+H]+ =446.3 | $^1$H NMR (400 MHz, Chloroform-d) δ 7.97 (s, 1H), 6.89 (d, J = 1.5 Hz, 1H), 6.81 (dd, J = 11.3, 1.4 Hz, 1H), 6.45 (s, 1H), 4.52 (d, J = 5.1 Hz, 1H), 4.47 - 4.38 (m, 1H), 4.02 (dd, J = 11.6, 5.4 Hz, 1H), 3.92 (dd, J = 11.8, 4.7 Hz, 1H), 3.67 (tt, J = 9.5, 4.7 Hz, 1H), 3.54 - 3.44 (m, 2H), 3.38 (td, J = 12.0, 2.2 Hz, 1H), 3.20 (s, 4H), 3.13 (dd, J = 11.4, 10.0 Hz, 1H), 2.32 (tt, J = 12.9, 5.2 Hz, 1H), 1.95 - 1.88 (m, 2H), 1.63 (qd, J = 12.2, 4.8 Hz, 2H), 1.41 (d, J = 6.6 Hz, 3H). |

(continued)

| Comp. | Structure | LC-MS | NMR |
|---|---|---|---|
| TA-29 | 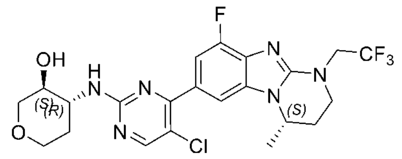 | [M+H]+ =515.3 | |
| TA-30 | 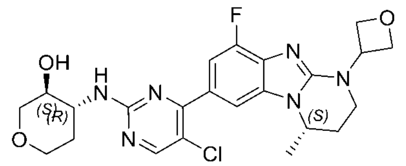 | [M+H]+ =489.2 | |
| TA-31 | 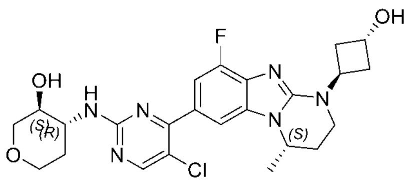 | [M+H]+ =503.3 | |
| TA-32 | 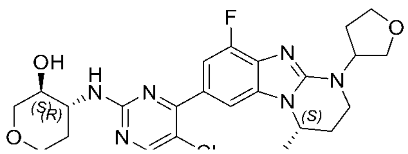 | [M+H]+ =503.2 | |
| TA-33 | 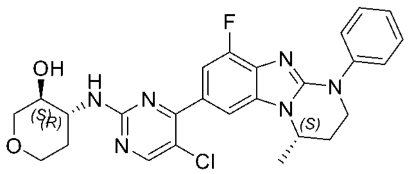 | [M+H]+ =509.1 | |
| TA-34 | 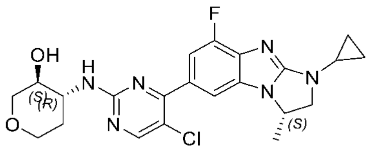 | [M+H]+ =459.3 | |
| TA-35 | 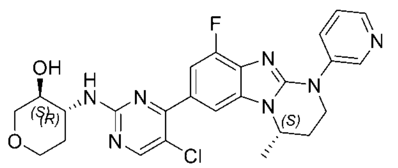 | [M+H]$^+$= 510.2 | |
| TC-1 | 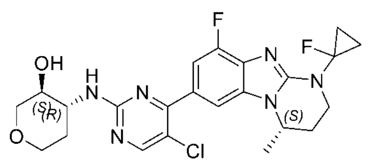 | [M+H]+ =491.2 | |
| TA-36 | 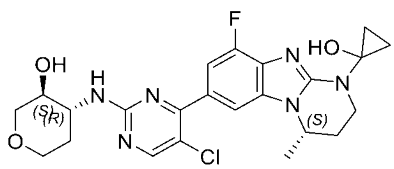 | [M+H]+ =489.3 | |

(continued)

| Comp. | Structure | LC-MS | NMR |
|---|---|---|---|
| TA-37 | 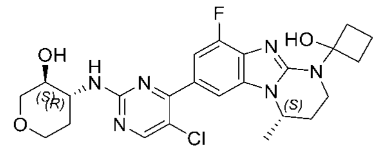 | [M+H]+ =503.4 | |
| TA-38 | 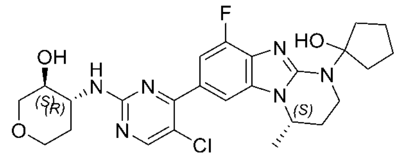 | [M+H]+ =517.3 | |
| TA-39 | 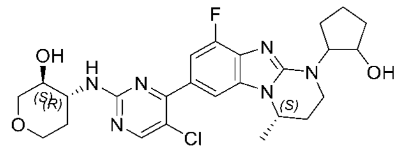 | [M+H]+ =517.2 | |
| TA-40 | 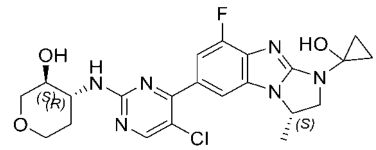 | [M+H]+ =475.2 | |
| TA-41 | 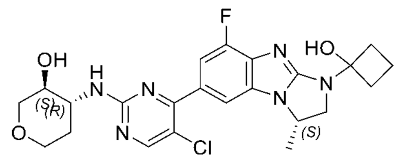 | [M+H]+ =489.2 | |
| TA-42 | 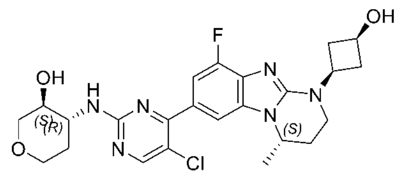 | [M+H]+ =503.2 | |
| TA-43 | 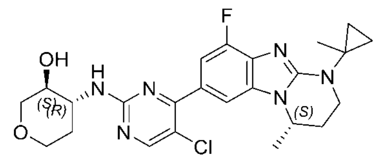 | [M+H]+ =487.1 | |
| TA-44 | 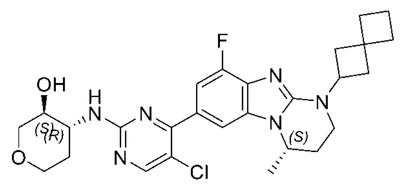 | [M+H]+ =527.2 | |
| TA-45 | 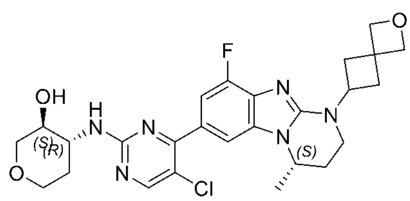 | [M+H]+ =529.3 | |

(continued)

| Comp. | Structure | LC-MS | NMR |
|---|---|---|---|
| TA-46 | 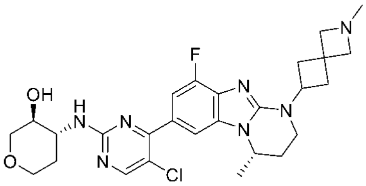 | [M+H]+ =542.1 | |
| TA-47 | 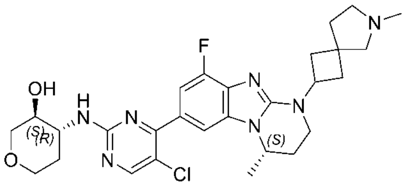 | [M+H]+ =556.3 | |
| TA-48 | 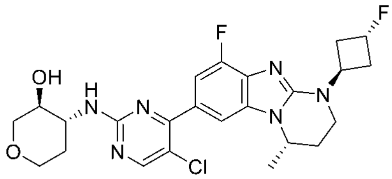 | [M+H]+ =505.3 | |
| TA-49 | 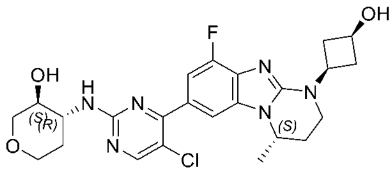 | [M+H]+ =503.2 | |
| TA-50 | 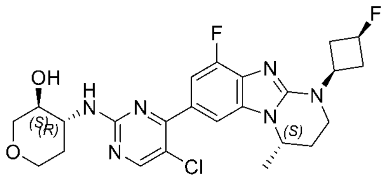 | [M+H]+ =505.3 | |
| TA-51 | 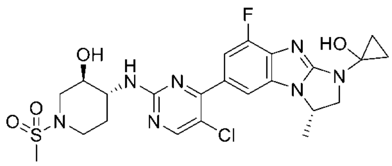 | [M+H]+ =552.1 | |
| TA-52 | 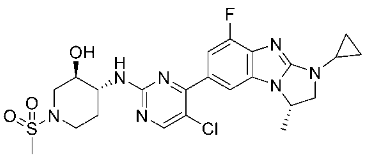 | [M+H]+ =536.3 | |
| TA-53 | 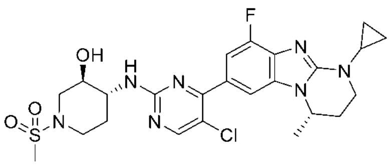 | [M+H]+ =550.1 | |
| TA-54 | 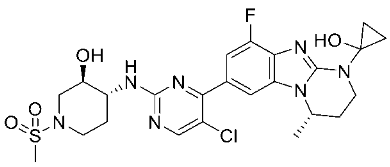 | [M+H]+ =566.1 | |

(continued)

| Comp. | Structure | LC-MS | NMR |
|---|---|---|---|
| TA-55 | 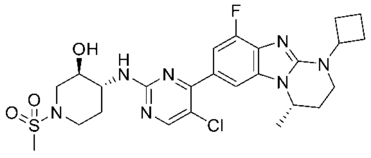 | [M+H]+ =564.2 | |
| TA-56 | 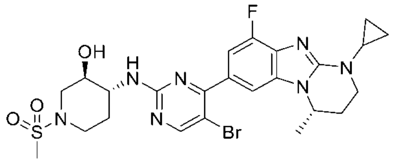 | [M+H]+ =594.2 | |
| TA-57 | 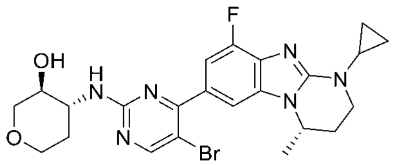 | [M+H]+ =517.2 | |
| TC-2 | 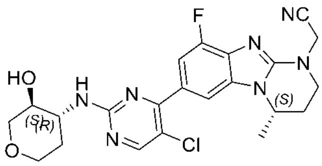 | [M+H]+ =472.4 | $^{1}$H NMR (400 MHz, Chloroform-d) δ 8.23 (s, 1H), 7.41 - 7.34 (m, 2H), 5.33 (d, J = 5.9 Hz, 1H), 4.81 (d, J = 17.6 Hz, 1H), 4.57 - 4.50 (m, 1H), 4.44 (d, J = 17.5 Hz, 1H), 3.99 (dd, J = 11.4, 4.9 Hz, 1H), 3.92 (dd, J = 11.9, 4.5 Hz, 1H), 3.78 (tt, J = 10.9, 5.2 Hz, 1H), 3.68 - 3.60 (m, 1H), 3.56 (dt, J = 9.3, 4.8 Hz, 1H), 3.44 - 3.34 (m, 2H), 3.11 (dd, J = 11.4, 9.9 Hz, 1H), 2.50 - 2.37 (m, 1H), 2.06 (dq, J = 14.1, 3.0 Hz, 1H), 2.00 - 1.94 (m, 1H), 1.65 (tt, J = 12.1, 6.1 Hz, 2H), 1.49 (d, J = 6.6 Hz, 3H). |
| TC-6 | 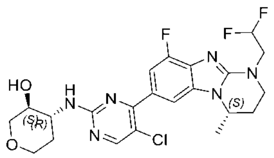 | [M+H]+ =497.2 | |
| TC-7 | 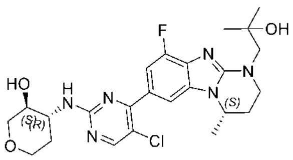 | [M+H]+ =505.2 | |
| TC-9 | 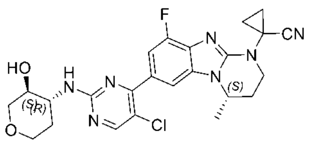 | [M+H]+ =498.2 | |

(continued)

| Comp. | Structure | LC-MS | NMR |
|---|---|---|---|
| TC-11 | 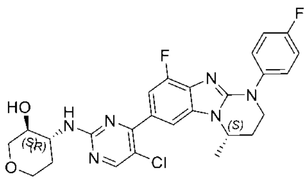 | [M+H]+ =527.3 | $^1$H NMR (400 MHz, Chloroform-d) δ 8.28 (s, 1H), 7.53 (dd, J = 8.8, 4.8 Hz, 2H), 7.45 (s, 1H), 7.42 (d, J = 11.8 Hz, 1H), 7.11 (t, J = 8.5 Hz, 2H), 5.29 (d, J = 5.9 Hz, 1H), 5.10 (s, 1H), 4.66 (m, 1H), 4.06 (m, 2H), 3.98 (dd, J = 11.8, 4.4 Hz, 1H), 3.84 (m, 1H), 3.75 (dt, J = 12.1, 3.8 Hz, 1H), 3.62 (m, 1H), 3.45 (t, J = 11.6 Hz, 1H), 3.17 (t, J = 10.6 Hz, 1H), 2.54 (m, 1H), 2.19 - 2.09 (m, 1H), 2.04 (m, 1H), 1.62 (d, J = 6.6 Hz, 3H). |
| TC-12 | 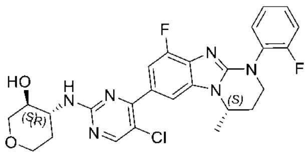 | [M+H]+ =527.3 | $^1$H NMR (400 MHz, Chloroform-d) δ 8.28 (s, 1H), 7.58 (td, J = 7.8, 1.8 Hz, 1H), 7.47 (d, J = 1.5 Hz, 1H), 7.41 (dd, J = 11.6, 1.4 Hz, 1H), 7.30 (dq, J = 7.8, 2.7, 2.3 Hz, 1H), 7.21 (dddd, J = 12.4, 10.0, 7.9, 1.6 Hz, 2H), 5.28 (d, J = 5.8 Hz, 1H), 4.67 (t, J = 6.5 Hz, 1H), 4.09 - 4.05 (m, 1H), 4.05 - 4.00 (m, 1H), 4.00 - 3.95 (m, 1H), 3.83 (tt, J = 10.7, 5.1 Hz, 1H), 3.71 - 3.65 (m, 1H), 3.62 (dd, J = 9.4, 4.9 Hz, 1H), 3.46 (td, J = 11.9, 2.2 Hz, 1H), 3.23 - 3.13 (m, 1H), 2.57 (ddt, J = 13.2, 10.0, 5.0 Hz, 1H), 2.11 (dq, J = 14.0, 2.9 Hz, 1H), 2.07 - 2.00 (m, 1H), 1.72 (td, J = 12.3, 4.8 Hz, 2H), 1.64 (d, J = 6.6 Hz, 3H). |
| TC-13 | 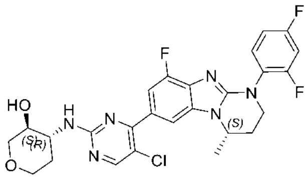 | [M+H]+ =545.5 | $^1$H NMR (400 MHz, Chloroform-d) δ 8.28 (s, 1H), 7.54 (m, 1H), 7.46 (s, 1H), 7.40 (dd, J = 11.6, 1.4 Hz, 1H), 6.95 (m, 2H), 5.29 (d, J = 5.8 Hz, 1H), 5.15 (brs, 1H), 4.67 (m, 1H), 4.08 - 4.03 (m, 1H), 4.03 - 3.95 (m, 2H), 3.89 - 3.76 (m, 1H), 3.68 - 3.57 (m, 2H), 3.45 (td, J = 11.9, 2.2 Hz, 1H), 3.17 (t, J = 10.9, 1H), 2.56 (m, 1H), 2.11 (dd, J = 13.9, 2.5 Hz, 1H), 2.04 - 2.01 (m, 1H), 1.73 (dd, J = 12.1, 4.9 Hz, 1H), 1.63 (d, J = 6.6 Hz, 3H). |
| TC-14 | 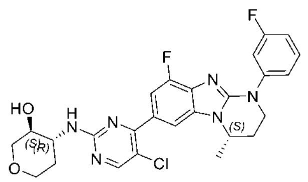 | [M+H]+ =527.3 | |
| TC-15 | 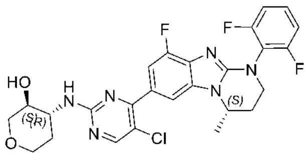 | [M+H]+ =545.1 | |

(continued)

| Comp. | Structure | LC-MS | NMR |
|---|---|---|---|
| TC-16 | 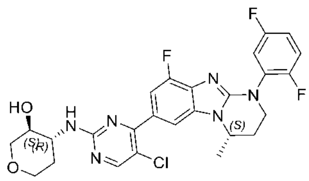 | [M+H]+ =545.3 | $^1$H NMR (400 MHz, Chloroform-d) δ 8.29 (s, 1H), 7.47 (s, 1H), 7.42 (d, J = 11.6 Hz, 1H), 7.35 (m, 1H), 7.14 (td, J = 9.6, 4.9 Hz, 1H), 7.04 - 6.92 (m, 1H), 5.30 (d, J = 5.9 Hz, 1H), 5.10 (brs, 1H), 4.68 (m, 1H), 4.02 (m, 3H), 3.83 (m, 1H), 3.65 (m, 2H), 3.46 (td, J = 11.8, 2.2 Hz, 1H), 3.18 (t, J = 11.3, 1H), 2.56 (m, 1H), 2.11 (dq, J = 13.9, 2.9 Hz, 1H), 2.05 (dt, J = 9.1, 2.8 Hz, 1H), 1.74 (dd, J = 12.1, 4.8 Hz, 1H), 1.64 (d, J = 6.9 Hz, 3H). |
| TC-17 | 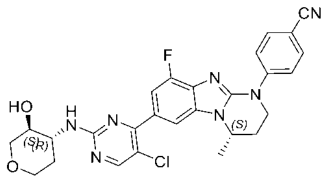 | [M+H]+ =534.3 | |
| TC-18 | 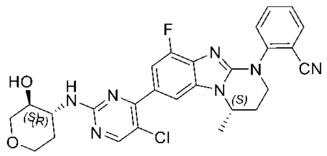 | [M+H]+ =534.3 | $^1$H NMR (400 MHz, Chloroform-d) δ 8.29 (s, 1H), 7.74 (d, J = 7.1 Hz, 1H), 7.70 - 7.65 (m, 2H), 7.49 (d, J = 1.4 Hz, 1H), 7.45 - 7.38 (m, 2H), 5.32 (d, J = 5.9 Hz, 1H), 5.10 (s, 1H), 4.70 (tt, J = 7.5, 5.0 Hz, 1H), 4.16 - 4.09 (m, 1H), 4.05 (dd, J = 11.4, 4.9 Hz, 1H), 3.98 (dd, J = 11.7, 4.5 Hz, 1H), 3.89 - 3.74 (m, 2H), 3.63 (dt, J = 9.4, 4.7 Hz, 1H), 3.45 (td, J = 11.9, 2.2 Hz, 1H), 3.17 (dd, J = 11.4, 9.9 Hz, 1H), 2.64 (tt, J = 13.1, 4.9 Hz, 1H), 2.16 (dd, J = 14.0, 2.7 Hz, 1H), 2.04 (q, J = 6.0, 4.7 Hz, 1H), 1.76 - 1.71 (m, 1H), 1.66 (d, J = 6.6 Hz, 3H). |

**Example B6**

Compound Synthesized in the Present Invention:

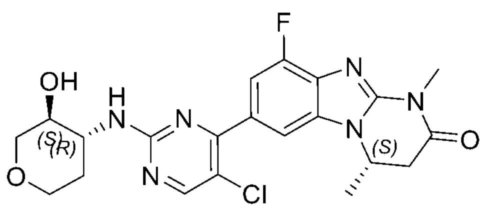

TB-98

The synthetic route was as follows:

Synthesis of Compound TB-98

The synthetic route was as follows:

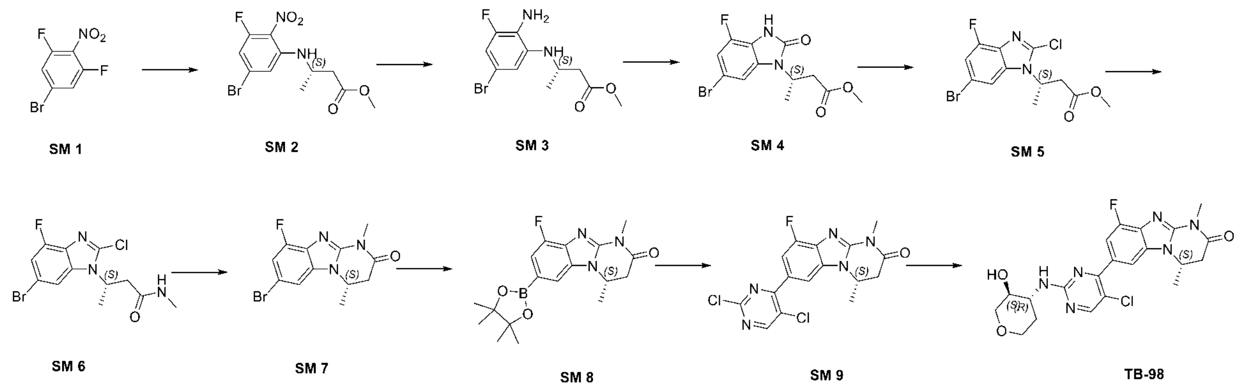

SM 1 SM 2 SM 3 SM 4 SM 5

SM 6 SM 7 SM 8 SM 9 TB-98

The experimental procedure was as follows:

Step 1

Compound SM 1 (7.7 g, 1.0 eq.) was dissolved in ultra-dry tetrahydrofuran. The mixture was cooled in an ice-water bath, and cesium carbonate solid (31.8 g, 3.0 eq.) was added, followed by the addition of methyl (S)-3-aminobutyrate hydrochloride (5.0 g, 1.0 eq.) under stirring. After the completion of addition, the mixture was allowed to warm to room temperature naturally. After the reaction was completed as monitored by TLC, the solid was removed by filtration, and the filter cake was washed with an appropriate amount of EA. Water was added for extraction and layer separation. The organic phase was dried over anhydrous sodium sulfate, filtered, concentrated under reduced pressure, separated and purified by column chromatography to afford SM 2 as a yellow solid (6.7 g, 61.5%), LCMS: $[M+H]^+$ = 335.2, 337.2.

Step 2

Compound SM **2** (6.7 g, 1.0 eq.) was dissolved in a mixture of methanol and water (v:v = 4:1). Iron powder (6.7 g, 6.0 eq.) and ammonium chloride (2.1 g, 2.0 eq.) were added, and the mixture was heated to reflux and reacted. After the reaction was completed as monitored by TLC, the mixture was cooled to room temperature and filtered. The filter cake was washed with an appropriate amount of EA. The filtrate was concentrated under reduced pressure to remove most of the solvent, then extracted with saturated sodium bicarbonate solution and EA for layer separation. The organic phase was dried over anhydrous sodium sulfate, filtered, concentrated under reduced pressure, separated and purified by column chromatography to obtain compound SM **3** (5.5 g, 90.0%), LCMS: $[M+H]^+$ = 305.1, 307.1.

Step 3

Compound SM **3** (5.5 g, 1.0 eq.) and CDI (4.4 g, 1.5 eq.) were successively dissolved in ultra-dry DMF to form a clear solution. The mixture was heated to 50 °C and reacted. After the reaction was completed as monitored by TLC, the mixture was cooled to room temperature, extracted with EA and water for layer separation. The organic phase was dried over anhydrous sodium sulfate, filtered, concentrated under reduced pressure, separated and purified by column chromatography to obtain compound SM **4** (5.4 g, 90.4%), LCMS: $[M+H]^+$ = 331.2, 333.1.

Step 4

Compound SM **4** (5.4 g, 1.0 eq.) was dissolved in phosphorus oxychloride (18.2 mL, 12.0 eq.). The mixture was heated to 100 °C and reacted for 12 hours. After the reaction was completed as monitored by TLC, the mixture was cooled to room temperature, poured into ice water to quench, and extracted with EA for layer separation. The organic phase was dried over anhydrous sodium sulfate, filtered, concentrated under reduced pressure, separated and purified by column chromatography to obtain compound SM 5 (5.2 g, 91.2%), LCMS: $[M+H]^+$ = 349.0, 351.1.

Step 5

Compound SM **5** (1.9 g, 1.0 eq.) was added to 50 mL of 30% methylamine in ethanol, and the reaction was carried out in an ice-water bath. After completion of the reaction, most solvent was removed by concentration under reduced pressure. The mixture was extracted with saturated brine and EA for layer separation. The organic phase was dried over anhydrous sodium sulfate, filtered, concentrated, separated and purified by silica gel column chromatography to obtain compound SM **6** (1.7 g, 89.4%), LCMS: $[M+H]^+$ = 348.1, 350.1.

Step 6

Compound SM 6 (1.5 g, 1.0 eq.) was dissolved in 50 mL of ultra-dry tetrahydrofuran to form a clear solution, cooled in an ice-water bath, and 60% NaH powder was added in batches. After the completion of addition, the temperature was maintained and the reaction was carried out for 3 hours. After the reaction was completed, the mixture was cooled to 0 °C, quenched with an appropriate amount of ice water, and extracted with saturated brine and EA for layer separation. The organic phase was dried over anhydrous sodium sulfate, filtered, concentrated, separated and purified by silica gel column chromatography to obtain compound SM **7** (811 mg, 60.5%), LCMS: $[M+H]^+$ = 312.1, 314.0.

Step 7

Under nitrogen protection, SM 7 (811 mg, 1.0 eq.), bis(pinacolato)diboron (1.32 g, 2.0 eq.), Pd(dppf)$Cl_2$ (95.1 mg, 0.05 eq.), and potassium acetate (764.7 mg, 3.0 eq.) were successively added to a dry 50 mL three-necked flask. The flask was purged with nitrogen three times, ultra-dry 1,4-dioxane was added, and the mixture was heated to reflux and reacted. After the reaction was completed as monitored by TLC, the mixture was cooled to room temperature, extracted with Ea and water for layer separation. The organic phase was dried over anhydrous sodium sulfate, filtered, concentrated under reduced pressure, separated and purified by column chromatography to obtain compound SM **8** (890 mg, 95.4%), LCMS: $[M+H]^+$ = 360.3.

Step 7

Under nitrogen protection, SM **8** (150 mg, 1.0 eq.), 2,4,5-trichloropyrimidine (115 mg, 1.5 eq.), tetratriphenylphosphine palladium (48.3 mg, 0.1 eq.), and sodium carbonate solid (66.4 mg, 1.5 eq.) were added to a 50 mL three-necked flask. A mixture of 1,4-dioxane and water (v:v = 10:3) was added, the flask was purged with nitrogen three times, and the mixture was heated to reflux and reacted. After the reaction was completed as monitored by TLC, the mixture was cooled to room temperature and extracted with EA and water for layer separation. The organic phase was dried over anhydrous sodium sulfate, filtered, concentrated under reduced pressure, separated and purified by column chromatography to obtain compound SM **9** (120 mg, 75.5%), LCMS: $[M+H]^+$ = 380.1.

Step 8

Under nitrogen atmosphere, SM **9** (150 mg, 1.0 eq.), (3s,4r)-4-aminooxan-3-ol hydrochloride (121.2 mg, 2.0 eq.), and DIPEA (0.28 mL, 4.0 eq.) were added to NMP solvent. The mixture was heated to 90 °C and reacted. After the reaction was completed, the mixture was cooled to room temperature, extracted with saturated brine, water, and EA, successively for layer separation. The organic phase was dried over anhydrous sodium sulfate, filtered, concentrated under reduced pressure, separated and purified by preparative HPLC to obtain compound TB-98 (76 mg, 40.7%) with an HPLC purity of 99.38%, LCMS: $[M+H]^+$ = 461.3.

$^1$H NMR (400 MHz, Chloroform-*d*) $\delta$ 8.32 (s, 1H), 7.53 (s, 1H), 7.48 (d, *J*= 11.4 Hz, 1H), 5.32 (d, *J* = 6.0 Hz, 1H), 4.75 (p, *J* = 6.9 Hz, 1H), 4.05 (dd, *J* = 11.5, 5.0 Hz, 1H), 3.99 (dd, *J* = 11.7, 4.1 Hz, 1H), 3.90 - 3.80 (m, 1H), 3.67 (dt, *J* = 8.5, 4.5 Hz, 1H), 3.63 (d, *J* = 4.9 Hz, 1H), 3.60 (s, 3H), 3.46 (ddd, *J* = 14.1, 9.8, 2.2 Hz, 2H), 3.27 - 3.14 (m, 2H), 2.11 - 1.99 (m, 1H), 1.76 - 1.69 (m, 1H), 1.47 (d, *J*= 6.7 Hz, 3H).

The following compounds were synthesized by referring to the synthesis method of Example B6:

| Compound | Structure | LC-MS | NMR |
|---|---|---|---|
| TB-46 | 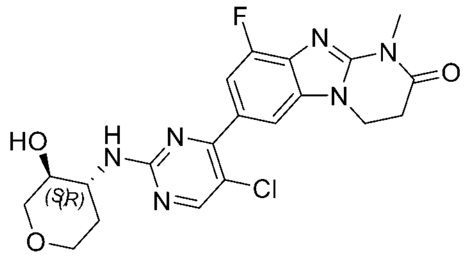 7-(5-chloro-2-(((3*S*,4*R*)-3-hydroxytetra hydro-2*H*-pyran-4-yl)amino)pyrimidin-4-yl)-9-fluoro-1-methyl-3,4-dihydrobenzo[4,5]imidazo [1,2-a]pyrimidin-2(1*H*)-one | $[M+H]^+$= 447.3 | $^1$H NMR (400 MHz, Chloroform-d) $\delta$ 8.31 (s, 1H), 7.51 (s, 1H), 7.48 (d, *J* = 12.0 Hz, 1H), 5.31 (d, *J* = 6.0 Hz, 1H), 4.81 (s, 1H), 4.28 (t, *J* = 7.2 Hz, 2H), 4.05 (dd, *J* = 12.0, 4.0 Hz, 1H), 4.02 - 3.94 (m, 1H), 3.85 (m, 1H), 3.69 - 3.59 (m, 2H), 3.59 (s, 3H), 3.46 (td, *J* = 12.0, 2.0 Hz, 1H), 3.23 - 3.14 (m, 1H), 3.09 (t, *J* = 7.2 Hz, 2H), 2.13 - 1.93 (m, 1H). |
| TB-47 | 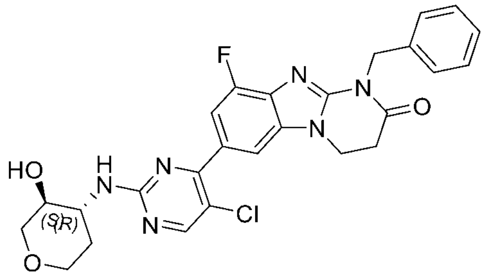 1-benzyl-7-(5-chloro-2-(((3*S*,4*R*)-3-hydroxytetrahydro-2*H*-pyran-4-yl)amino)pyrimidin-4-yl)-9-fluoro-3,4-dihydrobenzo[4,5]imidazo[1,2-a]pyrimidin-2(1*H*)-one | $[M+H]^+$= 523.8 | |
| TB-158 | 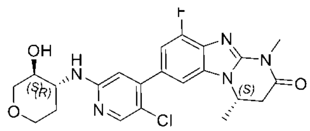 | $[M+H]^+$= 460.2 | |

**Example B7**

Compound Synthesized in the Present Invention:

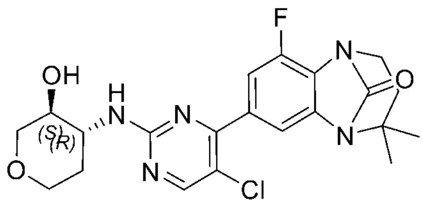

TB-164

The experimental procedure was as follows:

Synthesis of Compound TB-164

The synthetic route was as follows:

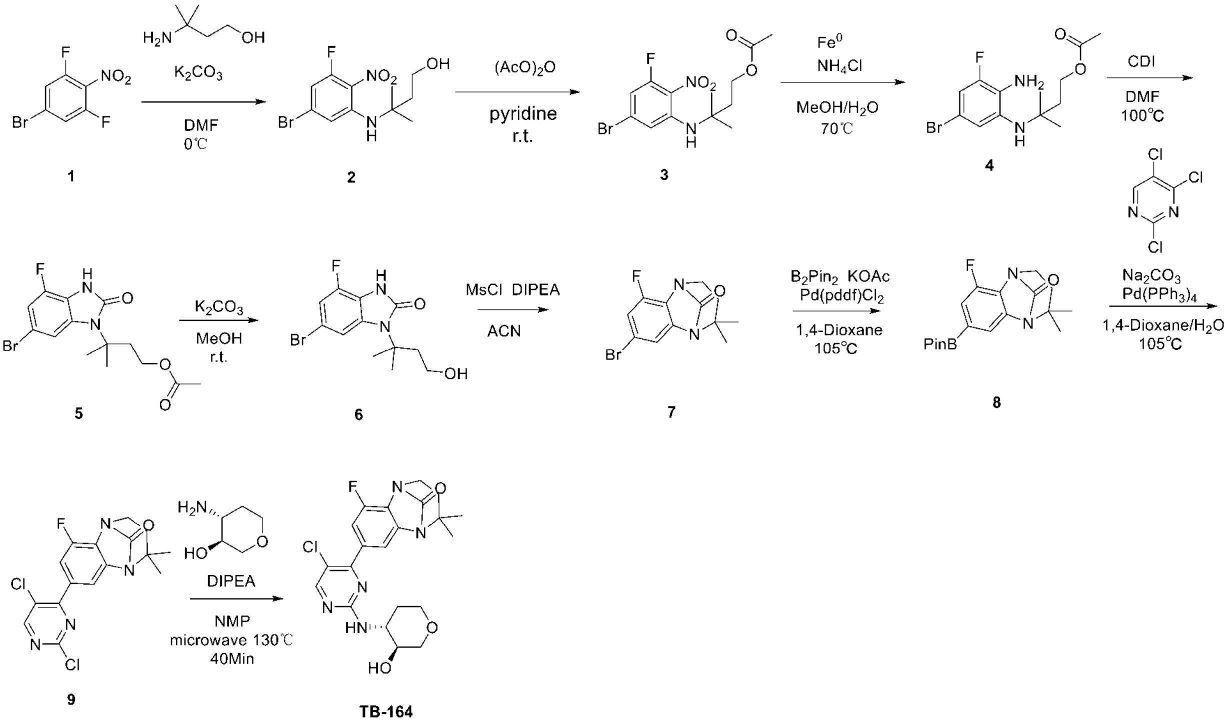


1 2 3 4 5 6 7 8 9 TB-164

The experimental procedure was as follows:

Step 1

SM **1** (11.5 g, 1.0 eq.) and potassium carbonate (13.4 g, 2.0 eq.) were added to a 500 mL three-necked flask. Under nitrogen protection, 100 mL of DMF was added. 3-Amino-3-methylbutan-1-ol (5 g, 1.0 eq.) was added dropwise at 0 °C in an ice-water bath. The flask was purged with nitrogen three times, and the mixture was allowed to warm to room temperature and reacted for 1-2 hours. When TLC indicated formation of a new spot with incomplete consumption of starting materials, the reaction was stopped. The mixture was filtered through Celite, washed with an appropriate amount of ethyl acetate, and the organic phase was concentrated directly. The residue was extracted with ethyl acetate and water. The organic phase was washed with brine several times, dried over anhydrous sodium sulfate, concentrated, separated and purified by silica gel column chromatography to obtain SM **2** as a yellow solid, yield: 60%.

Step 2

Compound SM **2** (9.3 g, 1.0 eq.) was added to a 100 mL three-necked flask, followed by the addition of 40 mL of pyridine. Acetic anhydride (8.2 mL, 3.0 eq.) was added dropwise, and the mixture was stirred at room temperature under nitrogen protection. TLC showed no starting material remained, the reaction was completed. The reaction solution was extracted with ethyl acetate and water. The organic phase was dried over anhydrous sodium sulfate, concentrated, separated and purified by silica gel column chromatography to obtain SM **3** as a yellow liquid, LCMS: $[M+Na]^+$ = 385.0, yield: 96%.

Step 3

Compound SM **3** (10.5 g, 1.0 eq.), reduced iron powder (9.8 g, 6.0 eq.), and $NH_4Cl$ (3.1 g, 2.0 eq.) were added to a 500 mL three-necked flask, followed by the addition of a mixture of methanol and water (v:v = 60 mL:30 mL). The mixture was heated to 70 °C for 1 hour under nitrogen protection. TLC showed no starting material remained, the reaction was completed. After cooled to room temperature, the mixture was filtered through Celite, washed with an appropriate amount of ethyl acetate. The organic phase was concentrated directly, extracted with ethyl acetate and water, dried over anhydrous sodium sulfate, concentrated, separated and purified by silica gel column chromatography to obtain SM **4** as a dark purple liquid, LCMS: $[M+H]^+$ = 333.1, $[M+Na]^+$ = 355.1, yield: 92%.

Step 4

SM 4 (8.8 g, 1.0 eq.) was added to a 250 mL three-necked flask. Under nitrogen protection, 80 mL of DMF and CDI (21.6 g, 5.0 eq.) were added. The flask was purged with nitrogen three times, and the mixture was heated to 100 °C and reacted for 1 hour. When TLC showed the spot of starting material disappeared, the reaction was completed. The mixture was extracted with ethyl acetate and water, the organic phase was washed with brine several times, dried over anhydrous sodium sulfate, concentrated, separated and purified by silica gel column chromatography to obtain SM 5 as a white solid, LCMS: $[M+H]^+$ = 359.10, $[M+Na]^+$ = 381.10, yield: 72%.

Step 5

Compound SM **5** (6.8 g, 1.0 eq.) and $K_2CO_3$ (2.6 g, 1.0 eq.) were added to a 250 mL three-necked flask, followed by the addition of 70 mL of methanol. The mixture was stirred at room temperature for 16 hours under nitrogen protection. TLC showed no starting material remained, the reaction was completed. The mixture was extracted with ethyl acetate and water. The organic phase was dried over anhydrous sodium sulfate, concentrated, separated and purified by silica gel column chromatography to obtain SM **6** as a white solid, yield: 98%.

Step 6

Compound SM **6** (5.9 g, 1.0 eq.) was added to a 250 mL three-necked flask, followed by the addition of 100 mL of acetonitrile and DIPEA (13.0 mL, 4.0 eq.). MsCl (4.4 mL, 1.5 eq.) in mixed solvent of methanol and water (v: v=60 mL: 30 mL) was added dropwise at 0 °C in an ice-water bath. The flask was purged with $N_2$ three times, and the mixture was allowed to warm to room temperature and reacted. When TLC showed no starting material remained, the reaction was completed. The organic phase was extracted with ethyl acetate and water. The organic phase was dried over anhydrous sodium sulfate, concentrated, separated and purified by silica gel column chromatography to afford SM **7** as a white solid, LCMS: $[M+H]^+$ = 299.10, yield: 60%.

Step 7

SM **7** (3.3 g, 1.0 eq.), $B_2Pin_2$ (5.6 g, 2.0 eq.), $Pd(dppf)Cl_2$ (404 mg, 0.05 eq.), and KOAc (3.3 g, 3.0 eq.) were added to a 250 mL three-necked flask. Under nitrogen protection, 60 mL of 1,4-dioxane was added, and the mixture was heated to 105 °C and reacted for 2 hours. When TLC showed the spot of starting material disappeared, the reaction was completed. The mixture was extracted with ethyl acetate and water. The organic phase was dried over anhydrous sodium sulfate, concentrated, separated and purified by silica gel column chromatography to afford SM **8** as a pale yellow solid, LCMS: $[M+H]^+$ = 347.40, yield: 95%.

Step 8

Compound SM **8** (3.6 g, 1.0 eq.), $Pd(PPh_3)_4$ (404 mg, 0.05 eq.), and $Na_2CO_3$ (3.3 g, 3.0 eq.) were added to a 250 mL three-necked flask in sequence. Under nitrogen protection, a mixed solvent of 1,4-dioxane and water (50 mL:15 mL) was added, followed by the addition of 2,4,6-trichloropyrimidine (1.78 mL, 1.5 eq.). The mixture was heated to 105 °C and reacted for 2 hours. When TLC showed no starting material remained, the reaction was completed. The mixture was extracted with ethyl acetate and water. The organic phase was dried over anhydrous sodium sulfate, concentrated, separated and purified by silica gel column chromatography to afford SM **9** as a white solid, LCMS: $[M+H]^+$ = 367.20, yield: 97%.

Step 9

Compound SM **9** (1.0 g, 1.0 eq.) and (3S,4R)-4-aminooxan-3-ol hydrochloride (840 mg, 2.0 eq.) were added to a microwave tube, followed by the addition of 10 mL of NMP and DIPEA (2.0 mL, 4.0 eq.). The mixture was heated to 130 °C and reacted for 1 hour. When TLC showed no starting material remained, the reaction was completed. The mixture was extracted with ethyl acetate and water. The organic phase was washed with brine several times, dried over anhydrous sodium sulfate, concentrated, separated and purified by silica gel column chromatography to afford TB-164 as a white solid, LCMS: $[M+H]^+$ = 448.40, yield: 38%.

$^1$H NMR (400 MHz, Chloroform-*d*) δ 8.31 (s, 1H), 7.70 (d, *J*= 1.4 Hz, 1H), 7.45 (d, *J* = 11.1 Hz, 1H), 5.39 (d, *J* = 6.0 Hz, 1H), 4.55 - 4.48 (m, 2H), 4.05 (dd, *J* = 11.3, 4.9 Hz, 1H), 4.02 - 3.95 (m, 1H), 3.85 (tt, *J* = 10.8, 5.4 Hz, 1H), 3.64 (td, *J*=9.5, 5.0 Hz, 1H), 3.46 (td, *J* = 11.9, 2.2 Hz, 1H), 3.17 (t, *J* = 10.6 Hz, 1H), 2.27 - 2.20 (m, 2H), 1.77 (s, 6H), 1.70 (td, *J* = 12.3, 4.8 Hz, 3H).

The following compounds were synthesized by referring to the synthesis method of Example B7:

| Compound No. | Structure | Characterization Data |
|---|---|---|
| TB-165 | 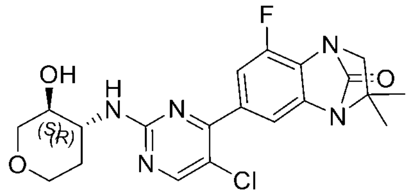 | $[M+H]^+$=434.3 |
| TB-146 | 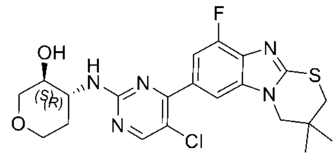 | $[M+H]^+$=464.1 |
| TB-148 | 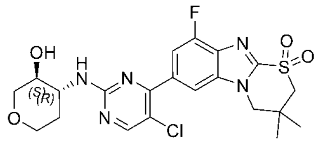 | $[M+H]^+$=496.1 |

**Example B8**

Compound Synthesized in the Present Invention:

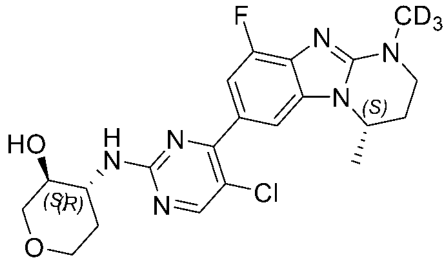

TA-28

The experimental procedure was as follows:

Synthesis of Compound TA-28

The synthetic route was as follows:

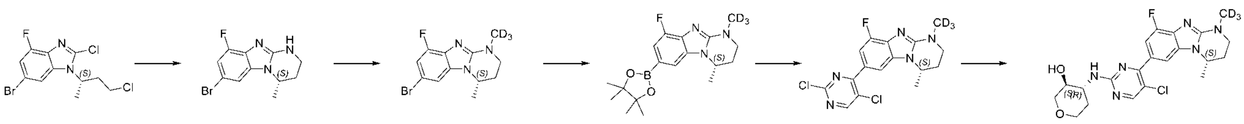

Int-3 Int-7 SM 1 SM 2 Int-8 TA-28

The experimental procedure was as follows:

Step 1

Int-**3** (1.0 g, 1.0 eq.), 2.0 M amino in ethanol (10 mL, 10.0 vol), sodium iodide (2.2 g, 5.0 eq.), and DMF (20 mL, 20 vol) were added to a 100 mL pressure vessel. The vessel was sealed and heated to 70 °C and reacted for 12 hours. After the reaction was completed as monitored by TLC, the mixture was cooled to room temperature, extracted with EA and

water for layer separation. The organic phase was washed with saturated brine three times, dried over sodium sulfate pentahydrate, filtered, concentrated under reduced pressure, separated and purified by silica gel column chromatography to afford target product Int-7 (400 mg, 47.8%), LCMS: $[M+H]^+$ = 284.1, 286.1.

Step 2

Under nitrogen protection, 60% NaH powder (35.6 mg, 1.1 eq.) was added to 15 mL of ultra-dry THF. The mixture was cooled in an ice-water bath, and Int-7 was added. After reacted for 30 minutes, a solution of deuterated iodomethane in THF (117 mg, 1.0 eq.) was added dropwise. After the addition, the temperature was maintained and the reaction was carried out for 2 hours. After the reaction was completed as monitored by TLC, the reaction was quenched with an appropriate amount of ice water and the mixture was extracted with saturated ammonium chloride solution and EA for layer separation. The organic phase was dried over anhydrous sodium sulfate, filtered, concentrated, separated and purified by silica gel column chromatography to obtain target compound SM **1** (200 mg, 82.3%), LCMS: $[M+H]^+$ = 301.2, 303.2.

Step 3

Under nitrogen protection, SM **1** (240 mg, 1.0 eq.), bis(pinacolato)diboron (406 mg, 2.0 eq.), $Pd(dppf)Cl_2$ (29 mg, 0.05 eq.), and potassium acetate (235 mg, 3.0 eq.) were added to a dry 100 mL three-necked flask. The flask was purged with nitrogen three times, 10 mL of ultra-dry 1,4-dioxane was added, and the mixture was heated to 110 °C and reacted. After the reaction was completed as monitored by TLC, the mixture was cooled to room temperature, extracted with EA and water for layer separation. The organic phase was dried over anhydrous sodium sulfate, filtered, concentrated under reduced pressure, separated and purified by column chromatography to obtain compound SM 2 (200 mg, 72.7%), LCMS: $[M+H]^+$ = 349.4.

Step 4

Under nitrogen protection, SM **2** (220 mg, 1.0 eq.), 2,4,5-trichloropyrimidine (0.11 mL, 1.5 eq.), tetratriphenylphosphine palladium (36 mg, 0.05 eq.), and sodium carbonate solid (201 mg, 3.0 eq.) were added to a 50 mL three-necked flask. A mixture of 1,4-dioxane and water (v:v = 10:3) was added, the flask was purged with nitrogen three times, and the mixture was heated to 110 °C and reacted. After the reaction was completed as monitored by TLC, the mixture was cooled to room temperature and extracted with Ea and water for layer separation. The organic phase was dried over anhydrous sodium sulfate, filtered, concentrated under reduced pressure, separated and purified by column chromatography to obtain compound Int-**8** (100 mg, 42.9%), LCMS: $[M+H]^+$ = 369.3.

Step 5

Int-**8** (100 mg, 1.0 eq.), (3s,4r)-4-aminooxan-3-ol hydrochloride (83.0 mg, 2.0 eq.), DIPEA (0.18 mL, 4.0 eq.), and NMP solvent (1.5 mL) were added to a 10 mL microwave tube. The mixture was heated to 130 °C under microwave and reacted for 30 minutes. After the reaction was completed as monitored by TLC, the mixture was extracted with EA and water for layer separation. The organic phase was dried over anhydrous sodium sulfate, filtered, concentrated under reduced pressure, separated and purified by silica gel column chromatography to obtain target compound TA-28 (80 mg, 65.6%), LCMS: $[M+H]^+$ = 450.4, HPLC purity: 95.6%.

$^1H$ NMR (400 MHz, Chloroform-d) $\delta$ 8.20 (s, 1H), 7.34 (d, J = 11.7 Hz, 1H), 7.30 (s, 1H), 5.21 (d, J = 5.8 Hz, 1H), 4.47 (s, 1H), 3.99 (dd, J = 11.4, 4.9 Hz, 1H), 3.92 (dd, J = 11.8, 4.5 Hz, 1H), 3.81 - 3.71 (m, 1H), 3.56 (d, J = 4.8 Hz, 1H), 3.55 (m, 1H), 3.44 - 3.34 (m, 1H), 3.24 (d, J = 12.4 Hz, 1H), 3.10 (t, J = 10.6 Hz, 1H), 2.33 (m, 1H), 1.98 (s, 1H), 1.95 (m, 1H), 1.73 (m, 1H), 1.65 (d, J = 4.5 Hz, 1H), 1.43 (d, J = 6.5 Hz, 3H).

The following compounds were synthesized by referring to the synthesis method of Example B8:

| Comp. | Structure | LC-MS | NMR |
|---|---|---|---|
| TC-8 | 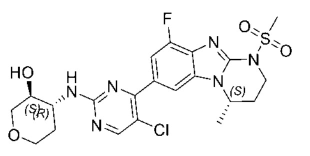 | $[M+H]^+$= 511.2 | |

(continued)

| Comp. | Structure | LC-MS | NMR |
|---|---|---|---|
| TC-10 | 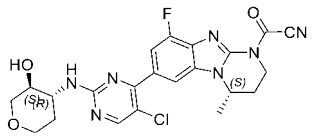 | $[M+H]^+$= 486.2 | |

**Test Example 1: Enzyme Activity Assay**

The biological activities of some compounds in the above Examples and Comparative Examples were tested below.

The biological activity test procedure was as follows:

1. Kinase Activity Assay

The $IC_{50}$ values of the test compounds against CDK4 and CDK6 kinases were determined.

(I) Reagent Information

| Reagent | Brand | Cat. No. |
|---|---|---|
| Palbociclib | MCE | HY-50767 |
| FL-Peptide18 | PerkinElmer | 760362 |
| FL-Peptide29 | PerkinElmer | 760429 |
| FL-Peptide34 | PerkinElmer | 760643 |

(II) Equipment Information

| Equipment | Brand | Cat. No. |
|---|---|---|
| Incubator | Thermo Scientific | - |
| Shaker | QILINBEIER | - |
| EZ Reader | PerkinElmer | 122919 |
| Liquid Handler | Labcyte Inc. | Echo 550 |
| Liquid Handler | TECAN | EVO200 |

(III) Study Design

(1) Compound Preparation

i. The test compound was prepared into a 0.5 mM solution in DMSO, and the positive control drug Palbociclib was also prepared into a 0.5 mM solution in DMSO.
ii. 3-fold dilution was performed to obtain compound solutions with 10 different concentrations.

(2) Enzyme Assay

i. A 1.3× enzyme solution containing enzyme, substrate and cofactor was prepared as described below.
ii. 15 μL of the 1.3× enzyme solution was added to each well and incubated at room temperature for 30 minutes.
iii. 5 μL of 4× ATP solution was added to initiate the reaction. Each test well contained the listed components with a final volume of 20 μL.
iv. After incubation for 150 minutes, 75 μL of buffer (containing 0.5 M EDTA) was added to terminate the reaction.
v. Data from each test well was read and analyzed using an EZ Reader.

(3) Data Analysis

The percent inhibition was calculated from the conversion ratio (CR) according to the following formula: Wells treated with DMSO served as positive control (PC), and wells without enzyme served as negative control (NC).

$$\% \text{ Inhibition} = 100 - 100 \times ((\text{CRPC} - \text{CRSample}) / (\text{CRPC} - \text{CRNC}))$$

| Enzyme | Vendor | Cat No. | Reference | Enzyme conc. (nM) | Assay | ATP conc.(μM) | Substrate | Cofactor | Reaction time |
|---|---|---|---|---|---|---|---|---|---|
| CDK4/CycD1 | ThermoFisher | PR8064A | Palbociclib | 2.50 | MSA | 180 | FL-Peptide34 | 10mM $MgCl_2$ | 120 minutes |
| CDK6/CyclinD3 | Carna | 04-107 | Palbociclib | 5.00 | MSA | 350 | FL-Peptide34 | 10mM MgCl2 | 120 minutes |

Based on the above tests, the $IC_{50}$ (nM) values of the inhibitory activities of the test samples against CDK4 and CDK6 kinases are shown in Table 1.

Table 1

| Compound | CDK4 kinase $IC_{50}$(nM) | CDK6 kinase $IC_{50}$(nM) |
|---|---|---|
| Palbociclib | 7.8 | 3.6 |
| TB-1 | 3.696 | 67.802 |
| TB-2 | 17.987 | 318.856 |
| TB-3 | 6.968 | 74.886 |
| TB-4 | 27.120 | >500 |
| TB-5 | 4.237 | 71.573 |
| TB-6 | 10.796 | 190.298 |
| TB-7 | 2.938 | 113.190 |
| TB-8 | 18.822 | 334.496 |
| TB-9 | 40.06 | 222.9 |
| TB-11 | 6.446 | 201.534 |
| TB-12 | 6.006 | 151.182 |
| TB-13 | 1.268 | 18.630 |
| TB-14 | 6.371 | 234.955 |
| TB-16 | 6.915 | 44.636 |
| TB-17 | 33.843 | 297.064 |
| TB-18 | 2.604 | 13.715 |
| TB-20 | 44.565 | 140.674 |
| TB-23 | 185 | >500 |
| TB-24 | 10.073 | 142.732 |
| TB-25 | 43.367 | >500 |
| TB-26 | 18.554 | 200.051 |
| TB-28 | 8.568 | 154.037 |
| TB-31 | 15.289 | 181.628 |
| TB-32 | 12.287 | 145.373 |
| TB-33 | 8.373 | 61.66 |
| TB-35 | 15.496 | 166.793 |
| TB-38 | 18.504 | 76.613 |
| TB-40 | 7.939 | 228.859 |
| TB-41 | 160.593 | >500 |
| TB-42 | 8.358 | 195.291 |
| TB-43 | 18.554 | 200.051 |
| TB-44 | 2.927 | 90.184 |
| TB-45 | 9.439 | 241.422 |
| TB-46 | 15.275 | 243.864 |
| TB-51 | 2.234 | 27.601 |
| TB-52 | 8.940 | 40.623 |

(continued)

| Compound | CDK4 kinase $IC_{50}$(nM) | CDK6 kinase $IC_{50}$(nM) |
|---|---|---|
| TB-53 | 10.572 | 56.852 |
| TB-54 | 101.298 | 410.857 |
| TB-68 | 301.466 | >500 |
| TB-70 | 18.504 | 76.613 |
| TB-90 | 12.665 | 95.239 |
| TB-91 | 4.154 | 68.376 |
| TB-92 | 4.546 | 123.496 |
| TB-93 | 51.787 | >500 |
| TB-94 | 44.364 | 458.443 |
| TB-95 | 103.169 | >500 |
| TB-96 | 2.1 | 20.3 |
| TB-130 | 28.567 | 154.898 |
| TB-132 | 46.788 | 245.603 |
| TB-133 | 17.931 | 64.836 |
| TB-137 | 28.3 | 174.8 |
| TB-142 | 69.533 | 361.303 |
| TB-143 | 7.899 | 58.343 |
| TB-144 | 13.574 | 125.787 |
| TB-146 | 11.9 | 60.1 |
| TB-148 | 71.4 | 222.2 |
| TB-149 | 5.3 | 90.8 |
| TB-154 | 3.0 | 75.8 |
| TA-4 | 4.4 | 63.9 |
| TA-33 | 3.4 | 94.2 |
| TC-2 | 4.5 | 75.7 |
| TC-7 | 3.7 | 153.7 |
| TC-12 | 1.9 | 45.7 |
| TC-13 | 3.3 | 74.4 |
| TC-17 | 4.5 | >500 |

As can be seen from Table 1, through *in vitro* biological activity screening, with Palbociclib as the control compound, the compounds synthesized in the present invention all exhibit excellent inhibitory activity against CDK4 kinase. Furthermore, the compounds synthesized in the present invention show excellent selectivity for CDK4 kinase over CDK6 kinase, which is highly likely to reduce hematological and other side effects caused by the inhibition of CDK6. Therefore, the compounds synthesized in the present invention are expected to be further developed as drugs for regulating CDK4 kinase activity or treating CDK4-related diseases.

**Test Example 2: Cell Anti-Proliferation Assay**

I. Experimental Materials and Equipment

Human breast cancer cells MCF-7, human ovarian cancer cells A2780, human mantle cell lymphoma cells JEKO-1. DMEM medium (Bio-Channel), DMSO (dimethyl sulfoxide), MTT (thiazolyl blue), 0.25% EDTA-Trypsin (trypsin

digestive solution), 1×PBS (phosphate buffered saline, pH 7.2), 96-well plate (Corning), fetal bovine serum (FBS), 10,000 U/mL penicillin-G/streptomycin, high-speed refrigerated centrifuge (EPPENDORF 5810R), and microplate reader (Tecan Spark).

II. Experimental Preparation

1. Cell Seeding

A) Tumor cells were cultured in DMEM (high glucose, containing 10% FBS and 100 U/mL penicillin-G/streptomycin) at 37 °C, 5% $CO_2$ and saturated humidity until 80-90% confluence.
B) The medium in the 10 cm culture dish was removed.
C) Cells were rinsed once with 10 mL of 1×PBS.
D) 4 mL of 0.25% EDTA-Trypsin was added, and the cells were digested in a 37 °C, 5% $CO_2$ incubator for 5 minutes. The mixture was transferred to a 15 mL centrifuge tube, centrifuged at 200 g for 5 minutes, and the supernatant was discarded to obtain cell pellet.
E) The cells were resuspended in 4 mL of DMEM medium, counted, and adjusted to 50,000 cells/mL.
F) 100 μL of cell suspension was added to each well of a 96-well plate, and the plate was incubated overnight at 37 °C in a 5% $CO_2$ incubator.

2. Compound Treatment

Compound Dilution

A) Gradient dilution solutions of the test compounds were prepared: the test compound was prepared into a 1 mM stock solution. Then 1.5 μL of the stock solution was dissolved in 1.5 mL of DMSO-free medium, followed by 3-fold serial gradient dilution with medium containing 0.1% DMSO to give 9 concentrations. The concentrations of diluted compound were as follows:
333.33 nM, 111.11 nM, 37.03 nM, 12.35 nM, 4.15 nM, 1.37 nM, 0.46 nM, and 0.15 nM.
B) After thorough mixing, 100 μL of the compound solution was used to replace the medium in the cell culture plate, with 4 replicate wells per concentration.
C) The cells were transferred to the incubator and incubated for 5 days.

3. MTT Assay

A) The cell culture plate was taken out, and 10 μL of 5 mg/mL MTT was added in a biological safety cabinet;
B) The plate was returned to the incubator and incubated for another 3 hours;
C) The cell culture plate was taken out and the medium was removed, 100 μL of isopropanol (containing 0.4 mM HCl, 0.1% NP-40) was added, and the plate was shaken at room temperature for 30 minutes;
D) The absorbance at 570 nm was measured using a TECAN microplate reader.

4. Data Analysis

Cell viability was calculated using the following formula:

$$\% \text{ Cell Viability} = 100\% \times (\text{Lum_Sample} - \text{Lum_LC}) / (\text{Lum_HC} - \text{Lum_LC})$$

Lum_HC: absorbance of cells treated with 0.1% DMSO control group
Lum_Sample: absorbance of cells treated with compound
Lum_LC: absorbance of blank medium
$IC_{50}$ values (unit: nM) were obtained by curve fitting using GraphPad Prism 8 software and are shown in Table 2, wherein, the meanings of A/B/C are as follows: A ≤ 500 nM, 500 nM < B ≤ 2000 nM, C > 2000 nM.

Table 2

| Compound | MCF7 $IC_{50}$(nM) | A2780 $IC_{50}$(nM) | JEKO-1 $IC_{50}$(nM) |
|---|---|---|---|
| TB-1 | A | A | A |
| TB-2 | A | A | A |
| TB-3 | A | A | A |
| TB-5 | A | A | A |
| TB-6 | B | A | A |
| TB-7 | A | A | A |
| TB-8 | B | A | A |
| TB-9 | B | A | A |
| TB-11 | A | A | A |
| TB-12 | A | A | A |
| TB-13 | A | A | A |
| TB-14 | A | A | A |
| TB-16 | A | A | A |
| TB-17 | B | B | B |
| TB-18 | A | A | A |
| TB-24 | A | A | A |
| TB-25 | B | A | A |
| TB-26 | A | A | A |
| TB-28 | A | A | A |
| TB-31 | A | A | A |
| TB-32 | A | A | A |
| TB-33 | A | A | A |
| TB-35 | A | A | A |
| TB-38 | A | A | A |
| TB-40 | A | A | A |
| TB-41 | B | B | B |
| TB-42 | A | A | A |
| TB-43 | A | A | A |
| TB-44 | A | A | A |
| TB-45 | A | A | A |
| TB-46 | A | A | A |
| TB-51 | A | A | A |
| TB-52 | A | A | A |
| TB-53 | A | A | A |
| TB-70 | A | A | A |
| TB-90 | B | A | A |
| TB-91 | A | A | A |
| TB-92 | A | A | A |
| TB-93 | B | A | A |

(continued)

| Compound | MCF7 $IC_{50}$(nM) | A2780 $IC_{50}$(nM) | JEKO-1 $IC_{50}$(nM) |
|---|---|---|---|
| TB-108 | A | A | A |
| TB-109 | B | A | A |
| TB-130 | A | A | A |
| TB-132 | B | A | A |
| TB-133 | A | A | A |
| TB-137 | A | A | A |
| TB-143 | A | A | A |
| TB-144 | A | A | A |
| TB-146 | A | A | A |
| TB-149 | A | A | A |
| TB-150 | A | A | A |
| TB-153 | A | A | A |
| TB-154 | A | A | A |
| TB-158 | A | A | A |
| TB-162 | A | A | A |
| TB-164 | A | A | A |
| TB-165 | A | A | A |
| TA-4 | A | A | A |
| TA-5 | B | A | A |
| TA-6 | A | A | B |
| TA-8 | A | B | A |
| TA-28 | A | A | A |
| TA-29 | A | A | A |
| TA-30 | C | C | C |
| TA-31 | C | B | A |
| TA-32 | A | A | B |
| TA-32 | A | A | A |
| TA-42 | C | B | B |
| TC-2 | B | A | A |
| TC-6 | B | A | A |
| TC-7 | A | A | A |
| TC-8 | C | B | C |
| TC-11 | A | A | A |
| TC-12 | A | A | A |
| TC-13 | A | A | A |
| TC-15 | B | B | B |
| TC-16 | A | A | A |
| TC-17 | A | A | A |
| TC-18 | A | A | A |

As can be seen from Table 2, through *in vitro* biological activity screening, the compounds synthesized in the present invention exhibit excellent inhibitory activity against human breast cancer cells MCF-7, human ovarian cancer cells A2780, and human mantle cell lymphoma cells JEKO-1.

**Test Example 3: Preclinical Pharmacokinetic Study in Rats**

I. Experimental Materials and Equipment

Healthy adult Sprague-Dawley (SD) rats, male, 6-8 weeks old, weighing 200-300 g. EDTA-Na2 anticoagulant. Analytical balance, animal weighing scale, magnetic stirrer, refrigerated centrifuge, single-channel manual pipette, etc.

II. Experimental Procedure

1. Drug Formulation

About 10 mg of the test sample was precisely weighed, dissolved in a calculated volume of 5% DMSO, followed by addition of 10% solutol HS-15 and 85% normal saline. The mixture was sonicated and vortexed to homogeneity to obtain a solution with a concentration of 1 mg/mL. The solution was freshly prepared before use.

0.2 mL of the sample was transferred into a 1.5 mL centrifuge tube and stored at -80 °C for analysis of the dosing solution concentration.

2. Animal Preparation

Animals were housed in rat cages and fasted overnight (no less than 10 h) before the test, with free access to water. On the day of the test, animals were weighed and marked on the tail. Blank blood samples were collected before administration. The blood was collected via tail vein.

3. Administration

Route of administration: oral gavage (p.o.)
Dose: 10 mg/kg
Dosing volume: 10 mL/kg
Procedure: The rat was held upright with the left hand wearing bite-resistant glove. A 16-gauge gavage needle was inserted through the mouth into the esophagus. The needle was advanced gently without obvious resistance, and the drug solution was injected into the stomach.

4. Sample Collection

Whole blood (0.1 mL) were collected from each test animal before administration and at 0.5 h, 1 h, 2 h, 4 h, 6 h, 8 h, 12 h, and 24 h after administration into EDTA-Na2 anticoagulant tubes. The tubes were inverted 3-4 times for mixing, then centrifuged at 10,000 g for 5 min at 4 °C to separate plasma. Plasma samples were stored at -80 °C until analysis. Blood was collected via tail vein.

Detailed procedure: The rat was fixed in a restrainer with the tail fully exposed. The tail was wiped with alcohol to absorb alcohol, thereby inducing vasodilation. A suitable vein on either side was selected, and the needle was inserted at approximately one-third of the tail length from the tip. An insulin syringe was used with the bevel facing upward. After puncturing the skin, the needle was immediately advanced parallelly into the vein. Low resistance during sliding of the needle in the vein and blood reflux in the syringe indicate that the needle had entered the vein. Approximately 0.1-0.2 mL of whole blood was withdrawn. After withdrawing the needle, pressure was applied to achieve hemostasis.

III. Sample Analysis

Preparation of standard curve: 25 μL of blank rat plasma was transferred into centrifuge tubes. 25 μL of the prepared standard series solution (prepared in methanol) and 200 μL of internal standard solution (prepared in methanol) were added sequentially. The mixture was vortexed for 2 min and centrifuged at 10,000 g for 10 min at 4 °C.

Processing of unknown plasma samples: 25 μL of drug-containing rat plasma was transferred, followed by addition of 25 μL of methanol and 200 μL of internal standard solution. The mixture was vortexed for 2 min and centrifuged

at 10,000 g for 10 min at 4 °C. The supernatant was collected for LC-MS/MS analysis.

IV. Data Processing

A quantitative method for the determination of the test compound was established using a Shimadzu liquid chromatography system coupled with Triple Quad™ 6500+ AB mass spectrometer. The concentration of the parent drug in plasma was determined. The plasma concentration-time curve was plotted, and the main pharmacokinetic parameters were calculated using a non-compartmental model in winnonlin Phoenix software. Detailed data are shown in Table 3.

Table 3

| Compound | Tmax (h) | Cmax (ng/ml) | AUClast (h*ng/ml) | **HL_ (h)** |
|---|---|---|---|---|
| TB-12 | 1.50 | 677 | 4502 | 3.14 |
| TB-24 | 1.67 | 677 | 4502 | 3.14 |
| TB-27 | 2.33 | 1503 | 11472 | 3.69 |
| TB-31 | 4.00 | 923 | 4891 | 1.34 |
| TB-92 | 0.50 | 1943 | 5025 | 1.21 |
| TB-108 | 0.60 | 503 | 1374 | 1.25 |
| TB-109 | 0.50 | 777 | 1774 | 5.32 |
| TB-137 | 0.83 | 1244 | 3668 | 4.10 |
| TB-149 | 1.17 | 4160 | 25428 | 1.99 |
| TB-165 | 0.50 | 1184 | 2801 | 1.29 |
| TA-4 | 1.0 | 4433 | 16214 | 3.19 |
| TA-28 | 0.50 | 990 | 2492 | 1.58 |
| TA-29 | 0.67 | 506 | 1493 | 1.60 |
| TA-33 | 4.67 | 262 | 2172 | / |
| TC-2 | 0.67 | 400 | 1239 | 2.87 |
| TC-12 | 1.0 | 417 | 1294 | 2.55 |

As can be seen from Table 3, the compounds synthesized in the present invention all exhibit excellent pharmacokinetic properties by PK test screening.

All documents mentioned herein are incorporated by reference in this application as if each document were individually incorporated by reference. In addition, it should be understood that after reading the above teaching content of the present invention, those skilled in the art can make various changes or modifications to the present invention, and these equivalent forms also fall within the scope defined by the appended claims of the present application.

## Claims

1. A compound used as a CDK4 kinase inhibitor, wherein the compound is a compound of formula II, or a pharmaceutically acceptable salt, a stereoisomer, a tautomer, a hydrate, a solvate, an isotope compound, or a prodrug thereof,

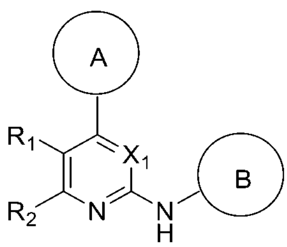

formula II

wherein,

$X_1$ is selected from the group consisting of N and $CR_3$;
$R_1$ is selected from the group consisting of H, $CF_3$, F, Cl, Br, methyl, ethyl, isopropyl, and cyclopropyl;
$R_2$ is selected from the group consisting of H, F, $CF_3$, $CF_2H$, $NH_2$, and methyl;
or, $R_1$ and $R_2$ together with the C to which they are attached form a 5-membered heteroaryl containing two N atoms;
each $R_3$ is independently selected from the group consisting of H, $CF_3$, F, Cl, Br, methyl, and cyano;
ring A is selected from the group consisting of

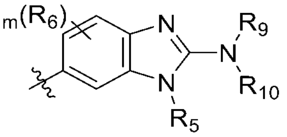 ,

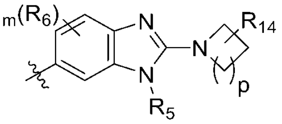 , 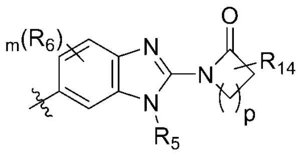 , 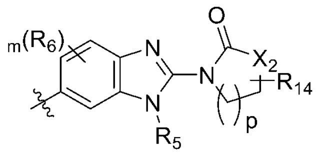 ,

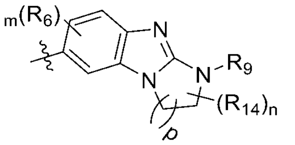 , 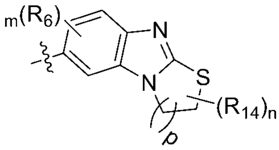 , 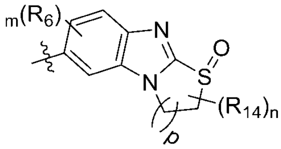 ,

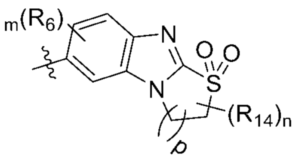 , 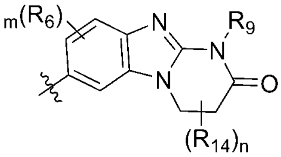 , 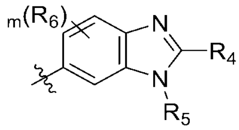 , 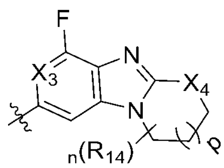 ,

and

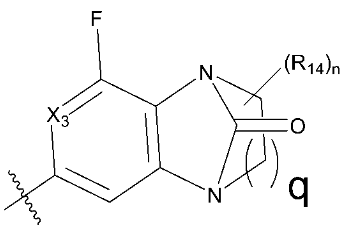

wherein, $X_2$ is O or NR, $X_3$ is N or $CR_3$, and $X_4$ is NR, SO or $SO_2$;
ring B is selected from the group consisting of

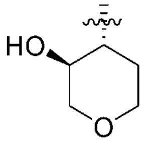 , 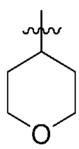 , 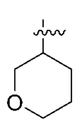 , 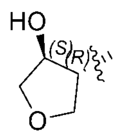 ,

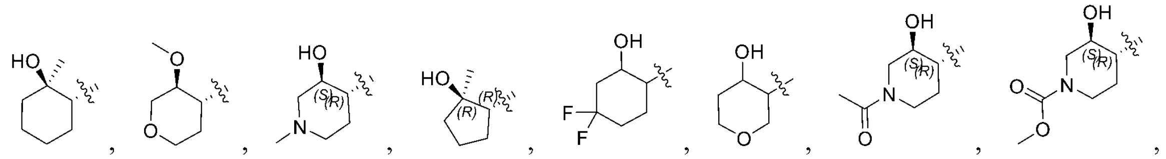 , , , , , , , ,

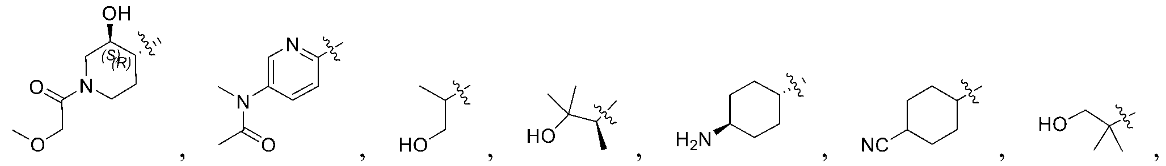 , , , , , , ,

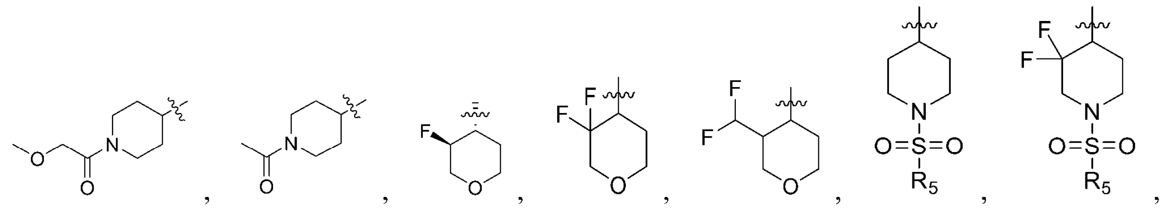 , , , , , , ,

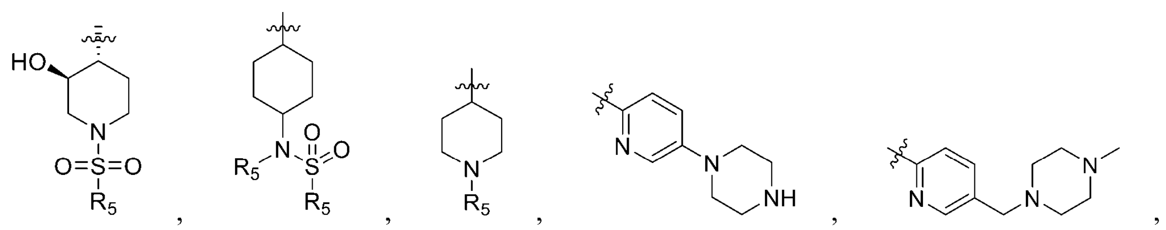 , , , , ,

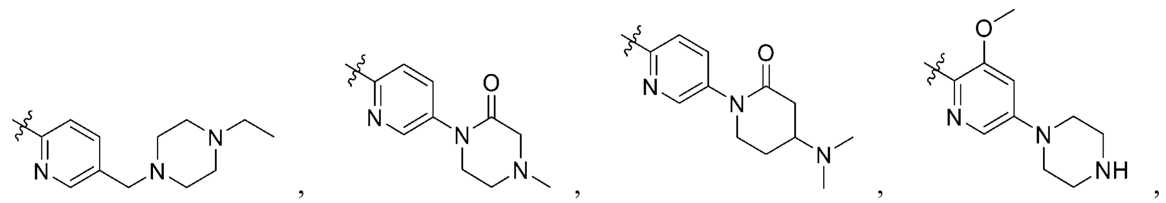 , , , ,

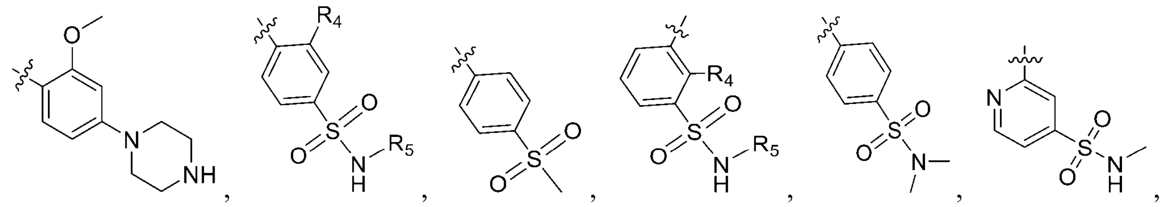 , , , , , ,

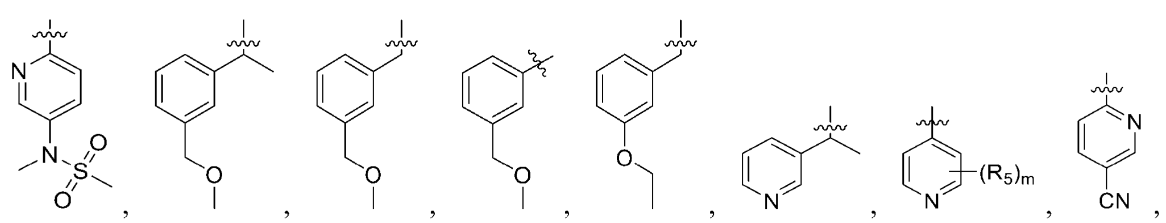 , , , , , , , ,

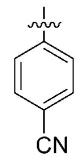, 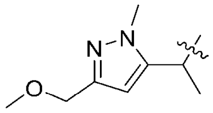, 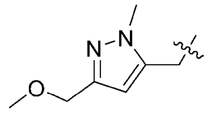, 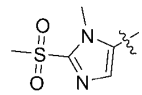, 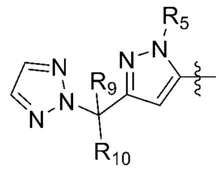,

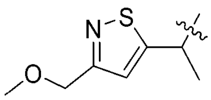, 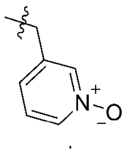, 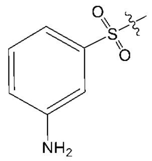, 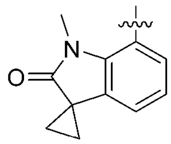, 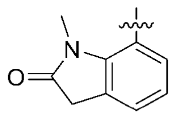, 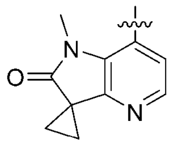,

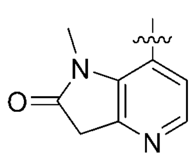, 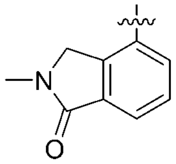, 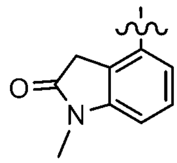, and 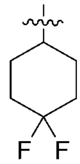;

wherein, each $R_4$ is independently selected from the group consisting of H, cyano, halogen, substituted or unsubstituted C1-C6 alkyl, and substituted or unsubstituted C3-C6 cycloalkyl;

each $R_5$, $R_6$, $R_9$, $R_{10}$ and $R_{14}$ is each independently selected from the group consisting of H, halogen, hydroxyl, amino,

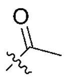, 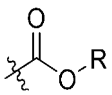, 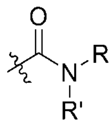,

oxo, substituted or unsubstituted C1-C6 alkyl, substituted or unsubstituted C3-C6 cycloalkyl, substituted or unsubstituted phenyl, substituted or unsubstituted $C_{1-6}$ alkoxy, and substituted or unsubstituted $-NR_mR_n$; or, two $R_{14}$ connected to the same C form a C3-C6 cycloalkyl together with the C to which they are attached;

or, $R_9$ and $R_{10}$ together with the N to which they are attached form

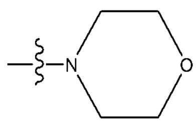

or

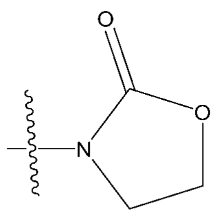;

each R and R' is each independently selected from the group consisting of H, C1-C6 alkyl, halogenated C1-C6 alkyl, C3-C6 cycloalkyl, halogenated C3-C6 cycloalkyl, and phenyl;

each $R_m$ is selected from the group consisting of H, $C_{1-6}$ alkyl, $C_{3-6}$ cycloalkyl, $C_{6-10}$ aryl, and 6-10 membered heteroaryl containing 1, 2, or 3 heteroatoms selected from N, O, and S;

each $R_n$ is $C_{1-6}$ alkyl;

the substituted in $R_4$, $R_5$, $R_6$, $R_9$, $R_{10}$ and $R_{14}$ each independently refers to being substituted by 1, 2 or 3 substituents selected from the group consisting of deuterium, halogen, hydroxyl, amino, $-N-(C1-C6\ alkyl)_2$, C1-

C6 alkoxy, phenyl, and cyano;
each m, n, and p is each independently selected from the group consisting of 0, 1, 2, 3, and 4;
q is selected from the group consisting of 1, 2, and 3.

**2.** A compound, wherein the compound is a compound of formula I, or a pharmaceutically acceptable salt, a stereoisomer, a tautomer, a hydrate, a solvate, an isotope compound, or a prodrug thereof,

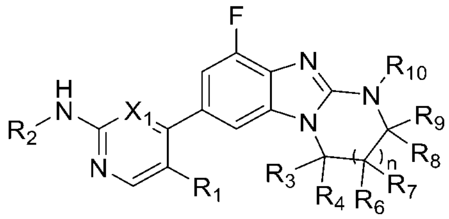

formula I

, .

wherein:

$X_1$ is selected from the group consisting of N and $CR_{11}$;
$R_1$ is selected from the group consisting of H, halogen, cyano, $C_{1-6}$ alkyl, and $C_{1-6}$ haloalkyl;
$R_2$ is selected from the group consisting of

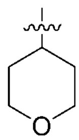 , 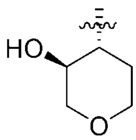 , 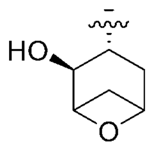 , 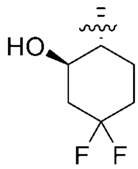 ,

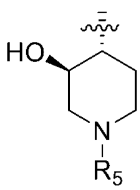 , 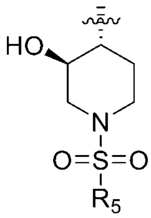 , 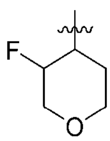 , 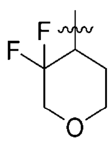 , 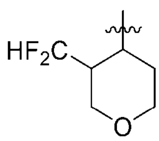 , 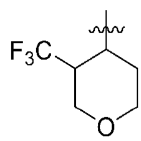 , and 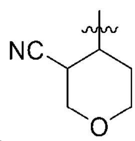 ;

$R_5$ is selected from the group consisting of H, halogen, hydroxyl, amino,

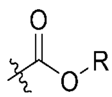 , 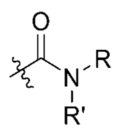 ,

$C_{1-6}$ alkyl, $C_{3-6}$ cycloalkyl, and phenyl;
R and R' are each independently selected from the group consisting of H and $C_{1-6}$ alkyl;
$R_3$, $R_4$, $R_6$, $R_7$, $R_8$, and $R_9$ are each independently selected from the group consisting of H, $C_{1-6}$ alkyl, $C_{3-6}$ cycloalkyl, hydroxyl, amino, ketocarbonyl, $C_{1-6}$ alkoxy, -$COC_{1-6}$ alkoxy, -$COC_{1-6}$ alkyl, $C_{1-6}$ haloalkyl, $C_{1-6}$ haloalkoxy, hydroxyl substituted $C_{1-6}$ alkyl, 5-8 membered heterocycloalkyl containing 1, 2, or 3 heteroatoms selected from N, O, and S, and 5-8 membered heteroaryl containing 1, 2, or 3 heteroatoms selected from N, O, and S;
$R_{10}$ is selected from the group consisting of H, $C_{1-6}$ alkyl, deuterated $C_{1-6}$ alkyl, $C_{3-8}$ cycloalkyl, $C_{1-6}$ alkoxy, -$COC_{1-6}$ alkoxy, -$COC_{1-6}$ alkyl, $C_{6-10}$ aryl, 5-8 membered heteroaryl containing 1, 2, or 3 heteroatoms selected from N, O, and S, 4-8 membered heterocycloalkyl containing 1, 2, or 3 heteroatoms selected from N, O, and S,

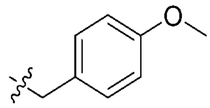,

$-S(=O)_2-C_{1-6}$ alkyl, and -CO-CN; or, $R_{10}$ together with adjacent $R_8$ or $R_9$ forms a 5-8 membered heterocycloalkyl containing 1, 2, or 3 heteroatoms selected from N, O, and S, wherein the alkyl, aryl, cycloalkyl, and heterocycloalkyl are optionally substituted with one or more groups selected from the group consisting of halogen, cyano, hydroxyl, $C_{1-6}$ alkyl, $C_{1-6}$ haloalkyl, $-CO-C_{1-6}$ alkyl, $C_{1-6}$ alkoxy, amino, -COOH, $-CONH_2$, $-CF_3C(F)_2$, and 5-8 membered heterocycloalkyl containing 1, 2, or 3 heteroatoms selected from N, O and S which is optionally substituted with $C_{1-6}$ alkyl, $-CH(F)_2$, $-COO-C_{1-6}$ alkyl, or $-S(O)_2-C_{6-10}$ aryl;

$R_{11}$ is selected from the group consisting of H, halogen, cyano, $C_{1-6}$ alkyl, and $C_{1-6}$ haloalkyl;

n is independently selected from the group consisting of 0, 1 and 2.

**3.** A compound, wherein the compound is a compound of formula I, or a pharmaceutically acceptable salt, a stereoisomer, a tautomer, a hydrate, a solvate, an isotope compound, or a prodrug thereof,

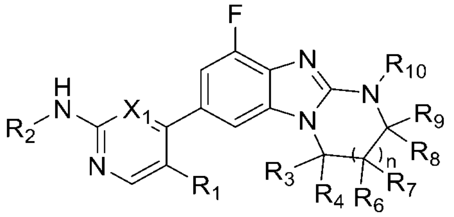

formula I

wherein:

$X_1$ is selected from the group consisting of N and $CR_{11}$;

$R_1$ is selected from the group consisting of H, halogen, cyano, $C_{1-6}$ alkyl, and $C_{1-6}$ haloalkyl;

$R_2$ is selected from the group consisting of

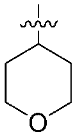, 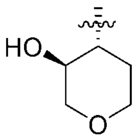, 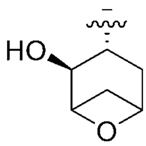, 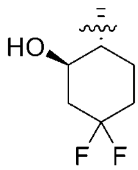,

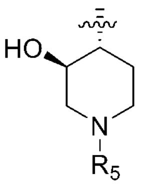, 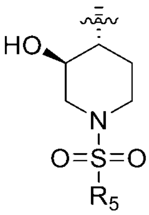, 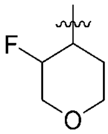, 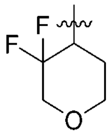, 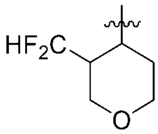, 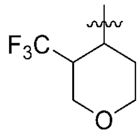, and 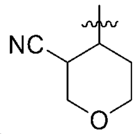;

$R_5$ is selected from the group consisting of H, halogen, hydroxyl, amino,

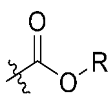, 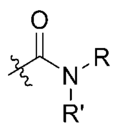,

$C_{1-6}$ alkyl, $C_{3-6}$ cycloalkyl, and phenyl;

R and R' are each independently selected from the group consisting of H and $C_{1-6}$ alkyl;

$R_3$, $R_4$, $R_6$, $R_7$, $R_8$, and $R_9$ are each independently selected from the group consisting of H, $C_{1-6}$ alkyl, $C_{3-6}$ cycloalkyl, hydroxyl, amino, ketocarbonyl, $C_{1-6}$ alkoxy, $-COC_{1-6}$ alkoxy, $-COC_{1-6}$ alkyl, $C_{1-6}$ haloalkyl, $C_{1-6}$

haloalkoxy, hydroxyl substituted $C_{1-6}$ alkyl, 5-8 membered heterocycloalkyl containing 1, 2, or 3 heteroatoms selected from N, O, and S, and 5-8 membered heteroaryl containing 1, 2, or 3 heteroatoms selected from N, O, and S;

$R_{10}$ is selected from the group consisting of H, $C_{1-6}$ alkyl, deuterated $C_{1-6}$ alkyl, $C_{3-8}$ cycloalkyl, $C_{1-6}$ alkoxy, -$COC_{1-6}$ alkoxy, -$COC_{1-6}$ alkyl, $C_{6-10}$ aryl, 5-8 membered heteroaryl containing 1, 2, or 3 heteroatoms selected from N, O, and S, 4-8 membered heterocycloalkyl containing 1, 2, or 3 heteroatoms selected from N, O, and S, and

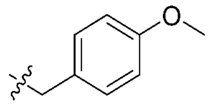

;

or, $R_{10}$ together with adjacent $R_8$ or $R_9$ forms a 5-8 membered heterocycloalkyl containing 1, 2, or 3 heteroatoms selected from N, O, and S, wherein the alkyl, aryl, cycloalkyl, and heterocycloalkyl are optionally substituted with one or more groups selected from the group consisting of halogen, cyano, hydroxyl, $C_{1-6}$ alkyl, -CO-$C_{1-6}$ alkyl, $C_{1-6}$ alkoxy, amino, -COOH, -$CONH_2$, -$CF_3C(F)_2$, and 5-8 membered heterocycloalkyl containing 1, 2, or 3 heteroatoms selected from N, O and S which is optionally substituted with $C_{1-6}$ alkyl, -$CH(F)_2$, -COO-$C_{1-6}$ alkyl, or -$S(O)_2$-$C_{6-10}$ aryl;

$R_{11}$ is selected from the group consisting of H, halogen, cyano, $C_{1-6}$ alkyl, and $C_{1-6}$ haloalkyl;

n is independently selected from the group consisting of 0, 1 and 2.

**4.** The compound according to claim 2 or 3, wherein $R_2$ is selected from the group consisting of

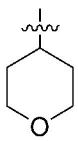 , 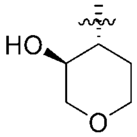 , 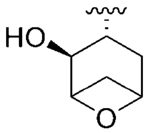 , 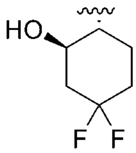 , 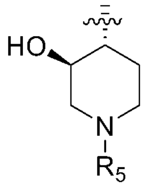 , 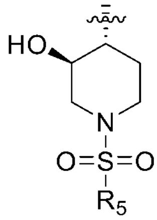 , 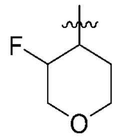 , 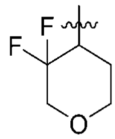 ,

and

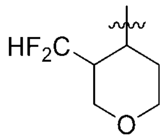

;

$R_{10}$ is selected from the group consisting of H, $C_{1-6}$ alkyl, deuterated $C_{1-6}$ alkyl, $C_{3-8}$ cycloalkyl, $C_{1-6}$ alkoxy, -$COC_{1-6}$ alkoxy, -$COC_{1-6}$ alkyl, $C_{6-10}$ aryl, 5-8 membered heteroaryl containing 1, 2, or 3 heteroatoms selected from N, O, and S, 5-8 membered heterocycloalkyl containing 1, 2, or 3 heteroatoms selected from N, O, and S, and

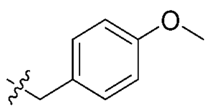

;

or, $R_{10}$ together with adjacent $R_8$ or $R_9$ forms a 5-8 membered heterocycloalkyl containing 1, 2, or 3 heteroatoms selected from N, O, and S, wherein the alkyl, aryl, cycloalkyl, and heterocycloalkyl are optionally substituted with one or more groups selected from the group consisting of halogen, cyano, hydroxyl, $C_{1-6}$ alkyl, -CO-$C_{1-6}$ alkyl, $C_{1-6}$ alkoxy, amino, -COOH, -$CONH_2$, -$CF_3C(F)_2$, and 5-8 membered heterocycloalkyl containing 1, 2, or 3 heteroatoms selected from N, O and S which is optionally substituted with $C_{1-6}$ alkyl, -$CH(F)_2$, -COO-$C_{1-6}$ alkyl, or -$S(O)_2$-$C_{6-10}$ aryl.

**5.** The compound according to claim 2 or 3, wherein $R_1$ is selected from the group consisting of Cl and Br; $R_2$ is selected from the group consisting of

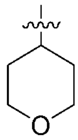 , 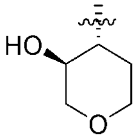 , 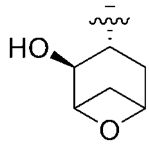 , 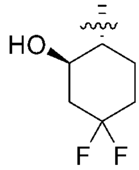 ,

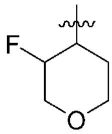 , 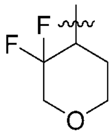 , 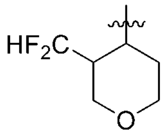 , 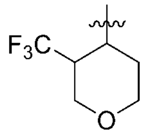 , and 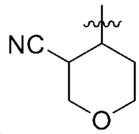 .

**6.** The compound according to claim 2 or 3, wherein $R_3$, $R_4$, $R_6$, $R_7$, $R_8$, and $R_9$ are each independently selected from the group consisting of H, $C_{1-6}$ alkyl, $C_{3-6}$ cycloalkyl, hydroxyl, amino, $C_{1-6}$ alkoxy, $-COC_{1-6}$ alkoxy, $-COC_{1-6}$ alkyl, $C_{1-6}$ haloalkyl, $C_{1-6}$ haloalkoxy, hydroxyl substituted $C_{1-6}$ alkyl, and 5-8 membered heterocycloalkyl containing 1, 2, or 3 heteroatoms selected from N, O, and S.

**7.** The compound according to claim 2 or 3, wherein $R_{10}$ is selected from the group consisting of H, $C_{1-6}$ alkyl, deuterated $C_{1-6}$ alkyl, $C_{3-8}$ cycloalkyl, $C_{6-10}$ aryl, 4-8 membered heterocycloalkyl containing 1, 2, or 3 heteroatoms selected from N, O, and S, and

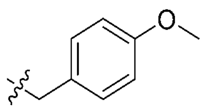 ;

or, $R_{10}$ together with adjacent $R_8$ or $R_9$ forms a 5-8 membered heterocycloalkyl containing 1, 2, or 3 heteroatoms selected from N, O, and S, wherein the alkyl, aryl, cycloalkyl, and heterocycloalkyl are optionally substituted with one or more groups selected from the group consisting of halogen, cyano, hydroxyl, $C_{1-6}$ alkyl, amino, and $-CF_3C(F)_2$.

**8.** A compound used as a CDK4 kinase inhibitor, or a pharmaceutically acceptable salt, a stereoisomer, a tautomer, a hydrate, a solvate, an isotope compound, or a prodrug thereof, wherein the compound is selected from the group consisting of:

| | | |
|---|---|---|
| 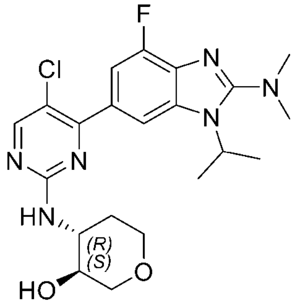 TB-1 | 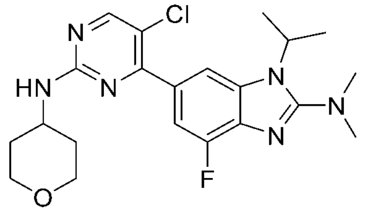 TB-2 | 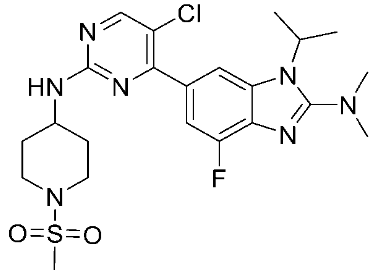 TB-3 |
| 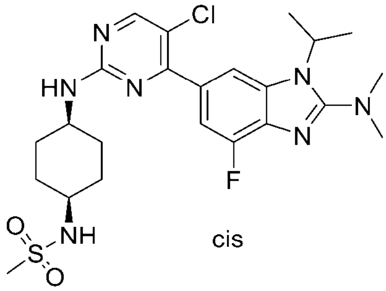 cis TB-4 | 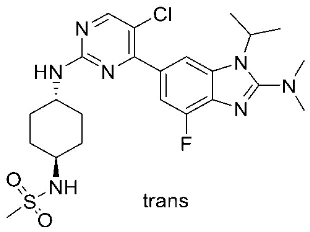 trans TB-5 | 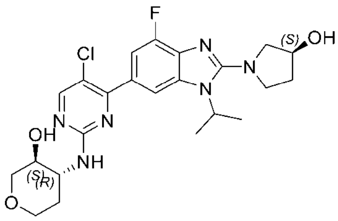 TB-6 |
| 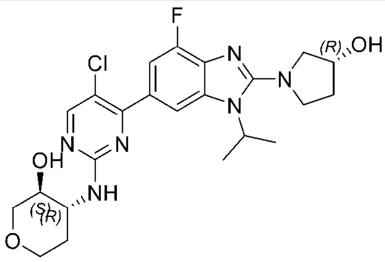 TB-7 | 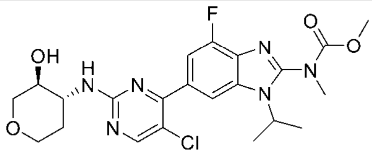 TB-8 | 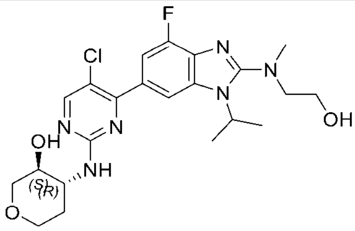 TB-9 |
| 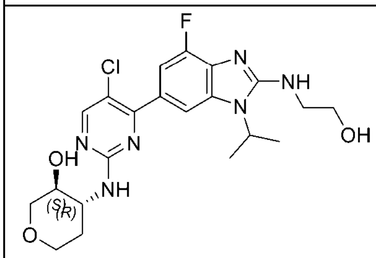 TB-10 | 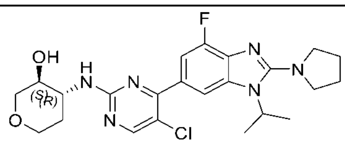 TB-11 | 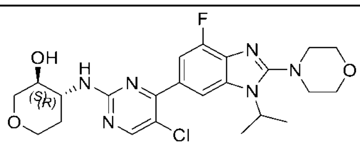 TB-12 |

| | | |
|---|---|---|
| 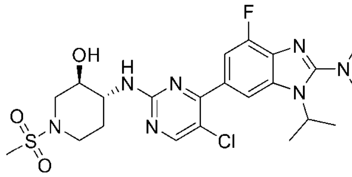 TB-13 | 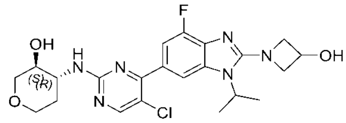 TB-14 | 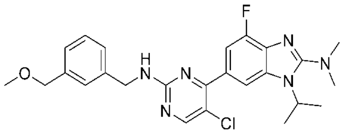 TB-15 |
| 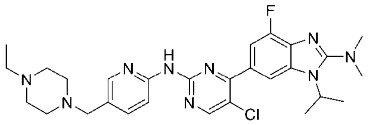 TB-16 | 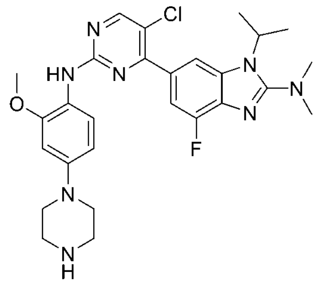 TB-17 | 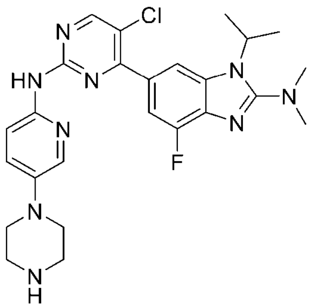 TB-18 |
| 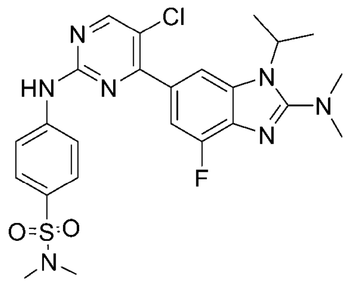 TB-19 | 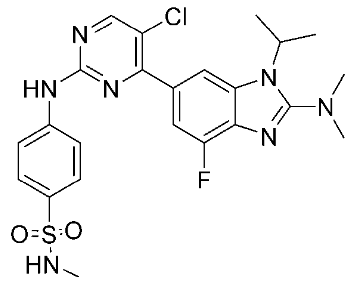 TB-20 | 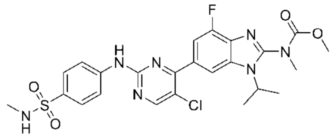 TB-21 |
| 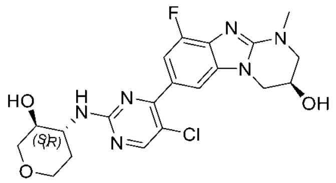 TB-22 | 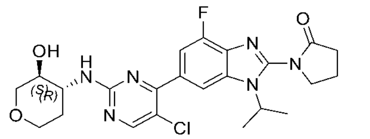 TB-23 | 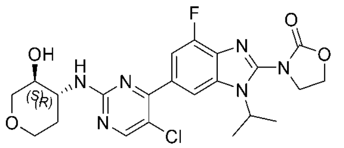 TB-24 |
| 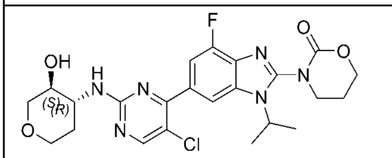 TB-25 | 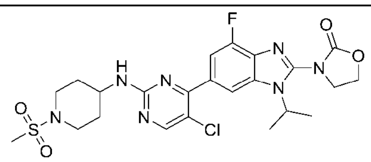 TB-26 | 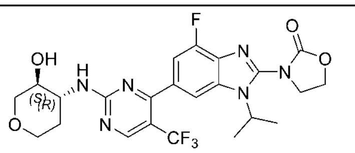 TB-27 |
| 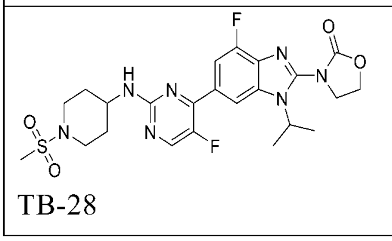 TB-28 | 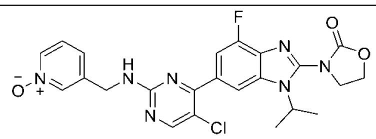 TB-29 | 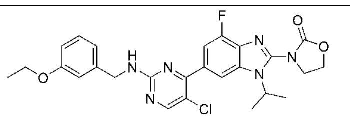 TB-30 |

| | | |
|---|---|---|
| 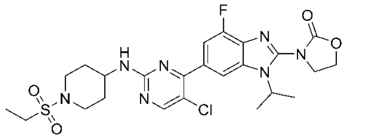 TB-31 | 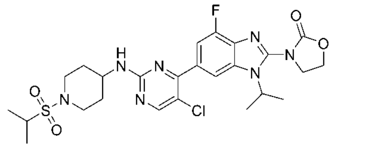 TB-32 | 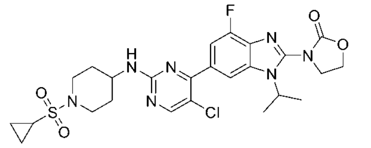 TB-33 |
| 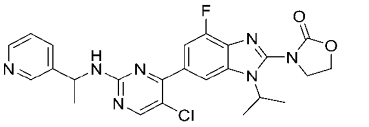 TB-34 | 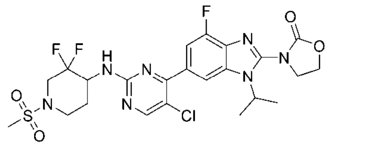 TB-35 | 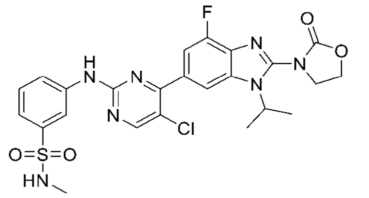 TB-36 |
| 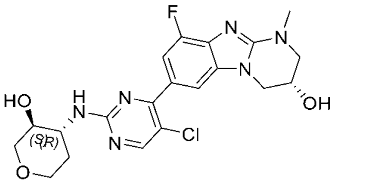 TB-37 | 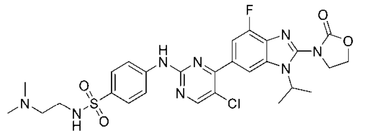 TB-38 | 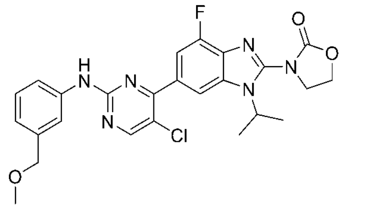 TB-39 |
| 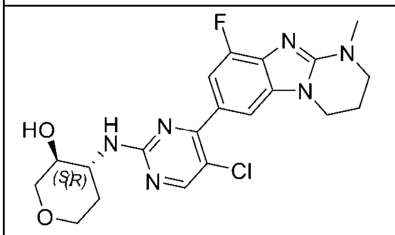 TB-40 | 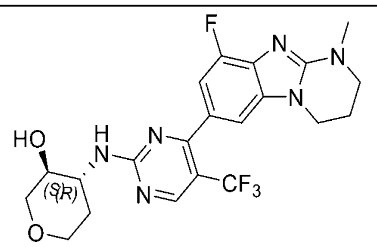 TB-41 | 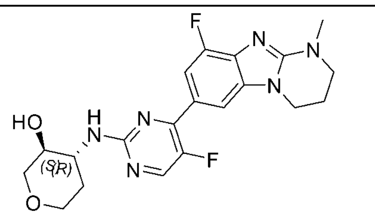 TB-42 |
| 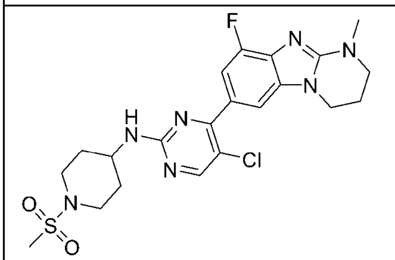 TB-43 | 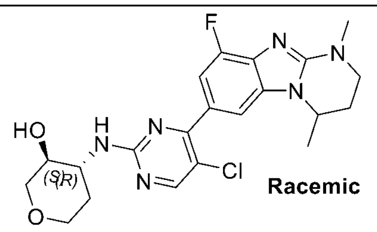 Racemic TB-44 | 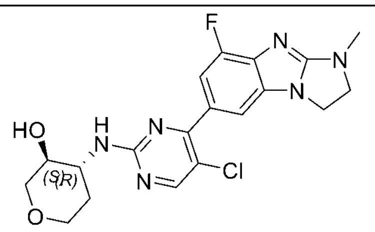 TB-45 |
| 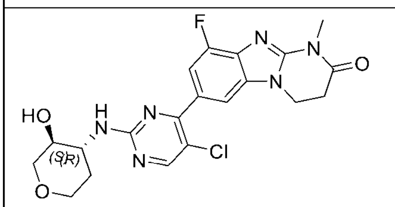 TB-46 | 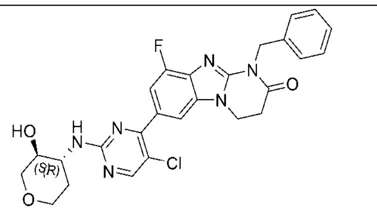 TB-47 | 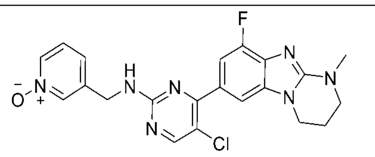 TB-48 |

| | | |
|---|---|---|
| 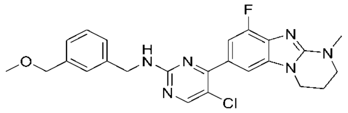 TB-49 | 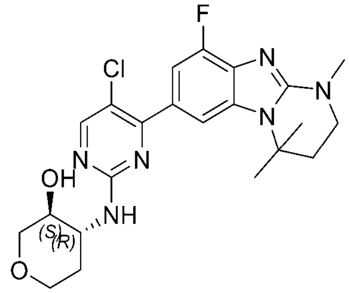 TB-50 | 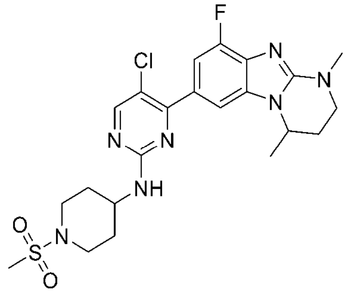 TB-51 |
| 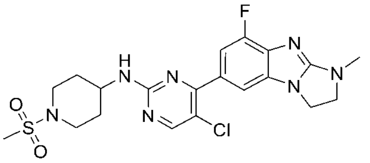 TB-52 | 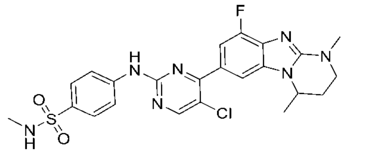 TB-53 | 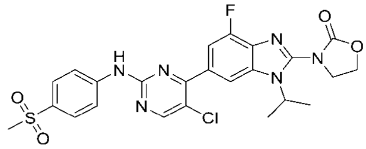 TB-54 |
| 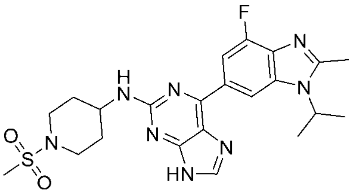 TB-55 | 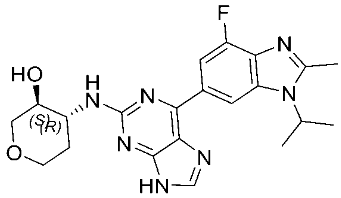 TB-56 | 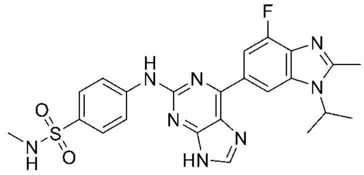 TB-57 |
| 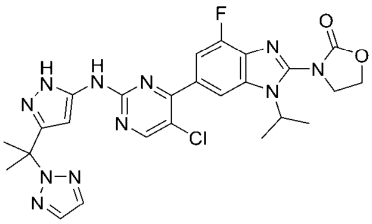 TB-58 | 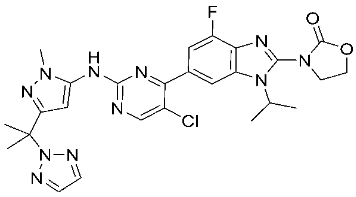 TB-59 | 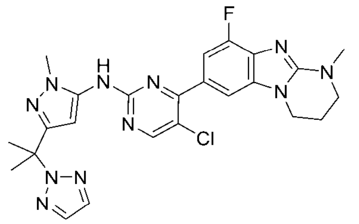 TB-60 |
| 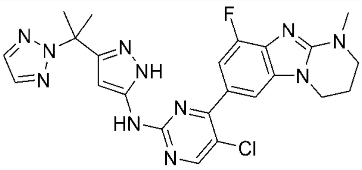 TB-61 | 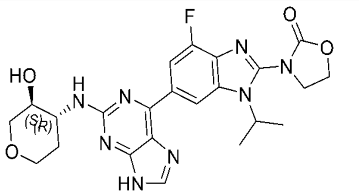 TB-62 | 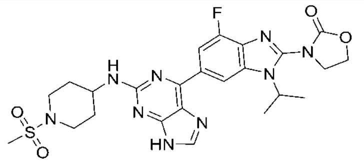 TB-63 |

| | | |
|---|---|---|
| 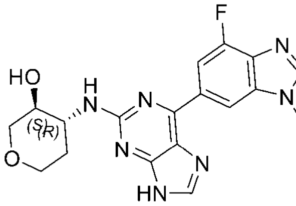<br>TB-64 | 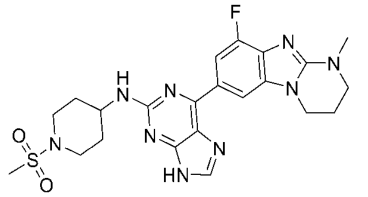<br>TB-65 | 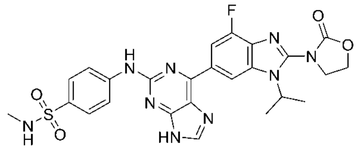<br>TB-66 |
| 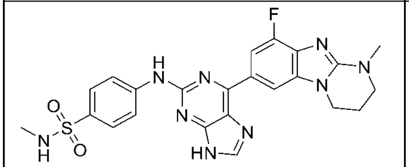<br>TB-67 | 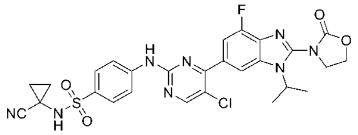<br>TB-68 | 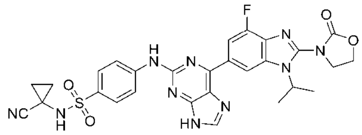<br>TB-69 |
| 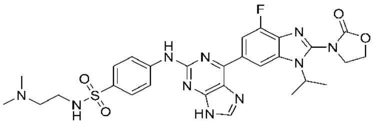<br>TB-70 | 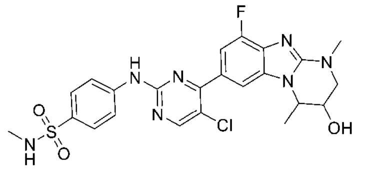<br>TB-71 | 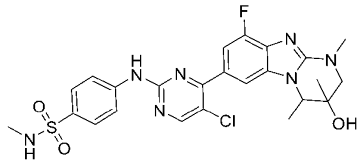<br>TB-72 |
| 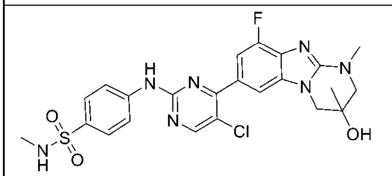<br>TB-73 | 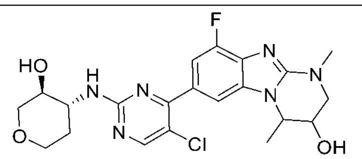<br>TB-74 | 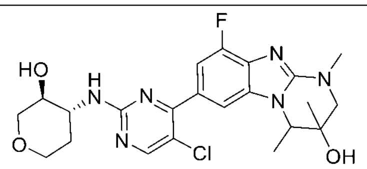<br>TB-75 |
| 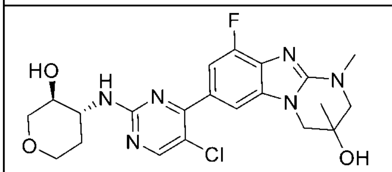<br>TB-76 | 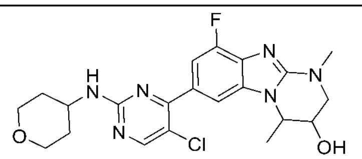<br>TB-77 | 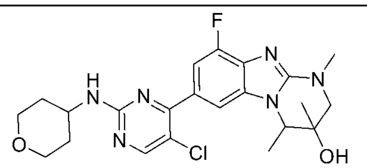<br>TB-78 |
| 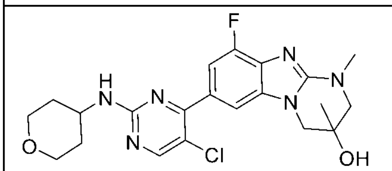<br>TB-79 | 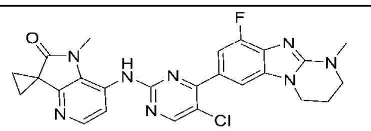<br>TB-80 | 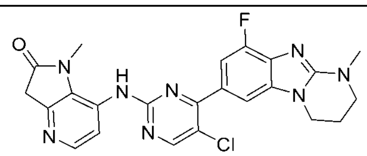<br>TB-81 |
| 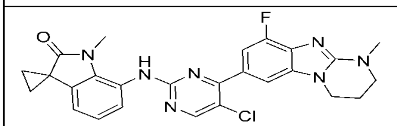<br>TB-82 | 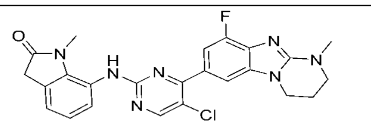<br>TB-83 | 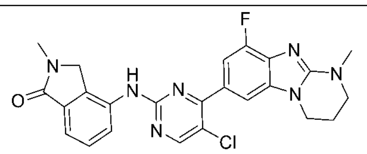<br>TB-84 |

| | | |
|---|---|---|
| 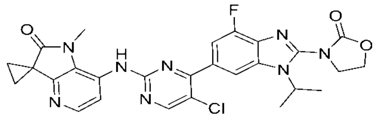 TB-85 | 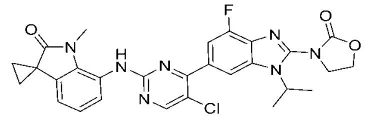 TB-86 | 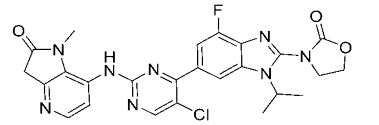 TB-87 |
| 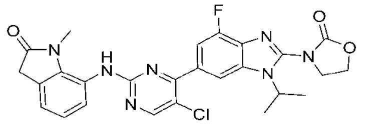 TB-88 | 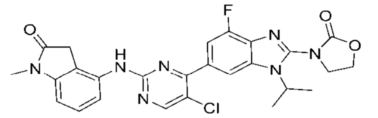 TB-89 | 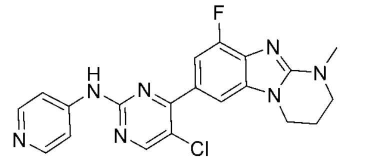 TB-90 |
| 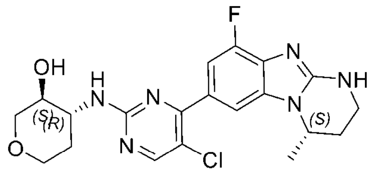 TB-91' | 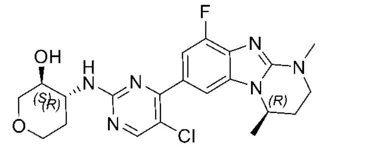 TB-91 | 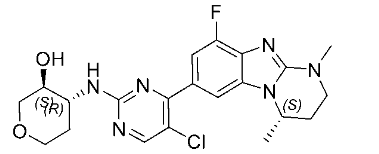 TB-92 (or TA-1) |
| 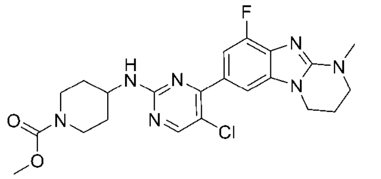 TB-93 | 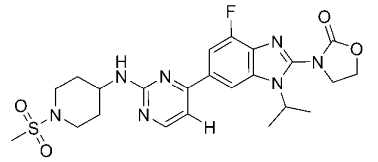 TB-94 | 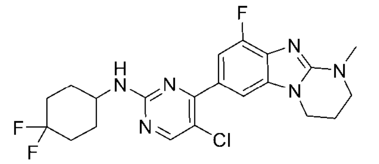 TB-95 |
| 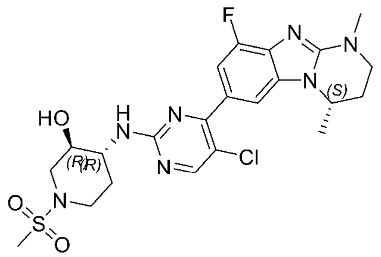 TB-96 | 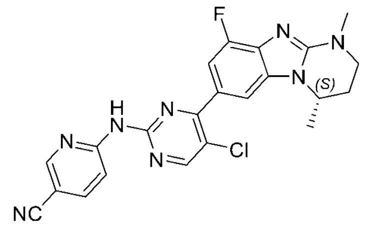 TB-97 | 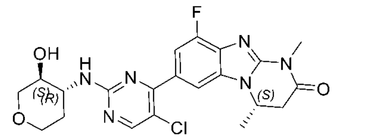 TB-98 |
| 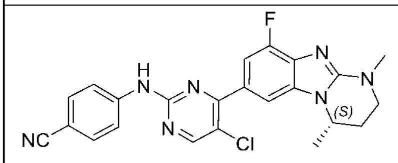 TB-99 | 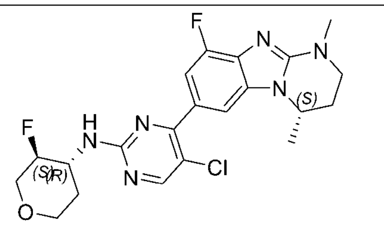 TB-100 | 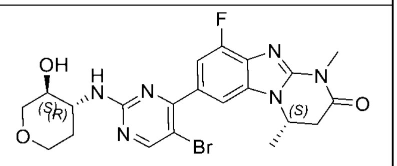 TB-101 |
| 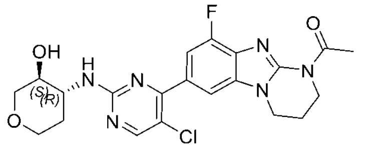 TB-102 | 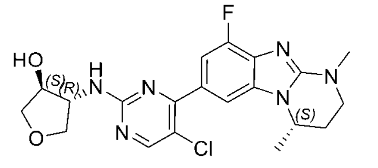 TB-103 | 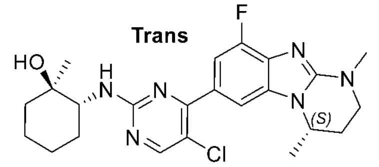  TB-104 |

| | | |
|---|---|---|
| 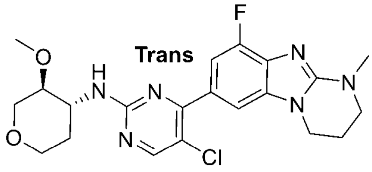 TB-105 | 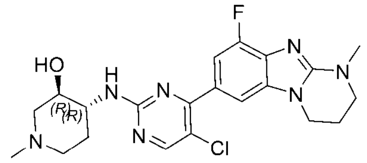 TB-106 | 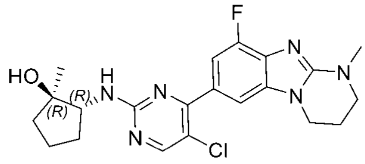 TB-107 |
| 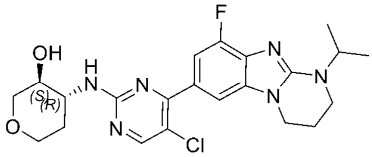 TB-108 | 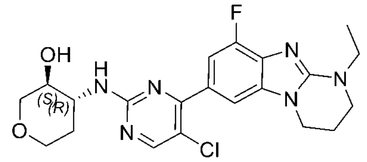 TB-109 | 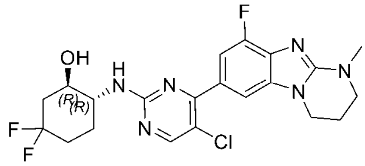 TB-110 |
| 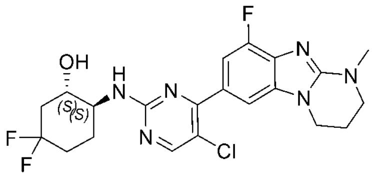 TB-111 | 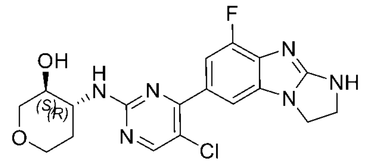 TB-112 | 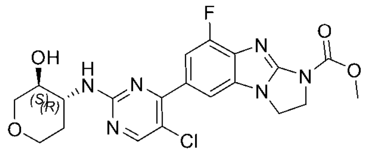 TB-113 |
| 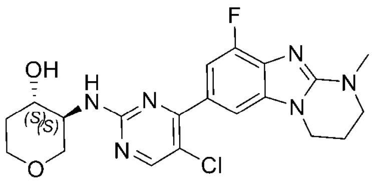 TB-114 | 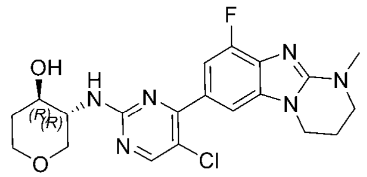 TB-115 | 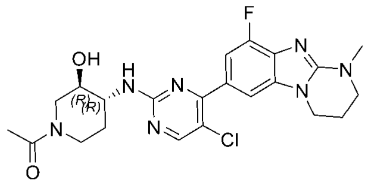 TB-116 |
| 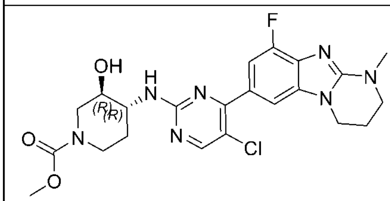 TB-117 | 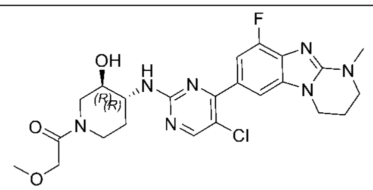 TB-118 | 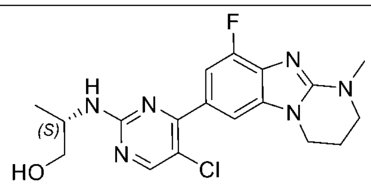 TB-119 |
| 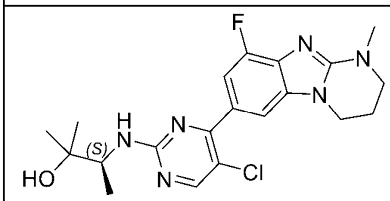 TB-120 | 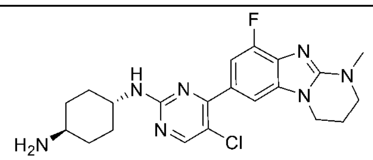 TB-121 | 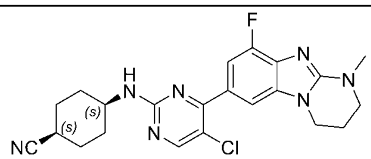 TB-122 |
| 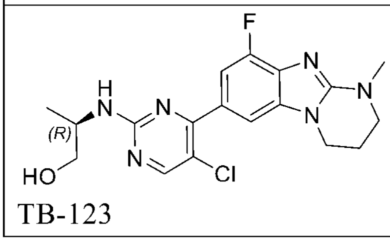 TB-123 | 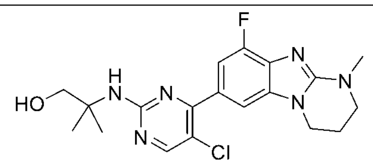 TB-124 | 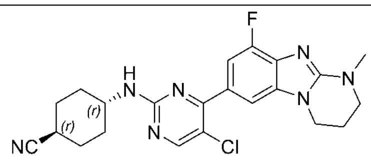 TB-125 |

| | | |
|---|---|---|
| 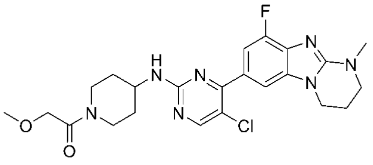 TB-126 | 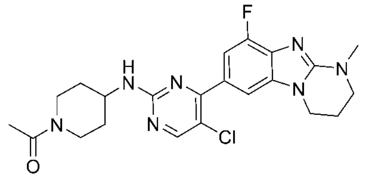 TB-127 | 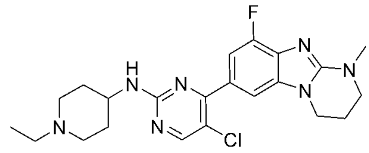 TB-128 |
| 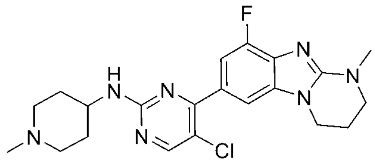 TB-129 | 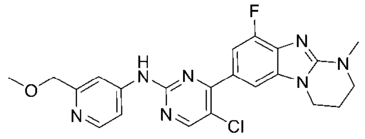 TB-130 | 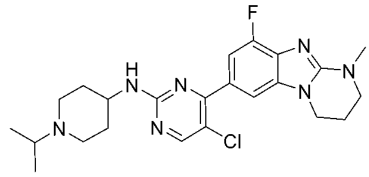 TB-131 |
| 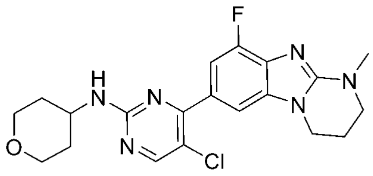 TB-132 | 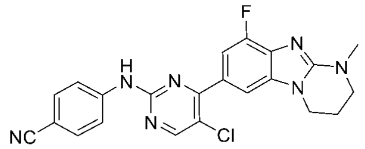 TB-133 | 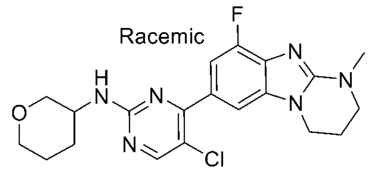  TB-134 |
| 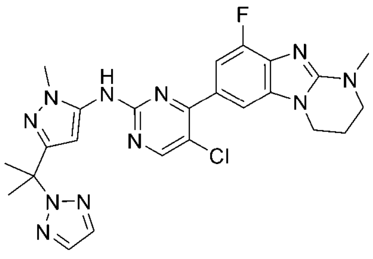 TB-135 | 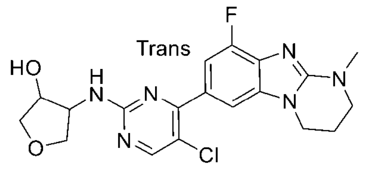  TB-136 | 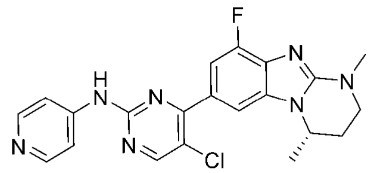 TB-137 |
| 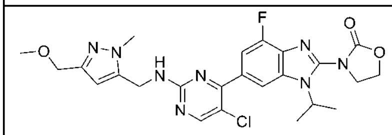 TB-138 | 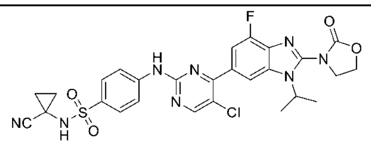 TB-139 | 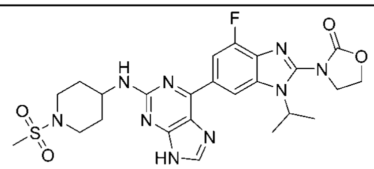 TB-140 |
| 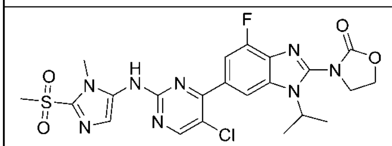 TB-141 | 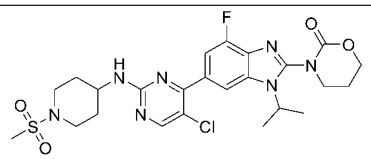 TB-142 | 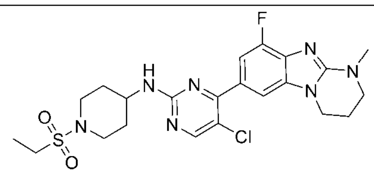 TB-143 |
| 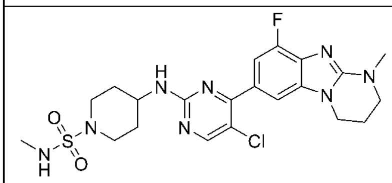 TB-144 | 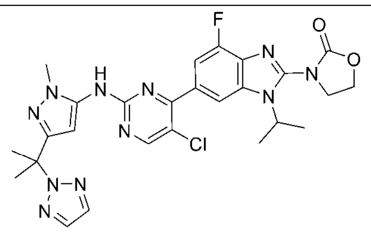 TB-145 | 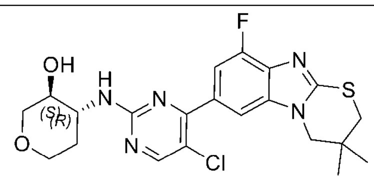 TB-146 |

| | | |
|---|---|---|
| 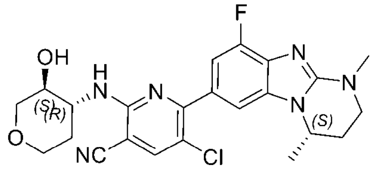 TB-147 | 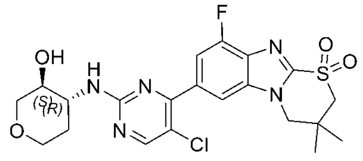 TB-148 | 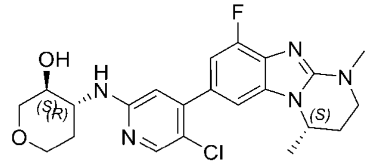 TB-149 (or TA-11) |
| 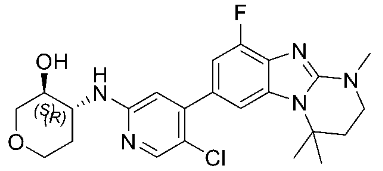 TB-150 | 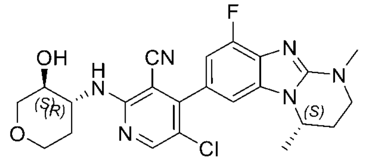 TB-151 | 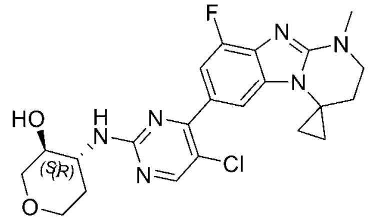 TB-152 |
| 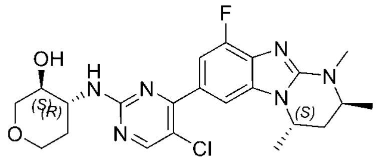 TB-153 | 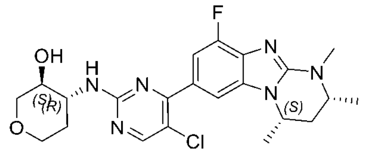 TB-154 | 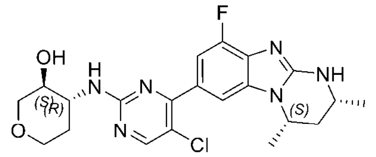 TB-155 |
| 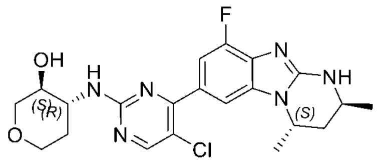 TB-156 | 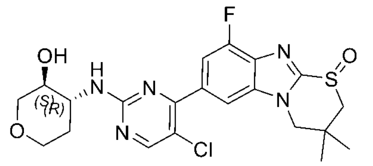 TB-157 | 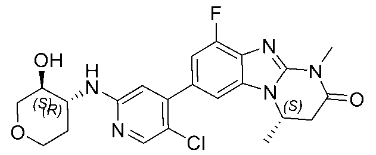 TB-158 |
| 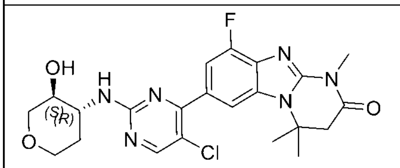 TB-159 | 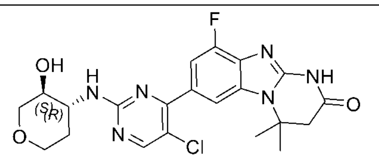 TB-160 | 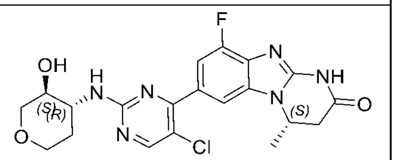 TB-161 |
| 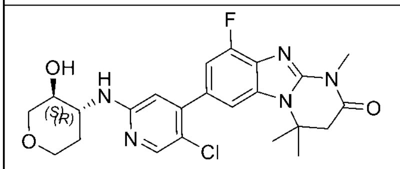 TB-162 | 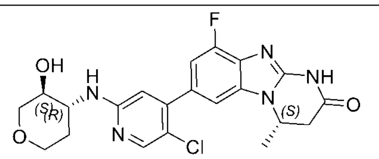 TB-163 | 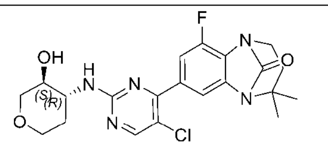 TB-164 |
| 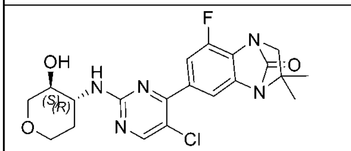 TB-165 | 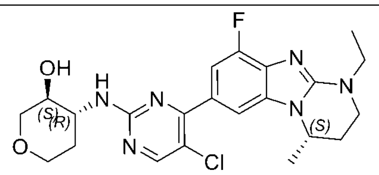 TA-2 | 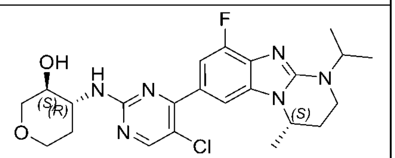 TA-3 |
| 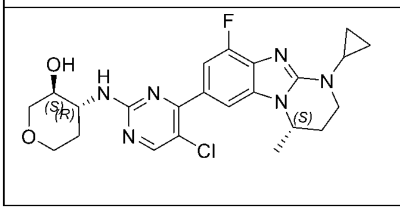 | 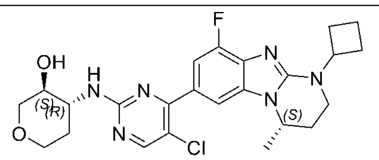 | 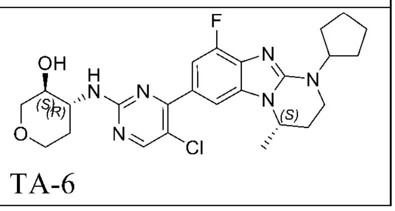 TA-6 |

| TA-4 | TA-5 | |
|---|---|---|
| 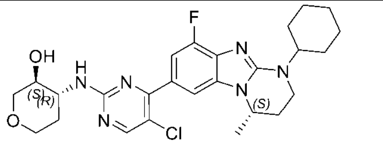 TA-7 | 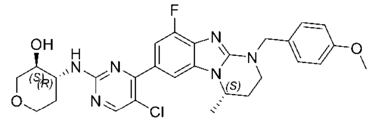 TA-8 | 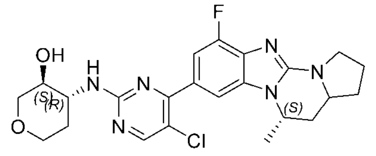 TA-9 |
| 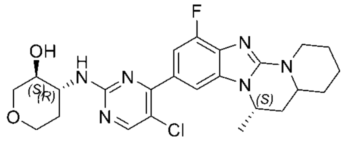 TA-10 | | 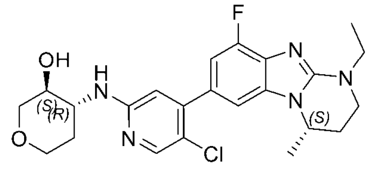 TA-12 |
| 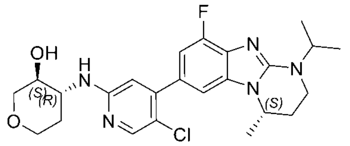 TA-13 | 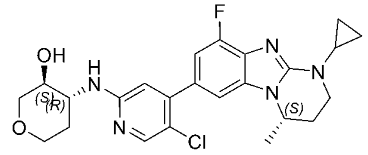 TA-14 | 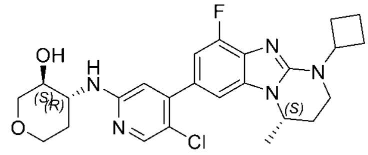 TA-15 |
| 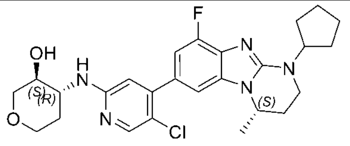 TA-16 | 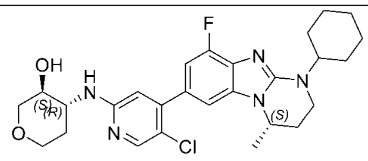 TA-17 | 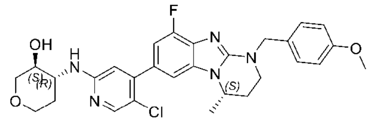 TA-18 |
| 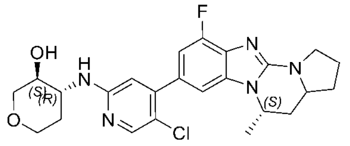 TA-19 | 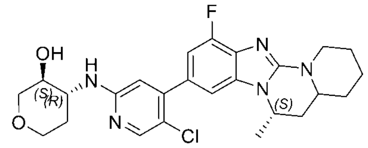 TA-20 | 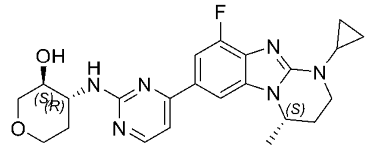 TA-21 |
| 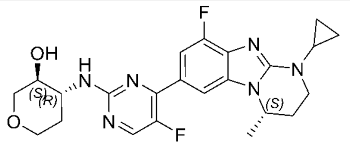 TA-22 | 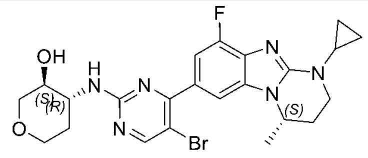 TA-23 | 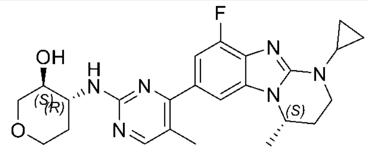 TA-24 |
| 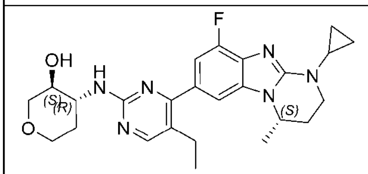 TA-25 | 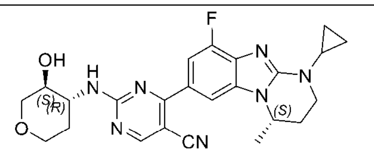 TA-26 | 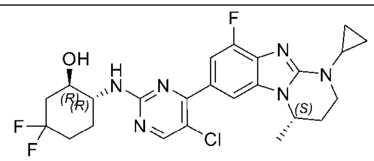 TA-27 |
| 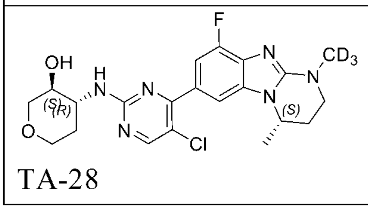 TA-28 | 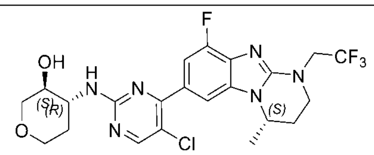 TA-29 | 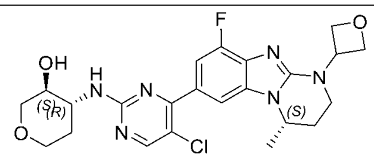 TA-30 |

| | | |
|---|---|---|
| 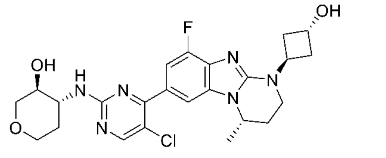 TA-31 | 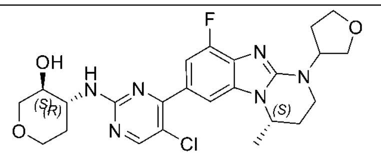 TA-32 | 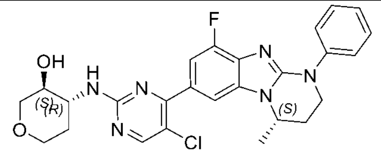 TA-33 |
| 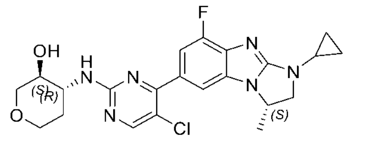 TA-34 | 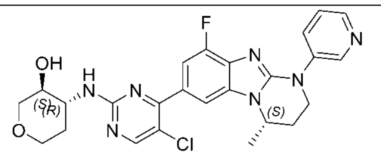 TA-35 | 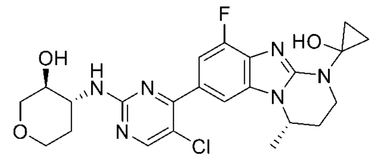 TA-36 |
| 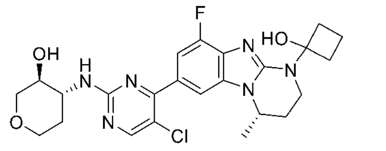 TA-37 | 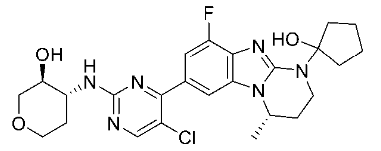 TA-38 | 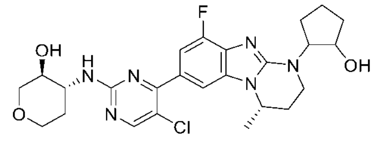 TA-39 |
| 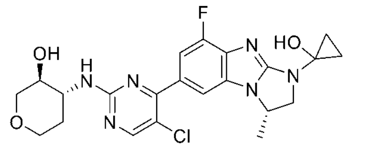 TA-40 | 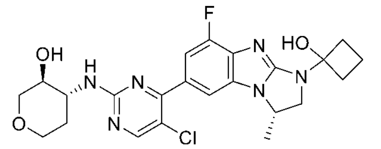 TA-41 | 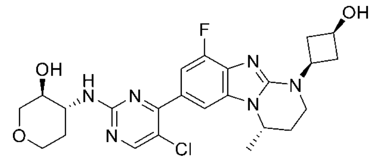 TA-42 |
| 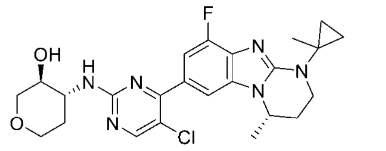 TA-43 | 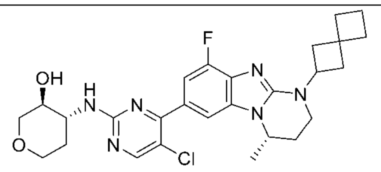 TA-44 | 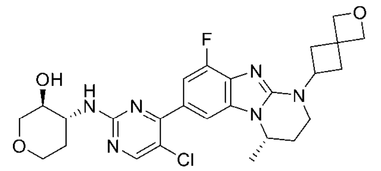 TA-45 |
| 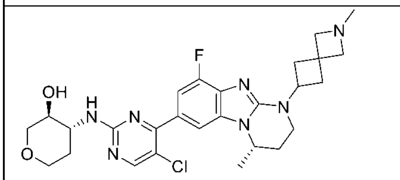 TA-46 | 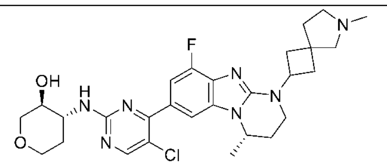 TA-47 | 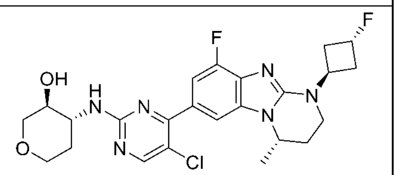 TA-48 |
| 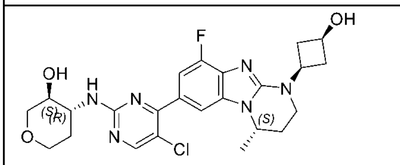 TA-49 | 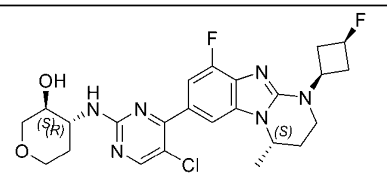 TA-50 | 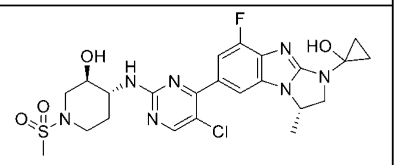 TA-51 |
| 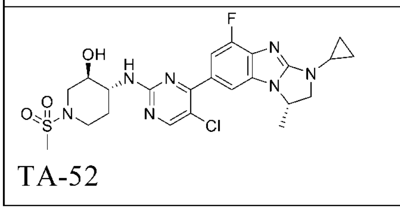 TA-52 | 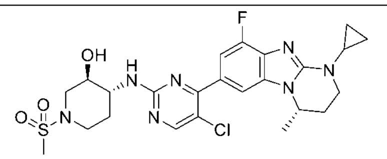 TA-53 | 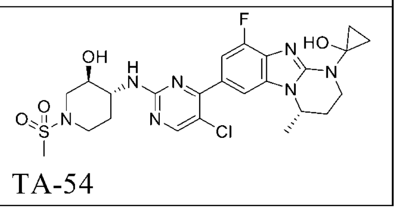 TA-54 |

| | | |
|---|---|---|
| 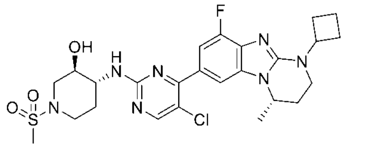 TA-55 | 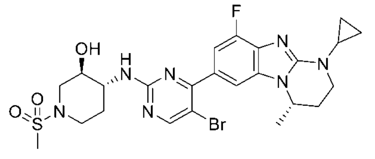 TA-56 | 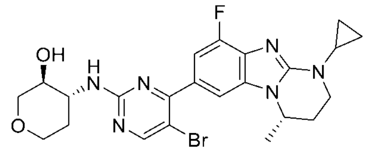 TA-57 |
| 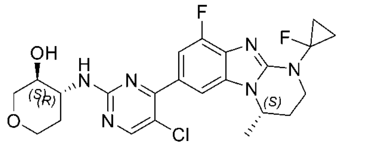 TC-1 | 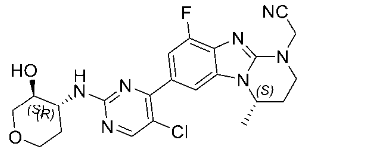 TC-2 | |
| 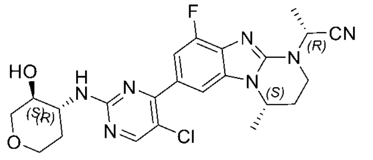 TC-3 | 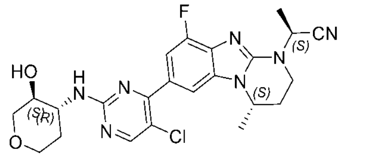 TC-4 | 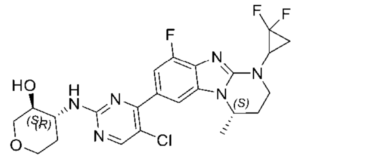 TC-5 |
| 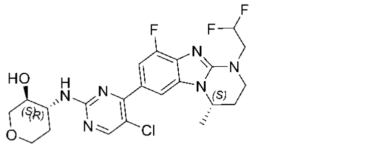 TC-6 | 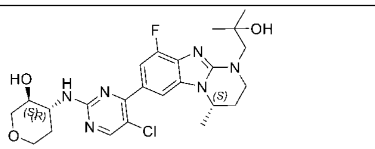 TC-7 | 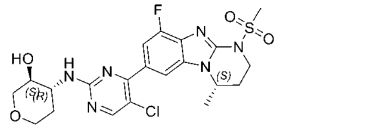 TC-8 |
| 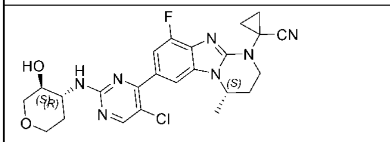 TC-9 | 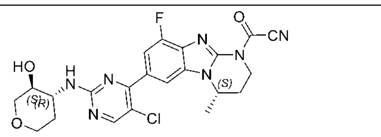 TC-10 | 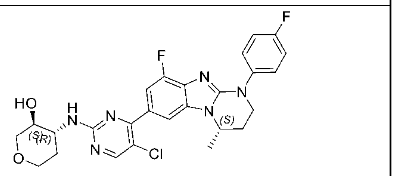 TC-11 |
| 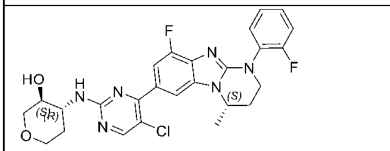 TC-12 | 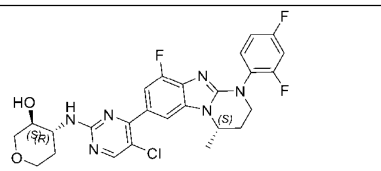 TC-13 | 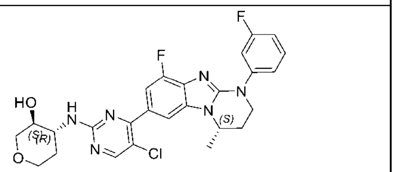 TC-14 |
| 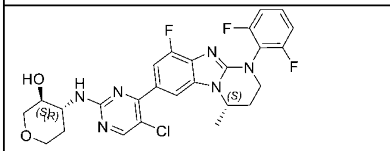 TC-15 | 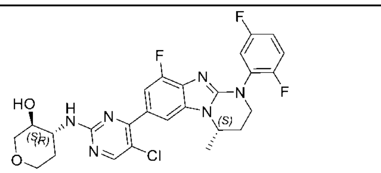 TC-16 | 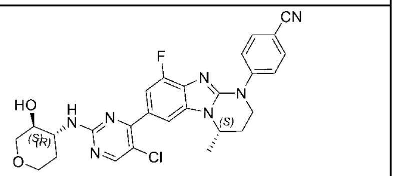 TC-17 |
| 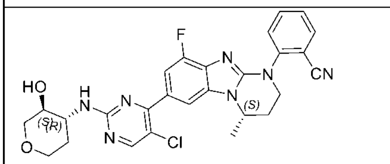 TC-18 | 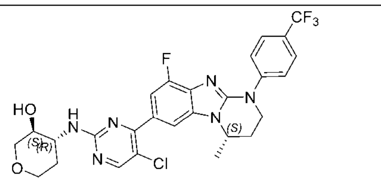 TC-19 | 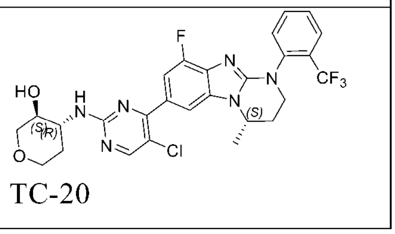 TC-20 |

| | | |
|---|---|---|
| 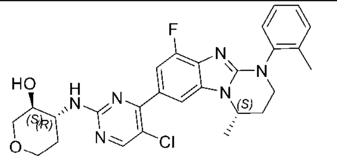 TC-21 | 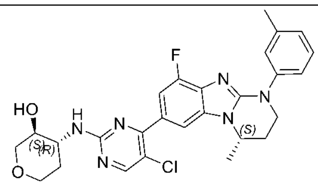 TC-22 | 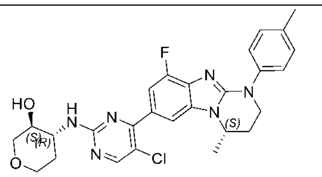 TC-23 |
| 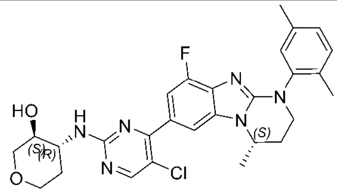 TC-24 | 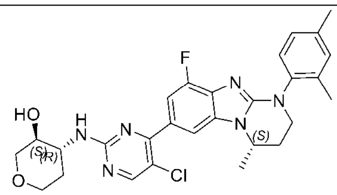 TC-25 | 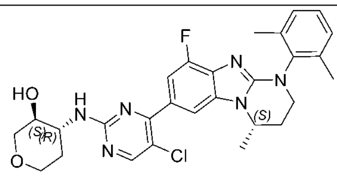 TC-26 |
| 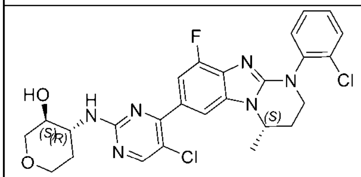 TC-27 | 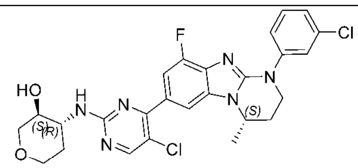 TC-28 | 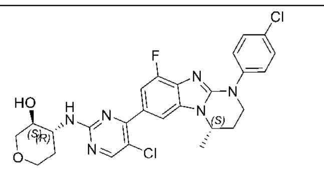 TC-29 |
| 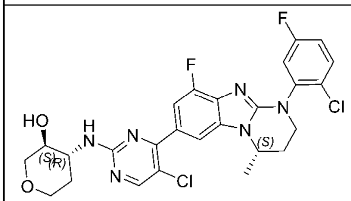 TC-30 | 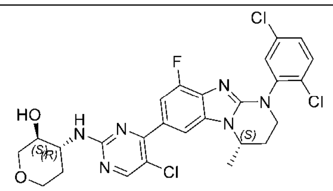 TC-31 | 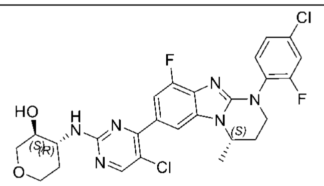 TC-32 |
| 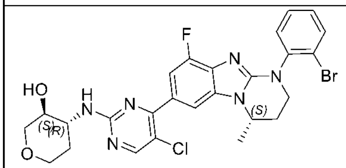 TC-33 | 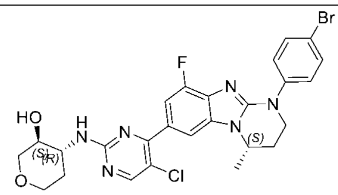 TC-34 | 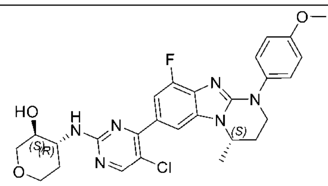 TC-35 |
| 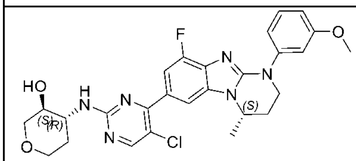 TC-36 | 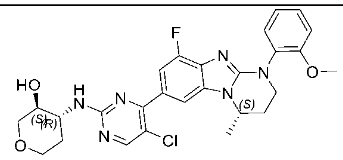 TC-37 | 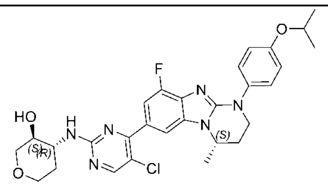 TC-38 |
| 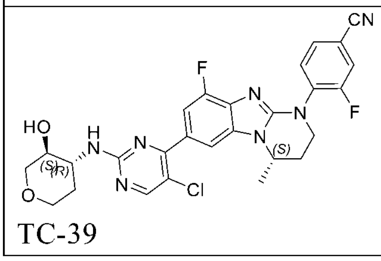 TC-39 | 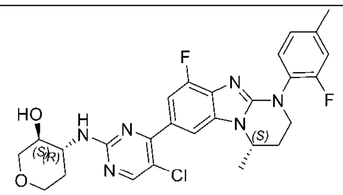 TC-40 | 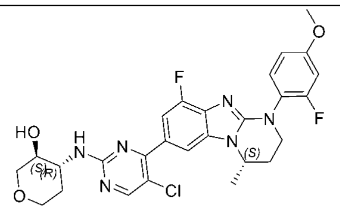 TC-41 |

| | | |
|---|---|---|
| 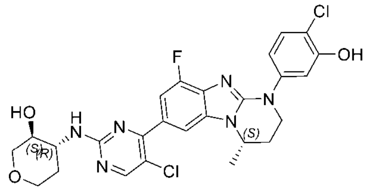 TC-42 | 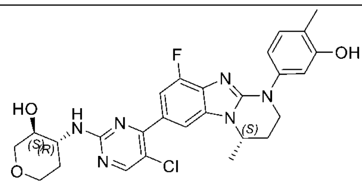 TC-43 | 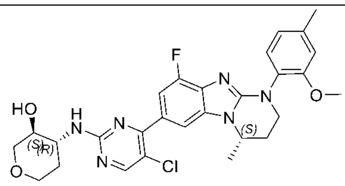 TC-44 |
| 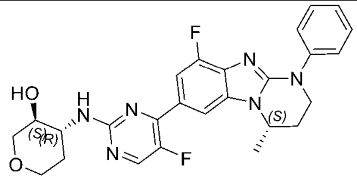 TC-45 | 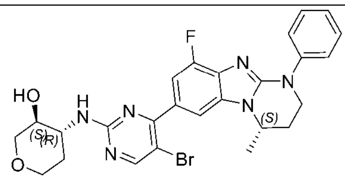 TC-46 | 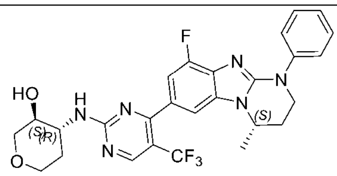 TC-47 |
| 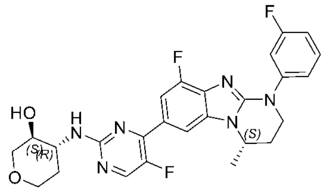 TC-48 | 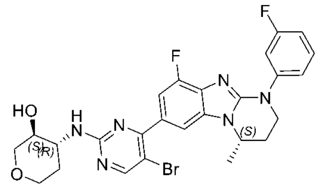 TC-49 | 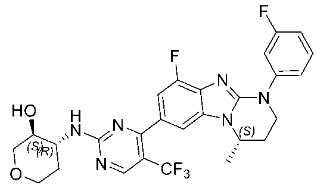 TC-50 |
| 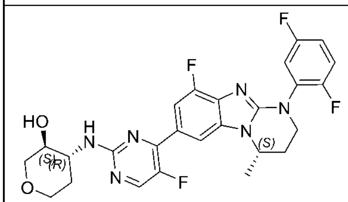 TC-51 | 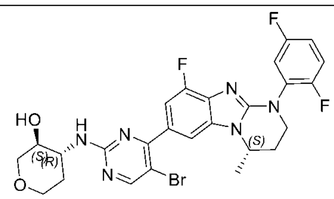 TC-52 | 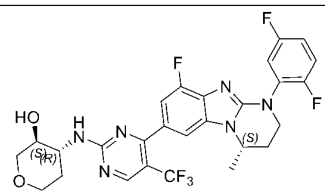 TC-53 |
| 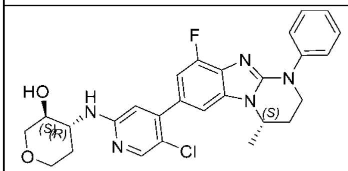 TC-54 | 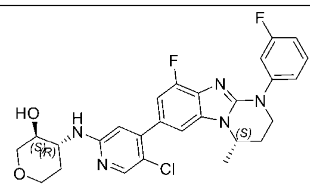 TC-55 | 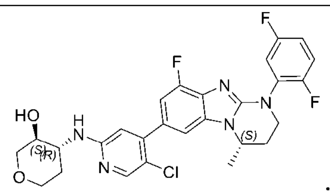 TC-56 |

**9.** A pharmaceutical composition comprising a safe and effective amount of the compound according to any one of claims 1-3, and a pharmaceutically acceptable carrier.

**10.** Use of the compound according to any one of claims 1-3 for the manufacture of a medicament for modulating CDK4 kinase activity or treating a CDK4-related disease.

**INTERNATIONAL SEARCH REPORT**

International application No.
**PCT/CN2024/117282**

**A. CLASSIFICATION OF SUBJECT MATTER**

C07D405/14(2006.01)i; C07D487/04(2006.01)i; C07D401/14(2006.01)i; C07D403/04(2006.01)i; C07D413/14(2006.01)i; C07D471/14(2006.01)i; C07D473/00(2006.01)i; C07D519/00(2006.01)i; C07D487/08(2006.01)i; C07D487/10(2006.01)i; C07D487/14(2006.01)i; C07D513/04(2006.01)i; A61P29/00(2006.01)i; A61P35/00(2006.01)i; A61P35/02(2006.01)i; A61P9/00(2006.01)i; A61P31/00(2006.01)i; A61P37/02(2006.01)i; A61P37/06(2006.01)i; A61P3/00(2006.01)i; A61K31/506(2006.01)i; A61K31/529(2006.01)i; A61K31/519(2006.01)i; A61K31/527(2006.01)i; A61K31/542(2006.01)i; A61K31/52(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNABS; DWPI; VEN; CJFD; CNKI; CNTXT; USTXT; WOTXT; EPTXT; STN-REG; STN-CAPLUS; ISI-Web of Science; 万方, WANFANG; 超星读秀, DUXIU: 浙江同源康, 梁阿朋, 王凯, 陈少清, 李钧, 吴豫生, 李美华, 尹洲, 龙易, 牛成山, 苯并咪唑, CDK4激酶, benzimidazole, CDK4 kinase, 结构式II, 结构式I

**C. DOCUMENTS CONSIDERED TO BE RELEVANT**

| Category* | Citation of document, with indication, where appropriate, of the relevant passages | Relevant to claim No. |
|---|---|---|
| PX | CN 117645604 A (TYK MEDICINES, INC.) 05 March 2024 (2024-03-05) claims 1-10 | 1-10 |
| X | CN 112313219 A (PFIZER INC.) 02 February 2021 (2021-02-02) claims 1-23, and description, pages 157, 158, and 221 | 1-10 |
| A | CN 109952295 A (JIANGSU HANSOH PHARMACEUTICAL GROUP CO., LTD. et al.) 28 June 2019 (2019-06-28) entire document | 1-10 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 November 2024** | **16 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/117282**

| Patent document cited in search report | Publication date (day/month/year) | Patent family member(s) | Publication date (day/month/year) |
|---|---|---|---|
| CN 117645604 A | 05 March 2024 | WO 2024051702 A1 | 14 March 2024 |
| CN 112313219 A | 02 February 2021 | US 2019330196 A1 | 31 October 2019 |
| | | US 10766884 B2 | 08 September 2020 |
| | | EP 3784664 A1 | 03 March 2021 |
| | | US 2022089580 A1 | 24 March 2022 |
| | | RU 2021136543 A | 16 December 2021 |
| | | TW 202000661 A | 01 January 2020 |
| | | BR 112020021689 A2 | 26 January 2021 |
| | | BR 112020021689 B1 | 28 February 2023 |
| | | US 2020354350 A1 | 12 November 2020 |
| | | US 11220494 B2 | 11 January 2022 |
| | | UA 126177 C2 | 25 August 2022 |
| | | JP 2021522275 A | 30 August 2021 |
| | | JP 7089061 B2 | 21 June 2022 |
| | | CA 3098283 A1 | 31 October 2019 |
| | | CA 3098283 C | 23 May 2023 |
| | | KR 20210002642 A | 08 January 2021 |
| | | KR 102596598 B1 | 03 November 2023 |
| | | KR 20230152182 A | 02 November 2023 |
| | | KR 102661053 B1 | 26 April 2024 |
| | | RU 2762557 C1 | 21 December 2021 |
| | | WO 2019207463 A1 | 31 October 2019 |
| | | JP 2022120096 A | 17 August 2022 |
| | | IL 278075 A | 30 November 2020 |
| | | PH 12020551692 A1 | 19 July 2021 |
| | | AU 2019259653 A1 | 29 October 2020 |
| | | AU 2019259653 B2 | 19 January 2023 |
| | | SG 11202009992 A1 | 27 November 2020 |
| | | VN 75481 A | 25 January 2021 |
| | | IN 202017045768 A | 05 February 2021 |
| | | ID 202102205 A1 | 01 April 2021 |
| | | TW 740135 B1 | 21 September 2021 |
| | | RU 2021136543 A | 16 December 2021 |
| | | IL 278075 B | 01 January 2023 |
| | | RU 2790006 C2 | 14 February 2023 |
| | | MX 401739 B | 13 April 2023 |
| | | CN 112313219 B | 26 April 2024 |
| | | CN 118286225 A | 05 July 2024 |
| CN 109952295 A | 28 June 2019 | WO 2018045957 A1 | 15 March 2018 |
| | | TW 201811771 A | 01 April 2018 |
| | | CN 109952295 B | 06 April 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Periodic Table of the Elements, CAS version. Handbook of Chemistry and Physics
- **J. SAMBROOK et al.** Molecular Cloning: A Laboratory Manual. Cold Spring Harbor Laboratory Press, 1989